(19) Europäisches Patentamt / European Patent Office / Office européen des brevets

(11) EP 2 721 030 B1

(12) **EUROPEAN PATENT SPECIFICATION**

(45) Date of publication and mention of the grant of the patent:
**16.12.2015 Bulletin 2015/51**

(21) Application number: **12728453.7**

(22) Date of filing: **12.06.2012**

(51) Int Cl.:
***C07D 487/04*** (2006.01) ***A61P 11/00*** (2006.01)
***A61K 31/519*** (2006.01)

(86) International application number:
**PCT/EP2012/061084**

(87) International publication number:
**WO 2012/171900 (20.12.2012 Gazette 2012/51)**

(54) **NOVEL PHTHALAZINONE-PYRROLOPYRIMIDINECARBOXAMIDE DERIVATIVES**
NEUARTIGE PHTHALAZINON-PYRROLOPYRIMIDINCARBOXAMIDDERIVATE
NOUVEAUX DÉRIVÉS DE PHTHALAZINONE-PYRROLOPYRIMIDINECARBOXAMIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.06.2011 EP 11170440**

(43) Date of publication of application:
**23.04.2014 Bulletin 2014/17**

(73) Proprietor: **Takeda GmbH**
**78467 Konstanz (DE)**

(72) Inventors:
- **STENGEL, Thomas**
  **76344 Eggenstein-Leopoldshafen (DE)**
- **MAIER, Thomas**
  **78333 Stockach (DE)**
- **MANN, Alexander**
  **78315 Radolfzell (DE)**
- **STADLWIESER, Josef**
  **6500 Landeck (AT)**
- **FLOCKERZI, Dieter**
  **78476 Allensbach (DE)**
- **PAHL, Andreas**
  **69115 Heidelberg (DE)**
- **BENEDIKTUS, Ewald**
  **88400 Biberach (DE)**
- **HESSMANN, Manuela**
  **22946 Grossensee (DE)**
- **KANACHER, Tobias**
  **88709 Hagnau (DE)**
- **HUSSONG, Ragna**
  **78652 Deißlingen (DE)**
- **ZITT, Christof**
  **78464 Konstanz (DE)**
- **HOLST, Hans Christof**
  **78467 Konstanz (DE)**
- **HUMMEL, Rolf-Peter**
  **78315 Radolfzell (DE)**
- **VIERTELHAUS, Martin**
  **68199 Mannheim (DE)**
- **TENOR, Hermann**
  **78467 Kostanz (DE)**
- **DUNKERN, Torsten**
  **41363 Jüchen (DE)**
- **HATZELMANN, Armin**
  **78467 Konstanz (DE)**
- **HESSLINGER, Christian**
  **78357 Zoznegg (DE)**

(74) Representative: **Wild, Robert et al**
**Takeda GmbH**
**Postfach 10 03 10**
**78403 Konstanz (DE)**

(56) References cited:
**WO-A1-2004/018451 WO-A1-2011/023693**

EP 2 721 030 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of application of the invention

The invention relates to novel phthalazinone -pyrrolopyrimidinecarboxamide derivatives, which are used in the pharmaceutical industry for the manufacture of pharmaceutical compositions.

### Known technical background

In the international patent applications WO02/064584, WO02/085906, WO04/017974, WO04/018449, WO04/018451, WO04/018457, WO 05/075456 and WO05/075457 phthalazinone- or pyridazinone derivatives with a piperidinyl substitutent are described as type 4 phosphodiesterase inhibitors. The international patent applications WO2009106531 and WO2011023693 describe pyrrolopyrimidinecarboxamide compounds representing inhibitors of the type 5 phosphodiesterase.

### Description of the invention

It has now been found that the piperidinyl pyridazinone- pyrrolopyrimidinecarboxamide derivates compounds, which are described in greater details below, have surprising and particularly advantageous properties.
The invention relates to a compound of formula (1)

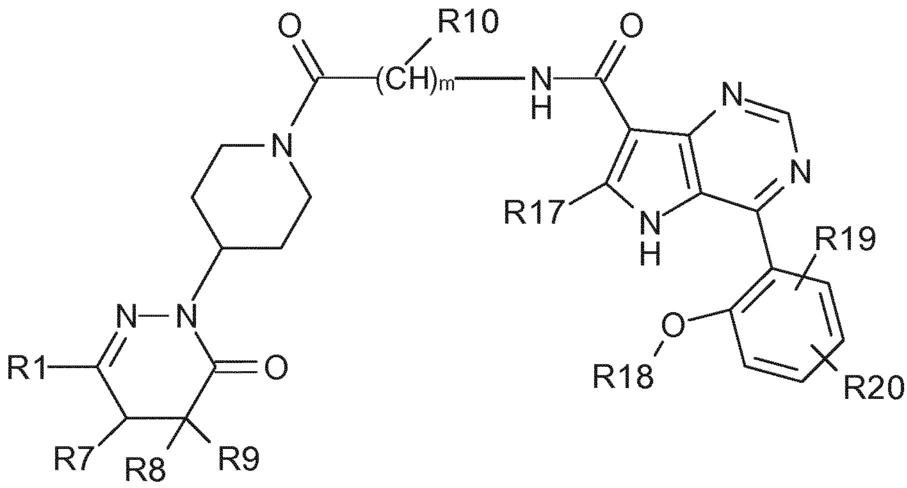

(1),

wherein

R1 represents a phenyl derivative of formulae (a) or (b)

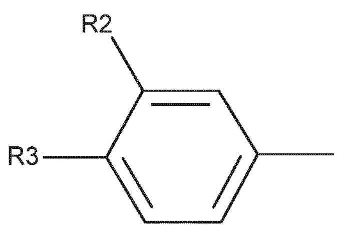

(a)

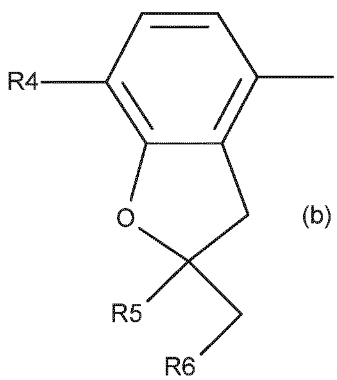

(b)

wherein

| | |
|---|---|
| R2 | is 1-4C-alkoxy or 1-4C-alkoxy predominantly or completely substituted by fluorine, |
| R3 | is 1-4C-alkoxy, 3-5C-cycloalkoxy, 3-5C-cycloalkoxymethoxy or 1-4C-alkoxy predominantly or completely substituted by fluorine, |
| R4 | is 1-4C-alkoxy or 1-4C-alkoxy predominantly or completely substituted by fluorine, |
| R5 | is 1-4C-alkyl, |
| R6 | is hydrogen or 1-4C-alkyl or wherein R5 and R6 together and with inclusion of the two carbon atoms, to which they are bonded, form a spiro-linked 5-, 6- or 7-membered hydrocarbon ring, optionally interrupted by an oxygen or sulphur atom, |
| R7 | is hydrogen, |
| R8 | is 1-4C-alkyl, |
| or R7 and R8 | together form a 3C- to 5C-alkylene group, |
| R9 | is hydrogen or 1-4C-alkyl, or wherein R8 and R9 together and with inclusion of the carbon atom, to which they are bonded, form a spiro-linked 5-, 6- or 7-membered hydrocarbon ring, |
| m | is 1 or 2, |
| R10 | is independently from each other hydrogen, 5-7C-cycloalkyl, 1-6C-alkyl, $-CH(CH_3)$-R11 or $-CH_2$-R12, wherein<br>R11 is unsubstituted phenyl or hydroxyl,<br>R12 is hydroxyl, 5-7C-cycloalkyl, -N-(1-2C-alkyl)$_2$, $-CH_2$-S-(1-2C-alkyl), benzyl, unsubstituted phenyl, phenyl substituted by R13, phenyl substituted by R13 and R14,<br>wherein<br>R13 is halogen, 1-4C-alkoxy, 1-6C-alkyl, 1-4C-fluoroalkyl, hydroxyl, phenyl, $-C(O)NH_2$, -CN, 2-oxoazetidin-1-yl or 2-oxopyrrolidin-1-yl,<br>R14 is halogen, 1-4C-alkoxy, 1-6C-alkyl, 1-4C-fluoroalkyl, hydroxyl, phenyl, $-C(O)NH_2$ or -CN,<br>or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or<br>R12 is $-CH_2-C(O)$-R15,<br>wherein<br>R15 is hydroxyl, -N(R16)$_2$, piperidin-1-yl, pyrrolidin-1-yl or benzyloxy, wherein<br>R16 is independently from each other hydrogen or 1-4C-alkyl, |
| R17 | is hydrogen or methyl, |
| R18 | is $-CH_2$-3-6C-cycloalkyl, |
| R19 | is halogen, hydroxyl, 1-4C-alkoxy, 1-4C-alkoxy predominantly or completely substituted by fluorine or 1-4C-fluoroalkyl, |
| R20 | is halogen, hydroxyl, 1-4C-alkoxy or 1-4C-alkoxy predominantly or completely substituted by fluorine, |
| or R19 and R20 | together form a 1-2C-alkylenedioxy group |

or a stereoisomer of the compound or a salt of the compound or a salt of the stereoisomer of the compound.

1-6C-Alkyl is a straight-chain or branched alkyl group having 1 to 6 carbon atoms. Examples are n-hexyl, 2-methylhexyl, 3-methylpentyl, 2,2,-dimethylbutyl, 2,3-dimethylbutyl, n-pentyl, 2-pentyl, 3-pentyl, 2-methylbutyl, 3-methylbutyl, 3-methylbut-2-yl, 2-methylbut-2-yl, 2,2-dimethylpropyl, butyl, isobutyl, sec-butyl, tert-butyl, propyl, isopropyl, ethyl and methyl.

1-4C-Alkyl is a straight-chain or branched alkyl group having 1 to 4 carbon atoms. Examples are butyl, isobutyl, sec-butyl, tert-butyl, propyl, isopropyl, ethyl and methyl.

1-3C-Alkyl is a straight-chain or branched alkyl group having 1 to 3 carbon atoms. Examples are propyl, isopropyl, ethyl and methyl.

1-2C-Alkyl is a straight-chain alkyl group having 1 to 2 carbon atoms. Examples are ethyl and methyl.

1-4C-Alkoxy is a group which, in addition to the oxygen atom, contains a straight-chain or branched alkyl group having 1 to 4 carbon atoms. Alkoxy groups having 1 to 4 carbon atoms which may be mentioned in this context are, for example, butoxy, isobutoxy, sec-butoxy, tert-butoxy, propoxy, isopropoxy, ethoxy and methoxy.

1-2C-Alkoxy is a group, which in addition to the oxygen atom, contains a straight-chain alkyl group having 1 to 2 carbon atoms. Examples are ethoxy and methoxy.

1-2C-Alkylenedioxy represents, for example, the methylenedioxy [O-$CH_2$-O-] and the ethylenedioxy [-O-$CH_2$-$CH_2$-O-] group.

1-4C-Alkoxy, which is completely or predominantly substituted by fluorine, is a group which, in addition to the oxygen atom, contains a straight-chain or branched alkyl group having 1 to 4 carbon atoms, wherein one or more of the hydrogen atoms of the alkyl group are replaced by fluorine. Examples include, but are not limited to, trifluoromethoxy, difluoromethoxy, fluoromethoxy, perfluoroethoxy, 1,1,1-trifluoro-2-fluoroethoxy, 1,1,1-trifluoroethoxy, 1,1-difluoro-2,2-difluoroethoxy, 1,1-difluoro-2-fluoroethoxy, 1,1-difluoroethoxy, 1-fluoro-2,2-difluoroethoxy, 1-fluoro-2-fluoroethoxy, 1-fluoroethoxy, 2,2-difluoroethoxy, 2-fluoroethoxy, 2,2,3,3,3-pentafluoropropoxy, n-perfluoropropoxy, and n-perfluorobutoxy group, of which the 1,1-difluoro-2,2-difuoroethoxy, the 1,1,1-trifluoroethoxy, the trifluoromethoxy and the fluoromethoxy group are preferred. Most preferred is the difluoromethoxy group. Predominantly" in this connection means that more than half of the hydrogen atoms of the 1-4C-alkoxy group are replaced by fluorine atoms.

1-2C-Alkoxy, which is completely or predominantly substituted by fluorine, is a group which, in addition to the oxygen atom, contains a straight-chain or branched alkyl group having 1 to 2 carbon atoms, wherein one or more of the hydrogen atoms of the alkyl group are replaced by fluorine. Examples h-clude, but are not limited to, perfluoroethoxy, 1,1-difluoro-2,2-difluoroethoxy, the 1,2,2-tetrafluoro-ethoxy, the 1,1,1-trifluoroethoxy, the trifluoromethoxy and the difluoromethoxy group, of which the difluoromethoxy group is preferred. "Predominantly" in this connection means that more than half of the hydrogen atoms of the 1-2C-alkoxy group are replaced by fluorine atoms.

1-4C-Fluoroalkyl is a straight-chain or branched alkyl group having 1 to 4 carbon atoms, wherein one or more of the hydrogen atoms of the 1-4C alkyl group are replaced by fluorine. Examples include, but are not limited to, a trifluoromethyl, difluoromethyl, fluoromethyl, perfluoroethyl, 1,1,1-trifluoro-2-fluoroethyl, 1,1,1-trifluoroethyl, 1,1-difluoro-2,2-difluoroethyl, 1,1-difluoro-2-fluoroethyl, 1,1-difluoroethyl, 1-fluoro-2,2-difluoroethyl, 1-fluoro-2-fluoroethyl, 1-fluoroethyl, 2,2-difluoroethyl, 2-fluoroethyl, n-perfluoropropyl and n-perfluorobutyl group. Preferably, 1-4C-Fluoroalkyl is a straight-chain or branched alkyl group having 1 to 4 carbon atoms, wherein one to three of the hydrogen atoms of the 1-4C alkyl group are replaced by fluorine. Examples include, but are not limited to, a trifluoromethyl, difluoromethyl, fluoromethyl, perfluoroethyl, 1,1,1-trifluoroethyl, 1,1-difluoro-2-fluoroethyl, 1,1-difluoroethyl, 1-fluoro-2,2-difluoroethyl, 1-fluoro-2-fluoroethyl, 1-fluoroethyl, 2,2-difluoroethyl and 2-fluoroethyl group.

1-2C-Fluoroalkyl is a straight-chain or branched alkyl group having 1 to 2 carbon atoms, wherein one or more of the hydrogen atoms of the 1-2C-alkyl group are replaced by fluorine. Examples include, but are not limited to, a trifluoromethyl, difluoromethyl, fluoromethyl, perfluoroethyl, 1,1,1-trifluoro-2-fluoroethyl, 1,1,1-trifluoroethyl, 1,1-difluoro-2,2-difluoroethyl, 1,1-difluoro-2-fluoroethyl, 1,1-difluoroethyl, 1-fluoro-2,2-difluoroethyl, 1-fluoro-2-fluoroethyl, 1-fluoroethyl, 2,2-difluoroethyl and 2-fluoroethyl group. Preferably, 1-2C-Fluoroalkyl is a straight-chain or branched alkyl group having 1 to 2 carbon atoms, wherein one to three of the hydrogen atoms of the 1-2C-alkyl group are replaced by fluorine. Examples include, but are not limited to, a trifluoromethyl, difluoromethyl, fluoromethyl, perfluoroethyl, 1,1,1-trifluoroethyl, 1,1-difluoro-2-fluoroethyl, 1,1-difluoroethyl, 1-fluoro-2,2-difluoroethyl, 1-fluoro-2-fluoroethyl, 1-fluoroethyl, 2,2-difluoroethyl and 2-fluoroethyl group.

3-5C-Cycloalkoxy stands for cyclopropyloxy, cyclobutyloxy or cyclopentyloxy. 3-5C-Cycloalkylmethoxy stands for cyclopropylmethoxy, cyclobutylmethoxy or cyclopentylmethoxy.

5-7C-cycloalkyl is a cycloalkyl group having 5 to 7 carbon atoms and stands for cyclopentyl, cyclohexyl or cycloheptyl, preferably cyclohexyl.

3-6C-cycloalkyl is a cycloalkyl group having 3 to 6 carbon atoms and stands for cyclopropyl, cyclobutyl cyclopentyl or cyclohexyl. 3-5C-cycloalkyl is preferred standing for cyclopropyl, cyclobutyl or cyclopentyl, wherein 3-4C-cycloalkyl is more preferred standing for cyclopropyl and cyclobutyl. The most preferred cycloalkyl is the cyclopropyl.

Halogen stands for fluorine, chlorine, bromine or iodine, with fluorine, chlorine or bromine being preferred and with fluorine and chlorine being more preferred.

According to the definition of the $-(CH)_m-R10$ group it is possible that this group is either derived from alpha-aminoacids such as phenylalanine, tyrosine, glycine, alanine, threonine or serine or from beta-aminoacids such as beta-alanine or beta-phenylalanine

It is to be understood that, if R12 represents a substituted phenyl ring, the substituent R13 can be attached in 2-position, 3-position or 4-position to the phenyl ring. It is further to be understood that the substituents R13 and R14 of the phenyl ring can be attached in 2- and 3-position, in 2- and 4-position, in 2- and 5-position, in 2- and 6-position, 3- and 4- position, in 3- and 5-position and in 3- and 6-position to the phenyl ring. Preferably, the substituents R13 and R14 can be attached in 3- and 4-position, 3-and 5-position and in 2- and 4-position to the phenyl ring.

If, R12 represents a phenyl ring, which is substituted by R13 or which is substituted by R13 and 14, exemplary substituted phenyl rings, which may be mentioned, are 3-methyl-phenyl, 4-methyl-phenyl, 4-tert-butyl-phenyl, 4-biphenyl, 4-methoxy-phenyl, 4-ethoxy-phenyl, 2-trifluoromethyl-phenyl, 4-trifluoromethyl-phenyl, 2-chloro-phenyl, 3-chloro-phenyl, 4-chloro-phenyl, 4-fluoro-phenyl, 4-cyano-phenyl, 4-hydroxy-phenyl, 4-carboxamide-phenyl, 3,4-difluoro-phenyl, 3,5-difluoro-phenyl, 2,4-dichloro-phenyl or 3,4-dimethoxy-phenyl.

It is further to be understood that the substituents R19 and R20 can be attached in 4- and 5-position and in 5- and 6-position to the phenyl ring, preferably R19 and R20 can be attached in 4- and 5-position to the phenyl ring. In case, R19 and R20 form a 1-2C-alkylenedioxygroup, this group can be attached in 4,5-position or in 5,6-position, preferably in 5,6-position, to the phenyl ring. This phenyl ring always has a $-O-CH_2$-3-6C-cycloalkyl group of which the- $O-CH_2$-cyclopropyl group is the most preferred one.

Exemplary phenyl rings substituted by R19 and R20, which may be listed are 2-(cyclopropyl-methoxy)-5-fluoro-4-methoxyphenyl, 2-(cyclopropylmethoxy)-4-fluoro-5-methoxyphenyl or 5-(cyclopropyl-methoxy)-1,3-benzodioxol-4-yl].

Salts of the compounds of formula (1) and the stereoisomers thereof include all inorganic and organic acid addition salts and salts with bases, especially all pharmaceutically acceptable inorganic and organic acid addition salts and salts with bases, particularly all pharmaceutically acceptable inorganic and organic acid addition salts and salts with bases customarily used in pharmacy.

Examples of acid addition salts include, but are not limited to, hydrochlorides, hydrobromides, phosphates, nitrates, sulfates, acetates, trifluoroacetates, citrates, gluconates including D-gluconates and L-gluconates, glucuronates including D-glucuronates and L-glucuronates, benzoates, 2-(4-hydroxybenzoyl)benzoates, butyrates, salicylates, sulfosalicylates, maleates, laureates, malates including L-malates and D-malates, lactates including L-lactates and D-lactates, fumarates, succinates, oxalates, tartarates including L-tartarates, D-tartarates and meso-tartarates, stearates, benzenesulfonates (besilates), toluenesulfonates (tosilates), methanesulfonates (mesilates), laurylsulfonates, 3-hydroxy-2-naphthoates, lactobionates (salts of 4-O-beta-D-galactopyranosyl-D-gluconic acid), galactarates, embonates and ascorbates.

Examples of salts with bases include, but are not limited to, lithium, sodium, potassium, calcium, aluminum, magnesium, titanium, ammonium, meglumine and guanidinium salts.

The salts include water-insoluble and, particularly, water-soluble salts.

The compounds of formula (1), the stereoisomers thereof, the salts of compounds of formula (1) or the stereosimoer thereof, may contain, e.g. when isolated in crystalline form, varying amounts of solvents. Included within the scope of the invention are, therefore, all solvates of the compounds of formula (I), the stereoisomers thereof, the salts of compounds of formula (1) and stereoisomers thereof. Hydrates are a preferred example of said solvates.

"Stereoisomer" as part of the phrase "or a stereoisomer of the compound" or of the phrase "or a stereoisomer of a salt of the compound" is meant to mean that the compounds of formula (1) have chiral centers in the positions 4a and 8a, in case R7 and R8 form together a 3C-5C alkylene group. In case, R1 represents a phenyl derivative of formula (b) there is one further chiral center in the dihydrofuran ring, if the substituents R5 and-$CH_2$-R6 are not identical. Compounds of formula (1) are preferred in which the hydrogen atoms in the positions 4a and 8a are cis-configurated, more preferred are compounds of formula (1) in which the absolute configuration is S in the position 4a and R in the position 8a (according to the rules of Cahn, Ingold and Prelog).

The numbering of the ring system, if R7 and R8 form together a 3C-5C alkylene group is shown in the below formula (1*). The dotted ring depicts the possible ring closure of R7 and R8.

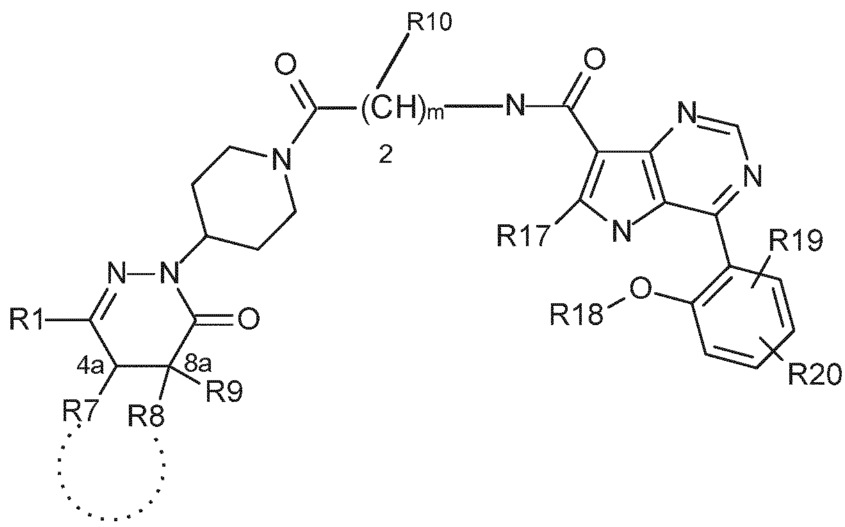

(1*)

However, those compounds are preferred, in which the substitutents R5 and-$CH_2$-R6 are identical or together and with inclusion of the two carbon atoms to which they are bonded form a spiro-connected 5-, 6- or 7-membered hydrocarbon ring.

Furthermore, the compounds of formula (1*) have an additional chiral center in position 2 and if, R10 represents a -$CH(CH_3)$-R11 group, a further chiral center is present.

All possible stereoisomers, i.e. pure diasteromers and pure enantiomers, as well as all mixtures thereof, independent from the ratio, including the racemates, are within the scope of the invention (respectively within the scope of the particular claim).

In a preferred embodiment, the invention relates to to a compound of formula (1), wherein

R1 represents a phenyl derivative of formulae (a) or (b),

wherein
R2 is 1-4C-alkoxy or 1-4C-alkoxy predominantly or completely substituted by fluorine,
R3 is 1-4C-alkoxy or 1-4C-alkoxy predominantly or completely substituted by fluorine,
R4 is 1-2C-alkoxy or 1-2C-alkoxy predominantly or completely substituted by fluorine,
R5 is 1-2C-alkyl,
R6 is hydrogen or 1-2C-alkyl,
or wherein R5 and R6 together and with inclusion of the two carbon atoms, to which they are bonded, form a spiro-linked 5-, 6- or 7-membered hydrocarbon ring,

R7 is hydrogen,
R8 is 1-4C-alkyl,
or R7 and R8 together form a 3C- to 5C-alkylene group,
R9 is hydrogen or 1-4C-alkyl,
or wherein R8 and R9 together and with inclusion of the carbon atom, to which they are bonded, form a spiro-linked 5-, 6- or 7-membered hydrocarbon ring,
R10 is independently from each other hydrogen, 5-7C-cycloalkyl, 1-4C-alkyl, $-CH(CH_3)$-R11 or $-CH_2$-R12,
wherein
R11 is unsubstituted phenyl or hydroxyl,
R12 is hydroxyl, 5-7C-cycloalkyl, -N-(1-2C-alkyl)$_2$, $-CH_2$-S-(1-2C-alkyl), benzyl, unsubstituted phenyl, phenyl substituted by R13, phenyl substituted by R13 and R14,
wherein
R13 is halogen, 1-4C-alkoxy, 1-4C-alkyl, 1-4C-fluoroalkyl, hydroxyl, phenyl, $-C(O)NH_2$, -CN, 2-oxoazetidin-1-yl or 2-oxopyrrolidin-1-yl,
R14 is halogen, 1-4C-alkoxy, 1-4C-alkyl, 1-4C-fluoroalkyl, hydroxyl, phenyl, $-C(O)NH_2$ or -CN,
or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is $-CH_2-C(O)$-R15,
wherein
R15 is hydroxyl, -N(R16)$_2$, piperidin-1-yl, pyrrolidin-1-yl or benzyloxy, wherein
R16 is independently from each other hydrogen or 1-4C-alkyl,
R17 is hydrogen or methyl,
R18 is $-CH_2$-3-6C-cycloalkyl,
R19 is halogen, hydroxyl, 1-4C-alkoxy, 1-4C-alkoxy predominantly or completely substituted by fluorine or 1-4C-fluoroalkyl,
R20 is halogen, hydroxyl, 1-4C-alkoxy or 1-4C-alkoxy predominantly or completely substituted by fluorine,
or R19 and R20 together form a 1-2C-alkylenedioxy group

or a stereoisomer of the compound or a salt of the compound or a salt of the stereoisomer of the compound.

In another preferred embodiment, the invention relates to a compound of formula (1), wherein

R1 represents a phenyl derivative of formulae (a) or (b),
wherein
R2 is 1-2C-alkoxy or 1-2C-alkoxy predominantly or completely substituted by fluorine,
R3 is 1-2C-alkoxy or 1-2C-alkoxy predominantly or completely substituted by fluorine,
R4 is 1-2C-alkoxy or 1-2C-alkoxy predominantly or completely substituted by fluorine,
R5 is 1-2C-alkyl,
R6 is hydrogen or 1-2C-alkyl,
or wherein R5 and R6 together and with inclusion of the two carbon atoms, to which they are bonded, form a spiro-linked 5- or 6- membered hydrocarbon ring,
R7 is hydrogen,
R8 is 1-2C-alkyl,
or R7 and R8 together form a 3C- to 5C-alkylene group,
R9 is hydrogen or 1-2C-alkyl,
or wherein R8 and R9 together and with inclusion of the carbon atom, to which they are bonded, form a spiro-linked 5- or 6- membered hydrocarbon ring,
m is 1 or 2,
R10 is independently from each other hydrogen, 5-7C-cycloalkyl, 1-4C-alkyl, $-CH(CH_3)$-R11 or $-CH_2$-R12,
wherein

R11 is unsubstituted phenyl or hydroxyl,
R12 is hydroxyl, 5-7C-cycloalkyl, -N-(1-2C-alkyl)$_2$, -$CH_2$-S-(1-2C-alkyl), benzyl, unsubstituted phenyl, phenyl substituted by R13, phenyl substituted by R13 and R14,
wherein
R13 is halogen, 1-4C-alkoxy, 1-4C-alkyl, 1-4C-fluoroalkyl, hydroxyl, phenyl, -C(O)$NH_2$, -CN, 2-oxoazetidin-1-yl or 2-oxopyrrolidin-1-yl,
R14 is halogen or 1-4C-alkoxy, 1-4C-alkyl,or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is -$CH_2$-C(O)-R15,
wherein
R15 is hydroxyl, -N(R16)$_2$, piperidin-1-yl, pyrrolidin-1-yl or benzyloxy, wherein
R16 is independently from each other hydrogen or 1-3C-alkyl,
R17 is hydrogen or methyl,
R18 is -$CH_2$-3-5C-cycloalkyl,
R19 is halogen or 1-4C-alkoxy,
R20 is halogen or 1-4C-alkoxy,
or R19 and R20 together form a 1-2C-alkylenedioxy group

or a stereoisomer of the compound or a salt of the compound or a salt of the stereoisomer of the compound.
In yet another preferred embodiment, the invention relates to a compound of formula (1), wherein

R1 represents a phenyl derivative of formulae (a) or (b),
wherein
R2 is methoxy,
R3 is methoxy,
R4 is methoxy,
R5 is methyl,
R6 is hydrogen,
R7 is hydrogen,
R8 is 1-2C-alkyl, or R7 and R8 together form a 3C- to 5C-alkylene group,
R9 is hydrogen or 1-2C-alkyl, or wherein R8 and R9 together and with inclusion of the carbon atom, to which they are bonded, form a spiro-linked 5- membered hydrocarbon ring,
m is 1 or 2,
R10 is independently from each other hydrogen, 5-7C-cycloalkyl, 1-4C-alkyl, -CH($CH_3$)-R11 or -$CH_2$-R12,
wherein
R11 is unsubstituted phenyl or hydroxyl,
R12 is hydroxyl, 5-7C-cycloalkyl, -N-(1-2C-alkyl)$_2$, -$CH_2$-S-(1-2C-alkyl), benzyl, unsubstituted phenyl, phenyl substituted by R13, phenyl substituted by R13 and R14,
wherein
R13 is fluorine, chlorine, bromine, 1-2C-alkoxy, 1-4C-alkyl, 1-2C-fluoroalkyl, hydroxyl, phenyl, -C(O)$NH_2$, -CN, 2-oxoazetidin-1-yl or 2-oxopyrrolidin-1-yl,
R14 is fluorine, chlorine, bromine or 1-2C-alkoxy,
or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is -$CH_2$-C(O)-R15,
wherein
R15 is hydroxyl, -N(R16)$_2$, piperidin-1-yl, pyrrolidin-1-yl or benzyloxy, wherein
R16 is independently from each other hydrogen or 1-3C-alkyl,
R17 is hydrogen or methyl,
R18 is -$CH_2$-3-4C-cycloalkyl,
R19 is 1-2C-alkoxy,
R20 is fluorine, chlorine or bromine,
or R19 and R20 together form a methylenedioxy group

or a stereoisomer of the compound or a salt of the compound or a salt of the stereoisomer of the compound.
In yet another preferred embodiment, the invention relates to a compound of formula (1), wherein

R1 represents a phenyl derivative of formulae (a) or (b),
wherein
R2 is methoxy,
R3 is methoxy,
R4 is methoxy,
R5 is methyl,
R6 is hydrogen,
R7 is hydrogen,
R8 is 1-2C-alkyl,
or R7 and R8 together form a 3C- or 4C-alkylene group,
R9 is hydrogen or 1-2C-alkyl,
or wherein R8 and R9 together and with inclusion of the carbon atom, to which they are bonded, form a spiro-linked 5- membered hydrocarbon ring,
m is 1 or 2,
R10 is independently from each other hydrogen, cyclohexyl, 1-4C-alkyl, $-CH(CH_3)$-R11 or $-CH_2$-R12,
wherein
R11 is unsubstituted phenyl or hydroxyl,
R12 is hydroxyl, cyclohexyl, $-N-(CH_3)_2$, $-CH_2-S-CH_3$, benzyl, unsubstituted phenyl, phenyl substituted by R13, phenyl substituted by R13 and R14,
wherein
R13 is fluorine, chlorine, 1-2C-alkoxy, methyl, tert-butyl, trifluoromethyl, hydroxyl, phenyl, $-C(O)NH_2$, -CN, 2-oxoazetidin-1-yl or 2-oxopyrrolidin-1-yl,
R14 is fluorine, chlorine or methoxy,
or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is $-CH_2-C(O)$-R15,
wherein
R15 is hydroxyl, $-N(R16)_2$, piperidin-1-yl, pyrrolidin-1-yl or benzyloxy, wherein
R16 is independently from each other hydrogen or isopropyl,
R17 is hydrogen or methyl,
R18 is $-CH_2$-3C-cycloalkyl,
R19 is methoxy,
R20 is fluorine,
or R19 and R20 together form a methylenedioxy group

or a stereoisomer of the compound or a salt of the compound or a salt of the stereoisomer of the compound.

In a further preferred embodiment, the invention relates to a compound of formula (1) or a stereoisomer of the compound or a salt of the compound or a salt of the stereoisomer of the compound, wherein m is 1 or 2, preferably m is 1, R7 and R8 together form a 3C-4C-alkylene group, preferably a 4C-alkylene group, R9 is hydrogen and R1, R10, R17, R18, R19 and R20 are as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 and R8 together form a 3C-4C-alkylene group, preferably a 4C-alkylene group, R9 is hydrogen and R1 represents a phenyl derivative of formula (a), wherein R2 is 1-4C-alkoxy and R3 is 1-4C-alkoxy, and R10, R17, R18, R19 and R20 are as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 and R8 together form a 3C-4C-alkylene group, preferably a 4C-alkylene group, R9 is hydrogen, and R1 represents a phenyl derivative of formula (a), wherein R2 is 1-4C-alkoxy and R3 is 1-4C-alkoxy, R18 is $-CH_2$-cyclopropyl, R19 is 1-4C-alkoxy or halogen, R20 is 1-4C-alkoxy or halogen or R19 and R20 together form a 1-2C-alkylenedioxy group and R10 and R17 are as defined above. Preferably, R2 represents 1-2C-alkoxy, more preferably methoxy, R2 represents 1-2C-alkoxy, more preferably methoxy, R16 represents-$CH_2$-cyclopropyl, R19 represents 1-2C-alkoxy, preferably methoxy, or halogen, preferably fluorine, chlorine or bromine, more preferably fluorine, R20 represents 1-2C-alkoxy, preferably methoxy, or halogen, preferably fluorine, chlorine or bromine, more preferably fluorine, or R19 and R20 preferably together form a methylenedioxy group and R10 and R17 are as defined above.

In a further preferred embodiment, the invention relates to a compound of formula (1) or a stereoisomer of the compound or a salt of the compound or a salt of the stereoisomer of the compound, wherein m is 1 or 2, preferably m

is 1, R7 and R8 together form a 3C-4C-alkylene group, preferably a 4C-alkylene group, R9 is hydrogen, and R1 represents a phenyl derivative of formula (a), wherein R2and R3 are methoxy , R18 is $-CH_2$-cyclopropyl, R19 and R20 together form a methylenedioxy group, preferably attached in 5-, 6-position to the phenyl ring, R10 represents hydrogen, 1-6C-alkyl, 5-7C-cycloalkyl or-CH(CH3)-R11, R11 is unsubstituted phenyl or hydroxyl and R17 is as defined above. If, R10 is 1-6C-alkyl, it is preferably methyl, ethyl, iso-propyl iso-butyl, sec-butyl or tert-butyl and if, R10 represents 5-7C-cycloalkyl it preferably is cyclohexyl.

In a further preferred embodiment, the invention relates to a compound of formula (1) or a stereoisomer of the compound or a salt of the compound or a salt of the stereoisomer of the compound, wherein m is 1 or 2, preferably m is 1, R7 and R8 together form a 3C-4C-alkylene group, preferably a 4C-alkylene group, R9 is hydrogen, and R1 represents a phenyl derivative of formula (a), wherein R2 and R3 are methoxy, R18 is $-CH_2$-cyclopropyl, R19 and R20 together form a methylenedioxy group, preferably attached in 5-, 6-position to the phenyl ring, R10 is-$CH_2$-R12, wherein R12 represents hydroxyl, 5-7C-cycloalkyl, preferably cyclohexyl, -N-(1-2C-alkyl)$_2$, preferably -$N(CH_3)_2$ or-$CH_2$-S-(1-2C-alkyl), preferably $-CH_2-S-CH_3$ or benzyl and R17 is as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 and R8 together form a 3C-4C-alkylene group, preferably a 4C-alkylene group, R9 is hydrogen, and R1 represents a phenyl derivative of formula (a), wherein R2 and R3 are methoxy, R18 is-$CH_2$-cyclopropyl, R19 and R20 together form a methylenedioxy group, preferably attached in 5-, 6-position to the phenyl ring, R10 is-$CH_2$-R12, wherein R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or R12 is $-CH_2$-C(O)-R15, wherein R15 is hydroxyl, -N(R16)$_2$, piperidin-1-yl, pyrrolidin-1-yl or benzyloxy, wherein R16 is independently from each other hydrogen or 1-4C-alkyl, preferably 1-3C-alkyl, more preferably isopropyl and R17 is as definded above.

In a further preferred embodiment, the invention relates to a compound of formula (1) or a stereoisomer of the compound or a salt of the compound or a salt of the stereoisomer of the compound, wherein m is 1 or 2, preferably m is 1 ,R7 and R8 together form a 3C-4C-alkylene group, preferably a 4C-alkylene group, R9 is hydrogen, and R1 represents a phenyl derivative of formula (a), wherein R2 is methoxy and R3 is methoxy, R18 is $-CH_2$-cyclopropyl, R19 methoxy or fluorine, preferably fluorine, R20 represents methoxy or fluorine, preferably methoxy, or R19 and R20 together form a methylenedioxy group, preferably attached in 5-, 6-position to the phenyl ring, R10 is $-CH_2$-R12, wherein R12 represents unsubstituted phenyl and R17 is as defined above.

In a further preferred embodiment, the invention relates to a compound of formula (1) or a stereoisomer of the compound or a salt of the compound or a salt of the stereoisomer of the compound, wherein m is 1 or 2, preferably 1, R7 and R8 together form a 3C-4C-alkylene group, preferably a 4C-alkylene group, R9 is hydrogen, and R1 represents a phenyl derivative of formula (a), wherein R2 and R3are methoxy, R18 is $-CH_2$-cyclopropyl, R19 represents methoxy or fluorine, preferably fluorine, R20 represents methoxy or fluorine, preferably methoxy, or R19 and R20 together form a methylenedioxy group, preferably attached in 5-, 6-position to the phenyl ring, R10 is $-CH_2$-R12, wherein R12 represents phenyl either substituted by R13 or substituted by R13 and R14, wherein R13 and R14 as well as R17 are as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 and R8 together form a 3C-4C-alkylene group, preferably a 4C-alkylene group, R9 is hydrogen, and R1 represents a phenyl derivative of formula (a), wherein R2 is methoxy and R3 is methoxy, R18 is $-CH_2$-cyclopropyl, R19 represents methoxy or fluorine, preferably fluorine, R20 represents methoxy or fluorine, preferably methoxy, or R19 and R20 together form a methylenedioxy group, preferably attached in 5-, 6-position to the phenyl ring, R10 is $-CH_2$-R12, wherein R12 represents phenyl either substituted by R13 or substituted by R13 and R14, and R17 is as defined above. If, R12 represents phenyl substituted by R13, R13 represents 1-2C-alkoxy, 1-4C-alkyl, 1-2C-fluoroalkyl, fluorine, chlorine, bromine, hydroxyl, phenyl, -C(O)$NH_2$ or-CN, preferably R13 represents 1-2C-alkoxy, methyl, tert-butyl, trifluoromethyl, fluorine, chlorine, hydroxyl, phenyl, -C(O)$NH_2$, -CN, 2-oxoazetidin-1-yl or 2-oxopyrrolidinyl. If, R12 represents phenyl substituted by R13 and R14, R13 and R14 independently of each other represent 1-2C-alkoxy, fluorine, chlorine or bromine, preferably methoxy, fluorine or chlorine, more preferably R13 and R14 are both methoxy, fluorine or chlorine.

In a further preferred embodiment, the invention relates to a compound of formula (1) or a stereoisomer of the compound or a salt of the compound or a salt of the stereoisomer of the compound, wherein m is 1, R7 and R8 together form a 3C-4C-alkylene group, preferably a 4C-alkylene group, R9 is hydrogen and R1 represents a phenyl derivative of formula (b), wherein R4 is 1-4C-alkoxy, preferably 1-2C-alkoxy, more preferably, methoxy, R5 is 1-4C-alkyl, preferably 1-2C-alkyl, more preferably methyl, R6 is hydrogen or R5 and R6 together and with inclusion of the two carbon atoms, to which they are bonded, form a spiro-linked 5- or 6-membered hydrocarbon ring, preferably a spiro-linked 5-or 6-membered hydrocarbon ring, and R10, R17, R18, R19 and R20 are as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 is hydrogen, R8 and R9 are 1-2C-alkyl, R1 represents a phenyl derivative of formula (a), wherein R2 is 1-4C-alkoxy and R3 is 1-4C-alkoxy, and R10, R17, R18, R19 and R20 are as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 is hydrogen, R8 and R9 are 1-2C-alkyl, preferably methyl, R1 represents a phenyl derivative of formula (a), wherein R2 is 1-2C-alkoxy, preferably methoxy, and R3 is 1-2C-alkoxy, preferably methoxy, R10 is $-CH_2$-R12, wherein R12 either represents unsubstituted phenyl or phenyl substituted by R13, wherein R13 is halogen, preferably fluorine, chlorine or bromine, more preferably fluorine, R18 is $-CH_2$-cyclopropyl, R19 represents 1-2C-alkoxy, preferably methoxy, or halogen, preferably fluorine, chlorine or bromine, more preferably fluorine, R20 represents 1-2C-alkoxy, preferably methoxy, or halogen, preferably fluorine, chlorine or bromine, more preferably fluorine, or R19 and R20 together form a methylenedioxy group, which is preferably attached in 5-, 6-position to the phenyl ring and R17 is as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 is hydrogen, R8 and R9 are 1-2C-alkyl, preferably methyl, R1 represents a phenyl derivative of formula (a), wherein R2 is 1-2C-alkoxy, preferably methoxy, and R3 is 1-2C-alkoxy, preferably methoxy, R10 is $-CH_2$-R12, wherein R12 represents 5-7C-cycloalkyl, preferably cyclohexyl, 1-4C-alkyl, preferably methyl or R12 is $-CH_2$-C(O)-R15, wherein R15 is $-N(R16)_2$ with R16 being independently from each other hydrogen or 1-4C-alkyl, preferably hydrogen or methyl, R18 is-$CH_2$-cyclopropyl, R19 and R20 together form a methylenedioxy group, which is preferably attached in 5-, 6-position to the phenyl ring and R17 is as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 is hydrogen, R8 is 1-2C-alkyl, preferably methyl, R9 is 1-2C-alkyl, preferably methyl and R1 represents a phenyl derivative of formula (b), wherein R4 is 1-4C-alkoxy, preferably 1-2C-alkoxy, more preferably methoxy, R5 is 1-4C-alkyl, preferably 1-2C-alkyl, more preferably methyl, R6 is hydrogen or R5 and R6 together and with inclusion of the two carbon atoms, to which they are bonded, form a spiro-linked 5- or 6-membered hydrocarbon ring, preferably a spiro-linked 5- or 6-membered hydrocarbon ring, and R10, R17, R18, R19 and R20 are as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 is hydrogen, R8 is 1-2C-alkyl, preferably methyl, R9 is 1-2C-alkyl, preferably methyl and R1 represents a phenyl derivative of formula (b), wherein R4 is 1-4C-alkoxy, preferably 1-2C-alkoxy, more preferably methoxy, R5 is 1-4C-alkyl, preferably 1-2C-alkyl, more preferably methyl, R6 is hydrogen and R10 represents hydrogen, 1-4C-alkyl, preferably hydrogen or 1-2C-alkyl, more preferably hydrogen, or R10 is $-CH_2$-R12, wherein R12 is pyridin-2-yl, pyridin-3-yl or pyridin4-yl, R18 is $-CH_2$-cyclopropyl, R19 and R20 together form a methylenedioxy group, which is preferably attached in 5-, 6-position to the phenyl ring and R17 is as defined above.

In a further preferred embodiment, the invention relates to a compound of formula (1) or a stereoisomer of the compound or a salt of the compound or a salt of the stereoisomer of the compound, wherein m is 1 or 2, preferably m is 1, R7 is hydrogen, R8 and R9 together and with inclusion of the carbon atom, to which they are bonded, form a spiro-linked 5-, 6- or 7-membered hydrocarbon ring, preferably a spiro-linked 5-membered hydrocarbon ring, and R1 represents a phenyl derivative of formula (a), wherein R2 is 1-4C-alkoxy and R3 is 1-4C-alkoxy, R18 is $-CH_2$-cyclopropyl, R19 is 1-4C-alkoxy or halogen, R20 is 1-4C-alkoxy or halogen or R19 and R20 together form a 1-2C-alkylenedioxy group and R10 and R17 are as defined above. Preferably, R2 represents 1-2C-alkoxy, more preferably methoxy, R2 represents 1-2C-alkoxy, more preferably methoxy, R18 represents $-CH_2$-cyclopropyl, R19 and R20 together form a methylenedioxy group, R10 is hydrogen or -CH2-R12, wherein R12 represents unsubstituted phenyl, pyridine-2-yl, pyridin-3-yl or pyridine-4-yl, preferably unsubstituted phenyl or pyridine-3-yl, and R17 is as defined above.

The compounds according to the invention can be prepared according to reaction schemes 1 to 3.

As shown in reaction scheme 1 the compounds of formula 1, wherein R1, R7, R8, R9, R10, R17, R18, R19 and R20 have the above-mentioned meanings can be prepared by coupling of a carboxylic acid compound of formula (3) with a primary amine compound of formula (2) using any standard amide bond coupling method, such as for example the use of coupling agents such as HBTU, HATU, TOTU, COMU, T3P® or the use of activated acid compounds such as imidazolides. A review of suitable amide bond coupling methods can be found, for example, in C. A. G. N. Montalbetti, V. Falque, Tetrahedron, 61 (2005), 10827-10852 and in A. El-Faham, R. S. Funosas, R. Prohens, F. Albericio, Chemistry-A European Journal, 15 (2009), 9404-9416 and in J. Glauder, Speciality Chemicals Magazine, 24(2004), 30-31.

**Reaction Scheme 1:**

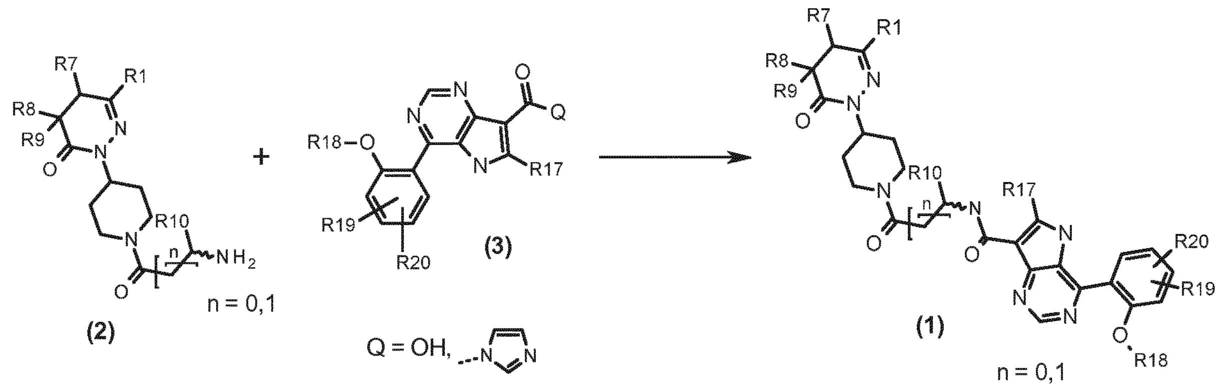

(2) + (3) → (1)

n = 0,1

Q = OH,

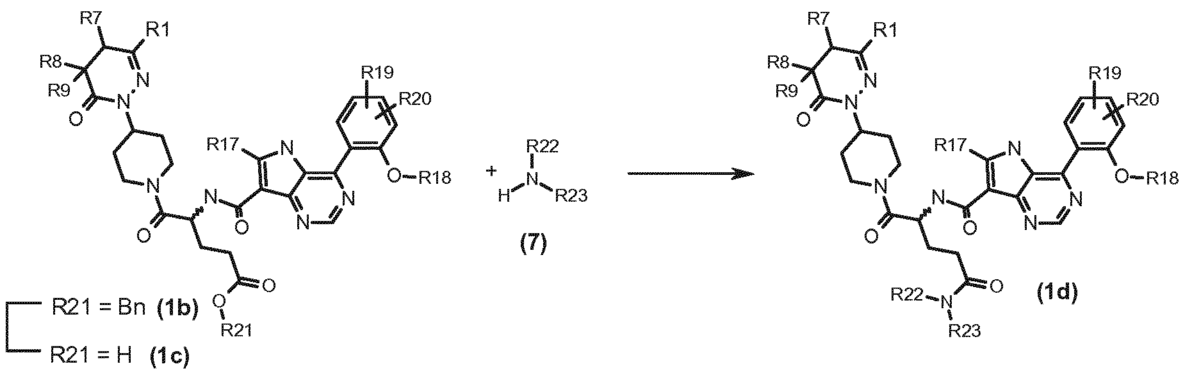

R21 = Bn (1b)

R21 = H (1c)

(7)

(1d)

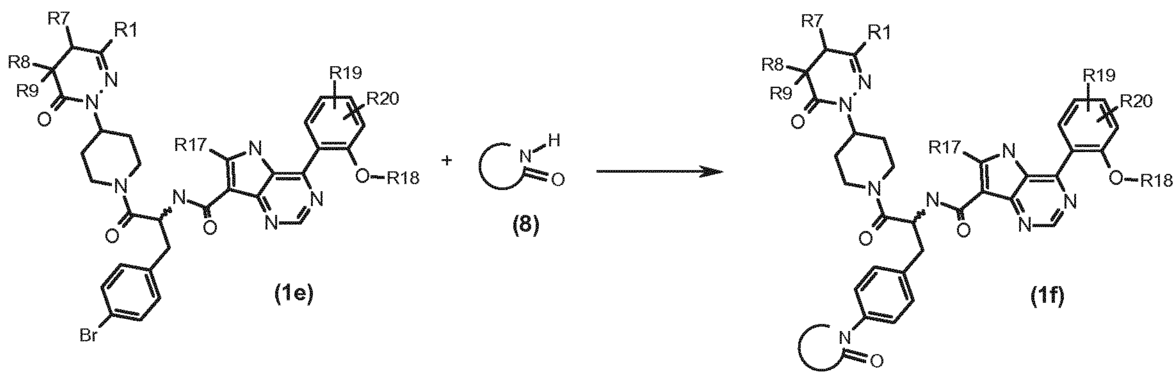

(1e)

(8)

(1f)

Compounds of the formula (1b), (1c) and (1e) also serve as starting materials for further reactions. The benzyl ester of compounds (1b) can be cleaved by hydrogenolysis which can be carried out according to standard methods known to the person skilled in the art, preferably using $H_2$ / Pd-C in an alcohol, such as methanol or ethanol as a solvent at ambient temperature under atmospheric hydrogen pressure to give the corresponding carboxylic acid derivates of formula (1c). Compounds of the formula (1d) can be prepared by coupling of a carboxylic acid compound of formula (1c) with a primary or secondary amine compound of formula (7) using any standard amide bond coupling method, such as for example the use of coupling agents such as HBTU, HATU, TOTU or COMU. An alternative synthesis route to compounds of the formula (1d) is described in scheme 2 and comprises the introduction of substituents R22 and R23 in intermediate (2) which can be reacted with (3) to the final compounds of formula (1d) according to scheme 1.

Compounds of the formula (1f) can be prepared via Palladium-catalyzed coupling of compounds (1e) and amides of the formula (8) using $Pd(dba)_2$ as Palladium source, Xantphos as the ligand, $Cs_2CO_3$ as the base and 1,4-dioxane as the solvent at elevated temperatures (preferably at about 140 °C) and additionally, under microwave irradiation.

The preparation of carboxyclic acid compounds of the formula (3) is described in WO2011/023693 and WO2009/106531 or can be prepared in analogy to methods described therein. In case, Q represents an imidazolyl the preparation of these compounds is known to a person skilled in the art.

Reaction scheme 2 illustrates the synthesis of compounds of the formula (2). In a first step compounds of the formula (4) are reacted with of the formula (5) using any standard amide bond coupling method, such as for example the use of coupling agents such as HBTU, HATU, TOTU, COMU, T3P® or the use of activated acid compounds such as imidazolides. A review of suitable amide bond coupling methods can be found, for example, in C. A. G. N. Montalbetti, V. Falque, Tetrahedron, 61 (2005), 10827-10852 and in A. El-Faham, R. S. Funosas, R. Prohens, F. Albericio, Chemistry - A European Journal, 15 (2009), 9404-9416 and in J. Glauder, Speciality Chemicals Magazine, 24(2004), 30-31. The primary amine of formula (2) can be prepared from the corresponding N-tert-butyloxycarbonyl protected compounds of formula (6) by using standard conditions for the removal of the tert-butyloxycarbonyl group, such as for example hydrogen chloride or trifluoroacetic acid in an appropriate solvent, such as dioxane, tetrahydrofuran or dichloromethane.

Compounds of the formula (5) are commercially available or can be prepared starting from commercially available precursors according to standard methods known to the person skilled in the art. N-Boc protected amino acids of formula (5c) can be synthesized in a two step sequence starting with an amide bond formation reaction of (5a) with a primary amine (14) using any standard amide bond coupling method, such as for example the use of coupling agents such as HBTU, HATU, TOTU or COMU followed by cleavage of the benzyl ester by hydrogenolysis which can be carried out according to standard methods known to the person skilled in the art, preferably using $H_2$ / Pd-C in an alcohol, such as methanol or ethanol as a solvent at ambient temperature under atmospheric hydrogen pressure.

**Reaction Scheme 2**:

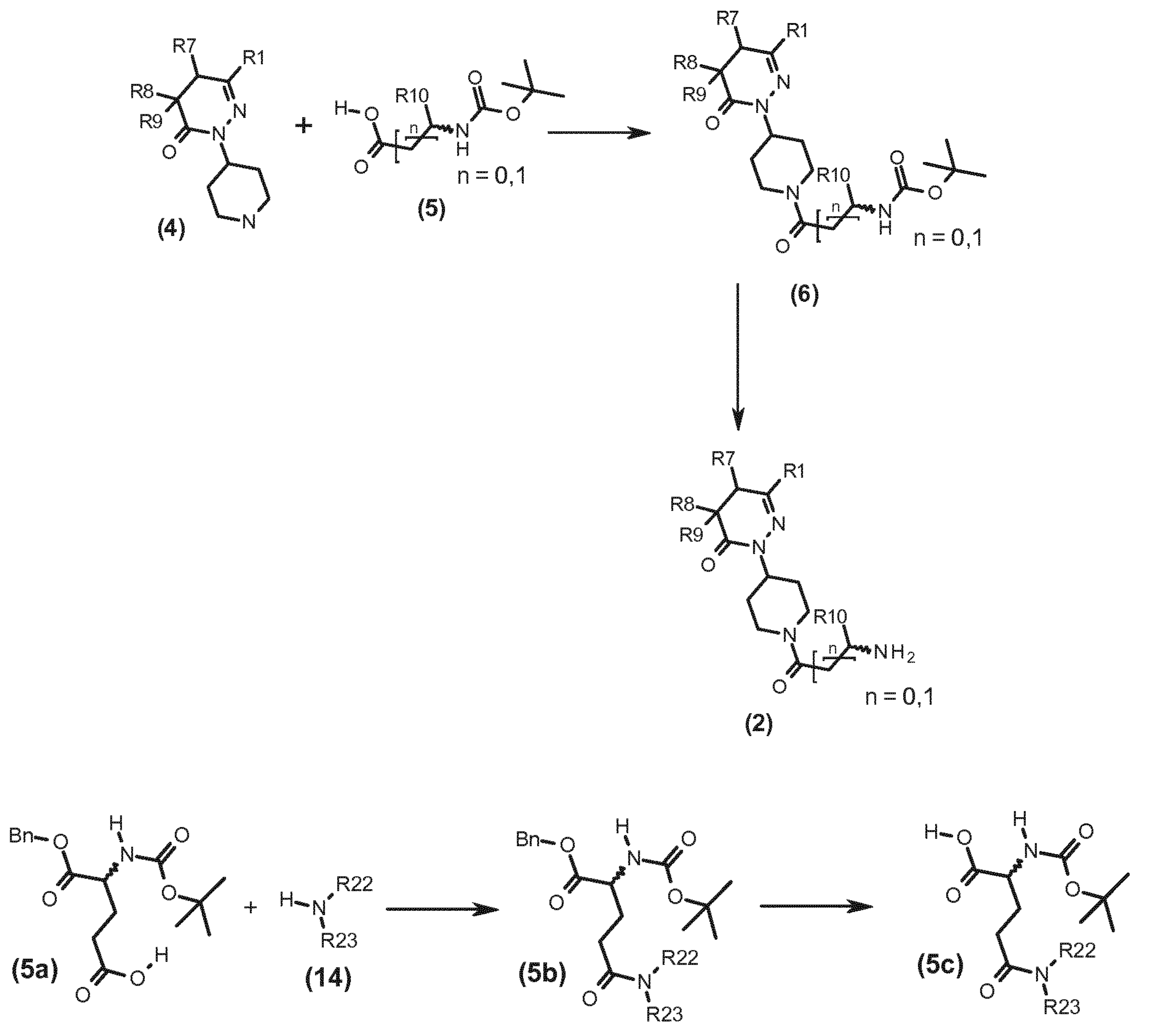
(4) (5) (6) (2)
(5a) (14) (5b) (5c)

The synthesis of intermediate (9) is depicted in scheme 3. Friedels-Crafts reaction of 1,2-dimethoxy benzene

(10) with compound (11) in the presence of aluminium chloride yields γ-keto acid (12) which can be reacted with 4-hydrazinylpiperidine (13) to give compound (9).

The preparation of the compounds of the formula (4) is described in WO2005075457 and WO2005075456 or these compounds can be prepared in analogy to methods described therein.

Amide coupling reactions to compounds of the formula (6) and of the formula (1) may lead to mixtures of diastereomers or enantiomers under the reaction conditions used due to epimerization at the C-R10 stereogenic center.

**Reaction scheme 3:**

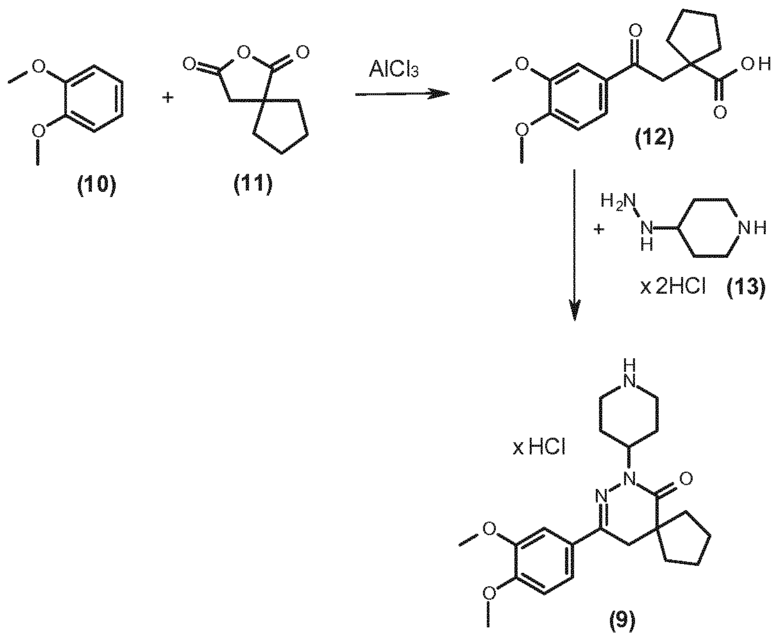

(10) (11) (12) (13) (9)

According to the definition of n and R10 it is possible that R10 is either derived from alpha-aminoacids such as phenylalanine, tyrosine, glycine, serine, alanine and threonine or from beta-aminoacids such such as beta-alanine or beta-phenylalanine.

Suitable starting materials for the synthesis of R10 are N-(tert-butoxycarbonyl)-3,5-difluoro-D-phenylalanine, N-(tert-butoxycarbonyl)-3-methyl-L-phenylalanine, N-(tert-butoxycarbonyl)-4-tert-butyl-D-phenylalanine, N-(tert-butoxycarbonyl)-4-carbamoyl-D-phenylalanine, N-(tert-butoxycarbonyl)-4-carbamoyl-D-phenylalanine, N-(tert-butoxycarbonyl)-O-ethyl-D-tyrosine, (2R)-3-(biphenyl-4-yl)-2-[(tert-butoxycarbonyl)amino]propanoic acid, N-(tert-butoxycarbonyl)-4-cyano-D-phenylalanine, N-(tert-butoxycarbonyl)-4-methyl-D-phenylalanine, N-(tert-butoxycarbonyl)-3-methyl-D-phenylalanine, N-(tert-butoxycarbonyl)-3,4-difluoro-D-phenylalanine, N-(tert-butoxycarbonyl)-3-methoxy-O-methyl-L-tyrosine, N-(tert-butoxycarbonyl)-4-chloro-D-phenylalanine, N-(tert-butoxycarbonyl)-4-fluoro-D-phenylalanine, N-(tert-butoxycarbonyl)-O-methyl-D-tyrosine, N-(tert-butoxycarbonyl)-3-chloro-D-phenylalanine, N-(tert-butoxycarbonyl)-D-phenylalanine, N-(tert-butoxycarbonyl)-2-(trifluoromethyl)-D-phenylalanine, N-(tert-butoxycarbonyl)-2-chloro-D-phenylalanine, N-(tert-butoxycarbonyl)-L-phenylalanine, (betaR)-N-(tert-butoxycarbonyl)-beta-methyl-D-phenylalanine, N-(tert-butoxycarbonyl)-3-pyridin-2-yl-L-alanine, N-(tert-butoxycarbonyl)-3-pyridin-4-yl-L-alanine, N-(tert-butoxycarbonyl)-3-pyridin-3-yl-L-alanine, N-(tert-butoxycarbonyl)-4-fluoro-L-phenylalanine, N-(tert-butoxycarbonyl)-2,4-dichloro-D-phenylalanine, N-(tert-butoxycarbonyl)-4-(trifluoromethyl)-D-phenylalanine, N-(tert-butoxycarbonyl)-D-tyrosine, N-(tert-butoxycarbonyl)-L-tyrosine, N-(tert-butoxycarbonyl)-2-chloro-L-phenylalanine, N-(tert-butoxycarbonyl)-O-methyl-D-tyrosine, N-(tert-butoxycarbonyl)glycine, N-(tert-butoxycarbonyl)-L-serine, N-(tert-butoxycarbonyl)-L-alanine, N-(tert-butoxycarbonyl)-D-alanine, N-(tert-butoxycarbonyl)-D-threonine, (2S)-2-[(tert-butoxycarbonyl)amino]butanoic acid and (2R)-2-[(tert-butoxycarbonyl)amino]-4-phenylbutanoic acid.

It is known to the person skilled in the art that, if there are a number of reactive centers on a starting or intermediate compound, it may be necessary to block one or more reactive centers temporarily by protective groups in order to allow a reaction to proceed specifically at the desired reaction center. A detailed description for the use of a large number of proven protective groups is found, for example, in T. W. Greene, Protective Groups in Organic Synthesis,

John Wiley & Sons, 1999, 3rd Ed., or in P. Kocienski, Protecting Groups, Thieme Medical Publishers, 2000.

The compounds according to the invention are isolated and purified in a manner known per se, e.g. by distilling off the solvent in vacuo and recrystallizing the residue obtained from a suitable solvent or subjecting it to one of the customary purification methods, such as column chromatography on a suitable support material.

As will be appreciated by persons skilled in the art, the invention is not limited to the particular embodiments described herein, but covers all modifications that are within the spirit and scope of the invention as defined by the appended claims.

The following examples illustrate the invention in greater detail, without restricting it. Further compounds according to the invention, of which the preparation is not explicitly described, can be prepared in an analogous way.

The compounds, which are mentioned in the examples, represent preferred embodiments of the invention.

Examples

The following abbreviations are used:

CDI: 1,1'-Carbonylbis-1H-imidazol ; TOTU: *O*-[(Ethoxycarbonyl)cyanomethylenamino]-*N*,*N*,*N'*,*N'*-tetramethyluronium tetrafluoroborate; COMU: (1-Cyano-2-ethoxy-2-oxoethylidenaminooxy)-dimethylamino-morpholino-carbenium hexafluorophosphate; HBTU: *O*-(Benzotriazol-1-yl)-*N*,*N*,*N'*,*N'*-tetramethyluronium hexafluorophosphate; HATU: *O*-(7-Azabenzotriazol-1-yl)-*N*,*N*,*N'*,*N'*-tetramethyl-uronium hexafluorophosphate; TBTU: O-(Benzotriazol-1-yl)-N,N,N',N'-tetramethyluronium tetrafluoroborate; HOAT: 1-hydroxy-7-azabenzotriazole; Boc: t-butoxycarbonyl; **HOBt:** N-Hydroxybenzo-trizole; **DIPEA:** diisopropylethylamine; DCM: dichloromethane; EtOAc: ethyl acetate; **MeOH:** methanol; THF: tetrahydrofuran; DMF: *N*,*N*-dimethylformamide; DIPCDI: N,N'-Diisopropylcarbodiimide; TEA: triethylamine; **XANTPHOS:** (9,9-Dimethyl-9H-xanthene-4,5-diyl)bis(diphenylphosphine); **$Pd(dba)_2$ :** Bis(dibenzylidenaceton)palladium(0); **RT**: room temperature; **h**: hour(s); **min**: minute(s); **d**: day(s); **calc.:** calculated; **(v/v)**: (volume/volume); **(v/v/v)**: (volume/volume/volume); **(v/v/v/v)**: (volume/volume/ volume /volume); **ESI:** electrospray ionization; **MS**: mass spectrometry; **HRMS**: high resolution mass spectrometry; **TLC**: thin layer chromatography; **HPLC**: high-performance liquid chromatography.

Unless otherwise stated compound purification is achieved by flash column chromatography, preparative TLC and preparative HPLC. HPLC purifications are carried out using a Phenomenex Gemini 5 μm C18 (75 x 30 mm) or a Phenomenex Gemini 5 μm C6-Phenyl (75 x 30 mm) or a Phenomenex Gemini 5 μm C18 Axia (75 x 30 mm) column, a binary gradient (solvent A: water, solvent B: acebnitrile), a flow rate of 40 ml/min, formic acid as a buffer or a buffer system consisting of formic acid and ammonium formiate and UV detection at 240 nm.

As used herein the term "e.e." or "enantiomeric excess" refers to the percent by which one enantiomer, E1 is in excess in a mixture of both enantiomers (E1+E2), as calculated by the equation, [(E1-E2)/(E1+E2)] x100% = e.e.

The enantiomeric excess (e.e.) was determined by HPLC using a Chiralcel OD-RH (150 x 4.6 mm) column [mobile phase: 100mM KPF6 pH=2 / acetonitrile (70/30)], a flow rate of 0.6 ml / min and UV detection at 220 & 316 nm.

All mass spectra are obtained using ESI technique. HRMS data of examples 1 to 125 are reported as $MH^+$.

**Final products**

The chemical names have been generated using the software ACD/NAME Library DLL: NAMIPLIB.dll; Version: 11.1.0.22379.

**1. 4-[5-(cyclopropylmethoxy)-1,3-benzodioxol-4-yl]-N-[(2R)-3-(3,5-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (110 mg; compound B71) and DIPEA (0.20 ml) in DCM (3 ml) was added (4aS,8aR)-2-{1-[(2R)-2-amino-3-(3,5-difluorophenyl)propanoyl]piperidin-4-yl}-4-(3,4-dimethoxyphenyl)-4a,5,6,7,8,8a-hexahydrophthalazin-1(2H)-one (172 mg; compound B14) and COMU (146 mg) and the reaction mixture was stirred for 4 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified twice by flash column chromatography [1) amino phase silica gel, eluent: EtOAc / MeOH, 98/2 (v/v); 2) silica gel, eluent: DCM / MeOH, 98/2 (v/v)]. After lyophilisation from acetonitrile / water the title compound was obtained as a solid.

HRMS [$C_{48}H_{49}N_7O_8F_2$]: calc.: 890.3683 found: 890.3681

**2. 4-[2-(cyclopropylmethoxy)-5-fluoro-4-methoxyphenyl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide**

To a mixture of 4-[2-(cyclopropylmethoxy)-5-fluoro-4-methoxyphenyl]-6-methyl-5H-pyrrolo[3,2-d]pyrimidine-7-carboxylic acid (139 mg; compound B12)) and DIPEA (0.25 ml) in DCM (2 ml) was added (4aS,8aR)-2-{1-[(2S)-2-amino-3-(3-methylphenyl)propanoyl]piperidin-4-yl}-4-(3,4-dimethoxy-phenyl)-4a,5,6,7,8,8a-hexahydrophthalazin-1(2H)-one (200 mg; compound B13) and COMU (177 mg) and the reaction mixture was stirred for 3 h at RT. Afterwards a half-saturated aqueous sodium bicarbonate solution was added and the mixture was extracted twice with DCM . The combined organic phases were dried over magnesium sulphate and the organic layer was concentrated under reduced pressure. The resulting residue was purified by preparative HPLC to give the title compound as a solid.

HRMS [$C_{50}H_{56}N_7O_7F$]: calc.: 886.4298 found: 886.4297

**3. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (71 mg; compound B71) and DIPEA (0.13 ml) in DCM (2 ml) was added (4aS,8aR)-2-{1-[(2S)-2-amino-3-(3-methylphenyl)propanoyl]piperidin-4-yl}-4-(3,4-dimethoxyphenyl)-4a,5,6,7,8,8a-hexahydrophthalazin-1(2H)-one (106 mg; compound B13) and COMU (94 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{49}H_{53}N_7O_8$]: calc.: 868.4028 found: 868.4025

**4. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (138 mg; compound B72) and DIPEA (0.25 ml) in DCM (3 ml) was added (4aS,8aR)-2-{1-[(2S)-2-amino-3-(3-methylphenyl)propanoyl]piperidin-4-yl}-4-(3,4-dimethoxyphenyl)-4a,5,6,7,8,8a-hexahydrophthalazin-1(2H)-one (200 mg; compound B13) and COMU (177 mg) and the reaction mixture was stirred for 3 h at RT. Afterwards a half-saturated aqueous sodium bicarbonate solution was added and the mixture was extracted twice with DCM . The combined organic phases were dried over magnesium sulphate and the organic layer was concentrated under reduced pressure. The resulting residue was purified by preparative HPLC to give the title compound as a solid.

HRMS [$C_{50}H_{55}N_7O_8$]: calc.: 882.4185 found: 882.4176

**5. N-[(2R)-3-(4-tert-butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[2-(cyclopropylmethoxy)-5-fluoro-4-methoxyphenyl]-6-methyl-5H-pyrrolo[3,2-d]pyrimidine-7-carboxamide**

To a mixture of 4-[2-(cyclopropylmethoxy)-5-fluoro-4-methoxyphenyl]-6-methyl-5H-pyrrolo[3,2-d]pyrimidine-7-carboxylic acid (142 mg; compound B12) and DIPEA (0.25 ml) in DCM (3 ml) was added (4aS,8aR)-2-{1-[(2R)-2-amino-3-(4-tert-butylphenyl)propanoyl]piperidin-4-yl}-4-(3,4-dimethoxyphenyl)-4a,5,6,7,8,8a-hexahydrophthalazin-1(2H)-one (224 mg; compound B11) and COMU (180 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{53}H_{62}N_7O_7F$]: calc.: 928.4768 found: 928.4763

**6. N-[(2R)-3-(4-tert-butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (71 mg; compound B71) and DIPEA (0.13 ml) in DCM (3 ml) was added (4aS,8aR)-2-{1-[(2R)-2-amino-3-(4-tert-butyl-

phenyl)propanoyl]piperidin-4-yl}-4-(3,4-dimethoxyphenyl)-4a,5,6,7,8,8a-hexahydrophthalazin-1(2H)-one (115 mg; compound B11) and COMU (95 mg) and the reaction mixture was stirred for 4 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{52}H_{59}N_7O_8$]: calc.: 910.4498 found: 910.4492

**7. N-[(2R)-3-(4-carbamoylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (138 mg; compound B71)and DIPEA (0.26 ml) in DCM (3 ml) was added 4-[(2R)-2-amino-3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl]benzamide (220 mg; compound B9) and COMU (184 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC and afterwards by flash column chromatography [amino phase silica gel, eluent: EtOAc / MeOH, 98/2 (v/v)]. After lyophilisation from acetonitrile / water the title compound was obtained as a solid.

HRMS [$C_{49}H_{52}N_8O_9$]: calc.: 897.3930 found: 897.3924

**8. 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-6-methyl-5H-pyrrolo[3,2-d]pyrimidine-7-carboxamide**

To a mixture of 4-[2-(cyclopropylmethoxy)-5-fluoro-4-methoxyphenyl]-6-methyl-5H-pyrrolo[3,2-d]pyrimidine-7-carboxylic acid (144 mg; compound B12) and DIPEA (0.25 ml) in DCM (3 ml) was added (4aS,8aR)-2-{1-[(2R)-2-amino-3-(4-ethoxyphenyl)propanoyl]piperidin-4-yl}-4-(3,4-dimethoxyphenyl)-4a,5,6,7,8,8a-hexahydrophthalazin-1(2H)-one (219 mg; compound B10) and COMU (183 mg) and the reaction mixture was stirred for 3 h at RT. Afterwards a half-saturated aqueous sodium bicarbonate solution was added and the mixture was extracted twicewith DCM. The combined organic phases were dried over magnesium sulphate and the organic layer was concentrated under reduced pressure. The resulting residue was purified by preparative HPLC to give the title compound as a solid.

HRMS [$C_{51}H_{58}N_7O_8F$]: calc.: 916.4404 found: 916.4396

**9. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (71 mg; compound B71) and DIPEA (0.13 ml) in DCM (3 ml) was added (4aS,8aR)-2-{1-[(2R)-2-amino-3-(4-ethoxyphenyl)propanoyl]piperidin-4-yl}-4-(3,4-dimethoxyphenyl)-4a,5,6,7,8,8a-hexahydrophthalazin-1(2H)-one (113 mg; compound B10) and COMU (95 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{50}H_{55}N_7O_9$]: calc.: 898.4134 found: 898.4123

**10. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (143 mg; compound B72) and DIPEA (0.25 ml) in DCM (3 ml) was added (4aS,8aR)-2-{1-[(2R)-2-amino-3-(4-ethoxyphenyl)propanoyl]piperidin-4-yl}-4-(3,4-dimethoxyphenyl)-4a,5,6,7,8,8a-hexahydrophthalazin-1(2H)-one (219 mg; compound B10) and COMU (183 mg) and the reaction mixture was stirred for 3 h at RT. Afterwards a half-saturated aqueous sodium bicarbonate solution was added and the mixture was extracted with DCM. The combined organic phases were dried over magnesium sulphate and the organic layer was concentrated under reduced pressure. The resulting residue was purified by preparative HPLC to give the title compound as a solid.

HRMS [$C_{51}H_{57}N_7O_9$]: calc.: 912.4291 found: 912.4283

**11. N-[(2R)-3-(4-carbamoylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-**(cyclopropylmethoxy)-1,3-benzodioxol-4-yl]-6-methyl-5H-pyrrolo[3,2-d]pyrimidine-7-**carboxamide**

To a mixture of 4-[5-(cyclopropylmethoxy)-1,3-benzodioxol-4-yl]-6-methyl-5H-pyrrolo[3,2-d]pyrimidine-7-carboxylic acid (144 mg; compound B72) and DIPEA (0.26 ml) in DCM (3 ml) was added 4-[(2R)-2-amino-3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl]benzamide (220 mg; compound B9) and COMU (184 mg) and the reaction mixture was stirred for 5 h at RT. Additional COMU (40 mg) was added and the reaction mixture was stirred for 12 h in order to complete the reaction. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.
HRMS [$C_{50}H_{54}N_8O_9$]: calc.: 911.4087 found: 911.4082

**12. N-[(2R)-3-(biphenyl-4-yl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (140 mg; compound B72) and DIPEA (0.25 ml) in DCM (3 ml) was added (4aS,8aR)-2-{1-[(2R)-2-amino-3-(biphenyl-4-yl)propanoyl]piperidin-4-yl}-4-(3,4-dimethoxyphenyl)-4a,5,6,7,8,8a-hexahydrophthalazin-1(2H)-one (200 mg; compound B8) and COMU (179 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.
HRMS [$C_{55}H_{57}N_7O_8$]: calc.: 944.4341 found: 944.4368

**13. N-[(2R)-3-(biphenyl-4-yl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (134 mg; compound B71) and DIPEA (0.25 ml) in DCM (3 ml) was added (4aS,8aR)-2-{1-[(2R)-2-amino-3-(biphenyl-4-yl)propanoyl]piperidin-4-yl}-4-(3,4-dimethoxyphenyl)-4a,5,6,7,8,8a-hexahydrophthalazin-1(2H)-one (226 mg; compound B8) and HBTU (158 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [amino phase silica gel, eluent: EtOAc/ MeOH, 98/2 (v/v)] and twice by flash column chromatography using silica gel [silica gel, eluent: DCM/MeOH, 98/2 (v/v)]. After lyophilsation from acetonitrile / water the title compound was obtained as a solid.
HRMS [$C_{54}H_{55}N_7O_8$]: calc.: 930.4185 found: 930.4171

**14. N-[(2R)-3-(4-cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (135 mg; compound B72) and DIPEA (0.24 ml) in DCM (3 ml) was added 4-[(2R)-2-amino-3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl]benzonitrile (200 mg; compound B3) and COMU (173 mg) and the reaction mixture was stirred for 5 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [amino phase silica gel, eluent: EtOAc/MeOH, 98/2 (v/v)] and afterwards by preparative HPLC to give the title compound as a solid.
HRMS [$C_{50}H_{52}N_8O_8$]: calc.: 893.3981 found: 893.3963

**15. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (129 mg; compound B72) and DIPEA (0.23 ml) in DCM (3 ml) was added (4aS,8aR)-2-{1-[(2R)-2-amino-3-(4-methylphenyl)propanoyl]piperidin-4-yl}-4-(3,4-dimethoxyphenyl)-4a,5,6,7,8,8a-hexahydrophthalazin-1(2H)-one hydrochloride (200 mg; compound B5) and COMU (166 mg) and the reaction mixture was stirred for 3 h at RT. Additional COMU (80 mg) was added in order to complete the reaction. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{50}H_{55}N_7O_8$]: calc.: 882.4185 found: 882.4175

**16. N-[(2R)-3-(4-cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (130 mg; compound B71) and DIPEA (0.24 ml) in DCM (3 ml) waswas added 4-[(2R)-2-amino-3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl]benzonitrile (200 mg; compound B3) and COMU (173 mg) and the reaction mixture was stirred for 2 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified twice by flash column chromatography [1) amino phase silica gel, eluent: EtOAc/MeOH, 98/2 (v/v); 2) silica gel, eluent: DCM/MeOH, 98/2 (v/v)]. After lyophilisation from acetonitrile / water the title compound was obtained as a solid.

HRMS [$C_{49}H_{50}N_8O_8$]: calc.: 879.3824 found: 879.3811

**17. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (129 mg; compound B72) and DIPEA (0.23 ml) in DCM (3 ml) was added (4aS,8aR)-2-{1-[(2R)-2-amino-3-(3-methylphenyl)propanoyl]piperidin-4-yl}-4-(3,4-dimethoxyphenyl)-4a,5,6,7,8,8a-hexahydrophthalazin-1(2H)-one hydrochloride (200 mg; compound B4) and COMU (166 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{50}H_{55}N_7O_8$]: calc.: 882.4185 found: 882.4176

**18. 4-[5-(cyclopropylmethoxy)-1,3-benzodioxol-4-yl]-N-[(2R)-3-(3,4-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (124 mg; compound B72) and DIPEA (0.22 ml) in DCM (3 ml) was added (4aS,8aR)-2-{1-[(2R)-2-amino-3-(3,4-difluorophenyl)propanoyl]piperidin-4-yl}-4-(3,4-dimethoxyphenyl)-4a,5,6,7,8,8a-hexahydrophthalazin-1(2H)-one hydrochloride (200 mg; compound B7) and COMU (159 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{49}H_{51}N_7O_8F_2$]: calc.: 904.3840 found: 904.3819

**19. 4-[5-(cyclopropylmethoxy)-1,3-benzodioxol-4-yl]-N-[(2R)-3-(3,4-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (119 mg; compound B71) and DIPEA (0.22 ml) in DCM (3 ml) was added (4aS,8aR)-2-{1-[(2R)-2-amino-3-(3,4-difluorophenyl)propanoyl]piperidin-4-yl}-4-(3,4-dimethoxyphenyl)-4a,5,6,7,8,8a-hexahydrophthalazin-1(2H)-one hydrochloride (200 mg; compound B7)and COMU (159 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{48}H_{49}N_7O_8F_2$]: calc.: 890.3683 found: 890.3680

**20. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (124 mg; compound B71) and DIPEA (0.23 ml) in DCM (3 ml) was added (4aS,8aR)-2-{1-[(2R)-2-amino-3-(3-methylphenyl)propanoyl]piperidin-4-yl}-4-(3,4-dimethoxyphenyl)-4a,5,6,7,8,8a-hexahydrophthalazin-1(2H)-one hydrochloride (200 mg; compound B4)and COMU (166 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified twice by flash column chromatography [1) amino phase silica gel, eluent: EtOAc/MeOH, 98/2 (v/v); 2) silica gel, eluent: DCM/MeOH, 98/2 (v/v)]. After lyophilisation from acetonitrile / water the title compound was obtained as a solid.

HRMS [$C_{49}H_{53}N_7O_8$]: calc.: 868.4028 found: 868.4014

**21. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (124 mg; compound B71) and DIPEA (0.23 ml) in DCM (3 ml) was added (4aS,8aR)-2-{1-[(2R)-2-amino-3-(4-methylphenyl)propanoyl]piperidin-4-yl}-4-(3,4-dimethoxyphenyl)-4a,5,6,7,8,8a-hexahydrophthalazin-1(2H)-one hydrochloride (200 mg; compound B5) and COMU (166 mg) and the reaction mixture was stirred for 3 h at RT. Afterwards a half-saturated aqueous sodium bicarbonate solution was added and the mixture was extracted twice with DCM. The combined organic phases are dried over magnesium sulphate and the organic layer was concentrated under reduced pressure. The resulting residue was purified twice by flash column chromatography [1) amino phase silica gel, eluent: EtOAc/MeOH, 98/2 (v/v); 2) silica gel, eluent: DCM/MeOH, 98/2 (v/v)]. After lyophilisation from acetonitrile / water the title compound was obtained as a solid.

HRMS [$C_{49}H_{53}N_7O_8$]: calc.: 868.4028 found: 868.4021

**22. 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-6-methyl-5H-pyrrolo[3,2-d]pyrimidine-7-carboxamide**

To a suspension of (4aS,8aR)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one hydrochloride (556 mg; compound B59), 4-[2-(cyclopropylmethoxy)-5-fluoro-4-methoxyphenyl]-6-methyl-5H-pyrrolo[3,2-d]pyrimidine-7-carboxylic acid (371 mg; compound B12)and HATU (434 mg) in DCM (15 ml) was added DIPEA (0.49 ml) and the mixture was stirred for 75 min at RT. Afterwards a saturated aqueous sodium bicarbonate solution (10 ml) and DCM (15 ml) were added, the phases are separated using a phase separator and the organic layer was concentrated under reduced pressure. The resulting residue was purified by flash column chromatography [amino phase silica gel, eluation gradient: EtOAc/MeOH, 100/0 to 95/5 (v/v)] to give the title compound as a solid.

HRMS [$C_{49}H_{55}FN_7O_7$]: calc.: 872.4142 found: 872.4147

**23. 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide**

To a suspension of (4aS,8aR)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one hydrochloride (390 mg; compound B59), 4-[2-(cyclopropylmethoxy)-5-fluoro-4-methoxyphenyl]-5H-pyrrolo[3,2-d]pyrimidine-7-carboxylic acid (250 mg; compound B17) and HATU (319 mg) in DCM (15 ml) was added DIPEA (0.36 ml) and the mixture was stirred for 75 min at RT. Afterwards a saturated aqueous sodium bicarbonate solution (10 ml) and DCM (15 ml) were added, the phases were separated using a phase separator and the organic layer was concentrated under reduced pressure. The resulting residue was purified by flash column chromatography [amino phase silica gel, eluation gradient: EtOAc/Cyclohexane to EtOAc to EtOAc/MeOH, 1/1 to 95/5 (v/v)] to give the title compound as a solid.

HRMS [$C_{48}H_{53}FN_7O_7$]: calc.: 858.3985 found: 858.3996

**24. 4-[2-(cyclopropylmethoxy)-4-fluoro-5-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide**

To a suspension of (4aS,8aR)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one hydrochloride (278 mg; compound B59), 4-[2-(cyclopropylmethoxy)-4-fluoro-5-methoxyphenyl]-5H-pyrrolo[3,2-d]pyrimidine-7-carboxylic acid (179 mg; compound B18) and HATU (228 mg) in DCM (15 ml) was added DIPEA (0.31 ml) and the mixture was stirred for 75 min at RT. Afterwards a saturated aqueous sodium bicarbonate solution (10 ml) and DCM (15 ml) were added, the phases were separated using a phase separator and the organic layer was concentrated under reduced pressure. The resulting residue was purified by flash column chromatography [amino phase silica gel, eluation gradient: EtOAc/Cyclohexane to EtOAc to EtOAc/MeOH, 1/1 to 95/5 (v/v)] to give the title compound as a solid.

HRMS [$C_{48}H_{53}FN_7O_7$]: calc.: 858.3985 found: 858.3979

**25. 4-[5-(cyclopropylmethoxy)-1,3-benzodioxol-4-yl]-N-[(2S)-3-(3,4-dimethoxyphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (246 mg; compound B71) and DIPEA (0.46 ml) in DCM (5 ml) was added HBTU (291 mg). The reaction mixture was stirred for 0.5 h at RT and afterwards (4aS,8aR)-2-{1-[(2S)-2-amino-3-(3,4-dimethoxyphenyl)propanoyl]piperidin-4-yl}-4-(3,4-dimethoxyphenyl)-4a,5,6,7,8,8a-hexahydro-phthalazin-1(2H)-one hydrochloride (403 mg; compound B6) was added and the reaction mixture was stirred for 2 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified twice by flash column chromatography [1) amino phase silica gel, eluation gradient: EtOAc/MeOH, 100/0 to 98/2 (v/v); 2) silica gel, eluation gradient: DCM/MeOH, 98/2 to 95/5 (v/v)]. After lyophilisation from acetonitrile / water the title compound was obtained as a solid.

HRMS [$C_{50}H_{56}N_7O_{10}$]: calc.: 914.4083 found: 914.4095

**26. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-6-methyl-5H-pyrrolo[3,2-d]pyrimidine-7-carboxamide**

To a suspension of (4aS,8aR)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one (519 mg; compound B52), 4-[5-(cyclopropylmethoxy)-1,3-benzodioxol-4-yl]-6-methyl-5H-pyrrolo[3,2-d]pyrimidine-7-carboxylic acid (368 mg; compound B72) and COMU (493 mg) in DCM (10 ml) was added DIPEA (162 mg) and the mixture was stirred for 1 h at RT. Additional COMU (214 mg) was added and after stirring for 1.5 h another batch of COMU (428 mg) was added and the mixture was stirred for 12 h at RT in order to complete the reaction. The mixture was extracted with saturated aqueous sodium bicarbonate solution (3x5 ml) and filtered using a phase separator. The organic layer was concentrated under vacuo and the resulting residue was purified by flash column chromatography [amino phase silica gel, eluation gradient: Cyclohexane/EtOAc/MeOH, 100/0/0 to 0/100/0 to 0/94/6 (v/v/v)]. After lyophilisation from acetonitrile / water (4/1 (v/v)) the title compound was obtained as a solid.

HRMS [$C_{49}H_{53}N_7O_8$]: calc.: 868.4028 found: 868.4017

**27. N-[(2R)-3-(4-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a suspension of (4aS,8aR)-2-{1-[(2R)-2-amino-3-(4-chlorophenyl)propanoyl]piperidin-4-yl}-4-(3,4-dimethoxyphenyl)-4a,5,6,7,8,8a-hexahydrophthalazin-1(2H)-one (1.51 g; compound B20), 4-[5-(cyclopropylmethoxy)-1,3-benzodioxol-4-yl]-5H-pyrrolo[3,2-d]pyrimidine-7-carboxylic acid (967 mg; compound B71) and COMU (1.29 g) in DCM (25 ml) was added DIPEA (1.86 ml) and the mixture was stirred for 45 min at RT. Afterwards a half-saturated aqueous sodium bicarbonate solution (100 ml) was added and the mixture was extracted with DCM (2 x 200 ml). The combined organic phases were dried over magnesium sulphate and the organic layer was concentrated under reduced pressure. The resulting residue was purified first by flash column chromatography [amino phase silica gel, eluation gradient: Cyclohexane to EtOAc to EtOAc / MeOH, 9 /1 (v/v)] second by flash column chromatography [amino phase silica gel, eluation gradient: DCM / MeOH, 1 / 0 to 9 /1 (v/v)] third by preparative TLC [20x20cm TLC plates with 2 mm thickness, eluent: DCM/MeOH/$NEt_3$, 87/10/3 (v/v/v) and afterwards with eluent: DCM/EtOAc/MeOH, 80/12/8 (v/v/v)] and finally by preparative HPLC togive the title compound as a solid.

HRMS [$C_{48}H_{51}ClN_7O_8$]: calc.: 888.3482 found: 888.3494

**28. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (1.05 g; compound B71) and DIPEA (1.94 g) in DCM (40 ml) was added COMU (1.39 g) after stirring for 5 minutes (4aS,8aR)-2-{1-[(2R)-2-amino-3-(4-fluorophenyl)propanoyl]piperidin-4-yl}-4-(3,4-dimethoxyphenyl)-4a,5,6,7,8,8a-hexahydrophthalazin-1(2H)-one hydrochloride (1.7 g; compound B26) was added and and the reaction mixture was stirred for 1 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified twice by flash column chromatography [1) amino phase silica gel, eluent: DCM/MeOH, 100/0 to 98/2 to 97.3 (v/v/v); 2) silica gel, eluent: DCM/MeOH, 100/0 to 98/2 to 97/3 (v/v/v)]. The isolated product was dissolved in methanol treated with charcoal and filtered through a plug of Celite. The solvent was removed under vacuo and after lyophilisation from acetonitrile / water the title compound was obtained as a solid.

HRMS [$C_{48}H_{51}FN_7O_8$]: calc.: 872.3778 found: 872.3777

**29. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (490 mg; compound B71) and DIPEA (0.91 ml) in DCM (20 ml) was added HBTU (579 mg) after stirring for 5 minutes (4aS,8aR)-2-{1-[(2R)-2-amino-3-(4-methoxyphenyl)propanoyl]piperidin-4-yl}-4-(3,4-dimethoxyphenyl)-4a,5,6,7,8,8a-hexahydrophthalazin-1(2H)-one hydrochloride (812 mg; compound B25) was added and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified twice by flash column chromatography [1) amino phase silica gel, eluent: EtOAc/MeOH, 100/0 to 98/2 (v/v); 2) silica gel, eluent: DCM/MeOH, 100/0 to 98/2 (v/v)]. After lyophilisation from acetonitrile / water the title compound was obtained as a solid.

HRMS [$C_{49}H_{54}N_7O_9$]: calc.: 884.3976 found: 884.3995

**30. N-[(2R)-3-(3-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a suspension of (4aS,8aR)-2-{1-[(2R)-2-amino-3-(3-chlorophenyl)propanoyl]piperidin-4-yl}-4-(3,4-dimethoxyphenyl)-4a,5,6,7,8,8a-hexahydrophthalazin-1(2H)-one (1.38 g; compound B19), 4-[5-(cyclopropylmethoxy)-1,3-benzodioxol-4-yl]-5H-pyrrolo[3,2-d]pyrimidine-7-carboxylic acid (879 mg; compound B71) and COMU (1.17 g) in DCM (25 ml) was added DIPEA (1.69 ml) and the mixture was stirred for 75 min at RT. Afterwards a half-saturated aqueous sodium bicarbonate solution (100 ml) was added and the mixture was extracted with DCM (2 x 200 ml). The combined

organic phases were dried over magnesium sulphate and the organic layer was concentrated under reduced pressure. The resulting residue was purified first by flash column chromatography [amino phase silica gel, eluation gradient: Cyclohexane to EtOAc to EtOAc / MeOH, 9/1 (v/v)] second by flash column chromatography [amino phase silica gel, eluation gradient: DCM / MeOH, 1/0 to 9/1 (v/v)] and finally by preparative TLC [20x20cm TLC plates with 2 mm thickness, eluent DCM / MeOH / $NEt_3$, 87/10/3 (v/v/v). After lyophilisation from acetonitrile / water the title compound was obtained as a solid.

HRMS [$C_{48}H_{51}ClN_7O_8$]: calc.: 888.3482 found: 888.3491

**31. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide**

To a suspension of (4aS,8aR)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one (519 mg; compound B52), 4-[5-(cyclopropylmethoxy)-1,3-benzodioxol-4-yl]-5H-pyrrolo[3,2-d]pyrimidine-7-carboxylic acid (353 mg; compound B71) and COMU (493 mg) in DCM (10 ml) was added DIPEA (162 mg) and the mixture was stirred for 1 h at RT. Additional COMU (214 mg) was added and the reaction mixture was stirred for another 1.5 h at RT. The mixture was extracted with saturated aqueous sodium bicarbonate solution (3x5 ml) and filtered using a phase separator. The organic layer was concentrated under vacuo and the resulting residue was purified by flash column chromatography [amino phase silica gel, eluation gradient: Cyclohexane/EtOAc/MeOH, 100/0/0 to 0/100/0 to 0/94/6 (v/v/v)]. After lyophilisation from acetonitrile / water (5/1 (v/v)) the title compound was obtained as a solid.

HRMS [$C_{48}H_{51}N_7O_8$]: calc.: 854.3872 found: 854.3872

**32. 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[2-(trifluoromethyl)phenyl]propan-2-yl}-6-methyl-5H-pyrrolo[3,2-d]pyrimidine-7-carboxamide**

To a suspension of (4aS,8aR)-2-(1-{(2R)-2-amino-3-[2-(trifluoromethyl)phenyl]propanoyl}piperidin-4-yl)-4-(3,4-dimethoxyphenyl)-4a,5,6,7,8,8a-hexahydrophthalazin-1(2H)-one hydrochloride (280 mg; compound B1), 4-[5-(cyclopropylmethoxy)-1,3-benzodioxol-4-yl]-6-methyl-5H-pyrrolo[3,2-d]pyrimidine-7-carboxylic acid (165 mg; compound B72)and HATU (190 mg) in DCM (4 ml) was added DIPEA (0.31 ml) and the mixture was stirred for 0.5 h at RT. Additional (4aS,8aR)-2-(1-{(2R)-2-amino-3-[2-(trifluoromethyl)phenyl]propanoyl}piperidin-4-yl)-4-(3,4-dimethoxyphenyl)-4a,5,6,7,8,8a-hexahydrophthalazin-1(2H)-one hydrochloride (140 mg) and HATU (95 mg) were added and the mixture was stirred for 20 min at RT in order to complete the reaction. Afterwards a saturated aqueous sodium bicarbonate solution (3 ml) was added, the phases were separated using a phase separator and the organic layer was concentrated under reduced pressure. The resulting residue was purified by flash column chromatography [amino phase silica gel, eluation gradient: EtOAc / MeOH, 100/0 to 95/5 (v/v)] and afterwards by preparative HPLC to yield the title compound as a solid.

HRMS [$C_{50}H_{53}F_3N_7O_8$]: calc.: 936.3902 found: 936.3901

**33. 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[2-(trifluoromethyl)phenyl]propan-2-yl}-5H-pyrrolo[3,2-d]pyrimidine-7-carboxamide**

To a suspension of (4aS,8aR)-2-(1-{(2R)-2-amino-3-[2-(trifluoromethyl)phenyl]propanoyl}piperidin-4-yl)-4-(3,4-dimethoxyphenyl)-4a,5,6,7,8,8a-hexahydrophthalazin-1(2H)-one hydrochloride (280 mg; compound B1), 4-[5-(cyclopropylmethoxy)-1,3-benzodioxol-4-yl]-5H-pyrrolo[3,2-d]pyrimidine-7-carboxylic acid (165 mg; compound B71) and HATU (180 mg) in DCM (4 ml) was added DIPEA (0.31 ml) and the mixture was stirred for 1 h at RT. Afterwards a saturated aqueous sodium bicarbonate solution (3 ml) was added, the phases were separated using a phase separator and the organic layer was concentrated under reduced pressure. The resulting residue was purified by flash column chromatography [amino phase silica gel, eluation gradient: EtOAc / MeOH, 97/3 to 95/5 (v/v)] and afterwards by preparative HPLC to yield the title compound as a solid.

HRMS [$C_{49}H_{51}F_3N_7O_8$]: calc.: 922.3746 found: 922.3766

**34. N-[(2R)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide**

To a suspension of (4aS,8aR)-2-{1-[(2R)-2-amino-3-(2-chlorophenyl)propanoyl]piperidin-4-yl}-4-(3,4-dimethoxyphenyl)-4a,5,6,7,8,8a-hexahydrophthalazin-1(2H)-one hydrochloride (265 mg; compound B23), 4-[5-(cyclopropylmethoxy)-1,3-benzodioxol-4-yl]-6-methyl-5H-pyrrolo[3,2-d]pyrimidine-7-carboxylic acid (195 mg; compound B72) and COMU (289 mg) in DCM (10 ml) was added DIPEA (0.46 ml) and the mixture was stirred for 1 h at RT. Additional (4aS,8aR)-2-{1-[(2R)-2-amino-3-(2-chlorophenyl)propanoyl]piperidin-4-yl}-4-(3,4-dimethoxyphenyl)-4a,5,6,7,8,8a-hexahydrophthalazin-1(2H)-one hydrochloride (106 mg; compound B23) and COMU (289 mg) were added in order to complete the reaction. Afterwards a saturated aqueous sodium bicarbonate solution (2.5 ml) was added, the phases were separated using a phase separator and the organic layer was concentrated under reduced pressure. The resulting residue was purified twice by flash column chromatography [first run: amino phase silica gel, eluation gradient: Cyclohexane to EtOAc to EtOAc / MeOH, 9/1 (v/v); second run: Cyclohexane to EtOAc to EtOAc / MeOH, 94/6 (v/v)] and afterwards by preparative HPLC to give the title compound as a solid.
HRMS [$C_{49}H_{53}ClN_7O_8$]: calc.: 902.3639 found: 902.3654

**35. N-[(2R)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a suspension of (4aS,8aR)-2-{1-[(2R)-2-amino-3-(2-chlorophenyl)propanoyl]piperidin-4-yl}-4-(3,4-dimethoxyphenyl)-4a,5,6,7,8,8a-hexahydrophthalazin-1(2H)-one hydrochloride (265 mg; compound B23), 4-[5-(cyclopropylmethoxy)-1,3-benzodioxol-4-yl]-5H-pyrrolo[3,2-d]pyrimidine-7-carboxylic acid (159 mg; compound B71) and COMU (289 mg) in DCM (10 ml) was added DIPEA (0.46 ml) and the mixture was stirred for 1 h at RT. Afterwards a saturated aqueous sodium bicarbonate solution (2.5 ml) was added, the phases were separated using a phase separator and the organic layer was concentrated under reduced pressure. The resulting residue was purified by flash column chromatography [amino phase silica gel, eluation gradient: Cyclohexane to EtOAc to EtOAc / MeOH, 9/1 (v/v)].
HRMS [$C_{48}H_{51}ClN_7O_8$]: calc.: 888.3482 found: 888.3483

**36. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide**

To a suspension of (4aS,8aR)-2-{1-[(2S)-2-amino-3-phenylpropanoyl]piperidin-4-yl}-4-(3,4-dimethoxyphenyl)-4a,5,6,7,8,8a-hexahydrophthalazin-1(2H)-one hydrochloride (333 mg; compound B58), 4-[5-(cyclopropylmethoxy)-1,3-benzodioxol-4-yl]-5H-pyrrolo[3,2-d]pyrimidine-7-carboxylic acid (212 mg; compound B71) and HBTU (250 mg) in DCM (5 ml) was added DIPEA (0.42 ml) and the mixture was stirred for 45 min at RT. Afterwards a saturated aqueous sodium bicarbonate solution (10 ml) was added to the reaction mixture, the phases were separated using a phase separator and the organic layer was concentrated under reduced pressure. The resulting residue was purified first by flash column chromatography [amino phase silica gel, eluation gradient: EtOAc/MeOH, 95/5 to 90/10 (v/v)]. After lyophilisation from acetonitrile / water the title compound was obtained as a solid.
HRMS [$C_{48}H_{52}N_7O_8$]: calc.: 854.3872 found: 854.3870

**37. 4-[2-(cyclopropylmethoxy)-5-fluoro-4-methoxyphenyl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-6-methyl-5H-pyrrolo[3,2-d]pyrimidine-7-carboxamide**

To a mixture of 4-[2-(cyclopropylmethoxy)-5-fluoro-4-methoxyphenyl]-6-methyl-5H-pyrrolo[3,2-d]pyrimidine-7-carboxylic acid (114 mg; compound B12)and DIPEA (0.20 ml) in DCM (3 ml) was added (4aS,8aR)-2-{1-[(2R,3R)-2-amino-3-phenylbutanoyl]piperidin-4-yl}-4-(3,4-dimethoxyphenyl)-4a,5,6,7,8,8a-hexahydrophthalazin-1(2H)-one (164 mg; compound B40) and COMU (145 mg) and the reaction mixture was stirred for 4 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified twice by flash column chromatography [1) amino phase silica gel, eluent: EtOAc / MeOH, 98/2 (v/v); 2) silica gel, eluent: DCM / MeOH, 98/2 (v/v)] and by preparative HPLC to give the title compound as a solid.
HRMS [$C_{50}H_{56}N_7O_7F$]: calc.: 886.4298 found: 886.4290

**38. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]-6-methyl-5H-pyrrolo[3,2-d]pyrimidine-7-carboxamide**

To a suspension of (4aS,8aR)-2-{1-[(2S)-2-amino-3-(pyridin-2-yl)propanoyl]piperidin-4-yl}-4-(3,4-dimethoxyphenyl)-4a,5,6,7,8,8a-hexahydrophthalazin-1(2H)-one hydrochloride (260 mg; compound B21)4-[5-(cyclopropylmethoxy)-1,3-benzodioxol-4-yl]-6-methyl-5H-pyrrolo[3,2-d]pyrimidine-7-carboxylic acid (184 mg; compound B72) and HATU (175 mg) in DCM (4 ml) was added DIPEA (0.35 ml) and the mixture was stirred for 40 min at RT. Additional HATU (around 170 mg) was added and after stirring for 30 min further HATU (around 170 mg), (4aS,8aR)-2-{1-[(2S)-2-amino-3-(pyridin-2-yl)propanoyl]piperidin-4-yl}-4-(3,4-dimethoxyphenyl)-4a,5,6,7,8,8a-hexahydrophthalazin-1(2H)-one hydrochloride (260 mg) and DIPEA (around 0.18 ml) were added in order to complete the reaction. After stirring for 30 min at RT a saturated aqueous sodium bicarbonate solution (2 ml) was added to the reaction mixture, the phases were separated using a phase separator and the organic layer was concentrated under reduced pressure. The organic layer was concentrated under reduced pressure and the resulting residue was purified by flash column chromatography [amino phase silica gel, eluation gradient: EtOAc / MeOH, 100 / 0 to 93 / 7 (v/v)] and afterwards by preparative HPLC to give the title compound as a solid.
HRMS [$C_{48}H_{53}N_8O_8$]: calc.: 869.3981 found: 869.3983

**39. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]-5H-pyrrolo[3,2-d]pyrimidine-7-carboxamide**

To a suspension of (4aS,8aR)-2-{1-[(2S)-2-amino-3-(pyridin-2-yl)propanoyl]piperidin-4-yl}-4-(3,4-dimethoxyphenyl)-4a,5,6,7,8,8a-hexahydrophthalazin-1(2H)-one hydrochloride (260 mg; compound B21), 4-[5-(cyclopropylmethoxy)-1,3-benzodioxol-4-yl]-5H-pyrrolo[3,2-d]pyrimidine-7-carboxylic acid (177 mg; compound B71) and COMU (236 mg) in DCM (3 ml) was added DIPEA (0.35 ml) and the mixture was stirred for 3 h at RT. Afterwards a saturated aqueous sodium bicarbonate solution (2 ml) was added, the phases were separated and the organic phase was dried over sodium sulphate. The organic layer was concentrated under reduced pressure and the resulting residue was purified first by flash column chromatography [amino phase silica gel, eluation gradient: EtOAc / MeOH, 100 / 0 to 92.5 / 7.5 (v/v)]. After lyophilisation from acetonitrile / water the title compound was obtained as a solid.
HRMS [$C_{47}H_{51}N_8O_8$]: calc.: 855.3824 found: 855.3822

**40. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-6-methyl-5H-pyrrolo[3,2-d]pyrimidine-7-carboxamide**

To a suspension of (4aS,8aR)-2-{1-[(2S)-2-amino-3-(pyridin-4-yl)propanoyl]piperidin-4-yl}-4-(3,4-dimethoxyphenyl)-4a,5,6,7,8,8a-hexahydrophthalazin-1(2H)-one hydrochloride (545 mg; compound B64), 4-[5-(cyclopropylmethoxy)-1,3-benzodioxol-4-yl]-6-methyl-5H-pyrrolo[3,2-d]pyrimidine-7-carboxylic acid (386 mg; compound B72) and COMU (493 mg) in DCM (4 ml) was added DIPEA (0.71 ml) and the mixture was stirred for at RT. After 1 h and after 3 h additional COMU (493 mg, respective-ly) was added in order to complete the reaction. Afterwards a saturated aqueous sodium bicarbonate solution (10 ml) and DCM (10 ml) were added and the phases were separated and dried over sodium sulphate. The organic layer was concentrated under reduced pressure and the resulting residue was purified by flash column chromatography [amino phase silica gel, eluation gradient: EtOAc/MeOH, 100/0 to 95/5 (v/v)] and afterwards by preparative HPLC to give the title compound as a solid.
HRMS [$C_{48}H_{53}N_8O_8$]: calc.: 869.3981 found: 869.4003

**41. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-5H-pyrrolo[3,2-d]pyrimidine-7-carboxamide**

To a suspension of (4aS,8aR)-2-{1-[(2S)-2-amino-3-(pyridin-4-yl)propanoyl]piperidin-4-yl}-4-(3,4-dimethoxyphenyl)-4a,5,6,7,8,8a-hexahydrophthalazin-1(2H)-one hydrochloride (545 mg; compound B64), 4-[5-(cyclopropylmethoxy)-1,3-benzodioxol-4-yl]-5H-pyrrolo[3,2-d]pyrimidine-7-carboxylic acid (371 mg; compound B71) and COMU (493 mg) in DCM (4 ml) was added DIPEA (0.71 ml) and the mixture was stirred for 45 min at RT. Afterwards a saturated aqueous sodium bicarbonate solution (10 ml) and DCM (10 ml) were added and the phases were separated using a phase separator. The organic layer was concentrated under reduced pressure and the resulting residue was purified by flash column chromatography [amino phase silica gel, eluation gradient: EtOAc/MeOH, 100/0 to 95/5 to 90/10 (v/v)]. After

lyophilisation from acetonitrile/waterthe title compound was obtained as a solid.

HRMS [$C_{47}H_{51}N_8O_8$]: calc.: 855.3824 found: 855.3822

**42. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]-6-methyl-5H-pyrrolo[3,2-d]pyrimidine-7-carboxamide**

To a suspension of (4aS,8aR)-2-{1-[(2S)-2-amino-3-(pyridin-3-yl)propanoyl]piperidin-4-yl}-4-(3,4-dimethoxyphenyl)-4a,5,6,7,8,8a-hexahydrophthalazin-1(2H)-one (368 mg; compound B22), 4-[5-(cyclopropylmethoxy)-1,3-benzodioxol-4-yl]-6-methyl-5H-pyrrolo[3,2-d]pyrimidine-7-carboxylic acid (260 mg; compound B72) and HBTU (295 mg) in DCM (12 ml) was added DIPEA (0.48 ml) and the mixture was stirred for 1 h at RT. Afterwards DCM (25 ml) and a saturated aqueous sodium bicarbonate solution (10 ml) were added, the phases were separated using a phase separator and the organic layer was concentrated under reduced pressure. The organic layer was concentrated under reduced pressure and the resulting residue was purified by flash column chromatography [1) amino phase silica gel, eluation gradient: Cyclohexane to EtOAc / MeOH, 92 / 8 (v/v); 2)Cyclohexane to EtOAc / MeOH, 95 / 5 (v/v)]. After lyophilisation from acetonitrile / water the title compound was obtained as a solid.

HRMS [$C_{48}H_{53}N_8O_8$]: calc.: 869.3981 found: 869.3984

**43. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]-5H-pyrrolo[3,2-d]pyrimidine-7-carboxamide**

To a suspension of (4aS,8aR)-2-{1-[(2S)-2-amino-3-(pyridin-3-yl)propanoyl]piperidin-4-yl}-4-(3,4-dimethoxyphenyl)-4a,5,6,7,8,8a-hexahydrophthalazin-1(2H)-one (260 mg; compound B22), 4-[5-(cyclopropylmethoxy)-1,3-benzodioxol-4-yl]-5H-pyrrolo[3,2-d]pyrimidine-7-carboxylic acid (177 mg; compound B71) and HBTU (209 mg) in DCM (10 ml) was added DIPEA (0.34 ml) and the mixture was stirred for 1.5 h at RT. Afterwards DCM (15 ml) and a saturated aqueous sodium bicarbonate solution (5 ml) were added, the phases were separated using a phase separator and the organic layer was concentrated under reduced pressure. The organic layer was concentrated under reduced pressure and the resulting residue was purified by flash column chromatography [amino phase silica gel, eluation gradient: Cyclohexane to EtOAc to EtOAc/MeOH, 90/10 (v/v). After lyophilisation from acetonitrile/water the title compound was obtained as a solid.

HRMS [$C_{47}H_{51}N_8O_8$]: calc.: 855.3824 found: 855.3827

**44. 4-[5-(cyclopropylmethoxy)-1,3-benzodioxol-4-yl]-N-[(2S)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide**

To a suspension of (4aR,8aS)-2-{1-[(2S)-2-amino-3-phenylpropanoyl]piperidin-4-yl}-4-(3,4-dimethoxyphenyl)-4a,5,6,7,8,8a-hexahydrophthalazin-1(2H)-one hydrochloride (550 mg; compound B67, , 4-[5-(cyclopropylmethoxy)-1,3-benzodioxol-4-yl]-5H-pyrrolo[3,2-d]pyrimidine-7-carboxylic acid (349 mg; compound B71) and HATU (429 mg) in DCM (10 ml) was added DIPEA (0.48 ml) and the mixture was stirred for 12 h at RT. Afterwards a saturated aqueous sodium bicarbonate solution (8 ml) and DCM (20 ml) were added, the phases were separated using a phase separator and the organic layer was concentrated under reduced pressure. The resulting residue was purified by flash column chromatography [amino phase silica gel, eluation gradient: EtOAc / MeOH, 100/0 to 95 / 5 (v/v)] to give the title compound as a solid.

HRMS [$C_{48}H_{51}N_7O_8$]: calc.: 854.3872 found: 854.3862

**45. 4-[5-(cyclopropylmethoxy)-1,3-benzodioxol-4-yl]-N-[(2R)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide**

To a suspension of (4aR,8aS)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one (330 mg; 78% purity, compound B70), 4-[5-(cyclopropylmethoxy)-1,3-benzodioxol-4-yl]-5H-pyrrolo[3,2-d]pyrimidine-7-carboxylic acid (177 mg; compound B71) and HATU (202 mg) in DCM (10 ml) was added DIPEA (0.26 ml) and the mixture was stirred for about 1 h at RT. Afterwards a saturated aqueous sodium bicarbonate solution (10 ml) and DCM (25 ml) were added, the phases were separated using a phase separator and the organic layer was concentrated under reduced pressure. The resulting residue was purified twice by flash column chromatography [1) amino phase silica gel, eluation gradient EtOAc / MeOH, 100/0 to 95 / 5 (v/v); 2) EtOAc / MeOH,

100/0 to 97 / 3 (v/v)]. After lyophilisation from acetonitrile / water (30 ml, 1/1 (v/v)) the title compound was obtained as a solid.
HRMS [$C_{48}H_{51}N_7O_8$]: calc.: 854.3872 found: 854.3862

**46. N-[(2R)-3-(4-tert-butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (143 mg; compound B72) and DIPEA (0.25 ml) in DCM (3 ml) was added (4aS,8aR)-2-{1-[(2R)-2-amino-3-(4-tert-butylphenyl)propanoyl]piperidin-4-yl}-4-(3,4-dimethoxyphenyl)-4a,5,6,7,8,8a-hexahydrophthalazin-1(2H)-one (224 mg; compound B11) and COMU (183 mg) and the reaction mixture was stirred for 4 h at RT. Afterwards a half-saturated aqueous sodium bicarbonate solution was added and the mixture was extracted with DCM. The combined organic phases were dried over magnesium sulphate and the organic layer was concentrated under reduced pressure. The resulting residue was purified by preparative HPLC to give the title compound as a solid.
HRMS [$C_{53}H_{61}N_7O_8$]: calc.: 924.4654 found: 924.4656

**47. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (101 mg; compound B71) and DIPEA (0.19 ml) in DCM (3 ml) was added (4aS,8aR)-2-{1-[(2S)-2-amino-3-(4-fluorophenyl)propanoyl]piperidin-4-yl}-4-(3,4-dimethoxyphenyl)-4a,5,6,7,8,8a-hexahydrophthalazin-1(2H)-one (154 mg; compound B15) and COMU (135 mg) and the reaction mixture was stirred for 3 h at RT. Afterwards a half-saturated aqueous sodium bicarbonate solution was added and the mixture was extracted with DCM. The combined organic phases were dried over magnesium sulphate and the organic layer was concentrated under reduced pressure. The resulting residue was purified by preparative HPLC to give the title compound as a solid.
HRMS [$C_{48}H_{50}N_7O_8F$]: calc.: 872.3778 found: 872.3772

**48. 4-[5-(cyclopropylmethoxy)-1,3-benzodioxol-4-yl]-N-[(2R)-3-(2,4-dichlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (102 mg; compound B71) and DIPEA (0.19 ml) in DCM (3 ml) was added (4aS,8aR)-2-{1-[(2R)-2-amino-3-(2,4-dichlorophenyl)propanoyl]piperidin-4-yl}-4-(3,4-dimethoxyphenyl)-4a,5,6,7,8,8a-hexahydrophthalazin-1(2H)-one (170 mg; compound B16) and COMU (136 mg) and the reaction mixture was stirred for 2 h at RT. Afterwards a half-saturated aqueous sodium bicarbonate solution was added and the mixture was extracted with DCM. The combined organic phases were dried over magnesium sulphate and the organic layerwas concentrated under reduced pressure. The resulting residue was purified twice by flash column chromatography [1) amino phase silica gel, eluent: EtOAc / MeOH, 98/2 (v/v); 2) silica gel, eluent: DCM / MeOH, 98/2 (v/v)]. After lyophilisation from acetonitrile / water the title compound was obtained as a solid.
HRMS [$C_{48}H_{49}N_7O_8Cl_2$]: calc.: 922.3092 found: 922.3086

**49. 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(trifluoromethyl)phenyl]propan-2-yl}-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (116 mg; compound B71) and DIPEA (0.22 ml) in DCM (3 ml) was added (4aS,8aR)-2-(1-{(2R)-2-amino-3-[4-(trifluoromethyl)phenyl]propanoyl}piperidin-4-yl)-4-(3,4-dimethoxyphenyl)-4a,5,6,7,8,8a-hexahydrophthalazin-1(2H)-one (193 mg; compound B24) and COMU (155 mg) and the reaction mixture was stirred for 2 h at RT. Afterwards a half-saturated aqueous sodium bicarbonate solution was added and the mixture was extracted with DCM. The combined organic phases were dried over magnesium sulphate and the organic layerwas concentrated under reduced pressure. The resulting residue was purified by flash column chromatography [amino phase silica gel, eluent: EtOAc / MeOH, 98/2 (v/v)]. After lyophilisation from acetonitrile / water the title compound was obtained as a solid.
HRMS [$C_{49}H_{50}N_7O_8F_3$]: calc.: 922.3746 found: 922.3749

**50. 4-[5-(cyclopropylmethoxy)-1,3-benzodioxol-4-yl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (113 mg; compound B71) and DIPEA (0.21 ml) in DCM (3 ml) was added (4aS,8aR)-2-{1-[(2R,3R)-2-amino-3-phenylbutanoyl]piperidin-4-yl}-4-(3,4-dimethoxyphenyl)-4a,5,6,7,8,8a-hexahydrophthalazin-1(2H)-one (170 mg; compound B40) and COMU (151 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified twice by flash column chromatography [1) amino phase silica gel, eluent: EtOAc / MeOH, 98/2 (v/v); 2) silica gel, eluent: DCM / MeOH, 98/2 (v/v)]. After lyophilisation from acetonitrile / water the title compound was obtained as a solid.

HRMS [$C_{49}H_{53}N_7O_8$]: calc.: 868.4028 found: 868.4026

**51. 4-[5-(cyclopropylmethoxy)-1,3-benzodioxol-4-yl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (138 mg; compound B72) and DIPEA (0.25 ml) in DCM (3 ml) was added (4aS,8aR)-2-{1-[(2R,3R)-2-amino-3-phenylbutanoyl]piperidin-4-yl}-4-(3,4-dimethoxyphenyl)-4a,5,6,7,8,8a-hexahydrophthalazin-1(2H)-one (200 mg; compound B40) and COMU (177 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by preparative HPLC to give the title compound as a solid.

HRMS [$C_{50}H_{55}N_7O_8$]: calc.: 882.4185 found: 882.4181

**52. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]-5H-pyrrolo[3,2-d]pyrimidine-7-carboxamide**

A mixture of (4aS,8aR)-2-{1-[(2R)-2-amino-3-(4-hydroxyphenyl)propanoyl]piperidin-4-yl}-4-(3,4-dimethoxyphenyl)-4a,5,6,7,8,8a-hexahydrophthalazin-1(2H)-one hydrochloride (300 mg; compound B54) and {4-[5-(cyclopropylmethoxy)-1,3-benzodioxol-4-yl]-5H-pyrrolo[3,2-d]pyrimidin-7-yl}(1H-imidazol-1-yl)methanone (212 mg; compound B53)and DIPEA (204 mg) in DCM (15 ml) was stirred for 10 min at RT, then for ca 15 h under reflux conditions and afterwards for around 2 d at RT. Afterwards DCM was added (35 ml) and the mixture was extracted with a saturated aqueous sodium bicarbonate solution (3x10 ml). The organic phase was concentrated under reduced pressure and the resulting residue was treated with DCM and filtered through a plug of silica gel (eluent: EtOAc). The solvent was removed under vacuo and the residue was purified twice by flash column chromatography [amino phase silica gel, eluent first run: Cyclohexane / EtOAc / MeOH, 100/0/0 to 0/100/0 to 0/90/10 (v/v/v), eluent second run: Cyclohexane / EtOAc / MeOH, 100/0/0 to 0/100/0 to 0/95/5 (v/v/v)]. After lyophilisation from acetonitrile / water (20 ml, 1/1 (v/v)) the title compound was obtained as a solid.

HRMS [$C_{48}H_{51}N_7O_9$]: calc.: 870.3821 found: 870.3823

**53. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]-5H-pyrrolo[3,2-d]pyrimidine-7-carboxamide**

A mixture of (4aS,8aR)-2-{1-[(2S)-2-amino-3-(4-hydroxyphenyl)propanoyl]piperidin-4-yl}-4-(3,4-dimethoxyphenyl)-4a,5,6,7,8,8a-hexahydrophthalazin-1(2H)-one (267 mg; compound B55) and {4-[5-(cyclopropylmethoxy)-1,3-benzodioxol-4-yl]-5H-pyrrolo[3,2-d]pyrimidin-7-yl}(1 H-imidazol-1-yl)methanone (202 mg; compound B53) and DIPEA (162 mg) in DCM (10 ml) was stirred for 7.5 h under reflux conditions and then for around 18 h at RT. Afterwards DCM was added (10 ml) and the mixture was extracted with a saturated aqueous sodium bicarbonate solution (3x5 ml). The organic phase was concentrated under reduced pressure and the resulting residue was purified four times by flash column chromatography [amino phase silica gel, eluent first run: EtOAc / MeOH, 9/1; eluation gradient second run: EtOAc / MeOH, 1/0 to 98/2 to 95/5 (v/v/v); eluent third run: EtOAc / MeOH, 9/1, eluent fourth run: DCM / MeOH, 95 / 5]. After lyophilisation from acetonitrile / water (15 ml, 4/1 (v/v)) the title compound was obtained as a solid.

HRMS [$C_{48}H_{51}N_7O_9$]: calc.: 870.3821 found: 870.3817

**54. N-[(2S)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

A mixture of (4aS,8aR)-2-{1-[(2S)-2-amino-3-(2-chlorophenyl)propanoyl]piperidin-4-yl}-4-(3,4-dimethoxyphenyl)-4a,5,6,7,8,8a-hexahydrophthalazin-1(2H)-one (277 mg; compound B74), 4-[5-(cyclopropylmethoxy)-1,3-benzodioxol-4-yl]-5H-pyrrolo[3,2-d]pyrimidine-7-carboxylic acid (177 mg; compound B71), HBTU (380 mg) and DIPEA (259 mg) in DCM (7.5 ml) was stirred for 2.5 h at RT. Afterwards a saturated aqueous sodium bicarbonate solution (5 ml) was added and the organic phase was separated by using a phase separator. The organic phase was concentrated under reduced pressure and the resulting residue was purified twice by flash column chromatography [amino phase silica gel, eluation gradient for first run: Cyclohexane/EtOAc/MeOH, 1/0/0 to 0/9/1(v/v/v); silica gel, eluation gradient for second run: Cyclohexane/EtOAc/DCM/MeOH, 1/0/0/0 to 0/9/1/0 to 0/8/1/1 (v/v/v/v)]. After lyophilisation from acetonitrile/water (10 ml, 1/1 (v/v)) the title compound was obtained as a solid.
HRMS [$C_{48}H_{50}N_7O_8Cl$]: calc.: 888.3482 found: 888.3473

**55. 4-[5-(cyclopropylmethoxy)-1,3-benzodioxol-4-yl]-N-[(2R)-1-{4-[(4aS,7aR)-4-(3,4-dimethoxyphenyl)-1-oxo-1,4a,5,6,7,7a-hexahydro-2H-cyclopenta[d]pyridazin-2-yl]piperidin-1-yl}-1-oxo-3-phenylpropan-2-yl]-5H-pyrrolo[3,2-d]pyrimidine-7-carboxamide**

To a mixture of (4aS,7aR)-2-{1-[(2R)-2-amino-3-phenylpropanoyl]piperidin-4-yl}-4-(3,4-dimethoxy-phenyl)-2,4a,5,6,7,7a-hexahydro-1H-cyclopenta[d]pyridazin-1-one hydrochloride (171 mg; compound B90) and DIPEA (123 mg) in DCM (10 ml) was added {4-[5-(cyclopropylmethoxy)-1,3-benzodioxol-4-yl]-5H-pyrrolo[3,2-d]pyrimidin-7-yl}(1H-imidazol-1-yl)methanone (212 mg; compound B53) and the reaction mixture was stirred for 48 h at 45 °C and for 72 h at RT. Afterwards the solvent was evaporated and the residue was purified by flash column chromatography [amino phase silica gel, eluation gradient: EtOAc / MeOH, 1/0 to 9/1 (v/v)]. After lyophilisation from acetonitrile / water (6 ml, 4/1 (v/v)) the title compound was obtained as a solid.
HRMS [$C_{47}H_{49}N_7O_8$]: calc.: 840.3715 found: 840.3712

**56. N-[(2S)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide**

A mixture of (4aS,8aR)-2-{1-[(2S)-2-amino-3-(2-chlorophenyl)propanoyl]piperidin-4-yl}-4-(3,4-dimethoxyphenyl)-4a,5,6,7,8,8a-hexahydrophthalazin-1(2H)-one (277 mg; compound B74) 4-[5-(cyclopropylmethoxy)-1,3-benzodioxol-4-yl]-6-methyl-5H-pyrrolo[3,2-d]pyrimidine-7-carboxylic acid (184 mg; compound B72), HBTU (380 mg) and DIPEA (259 mg) in DCM (7.5 ml) was stirred for 2.5 h at RT. Afterwards a saturated aqueous sodium bicarbonate solution (5 ml) was added and the organic phase was separated by using a phase separator. The organic phase was concentrated under reduced pressure and the resulting residue was purified by flash column chromatography [amino phase silica gel, eluation gradient for first run: Cyclohexane/EtOAc/MeOH, 1/0/0 to 0/1/0 to 0/9/1(v/v/v); silica gel, eluation gradient for second run: Cyclohexane/EtOAc/DCM/MeOH, 1/0/0/0 to 0/9/1/0 to 0/8/1/1 (v/v/v/v); silica gel, eluation gradient for third run: Cyclohexane/EtOAc/MeOH, 1/0/0 to 2/8/0 to 0/1/0 to 0/9.5/0.5 (v/v/v)]. After lyophilisation from acetonitrile/water (15 ml, 2/1 (v/v)) the title compound was obtained as a solid.
HRMS [$C_{49}H_{52}N_7O_8Cl$]: calc.: 902.3639 found: 902.3640

**57. 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide**

To a mixture of 4-[2-(cyclopropylmethoxy)-5-fluoro-4-methoxyphenyl]-6-methyl-5H-pyrrolo[3,2-d]pyrimidine-7-carboxylic acid (116.5 mg; compound B12) and DIPEA (0.2 ml) in DCM (3 ml) was added (4aS,8aR)-2-{1-[(2R)-2-amino-3-(3-methylphenyl)propanoyl]piperidin-4-yl}-4-(3,4-dimethoxyphenyl)-4a,5,6,7,8,8a-hexahydrophthalazin-1(2H)-one hydrochloride (173 mg; compound B4) and COMU (143 mg) and the reaction mixture was stirred for 2.5 h at RT. Afterwards a half-saturated aqueous sodium bicarbonate solution was added, the mixture was extracted twice with DCM and the solvent was removed under vacuo. The residue was purified twice by flash column chromatography [1) amino phase silica gel, eluent: EtOAc / MeOH, 98/2 (v/v); 2) silica gel, eluation gradient: DCM / MeOH, 97/3 to 98/2 (v/v)] and finally by preparative HPLC to give the title compound as a solid.

HRMS [$C_{50}H_{56}N_7O_7F$]: calc.: 886.4298 found: 886.4296

**58. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxopropan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (107 mg; compound B72) and DIPEA (0.19 ml) in DCM (3 ml) was added (4aS,8aR)-2-{1-[(2R)-2-amino-3-(4-methoxyphenyl)propanoyl]piperidin-4-yl}-4-(3,4-dimethoxyphenyl)-4a,5,6,7,8,8a-hexahydrophthalazin-1(2H)-one hydrochloride (171 mg; compound B25) and COMU (138 mg) and the reaction mixture was stirred for 4 h at RT. Afterwards a half-saturated aqueous sodium bicarbonate solution was added, the mixture was extracted twice with DCM and the solvent was removed under vacuo. The residue was purified by preparative HPLC to give the title compound as a solid.

HRMS [$C_{50}H_{55}N_7O_9$]: calc.: 898.4134 found: 898.4129

**59. 4-[5-(cyclopropylmethoxy)-1,3-benzodioxol-4-yl]-N-(2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl)-5H-pyrrolo[3,2-d]pyrimidine-7-carboxamide**

To a suspension of 4-[5-(cyclopropylmethoxy)-1,3-benzodioxol-4-yl]-5H-pyrrolo[3,2-d]pyrimidine-7-carboxylic acid (2.30 g; compound B71) (4aS,8aR)-2-[1-(aminoacetyl)piperidin-4-yl]-4-(3,4-dimethoxyphenyl)-4a,5,6,7,8,8a-hexahydrophthalazin-1(2H)-one hydrochloride (2.80 g; compound B80) and HBTU (2.71 g) in DCM (50 ml) was added DIPEA (4.5 ml) and the reaction mixture was stirred for 30 min at RT. Afterwards a saturated aqueous sodium bicarbonate solution (30 ml) was added, the organic phase was separated, dried over sodium sulphate and the organic layer was concentrated under reduced pressure. The resulting residue was purified twice by flash column chromatography [amino phase silica gel, eluation gradient of first run: EtOAc/MeOH, 98/2 to 90/10 (v/v), eluent of second run: EtOAc/MeOH, 90/10 (v/v)]. The organic solvent of all title compound containing fractions was removed under vacuo and the resulting residue was treated with diethyl ether (20 ml), filtered off and washed with ethyl ether and finally dried under vacuo to give the title compound as a solid.

HRMS [$C_{41}H_{45}N_7O_8$]: calc.: 764.3402 found: 764.3398

**60. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxopropan-2-yl]-5H-pyrrolo[3,2-d]pyrimidine-7-carboxamide**

A solution of 4-[5-(cyclopropylmethoxy)-1,3-benzodioxol-4-yl]-5H-pyrrolo[3,2-d]pyrimidine-7-carboxylic acid (2.47 g; compound B71), TOTU (2.30 g), HOAT (952 mg) and DIPEA (3.6 ml) in DMF (50 ml) was stirred for 30 min at RT and afterwards (4aS,8aR)-2-{1-[(2S)-2-amino-3-hydroxypropanoyl]piperidin-4-yl}-4-(3,4-dimethoxyphenyl)-4a,5,6,7,8,8a-hexahydrophthalazin-1(2H)-one trifluoroacetate (3.21 g; compound B78) was added. The reaction mixture was stirred for 1.5 h at RT, then all volatiles were removed under vacuo and the resulting residue was purified by flash column chromatography [amino phase silica gel, eluation gradient: EtOAc/MeOH, 100/0 to 90/10 (v/v)] to give the title compound as a solid.

HRMS [$C_{42}H_{47}N_7O_9$]: calc.: 794.3508 found: 794.3517

**61. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide**

A mixture of 4-[5-(cyclopropylmethoxy)-1,3-benzodioxol-4-yl]-5H-pyrrolo[3,2-d]pyrimidine-7-carboxylic acid (2.41 g; compound B71), (4aS,8aR)-2-{1-[(2S)-2-aminopropanoyl]piperidin-4-yl}-4-(3,4-dimethoxyphenyl)-4a,5,6,7,8,8a-hexahydrophthalazin-1(2H)-one (3.02 g; compound B82), HBTU (2.85 g) and DIPEA (15.91 ml) in DCM (120 ml) was stirred for 2 h at RT. Afterwards the mixture was extracted with a saturated aqueous sodium bicarbonate solution (50 ml), and then the separated organic phase was extracted with half-saturated citric acid solution (75ml) and afterwads with brine and a half-saturated solution of sodium bicarbonate. The organic phase was treated with charcoal (8 g) and DCM (100ml) and filtered through a plug of Celite. The solvent was removed under vacuo and the resulting residue was purified by flash column chromatography [amino phase silica gel, eluation gradient: DCM/Cyclohexane/EtOAc/MeOH, 1/0/0/0 to 1/1/0/0 to 0/1/0/0 to 1/0/0/0 to 0/0/9/1 (v/v/v/v)] to give the title compound as a solid.

HRMS [$C_{42}H_{47}N_7O_8$]: calc.: 778.3559 found: 778.3556

**62. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide**

To a suspension of 4-[5-(cyclopropylmethoxy)-1,3-benzodioxol-4-yl]-5H-pyrrolo[3,2-d]pyrimidine-7-carboxylic acid (212 mg; compound B71), (4aS,8aR)-2-{1-[(2R)-2-aminopropanoyl]piperidin-4-yl}-4-(3,4-dimethoxyphenyl)-4a,5,6,7,8,8a-hexahydrophthalazin-1(2H)-one hydrochloride (266 mg; compound B62)and HBTU (250 mg) in DCM (5 ml) was added DIPEA (0.42 ml) and the reaction mixture was stirred for 30 min at RT. Afterwards a saturated aqueous sodium bicarbonate solution (3 ml) was added, the organic phase was separated using a phase separator and the organic layer was concentrated under reduced pressure. The resulting residuewas purified by flash column chromatography [amino phase silica gel, eluation gradient: EtOAc/MeOH, 95/5 to 90/10 (v/v)]. After lyophilisation from acetonitrile / water (20 ml, 3/1 (v/v)) the title compound was obtained as a solid.

HRMS [$C_{42}H_{47}N_7O_8$]: calc.: 778.3559 found: 778.3561

**63. 4-[5-(cyclopropylmethoxy)-1,3-benzodioxol-4-yl]-N-[(2S,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxobutan-2-yl]-5H-pyrrolo[3,2-d]pyrimidine-7-carboxamide**

To a suspension of 4-[5-(cyclopropylmethoxy)-1,3-benzodioxol-4-yl]-5H-pyrrolo[3,2-d]pyrimidine-7-carboxylic acid (2.47 g; compound B71) (4aS,8aR)-2-{1-[(2S,3R)-2-amino-3-hydroxybutanoyl]-piperidin-4-yl}-4-(3,4-dimethoxyphenyl)-4a,5,6,7,8,8a-hexahydrophthalazin-1(2H)-one hydrochloride (3.31 g; compound B2) and HBTU (2.92 g) in DCM (50 ml) was added DIPEA (4.9 ml) and the reaction mixture was stirred for 30 min at RT. Afterwards a saturated aqueous sodium bicarbonate solution (20 ml) was added to the mixture, the organic phase was separated, dried over sodium sulphate and concentrated under reduced pressure. The resulting residue was purified twice by flash column chromatography [amino phase silica gel, eluation gradient: EtOAc/MeOH, 95/5 to 90/10 (v/v)]. The product containing fractions were collected, the solvent was removed under vacuo and the residue was treated with ethyl ether. The suspension was filtered off and the filter cake was dried under vacuo to give the title compound as a solid.

HRMS [$C_{43}H_{49}N_7O_9$]: calc.: 808.3665 found: 808.3662

**64. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxobutan-2-yl]-6-methyl-5H-pyrrolo[3,2-d]pyrimidine-7-carboxamide**

To a suspension of 4-[5-(cyclopropylmethoxy)-1,3-benzodioxol-4-yl]-6-methyl-5H-pyrrolo[3,2-d]pyrimidine-7-carboxylic acid (147 mg; compound B72), (4aS,8aR)-2-{1-[(2S)-2-aminobutanoyl]-piperidin-4-yl}-4-(3,4-dimethoxyphenyl)-4a,5,6,7,8,8a-hexahydrophthalazin-1(2H)-one hydrochloride (147 mg; compound B51) and HBTU (167 mg) in DCM (4 ml) was added DIPEA (0.28 ml) and the reaction mixture was stirred for 30 min at RT. Additional 4-[5-(cyclopropylmethoxy)-1,3-benzodioxol-4-yl]-6-methyl-5H-pyrrolo[3,2-d]pyrimidine-7-carboxylic acid (75 mg; compound B72) and HBTU (85 mg) were added and the reaction mixture was stirred for 48h at RT. Afterwards a saturated aqueous sodium bicarbonate solution (2 ml) was added to the mixture and the organic phase was separated using a phase separator. The organic layer was concentrated under reduced pressure and the resulting residue was purified by flash column chromatography [amino phase silica gel, eluation gradient: EtOAc/MeOH, 97/3 to 95/5 (v/v)] and by preparative HPLC to give the title compound as a solid.

HRMS [$C_{44}H_{51}N_7O_8$]: calc.: 806.3872 found: 806.3871

**65. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-4-phenylbutan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (799 mg; compound B71) and DIPEA (1.48 ml) in DCM (30 ml) was added HBTU (943 mg) and the suspension was stirred for 30 min at RT. Afterwards (4aS,8aR)-2-{1-[(2R)-2-amino-4-phenylbutanoyl]piperidin-4-yl}-4-(3,4-dimethoxyphenyl)-4a,5,6,7,8,8a-hexahydrophthalazin-1(2H)-one hydrochloride (1.29 g; compound B85) was added and the reaction mixture was stirred for 2 h at RT. Afterwards a half-saturated aqueous sodium bicarbonate solution was added, the mixture was extracted twice with DCM and the organic phase was dried over magnesium sulphate. The organic layer was concentrated under reduced pressure and the resulting residue was purified twice by flash column chromatography [1) amino phase silica gel, eluation gradient: EtOAc/MeOH, 100/0 to 98/2 (v/v); 2) silica gel, eluation gradient: DCM/MeOH, 100/0 to 98/2 (v/v)]. The isolated product was dissolved in acetonitrile and treated with charcoal and filtered through a

plug of Celite. Purification by flash column chromatography [amino phase silica gel, eluation gradient: EtOAc/MeOH, 100/0 to 98/2 (v/v)] and afterwards lyophilisation from acetonitrile/water gave the title compound as a solid.

HRMS [$C_{49}H_{54}N_7O_8$]: calc.: 868.4028 found: 868.4026

**66. 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidine-7-carboxamide**

To a mixture of 4-[5-(cyclopropylmethoxy)-1,3-benzodioxol-4-yl]-6-methyl-5H-pyrrolo[3,2-d]pyrimidine-7-carboxylic acid (153 mg; compound B72) and DIPEA (0.27 ml) in DCM (3 ml) was added 2-{1-[(2R)-2-amino-3-phenylpropanoyl]piperidin-4-yl}-6-(3,4-dimethoxyphenyl)-4,4-dimethyl-4,5-dihydropyridazin-3(2H)-one (205 mg; compound B87) and COMU (196 mg) and the reaction mixture was stirred for 3 h at RT. Afterwards a half-saturated aqueous sodium bicarbonate solution was added and the mixture was extracted with DCM. The combined organic phases were dried over magnesium sulphate and the organic layer was concentrated under reduced pressure. The resulting residue was purified by preparative HPLC to give the title compound as a solid.

HRMS [$C_{47}H_{51}N_7O_8$]: calc.: 842.3872 found: 842.3874

**67. 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (273 mg; compound B71) and DIPEA (0.51 ml) in DCM (5 ml) was added 2-{1-[(2R)-2-amino-3-phenylpropanoyl]piperidin-4-yl}-6-(3,4-dimethoxyphenyl)-4,4-dimethyl-4,5-dihydropyridazin-3(2H)-one (380 mg; compound B87) and HBTU (322 mg) and the reaction mixture was stirred for 2 h at RT. Afterwards a half-saturated aqueous sodium bicarbonate solution was added and the mixture was extracted with DCM. The organic layer was concentrated under reduced pressure and the resulting residue was purified twice by flash column chromatography [1) amino phase silica gel, eluent: EtOAc/MeOH, 98/2 (v/v); 2) silica gel, eluent: DCM/MeOH, 98/2 (v/v)]. After lyophilisation from acetonitrile/water the title compound was obtained as a solid.

HRMS [$C_{46}H_{49}N_7O_8$]: calc.: 828.3715 found: 828.3713

**68. 4-[2-(cyclopropylmethoxy)-5-fluoro-4-methoxyphenyl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide**

To a mixture of 4-[2-(cyclopropylmethoxy)-5-fluoro-4-methoxyphenyl]-5H-pyrrolo[3,2-d]pyrimidine-7-carboxylic acid (250 mg; compound B17) and DIPEA (0.13 ml) in DCM (2 ml) was added 2-{1-[(2R)-2-amino-3-phenylpropanoyl]piperidin-4-yl}-6-(3,4-dimethoxyphenyl)-4,4-dimethyl-4,5-dihydropyridazin-3(2H)-one (98.5 mg; compound B87) and COMU (94.2 mg) and the reaction mixture was stirred for 3 h at RT. Afterwards a half-saturated aqueous sodium bicarbonate solution was added and the mixture was extracted with DCM. The organic layer was concentrated under reduced pressure and the resulting residue was purified by preparative HPLC to give the title compound as a solid.

HRMS [$C_{46}H_{50}FN_7O_7$]: calc.: 832.3829 found: 832.3817

**69. 4-[2-(cyclopropylmethoxy)-5-fluoro-4-methoxyphenyl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidine-7-carboxamide**

To a mixture of 4-[2-(cyclopropylmethoxy)-5-fluoro-4-methoxyphenyl]-6-methyl-5H-pyrrolo[3,2-d]pyrimidine-7-carboxylic acid (155 mg; compound B12) and DIPEA (0.27 ml) in DCM (3 ml) was added 2-{1-[(2R)-2-amino-3-phenylpropanoyl]piperidin-4-yl}-6-(3,4-dimethoxyphenyl)-4,4-dimethyl-4,5-dihydropyridazin-3(2H)-one (205 mg; compound B87) and COMU (196 mg) and the reaction mixture was stirred for 3 h at RT. Afterwards a half-saturated aqueous sodium bicarbonate solution was added and the mixture was extracted with DCM. The organic layer was concentrated under reduced pressure and the resulting residue was purified by preparative HPLC to give the title compound as a solid.

HRMS [$C_{47}H_{52}FN_7O_7$]: calc.: 846.3985 found: 846.3978

**70. N-[(2S)-3-cyclohexyl-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropyl-methoxy)-1,3-benzodioxol-4-yl]-6-methyl-5H-pyrrolo[3,2-d]pyrimidine-7-carboxamide**

To a suspension of (4aS,8aR)-2-{1-[(2S)-2-amino-3-cyclohexylpropanoyl]piperidin-4-yl}-4-(3,4-di-methoxyphenyl)-4a,5,6,7,8,8a-hexahydrophthalazin-1(2H)-one hydrochloride (253 mg; compound B93), 4-[5-(cyclopropylmethoxy)-1,3-benzodioxol-4-yl]-6-methyl-5H-pyrrolo[3,2-d]pyrimidine-7-carboxylic acid (165 mg; compound B72) and HATU (190 mg) in DCM (4 ml) was added DIPEA (0.31 ml) and the mixture was stirred for 30 min at RT. Additional HATU (85 mg) was added and the reaction mixture was stirred for 20 min in order to complete the reaction. Afterwards the mixture was extracted with a saturated aqueous sodium bicarbonate solution (3 ml), the phases were separated using a phase separator and the organic layer was concentrated under reduced pressure. The resulting residue was purified by flash column chromatography [amino phase silica gel, eluation gradient: EtOAc/ MeOH, 100/0 to 95/5 (v/v)]. After lyophilisation from acetonitrile/ water the title compound was obtained as a solid.

HRMS [$C_{49}H_{60}N_7O_8$]: calc.: 874.4498 found: 874.4507

**71. 4-[5-(cyclopropylmethoxy)-1,3-benzodioxol-4-yl]-N-[(2S,3S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxopentan-2-yl]-5H-pyrrolo[3,2-d]pyrimidine-7-carboxamide**

To a suspension of (4aS,8aR)-2-{1-[(2S,3S)-2-amino-3-methylpentanoyl]piperidin-4-yl}-4-(3,4-di-methoxyphenyl)-4a,5,6,7,8,8a-hexahydrophthalazin-1(2H)-one hydrochloride (194 mg; compound B95), 4-[5-(cyclopropylmethoxy)-1,3-benzodioxol-4-yl]-5H-pyrrolo[3,2-d]pyrimidine-7-carboxylic acid (141 mg; compound B71) and HBTU (167 mg) in DCM (4 ml)was added DIPEA (0.28 ml) and the mixture was stirred for 20 min at RT. Additional HBTU (75 mg) was added and the reaction mixture was stirred for 30 min in order to complete the reaction. Afterwards the mixture was extracted with a saturated aqueous sodium bicarbonate solution (2 ml), the phases were separated using a phase separator and the organic layer was concentrated under reduced pressure. The resulting residue was purified twice by flash column chromatography [first column: amino phase silica gel, eluation gradient: EtOAc/MeOH, 97/3 to 95/5 (v/v); second column: amino phase silica gel, eluation gradient: EtOAc/MeOH, 100/0 to 90/10 (v/v)] to give the title compound as a solid.

HRMS [$C_{45}H_{53}N_7O_8$]: calc.: 820.4028 found: 820.4032

**72. N-[(1R)-1-cyclohexyl-2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl]-4-[5-(cyclopropylmethoxy)-1,3-benzodioxol-4-yl]-5H-pyrrolo[3,2-d]pyrimidine-7-carboxamide**

A mixture of (4aS,8aR)-2-{1-[(2R)-2-amino-2-cyclohexylacetyl]piperidin-4-yl}-4-(3,4-dimethoxyphenyl)-4a,5,6,7,8,8a-hexahydrophthalazin-1(2H)-one hydrochloride (175 mg; compound B97) and {4-[5-(cyclopropylmethoxy)-1,3-benzodioxol-4-yl]-5H-pyrrolo[3,2-d]pyrimidin-7-yl}(1H-imidazol-1-yl)-methanone (129 mg; compound B53) and DIPEA (124 mg) in DCM (10 ml) was stirred for 2 d at RT and for 20 h at 45 °C. The mixture was extracted with a saturated aqueous sodium bicarbonate solution (3x5 ml). The organic phase was concentrated under reduced pressure and the resulting residue was purified twice by flash column chromatography [first column: silica gel, eluation gradient: EtOAc/n-hexane, 70/30 to 100/0 (v/v); second column: amino phase silica gel, eluation gradient Cyclohexane/EtOAc/MeOH, 100/0/0 to 0/100/0 to 0/90/10 (v/v/v)]. After lyophilisation from acetonitrile/water (20 ml, 8/2 (v/v)) the title compound was obtained as a solid.

HRMS [$C_{47}H_{55}N_7O_8$]: calc.: 846.4185 found: 846.4183

**73. N-[(1S)-1-cyclohexyl-2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl]-4-[5-(cyclopropylmethoxy)-1,3-benzodioxol-4-yl]-5H-pyrrolo[3,2-d]pyrimidine-7-carboxamide**

A mixture of (4aS,8aR)-2-{1-[(2S)-2-amino-2-cyclohexylacetyl]piperidin-4-yl}-4-(3,4-dimethoxyphenyl)-4a,5,6,7,8,8a-hexahydrophthalazin-1(2H)-one hydrochloride (356 mg; compound B99) and {4-[5-(cyclopropylmethoxy)-1,3-benzodioxol-4-yl]-5H-pyrrolo[3,2-d]pyrimidin-7-yl}(1H-imidazol-1-yl)-methanone (262 mg; compound B53) and DIPEA (252 mg) in DCM (15 ml) was stirred altogether for about 2 d at RT and for 2 d at 45 °C. The mixture was extracted with a saturated aqueous sodium bicarbonate solution (3x20 ml). The organic phase was concentrated under reduced pressure and the resulting residue was purified by flash column chromatography [amino phase silica gel, eluation gradient: Cyclohexane/EtOAc/MeOH, 100/0/0 to 0/100/0 to 0/90/10 (v/v/v)]. After lyophilisation from acetonitrile/water (8/2 (v/v)) the title compound was obtained as a solid.

HRMS [$C_{47}H_{55}N_7O_8$]: calc.: 846.4185 found: 846.4179

**74. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(thiophen-2-yl)propan-2-yl]-6-methyl-5H-pyrrolo[3,2-d]pyrimidine-7-carboxamide**

To a suspension of (4aS,8aR)-2-{1-[(2S)-2-amino-3-(thiophen-2-yl)propanoyl]piperidin-4-yl}-4-(3,4-dimethoxyphenyl)-4a,5,6,7,8,8a-hexahydrophthalazin-1(2H)-one hydrochloride (253 mg; compound B101), 4-[5-(cyclopropylmethoxy)-1,3-benzodioxol-4-yl]-6-methyl-5H-pyrrolo[3,2-d]pyrimidine-7-carboxylic acid (195 mg; compound B72) and COMU (289 mg) in DCM (10 ml) was added DIPEA (289 mg) and the mixture was stirred for 1 h at RT. Additional COMU (482 mg) was added and the reaction mixture was stirred for 2 h in order to complete the reaction. Afterwards the mixture was extracted with a saturated aqueous sodium bicarbonate solution (2.5 ml), the phases were separated using a phase separator and the organic layer was concentrated under reduced pressure. The resulting residuewas purified by flash column chromatography [amino phase silica gel, eluation gradient: Cyclohexane/ EtOAc/MeOH, 100/0/0 to 0/50/50 to 0/90/10 (v/v)]. After lyophilisation from acetonitrile/water (20 ml, 1/1 (v/v)) the title compound was obtained as a solid.

HRMS [$C_{47}H_{52}N_7O_8S$]: calc.: 874.3593 found: 874.3599

**75. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(thiophen-2-yl)propan-2-yl]-5H-pyrrolo[3,2-d]pyrimidine-7-carboxamide**

To a suspension of (4aS,8aR)-2-{1-[(2S)-2-amino-3-(thiophen-2-yl)propanoyl]piperidin-4-yl}-4-(3,4-dimethoxyphenyl)-4a,5,6,7,8,8a-hexahydrophthalazin-1(2H)-one hydrochloride (253 mg; compound B101), 4-[5-(cyclopropylmethoxy)-1,3-benzodioxol-4-yl]-5H-pyrrolo[3,2-d]pyrimidine-7-carboxylic acid (159 mg; compound B71) and COMU (289 mg) in DCM (10 ml) was added DIPEA (289 mg) and the mixture was stirred for 1 h at RT. Afterwards the mixture was extracted with a saturated aqueous sodium bicarbonate solution (2.5 ml), the phases were separated using a phase separator and the organic layer was concentrated under reduced pressure. The resulting residue was purified by flash column chromatography [amino phase silica gel, eluation gradient: Cyclohexane/EtOAc/MeOH, 100/0/0 to 0/50/50 to 0/90/10 (v/v/v)]. After lyophilisation from acetonitrile/water (18 ml, 10/7.5 (v/v)) the title compound was obtained as a solid.

HRMS [$C_{46}H_{50}N_7O_8S$]: calc.: 860.3436 found: 860.3446

**76. 4-[5-(cyclopropylmethoxy)-1,3-benzodioxol-4-yl]-N-(3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl)-5H-pyrrolo[3,2-d]pyrimidine-7-carboxamide**

To a suspension of (4aS,8aR)-2-[1-(3-aminopropanoyl)piperidin-4-yl]-4-(3,4-dimethoxyphenyl)-4a,5,6,7,8,8a-hexahydrophthalazin-1(2H)-one hydrochloride (158 mg; compound B102), 4-[5-(cyclopropylmethoxy)-1,3-benzodioxol-4-yl]-5H-pyrrolo[3,2-d]pyrimidine-7-carboxylic acid (117 mg; compound B71) and HBTU (225 mg) in DCM (10 ml) was added DIPEA (0.28 ml) and the mixture was stirred for 1 h at RT. Afterwards the mixture was extracted with a saturated aqueous sodium bicarbonate solution (2 ml) and DCM (5 ml), the phases were separated using a phase separator and the organic layer was concentrated under reduced pressure. The resulting residue was purified by flash column chromatography [amino phase silica gel, eluation gradient: Cyclohexane/EtOAc/MeOH, 100/0/0 to 0/100/0 to 0/95/5 (v/v/v). After lyophilisation from acetonitrile / water (15 ml, 1/1 (v/v)) the title compound was obtained as a solid.

HRMS [$C_{42}H_{47}N_7O_8$]: calc.: 778.35589 found: 778.3559

**77. 4-[5-(cyclopropylmethoxy)-1,3-benzodioxol-4-yl]-N-[(2R)-4-{4-[(4aS,8aR)-4-(3,4-dimethoxyphenyl)-1-oxo-4a,5,6,7,8,8a-hexahydrophthalazin-2(1H)-yl]piperidin-1-yl}-4-oxo-1-phenylbutan-2-yl]-5H-pyrrolo[3,2-d]pyrimidine-7-carboxamide**

To a suspension of (4aS,8aR)-2-{1-[(3R)-3-amino-4-phenylbutanoyl]piperidin-4-yl}-4-(3,4-dimethoxyphenyl)-4a,5,6,7,8,8a-hexahydrophthalazin-1(2H)-one (238 mg; compound B105), 4-[5-(cyclopropyl-methoxy)-1,3-benzodioxol-4-yl]-5H-pyrrolo[3,2-d]pyrimidine-7-carboxylic acid (158 mg; compound B71) and COMU (211 mg) in DCM (3 ml) was added DIPEA (0.29 ml) and the mixture was stirred for 3 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified twice by flash column chromatography [first column: amino phase silica gel, eluent: EtOAc/MeOH, 98/2 (v/v); second column: silica gel, eluation gradient: DCM/MeOH, 98/2 to 97/3 (v/v)]. After lyophilisation from acetonitrile / water the title compound was obtained as a solid.

HRMS [$C_{49}H_{53}N_7O_8$]: calc.: 868.40284 found: 868.4023

**78. 4-[5-(cyclopropylmethoxy)-1,3-benzodioxol-4-yl]-N-[(2R)-4-{4-[3-(3,4-dimethoxyphenyl)-5,5-dimethyl-6-oxo-5,6-dihydropyridazin-1(4H)-yl]piperidin-1-yl}-4-oxo-1-phenylbutan-2-yl]-5H-pyrrolo[3,2-d]pyrimidine-7-carboxamide**

To a suspension of 2-{1-[(3R)-3-amino-4-phenylbutanoyl]piperidin-4-yl}-6-(3,4-dimethoxyphenyl)-4,4-dimethyl-4,5-dihydropyridazin-3(2H)-one (216 mg; compound B107), 4-[5-(cyclopropylmethoxy)-1,3-benzodioxol-4-yl]-5H-pyrrolo[3,2-d]pyrimidine-7-carboxylic acid (151 mg; compound B71) and COMU (201 mg) in DCM (3 ml) was added DIPEA (0.28 ml) and the mixture was stirred for 3 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified twice by flash column chromatography [first column: amino phase silica gel, eluent: EtOAc/MeOH, 98/2 (v/v); second column: silica gel, eluent: DCM/MeOH, 97/3 (v/v)]. After lyophilisation from acetonitrile / water the title compound was obtained as a solid.
HRMS [$C_{47}H_{51}N_7O_8$]: calc.: 842.38719 found: 842.3873

**79. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-5H-pyrrolo[3,2-d]pyrimidine-7-carboxamide**

To a stirred mixture of 4-[5-(cyclopropylmethoxy)-1,3-benzodioxol-4-yl]-5H-pyrrolo[3,2-d]pyrimidine-7-carboxylic acid (167 mg; compound B71) and DIPEA (0.31 ml) in DCM (3 ml) was added HATU (215 mg). After stirring for 30 min at RT a solution of (2S)-2-amino-1-{4-[(4aS,8aR)-4-(3,4-dimethoxy-phenyl)-1-oxo-4a,5,6,7,8,8a-hexahydrophthalazin-2(1H)-yl]piperidin-1-yl}-5-(piperidin-1-yl)pentane-1,5-dione hydrochloride (285 mg; compound B128) in DCM (4 ml) was added to the reaction mixture and the mixture was stirred for 2 h at RT. Afterwards a saturated aqueous sodium bicarbonate solution was added and the mixture was extracted with DCM (2x). The combined organic phases were dried over magnesium sulphate and the organic layer was concentrated under reduced pressure. The resulting residue was purified by preparative HPLC to give the title compound as a solid.
HRMS [$C_{49}H_{58}N_8O_9$]: calc.: 903.43995 found: 903.4398

**83. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-6-methyl-5H-pyrrolo[3,2-d]pyrimidine-7-carboxamide**

To a stirred mixture of 4-[5-(cyclopropylmethoxy)-1,3-benzodioxol-4-yl]-6-methyl-5H-pyrrolo[3,2-d]pyrimidine-7-carboxylic acid (87.5 mg; compound B72) and DIPEA (0.16 ml) in DCM (3 ml) was added COMU (112 mg) and (2S)-2-amino-1-{4-[(4aS,8aR)-4-(3,4-dimethoxyphenyl)-1-oxo-4a,5,6,7,8,8a-hexahydrophthalazin-2(1H)-yl]piperidin-1-yl}-5-(piperidin-1-yl)pentane-1,5-dione hydrochloride (144 mg; compound B128). After stirring for 5 h at RT an additional batch of 4-[5-(cyclopropylmethoxy)-1,3-benzodioxol-4-yl]-6-methyl-5H-pyrrolo[3,2-d]pyrimidine-7-carboxylic acid (33 mg; compound B72) was added and the reaction mixture was stirred for 12 h at rt. Afterwards additional COMU (50 mg) was added to the reaction mixture and stirring was continued for 12 h in order to complete the reaction. Then a half-saturated aqueous sodium bicarbonate solution was added and the mixture was extracted several times with DCM. The combined organic phases were dried over magnesium sulphate and the organic layer was concentrated under reduced pressure. The resulting residue was purified by flash column chromatography [amino phase silica gel, eluent: EtOAc/MeOH, 98/2 (v/v) and afterwards by preparative HPLC to give the title compound as a solid.
HRMS [$C_{50}H_{60}N_8O_9$]: calc.: 917.45560 found: 917.4557

**84. 4-[2-(cyclopropylmethoxy)-5-fluoro-4-methoxyphenyl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-6-methyl-5H-pyrrolo[3,2-d]pyrimidine-7-carboxamide**

To a stirred mixture of 4-[2-(cyclopropylmethoxy)-5-fluoro-4-methoxyphenyl]-6-methyl-5H-pyrrolo[3,2-d]pyrimidine-7-carboxylic acid (77 mg; compound B12) and DIPEA (0.13 ml) in DCM (2 ml) was added HBTU (83 mg) and (2S)-2-amino-1-{4-[(4aS,8aR)-4-(3,4-dimethoxyphenyl)-1-oxo-4a,5,6,7,8,8a-hexahydrophthalazin-2(1H)-yl]piperidin-1-yl}-5-(piperidin-1-yl)pentane-1,5-dione hydrochloride (122 mg; compound B128). The reaction mixture was stirred for 3 h at RT. Then a half-saturated aqueous sodium bicarbonate solution was added and the mixture was extracted several times with DCM. The combined organic phases were dried over magnesium sulphate and the organic layer was concentrated under reduced pressure. The resulting residue was purified by preparative HPLC to give the title compound as a solid.

HRMS [$C_{50}H_{61}N_8O_8F$]: calc.: 921.46691 found: 921.4670

**85. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-5H-pyrrolo[3,2-d]pyrimidine-7-carboxamide**

To a suspension of (4aS,8aR)-2-{1-[(2R)-2-amino-3-(1,3-thiazol-4-yl)propanoyl]piperidin-4-yl}-4-(3,4-dimethoxyphenyl)-4a,5,6,7,8,8a-hexahydrophthalazin-1(2H)-one hydrochloride (263 mg; compound B109), 4-[5-(cyclopropylmethoxy)-1,3-benzodioxol-4-yl]-5H-pyrrolo[3,2-d]pyrimidine-7-carboxylic acid (177 mg; compound B71) and HBTU (209 mg) in DCM (5 ml) was added DIPEA (0.35 ml) and the mixture was stirred for 0.5 h at RT. Afterwards the mixture was extracted with a saturated aqueous sodium bicarbonate solution (2 ml), the phases were separated using a phase separator and the organic layer was concentrated under reduced pressure. The resulting residue was purified by flash column chromatography [amino phase silica gel, eluation gradient: EtOAc/MeOH, 98/2 to 94/6 (v/v). After lyophilisation from acetonitrile / water the title compound was obtained as a solid.

HRMS [$C_{45}H_{49}N_8O_8S$]: calc.: 861.3389 found: 861.3388

**86. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-5H-pyrrolo[3,2-d]pyrimidine-7-carboxamide**

To a suspension of (4aS,8aR)-2-{1-[(2S)-2-amino-3-(1,3-thiazol-4-yl)propanoyl]piperidin-4-yl}-4-(3,4-dimethoxyphenyl)-4a,5,6,7,8,8a-hexahydrophthalazin-1(2H)-one hydrochloride (263 mg; compound B111), 4-[5-(cyclopropylmethoxy)-1,3-benzodioxol-4-yl]-5H-pyrrolo[3,2-d]pyrimidine-7-carboxylic acid (177 mg; compound B71)and HATU (209 mg) in DCM (5 ml) was added DIPEA (0.35 ml) and the mixture was stirred for 0.5 h at RT. Then additional HATU (95 mg) was added and the mixture was stirred for another 30 min at RT. Afterwards the mixture was extracted with a saturated aqueous sodium bicarbonate solution (3 ml), the phases were separated using a phase separator and the organic layer was concentrated under reduced pressure. The resulting residue was purified by flash column chromatography [amino phase silica gel, eluation gradient: EtOAc/MeOH, 95/5 to 90/10 (v/v). After lyophilisation from acetonitrile / water the title compound was obtained as a solid.

HRMS [$C_{45}H_{49}N_8O_8S$]: calc.: 861.3389 found: 861.3408

**87. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-6-methyl-5H-pyrrolo[3,2-d]pyrimidine-7-carboxamide**

To a suspension of (4aS,8aR)-2-{1-[(2S)-2-amino-3-(1,3-thiazol-4-yl)propanoyl]piperidin-4-yl}-4-(3,4-dimethoxyphenyl)-4a,5,6,7,8,8a-hexahydrophthalazin-1(2H)-one hydrochloride (263 mg; compound B111), 4-[5-(cyclopropylmethoxy)-1,3-benzodioxol-4-yl]-6-methyl-5H-pyrrolo[3,2-d]pyrimidine-7-carboxylic acid (184 mg; compound B72) and HATU (209 mg) in DCM (5 ml) was added DIPEA (0.35 ml) and the mixture was stirred for 0.5 h at RT. Then additional HATU (200 mg) and DIPEA (0.18 ml) was added and the mixture was stirred for 12 h at RT. Afterwards the mixture was extracted with a saturated aqueous sodium bicarbonate solution (3 ml), the phases were separated using a phase separator and the organic layer was concentrated under reduced pressure. The resulting residue was purified by flash column chromatography [amino phase silica gel, eluation gradient: EtOAc/MeOH, 95/5 to 90/10 (v/v). After lyophilisation from acetonitrile / water the title compound was obtained as a solid.

HRMS [$C_{46}H_{51}N_8O_8S$]: calc.: 875.3545 found: 875.3545

**88. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1H-pyrazol-1-yl)propan-2-yl]-5H-pyrrolo[3,2-d]pyrimidine-7-carboxamide**

To a suspension of (4aS,8aR)-2-{1-[(2S)-2-amino-3-(1H-pyrazol-1-yl)propanoyl]piperidin-4-yl}-4-(3,4-dimethoxyphenyl)-4a,5,6,7,8,8a-hexahydrophthalazin-1(2H)-one hydrochloride (3.81 g; compound B112), 4-[5-(cyclopropylmethoxy)-1,3-benzodioxol-4-yl]-5H-pyrrolo[3,2-d]pyrimidine-7-carboxylic acid (2.65 g; compound B71) and HBTU (3.13 g) in DCM (80 ml) was added DIPEA (5.2 ml) and the mixture was stirred for 1 h at RT. Then additional HBTU (1.5 g) and DIPEA (2.6 ml) were added and the mixture was stirred for 12 h at RT. Afterwards the mixture was extracted with a saturated aqueous sodium bicarbonate solution (40 ml), the phases were separated using a phase separator and the

organic layer was concentrated under reduced pressure. The resulting residue was purified triply by flash column chromatography [amino phase silica gel, eluation gradient: EtOAc/MeOH, 95/5 to 90/10 (v/v)] to give the title compound as a solid.

HRMS [$C_{45}H_{50}N_9O_8$]: calc.: 844.3777 found: 844.3790

**89. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]-5H-pyrrolo[3,2-d]pyrimidine-7-carboxamide**

To a suspension of (4aS,8aR)-2-{1-[(2R)-2-amino-3-(1H-imidazol-4-yl)propanoyl]piperidin-4-yl}-4-(3,4-dimethoxyphenyl)-4a,5,6,7,8,8a-hexahydrophthalazin-1(2H)-one (203 mg; compound B114), 4-[5-(cyclopropylmethoxy)-1,3-benzodioxol-4-yl]-5H-pyrrolo[3,2-d]pyrimidine-7-carboxylic acid (141 mg; compound B71) and HBTU (167 mg) in DCM (3 ml) was added DIPEA (0.28 ml) and the mixture was stirred for 1 h at RT. Then additional HBTU (160 mg) was added and the mixture was stirred for 1 h at RT. Afterwards the mixture was extracted with a saturated aqueous sodium bicarbonate solution (10 ml) and DCM (10 ml), the phases were separated using a phase separator, washed with water (10 ml) and the organic layer was concentrated under reduced pressure. The resulting residue was purified by flash column chromatography [amino phase silica gel, eluation gradient: DCM/MeOH, 99/1 to 95/5 (v/v)] and afterwards by preparative TLC [20x20cm TLC plates with 0.5 mm thickness, eluent: DCM/MeOH/ $NH_3$ (aqueous 28% solution), 89/10/1 (v/v/v)] to give the title compound as a solid.

HRMS [$C_{45}H_{50}N_9O_8$]: calc.: 844.3777 found: 844.3781

**90. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]-5H-pyrrolo[3,2-d]pyrimidine-7-carboxamide**

To a suspension of (4aS,8aR)-2-{1-[(2S)-2-amino-3-(1H-imidazol-4-yl)propanoyl]piperidin-4-yl}-4-(3,4-dimethoxyphenyl)-4a,5,6,7,8,8a-hexahydrophthalazin-1(2H)-one trifluoroacetate (249 mg; compound B116), 4-[5-(cyclopropylmethoxy)-1,3-benzodioxol-4-yl]-5H-pyrrolo[3,2-d]pyrimidine-7-carboxylic acid (141 mg; compound B71) and HBTU (167 mg) in DCM (3 ml) was added DIPEA (0.28 ml) and the mixture was stirred for 4 h at RT. Then additional HBTU (160 mg) was added and the mixture was stirred for 1 h at RT. Afterwards the mixture was extracted with a saturated aqueous sodium bicarbonate solution (10 ml) and DCM (10 ml), the phases were separated using a phase separator, washed with water (10 ml) and the organic layer was concentrated under reduced pressure. The resulting residue was purified by flash column chromatography [amino phase silica gel, eluation gradient: DCM/MeOH, 99/1 to 95/5 (v/v)] and afterwards by preparative TLC [20x20cm TLC plates with 0.5 mm thickness, eluent: DCM/MeOH/$NH_3$ (aqueous 28% solution), 89/10/1 (v/v/v)] to give the title compound as a solid.

HRMS [$C_{45}H_{50}N_9O_8$]: calc.: 844.3777 found: 844.3794

**91. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]-5H-pyrrolo[3,2-d]pyrimidine-7-carboxamide**

A mixture of (4aR,8aS)-2-{1-[(2R)-2-amino-3-(1H-indol-3-yl)propanoyl]piperidin-4-yl}-4-(3,4-dimethoxyphenyl)-4a,5,6,7,8,8a-hexahydrophthalazin-1(2H)-one (318 mg; compound B119), 4-[5-(cyclopropylmethoxy)-1,3-benzodioxol-4-yl]-5H-pyrrolo[3,2-d]pyrimidine-7-carboxylic acid (200 mg; compound B71), EDC (109 mg) and HOBt (88 mg) in DMF (5 ml) was stirred for 76 h at RT under argon atmosphere. Afterwards water was added (10 ml) and the mixture was extracted with DCM (3 x 10 ml). The combined organic layers were dried with sodium sulfate, the organic solvent was evaporated under vacuo and the resulting residue was purified by flash column chromatography [silica gel, eluation gradient: DCM/EtOH, 98/2 to 95/5 (v/v)]. The obtained product was treated with acetone / diisopropyl ether (1 ml / 10 ml) to give the title compound as a solid.

HRMS [$C_{50}H_{52}N_8O_8$]: calc.: 893.3981 found: 893.3979

**92. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]-5H-pyrrolo[3,2-d]pyrimidine-7-carboxamide**

To a solution of (4aS,8aR)-2-{1-[(2S)-2-amino-3-(1H-indol-3-yl)propanoyl]piperidin-4-yl}-4-(3,4-dimethoxyphenyl)-4a,5,6,7,8,8a-hexahydrophthalazin-1(2H)-one (611 mg; compound B118) and 4-[5-(cyclopropylmethoxy)-1,3-benzodioxol-4-yl]-5H-pyrrolo[3,2-d]pyrimidine-7-carboxylic acid (387 mg; compound B71) in DMF (10 ml) was added EDC

(210 mg) and HOBt (168 mg) and the reaction mixture was stirred for 12 h at RT. Afterwards water was added (120 ml) and the mixture was extracted with DCM (300 ml). The combined organic layers were dried with sodium sulfate, the organic solvent was evaporated under vacuo and the resulting residue was purified by flash column chromatography [silica gel, eluation gradient: DCM/EtOH, 100/0 to 90/10 (v/v)]. The obtained product was treated with acetone / diisopropyl ether (1 ml / 10 ml) to give the title compound as a solid.

HRMS [$C_{50}H_{52}N_8O_8$]: calc.: 893.3981 found: 893.3971

**93. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1-methyl-1H-indol-3-yl)-1-oxopropan-2-yl]-5H-pyrrolo[3,2-d]pyrimidine-7-carboxamide**

To a suspension of (4aS,8aR)-2-{1-[(2R)-2-amino-3-(1-methyl-1H-indol-3-yl)propanoyl]piperidin-4-yl}-4-(3,4-dimethoxyphenyl)-4a,5,6,7,8,8a-hexahydrophthalazin-1(2H)-one (188 mg; compound B122), 4-[5-(cyclopropylmethoxy)-1,3-benzodioxol-4-yl]-5H-pyrrolo[3,2-d]pyrimidine-7-carboxylic acid (116 mg; compound B71) and HBTU (136 mg) in DCM (2 ml) was added DIPEA (0.23 ml) and the mixture was stirred for 1 h at RT. Then additional HBTU (60 mg) was added and the mixture was stirred for 30 min at RT. Afterwards the mixture was extracted with a saturated aqueous sodium bicarbonate solution (10 ml) and DCM (10 ml), the phases were separated using a phase separator, washed with water (10 ml) and the organic layer was concentrated under reduced pressure. The resulting residue was purified by flash column chromatography [amino phase silica gel, eluation gradient: AcOEt/MeOH, 100/0 to 95/5 (v/v)] and afterwards by preparative TLC [20x20cm TLC plates with 0.5 mm thickness, eluent: DCM/MeOH/ $NH_3$ (aqueous 28% solution), 89/9/2 (v/v/v)] to give the title compound as a solid.

HRMS [$C_{51}H_{55}N_8O_8$]: calc.: 907.4137 found: 907.4141

**94. N-[(2S)-3-cyclohexyl-1-{4-[3-(3,4-dimethoxyphenyl)-5,5-dimethyl-6-oxo-5,6-dihydro-pyridazin-1(4H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (149 mg; compound B71) and DIPEA (0.28 ml) in DCM (3 ml) was added 2-{1-[(2S)-2-amino-3-cyclohexylpropanoyl]piperidin-4-yl}-6-(3,4-dimethoxyphenyl)-4,4-dimethyl-4,5-dihydro-pyridazin-3(2H)-one (210 mg; compound B157) and COMU (198 mg) and the mixture was stirred for 3 h at RT. Afterwards a half-saturated aqueous sodium bicarbonate solution was added and the mixture was extracted several times with DCM. The combined organic phase was dried over sodium sulphate. The organic solvent was removed under vacuo and the resulting residue was purified by preparative HPLC to give the title compound as a solid.

HRMS [$C_{46}H_{55}N_7O_8$]: calc.: 834.41849 found: 834.4181

**95. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-methyl-1-oxopentan-2-yl]-5H-pyrrolo[3,2-d]pyrimidine-7-carboxamide**

To a suspension of (4aS,8aR)-2-{1-[(2R)-2-amino-4-methylpentanoyl]piperidin-4-yl}-4-(3,4-di-methoxyphenyl)-4a,5,6,7,8,8a-hexahydrophthalazin-1(2H)-one (291 mg; compound B125), 4-[5-(cyclopropylmethoxy)-1,3-benzodioxol-4-yl]-5H-pyrrolo[3,2-d]pyrimidine-7-carboxylic acid (212 mg; compound B71) and HBTU (250 mg) in DCM (3 ml) was added DIPEA (0.42 ml) and the mixture was stirred for 1 h at RT. Then additional HBTU (240 mg) and DIPEA (0.21 ml) were added and the mixture was stirred for 1.5 h at RT. Afterwards the mixture was extracted with a saturated aqueous sodium bicarbonate solution (10 ml) and DCM (10 ml), the phases were separated and the organic phase was dried over sodium sulphate. The organic solvent was removed under vacuo and the resulting residue was purified by flash column chromatography [amino phase silica gel, eluation gradient: AcOEt/MeOH, 100/0 to 95/5 (v/v)] and afterwards by preparative HPLC to give the title compound as a solid.

HRMS [$C_{45}H_{53}N_7O_8$]: calc.: 820.4028 found: 820.4026

**96. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]-5H-pyrrolo[3,2-d]pyrimidine-7-carboxamide**

A solution of 4-[5-(cyclopropylmethoxy)-1,3-benzodioxol-4-yl]-5H-pyrrolo[3,2-d]pyrimidine-7-carboxylic acid (1.14 g; compound B71), TOTU (2.11 g), HOAT (1.10 g) and DIPEA (2.08 ml) in DCM/DMF (30 ml, 1/2 (v/v)) was stirred for 40 min at RT and afterwards (4aS,8aR)-2-{1-[(2R)-2-amino-3-(dimethyl-amino)propanoyl]piperidin-4-yl}-4-(3,4-dimethoxyphenyl)-4a,5,6,7,8,8a-hexahydrophthalazin-1(2H)-one trifluoroacetate (1.93 g; compound B144) was added. The reaction mixture was stirred for 6 h at RT. The mixture was partitioned between a saturated aqueous sodium bicarbonate solution (10 ml) and DCM (50 ml), the organic phase was separated using a phase separator and the organic layer was concentrated under reduced pressure. The resulting residue was purified twice by flash column chromatography [amino phase silica gel, eluation gradient for first column: EtOAc/MeOH, 100/0 to 92/8 (v/v); eluation gradient for second column: EtOAc/MeOH, 100/0 to 95/5 (v/v)] and afterwards by preparative HPLC to give the title compound as a solid.

HRMS [$C_{44}H_{52}N_8O_8$]: calc.: 821.39808 found: 821.3966

**97. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]-5H-pyrrolo[3,2-d]pyrimidine-7-carboxamide**

A solution of 4-[5-(cyclopropylmethoxy)-1,3-benzodioxol-4-yl]-5H-pyrrolo[3,2-d]pyrimidine-7-carboxylic acid (148 mg; compound B71), TOTU (179 mg), HOAT (86 mg) and DIPEA (0.21 ml) in DMF (8 ml) was stirred for 75 min at RT and afterwards (4aS,8aR)-2-{1-[(2S)-2-amino-3-(dimethylamino)-propanoyl]piperidin-4-yl}-4-(3,4-dimethoxyphenyl)-4a,5,6,7,8,8a-hexahydrophthalazin-1(2H)-one trifluo-roacetate (252 mg; compound B159) was added. The reaction mixture was stirred for 3.5 h at RT. The mixture was concentrated under vacuo and the resulting residue was purified by flash column chromatography [amino phase silica gel, eluation gradient: EtOAc/MeOH, 95/5 (v/v) and afterwards by preparative TLC [20x20cm TLC plates with 0.5 mm thickness, eluent: EtOAc/MeOH/$NEt_3$, 90/6/4 (v/v/v)]. After lyophilisation from a solvent mixture of acetonitrile (20 ml) / MeOH (5ml) / water (25 ml) the title compound was obtained as a solid.

HRMS [$C_{44}H_{52}N_8O_8$]: calc.: 821.39808 found: 821.3982

**98. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-(methylsulfanyl)-1-oxobutan-2-yl]-5H-pyrrolo[3,2-d]pyrimidine-7-carboxamide**

A mixture of 4-[5-(cyclopropylmethoxy)-1,3-benzodioxol-4-yl]-5H-pyrrolo[3,2-d]pyrimidine-7-carboxylic acid (117 mg; compound B71), (4aS,8aR)-2-{1-[(2R)-2-amino-4-(methylsulfanyl)butanoyl]piperidin-4-yl}-4-(3,4-dimethoxyphenyl)-4a,5,6,7,8,8a-hexahydrophthalazin-1(2H)-one hydrochloride (214 mg, compound B160), DIPEA (0.28 ml) and HBTU (225 mg) in DCM (10 ml) was stirred at RT for 1 h. Afterwards a saturated aqueous sodium bicarbonate solution was added and the mixture was extracted with DCM (5 ml). The phases were separated using a phase separator and the organic layer was concentrated under reduced pressure. The resulting residue was purified by flash column chromatography [amino phase silica gel, eluation gradient: Cyclohexane/EtOAc/MeOH, 100/0/0 to 0/100/0 to 0/95/5 (v/v/v)]. After lyophilisation from acetonitrile / water (20 ml, 1/1.5 (v/v)) the title compound was obtained as a solid.

HRMS [$C_{44}H_{51}N_7O_8S$]: calc.: 838.35926 found: 838.3585

**99. N-[(2R)-3-(4-bromophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide**

To a mixture of (4aS,8aR)-2-{1-[(2R)-2-amino-3-(4-bromophenyl)propanoyl]piperidin-4-yl}-4-(3,4-dimethoxyphenyl)-4a,5,6,7,8,8a-hexahydrophthalazin-1(2H)-one (1.89 g; compound B126) and DIPEA (2.07 ml) in DCM (30 ml) was added 4-[5-(cyclopropylmethoxy)-1,3-benzodioxol-4-yl]-5H-pyrrolo[3,2-d]pyrimidine-7-carboxylic acid (1.12 g; compound B71) and COMU (1.49 g) and the mixture was stirred for 3 h at RT. Afterwards a half-saturated aqueous sodium bicarbonate solution was added and the mixture was extracted several times with DCM. The combined organic phase was dried over sodium sulphate. The organic solvent was removed under vacuo and the resulting residue was purified twice by flash column chromatography [first column: amino phase silica gel, eluent: EtOAc/MeOH, 98:2 (v/v); second column: silica gel, eluent: DCM/MeOH, 98/2 (v/v)] to give the title compound as a solid.

HRMS [$C_{48}H_{50}N_7O_8Br$]: calc.: 934.29565 found: 934.2959

**100. Benzyl (4R)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate**

To a mixture of 4-[5-(cyclopropylmethoxy)-1,3-benzodioxol-4-yl]-5H-pyrrolo[3,2-d]pyrimidine-7-carboxylic acid (0.93 g; compound B71)and DIPEA (1.73 ml) in DCM (30 ml) was added benzyl (4R)-4-amino-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate (1.56 g; compound B155) and COMU (1.24 g) and the mixture was stirred for 3.5 h at RT. Afterwards a half-saturated aqueous

sodium bicarbonate solution was added and the mixture was extracted several times with DCM. The combined organic phase was dried over sodium sulphate. The organic solvent was removed under vacuo and the resulting residue was purified twice by flash column chromatography [first column: silica gel, eluent: Toluene/EtOAc, 8/2 (v/v); second column: amino phase silica gel, eluation gradient: Cyclohexane/EtOAc, 100/0 to 0/100] to give the title compound as a solid.

HRMS [$C_{51}H_{55}N_7O_{10}$]. calc.: 926.40832 found: 926.4087

**101. (4R)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoic acid**

To a solution of benzyl (4R)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate (921 mg; compound 100) in ethanol (40 ml) was added Pd/C (10%) (100 mg) and the mixture was stirred for 3 h under a hydrogen atmosphere. The mixture was filtered off, washed with DCM/MeOH (1:1, (v/v)) and dried under vacuo to give the title compound as a solid.

HRMS [$C_{44}H_{49}N_7O_{10}$]: calc.: 836.36137 found: 836.3613

**102. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(pyrrolidin-1-yl)pentan-2-yl]-5H-pyrrolo[3,2-d]pyrimidine-7-carboxamide**

To a suspension of (4S)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoic acid (167 mg; compound 103), pyrrolidine (14 mg), COMU (171 mg) in DCM (6 ml) was added DIPEA (103 mg) and the mixture was stirred for 1 h at RT. Afterwards the mixture was partitioned between a saturated aqueous sodium bicarbonate solution (10 ml) and DCM (10 ml), the phases were separated and the organic phase was dried over sodium sulphate. The organic solvent was removed under vacuo and the resulting residue was purified twice by flash column chromatography [first column: silica gel, eluation gradient: Cyclohexane/EtOAc/DCM, 100/0/0 to 0/90/10 (v/v/v) to EtOAc/MeOH/DCM, 88/7/5 (v/v/v) to EtOAc/MeOH/DCM, 70/15/15 (v/v/v); second column: amino phase silica gel, eluation gradient: Cyclohexane to EtOAc/MeOH/DCM, 75/10/15 (v/v/v)]. After lyophilisation from acetonitrile / water (20 ml, 1/1 (v/v)) the title compound was obtained as a solid.

HRMS [$C_{48}H_{56}N_8O_9$]: calc.: 889.42430 found: 889.4238

**103. (4S)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoic acid**

To a solution of benzyl (4S)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate (compound 104, 1.15 g) in methanol (40 ml) was added Pd/C (10%) (100 mg) and the mixture was stirred for 1.5 h under a hydrogen atmosphere. The mixture was filtered off, washed with DCM and dried under vacuo to give the title compound as a solid.

HRMS [$C_{44}H_{49}N_7O_{10}$]: calc.: 836.36137 found: 836.3609

**104. Benzyl (4S)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate**

To a suspension of benzyl (4S)-4-amino-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate (2.20 g; compound B133), 4-[5-(cyclo-propylmethoxy)-1,3-benzodioxol-4-yl]-5H-pyrrolo[3,2-d]pyrimidine-7-carboxylic acid (1.31 g; compound B71) and COMU (3.19 g) in DCM (50 ml) was added DIPEA (2.53 ml) and the mixture was stirred for 30 min at RT. Afterwards the mixture was extracted with a saturated aqueous sodium bicarbonate solution (50 ml), the phases were separated and the organic phase was dried over sodium sulphate. The organic solvent was removed under vacuo and the resulting residue was purified twice by flash column chromatography [first column: silica gel, eluation gradient: Cyclohexane/EtOAc, 100/0 to 0/100 (v/v)]; second column: amino phase silica gel, eluation gradient: Cyclohexane to EtOAc to EtOAc/MeOH 97/3 (v/v)] to give the title compound as a solid.

HRMS [$C_{51}H_{55}N_7O_{10}$]: calc.: 926.40832 found: 926.4082

**105. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(pyrrolidin-1-yl)pentan-2-yl]-5H-pyrrolo[3,2-d]pyrimidine-7-carboxamide**

To a suspension of (4R)-4-[({4-[5-(cyclopropylmethoxy)-1,3-benzodioxol-4-yl]-5H-pyrrolo[3,2-d]-pyrimidin-7-yl}carbonyl)amino]-5-{4-[(4aS,8aR)-4-(3,4-dimethoxyphenyl)-1-oxo-4a,5,6,7,8,8a-hexa-hydrophthalazin-2(1H)-yl]piperidin-1-yl}-5-oxopentanoic acid (105 mg, compound 101), pyrrolidine (9 mg), COMU (107 mg) in DCM (5 ml) was added DIPEA (65 mg) and the mixture was stirred for 45 min at RT. Afterwards the mixture was partitioned between a saturated aqueous sodium bicarbonate solution (5 ml) and DCM (10 ml), the phases were separated and the organic phase was dried over sodium sulphate. The organic solvent was removed under vacuo and the resulting residue was purified twice by flash column chromatography [first column: amino phase silica gel, eluation gradient: Cyclohexane/EtOAc/MeOH/DCM, 100/0/0/0 to 0/90/0/10 to 0/70/20/10 (v/v/v/v); second column: amino phase silica gel, eluation gradient: Cyclohexane/EtOAc/MeOH/DCM, 100/0/0/0 to 20/80/0/0 to 0/87/8/5 (v/v/v)]. After lyophilisation from acetonitrile / water (10 ml, 1/1 (v/v)) the title compound was obtained as a solid.

HRMS [$C_{48}H_{56}N_8O_9$]: calc.: 889.42430 found: 889.4236

**106. 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(propan-2-ylamino)pentan-2-yl]-5H-pyrrolo[3,2-d]pyrimidine-7-carboxamide**

To a suspension of (4S)-4-[({4-[5-(cyclopropylmethoxy)-1,3-benzodioxol-4-yl]-5H-pyrrolo[3,2-d]-pyrimidin-7-yl}carbonyl)amino]-5-{4-[(4aS,8aR)-4-(3,4-dimethoxyphenyl)-1-oxo-4a,5,6,7,8,8a-hexa-hydrophthalazin-2(1H)-yl]piperidin-1-yl}-5-oxopentanoic acid (167 mg, compound 103), propan-2-amine (12 mg), COMU (171 mg) in DCM (6 ml) was added DIPEA (103 mg) and the mixture was stirred for 1 h at RT. Afterwards the mixture was partitioned between a saturated aqueous sodium bicarbonate solution (5 ml) and DCM (5 ml), the phases were separated and the organic phase was dried over sodium sulphate. The organic solvent was removed under vacuo and the resulting residue was purified twice by flash column chromatography [first column: silica gel, eluation gradient: Cyclohexane/EtOAc/ DCM, 1/0/0 to 0/9/1 (v/v/v) to EtOAc/MeOH/DCM, 88/7/5 (v/v/v) to EtOAc/MeOH/DCM, 70/15/15 (v/v/v); second column: amino phase silica gel, eluation gradient: Cyclohexane to EtOAc/MeOH/DCM, 75/10/15 (v/v/v)]. After lyophilisation from acetonitrile / water (20 ml, 1/1 (v/v)) the title compound was obtained as a solid.

HRMS [$C_{47}H_{56}N_8O_9$]: calc.: 877.42430 found: 877.4240

**107. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(propan-2-ylamino)pentan-2-yl]-5H-pyrrolo[3,2-d]pyrimidine-7-carboxamide**

To a suspension of (4R)-4-[({4-[5-(cyclopropylmethoxy)-1,3-benzodioxol-4-yl]-5H-pyrrolo[3,2-d]-pyrimidin-7-yl}carbonyl)amino]-5-{4-[(4aS,8aR)-4-(3,4-dimethoxyphenyl)-1-oxo-4a,5,6,7,8,8a-hexa-hydrophthalazin-2(1H)-yl]piperidin-1-yl}-5-oxopentanoic acid (105 mg, compound 101), propan-2-amine (7 mg), COMU (107 mg) in DCM (6 ml) was added DIPEA (65 mg) and the mixture was stirred for 45 min at RT. Afterwards the mixture was partitioned between a saturated aqueous sodium bicarbonate solution (5 ml) and DCM (10 ml), the phases were separated and the organic phase was dried over sodium sulphate. The organic solvent was removed under vacuo and the resulting residue was purified twice by flash column chromatography [first column: silica gel, eluation gradient: Cyclohexane/EtOAc /MeOH/DCM, 100/0/0/0 to 90/0/0/10(v/v/v/v) to 0/70/20/10 (v/v/v/v); second column: amino phase silica gel, eluation gradient: Cyclohexane/EtOAc/MeOH/DCM, 100/0/0/0 to 20/80/0/0(v/v/v/v) to 0/87/8/5 (v/v/v/v)]. After lyophilisation from acetonitrile / water (10 ml, 1/1 (v/v)) the title compound was obtained as a solid.

HRMS [$C_{47}H_{56}N_8O_9$]: calc.: 877.42430 found: 877.4242

**108. 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-3-(4-fluorophenyl)-1-oxopropan-2-yl]-5H-pyrrolo[3,2-d]pyrimidine-7-carboxamide**

To a suspension of 2-{1-[(2R)-2-amino-3-(4-fluorophenyl)propanoyl]piperidin-4-yl}-6-(3,4-dimethoxy-phenyl)-4,4-dimethyl-4,5-dihydropyridazin-3(2H)-one (255 mg; compound B134), 4-[5-(cyclopropyl-methoxy)-1,3-benzodioxol-4-yl]-5H-pyrrolo[3,2-d]pyrimidine-7-carboxylic acid (177 mg; compound B71) and COMU (428 mg) in DCM (12 ml) was added DIPEA (0.34 ml) and the mixture was stirred for 100 min at RT. Afterwards the mixture was extracted with a saturated aqueous sodium bicarbonate solution (5 ml), the phases were separated using a phase separator and the organic layer was concentrated under reduced pressure. The resulting residue was purified by flash column chromatography [amino phase silica gel, eluation gradient: Cyclohexane to EtOAc to EtOAc /MeOH, 90/10 (v/v)] and afterwards by preparative HPLC to give the title compound as a solid.

HRMS [$C_{46}H_{48}N_7O_8F$]: calc.: 846.36211 found: 846.3623

**109. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-5H-pyrrolo[3,2-d]pyrimidine-7-carboxamide**

To a suspension of (4aS,8aR)-2-{1-[(2R)-2-amino-3-(pyridin-4-yl)propanoyl]piperidin-4-yl}-4-(3,4-dimethoxyphenyl)-4a,5,6,7,8,8a-hexahydrophthalazin-1(2H)-one (260 mg, compound 136), 4-[5-(cyclopropylmethoxy)-1,3-benzodioxol-4-yl]-5H-pyrrolo[3,2-d]pyrimidine-7-carboxylic acid (177 mg; compound B71) and COMU (428 mg) in DCM (12 ml) was added DIPEA (0.34 ml) and the mixture was stirred for 100 min at RT. Afterwards the mixture was extracted with a saturated aqueous sodium bicarbonate solution (5 ml), the phases were separated and the organic phase was dried over sodium sulphate. The organic solvent was removed under vacuo and the resulting residue was purified by flash column chromatography [amino phase silica gel, eluation gradient: Cyclohexane to EtOAc to AcOEt/MeOH, 90/10 (v/v)] and afterwards by preparative HPLC to give the title compound as a solid.

HRMS [$C_{47}H_{50}N_8O_8$]: calc.: 855.38243 found: 855.3812

**110. 4-[5-(cyclopropylmethoxy)-1,3-benzodioxol-4-yl]-N-[(2S)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide**

To a suspension of 2-{1-[(2S)-2-amino-3-phenylpropanoyl]piperidin-4-yl}-6-(3,4-dimethoxyphenyl)-4,4-dimethyl-4,5-dihydropyridazin-3(2H)-one (280 mg, compound 153), 4-[5-(cyclopropylmethoxy)-1,3-benzodioxol-4-yl]-5H-pyrrolo[3,2-d]pyrimidine-7-carboxylic acid (201 mg; compound B71) and COMU (487 mg) in DCM (12 ml) was added DIPEA (0.39 ml) and the mixture was stirred for 100 min at RT. Afterwards the mixture was extracted with a saturated aqueous sodium bicarbonate solution (5 ml), the phases were separated and the organic phase was dried over sodium sulphate. The organic solvent was removed under vacuo and the resulting residue was purified by flash column chromatography [amino phase silica gel, eluation gradient: Cyclohexane to EtOAc to AcOEt/MeOH, 90/10 (v/v)]. After lyophilisation from acetonitrile / water (25 ml, 1/1 (v/v)) the title compound was obtained as a solid.

HRMS [$C_{46}H_{49}N_7O_8$]: calc.: 828.37154 found: 828.3711

**111. 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxopropan-2-yl]-5H-pyrrolo[3,2-d]pyrimidine-7-carboxamide**

To a suspension of 2-{1-[(2R)-2-aminopropanoyl]piperidin-4-yl}-6-(3,4-dimethoxyphenyl)-4,4-dimethyl-4,5-dihydropyridazin-3(2H)-one (208 mg, compound B151), 4-[5-(cyclopropylmethoxy)-1,3-benzo-dioxol-4-yl]-5H-pyrrolo[3,2-d]pyrimidine-7-carboxylic acid (177 mg; compound B71) and COMU (428 mg) in DCM (12 ml) was added DIPEA (0.34 ml) and the mixture was stirred for 100 min at RT. Afterwards the mixture was extracted with a saturated aqueous sodium bicarbonate solution (5 ml), the phases were separated and the organic phase was dried over sodium sulphate. The organic solvent was removed under vacuo and the resulting residue was purified by flash column chromatography [amino phase silica gel, eluation gradient: Cyclohexane to EtOAc to AcOEt / MeOH, 90/10 (v/v)]. After lyophilisation from acetonitrile / water (25 ml, 1/1 (v/v)) the title compound was obtained as a solid.

HRMS [$C_{40}H_{45}N_7O_8$]: calc.: 752.34024 found: 752.3403

**112. 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(2-oxoazetidin-1-yl)phenyl]propan-2-yl}-5H-pyrrolo[3,2-d]pyrimidine-7-carboxamide**

A suspension of N-[(2R)-3-(4-bromophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropyl-methoxy)-1,3-benzodioxol-4-yl]-5H-pyrrolo[3,2-d]pyrimidine-7-carboxamide (110 mg; compound 99), azetidin-2-one (25 mg), Xantphos (34 mg), $Pd(dba)_2$ (34 mg) and $Cs_2CO_3$ (77mg) in 1,4-dioxane (2.5 ml) was placed in a microwave tube and subjected to microwave irradiation at 140 °C for 2.5 h. The reaction mixture was concentrated in vacuo and the residue was purified twice by flash column chromatography [silica gel, eluation gradient: Cyclohexane to EtOAc to EtOAc / MeOH, 90/10 (v/v)] to yield the title compound as a solid. After lyophilisation from acetonitrile / water (20 ml, 1/1 (v/v)) the title compound was obtained as a solid.

HRMS [$C_{51}H_{54}N_8O_9$]: calc.: 923.40865 found: 923.4088

**113. 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(2-oxopyrrolidin-1-yl)phenyl]propan-2-yl}-5H-pyrrolo[3,2-d]pyrimidine-7-carboxamide**

A suspension of N-[(2R)-3-(4-bromophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropyl-methoxy)-1,3-benzodioxol-4-yl]-5H-pyrrolo[3,2-d]pyrimidine-7-carboxamide (110 mg; compound 99), pyrrolidin-2-one (30 mg), Xantphos (34 mg), $Pd(dba)_2$ (34 mg) and $Cs_2CO_3$ (77mg) in 1,4-dioxane (2.5 ml) was placed in a microwave tube and subjected to microwave irradiation at 140 °C for 2.5 h. The reaction mixture was concentrated in vacuo and the residue was purified twice by flash column chromatography [silica gel, eluation gradient: Cyclohexane to EtOAc to EtOAc / MeOH, 9/1 (v/v)] to yield the title compound as a solid. After lyophilisation from acetonitrile / water (20 ml, 1/1 (v/v)) the title compound was obtained as a solid.

HRMS [$C_{52}H_{56}N_8O_9$]: calc.: 937.42430 found: 937.4238

**114. 4-[5-(cyclopropylmethoxy)-1,3-benzodioxol-4-yl]-N-[(2S)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-(pyridin-3-yl)propan-2-yl]-5H-pyrrolo[3,2-d]pyrimidine-7-carboxamide**

To a mixture of 4-[5-(cyclopropylmethoxy)-1,3-benzodioxol-4-yl]-5H-pyrrolo[3,2-d]pyrimidine-7-carboxylic acid (151 mg; compound B71) and DIPEA (0.28 ml) in DCM (3 ml) was added COMU (201 mg) and 2-{1-[(2S)-2-amino-3-(pyridin-3-yl)propanoyl]piperidin-4-yl}-6-(7-methoxy-2,2-dimethyl-2,3-dihydro-1-benzofuran-4-yl)-4,4-dimethyl-4,5-dihydropyridazin-3(2H)-one (228 mg; compound B139). The reaction mixture was stirred for 3 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified by preparative HPLC to give the title compound as a solid.

HRMS [$C_{48}H_{52}N_8O_8$]: calc.: 869.39808 found: 869.3970

**115. 4-[5-(cyclopropylmethoxy)-1,3-benzodioxol-4-yl]-N-[(2R)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidine-7-carboxamide**

To a suspension of 2-{1-[(2R)-2-amino-3-phenylpropanoyl]piperidin-4-yl}-6-(7-methoxy-2,2-dimethyl-2,3-dihydro-1-benzofuran-4-yl)-4,4-dimethyl-4,5-dihydropyridazin-3(2H)-one (186 mg; compound B141), 4-[5-(cyclopropylmethoxy)-1,3-benzodioxol-4-yl]-5H-pyrrolo[3,2-d]pyrimidine-7-carboxylic acid (124 mg; compound B71) and HBTU (266 mg) in DCM (10 ml) was added DIPEA (0.12 ml) and the mixture was stirred for 1 h at RT. Afterwards the mixture was extracted with a saturated aqueous sodium bicarbonate solution (3 x 5 ml), the phases were separated and the organic phase was dried over sodium sulphate. The organic solvent was removed under vacuo and the resulting residue was purified by flash column chromatography [amino phase silica gel, eluation gradient: Cyclohexane to EtOAc to AcOEt/MeOH, 95/5 (v/v)]. After lyophilisation from acetonitrile / water (25 ml, 2/1 (v/v)) the title compound was obtained as a solid.

HRMS [$C_{49}H_{53}N_7O_8$]: calc.: 868.40284 found: 868.4021

**116. 4-[5-(cyclopropylmethoxy)-1,3-benzodioxol-4-yl]-N-[(2R)-1-{4-[3-(3,4-dimethoxyphenyl)-5,5-diethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide**

To a suspension of 2-{1-[(2R)-2-amino-3-phenylpropanoyl]piperidin-4-yl}-6-(3,4-dimethoxyphenyl)-4,4-diethyl-4,5-dihydropyridazin-3(2H)-one (164 mg; compound B142), 4-[5-(cyclopropylmethoxy)-1,3-benzodioxol-4-yl]-5H-pyrrolo[3,2-d]pyrimidine-7-carboxylic acid (111 mg; compound B71) and COMU (270 mg) in DCM (7.5 ml) was added DIPEA (0.16 ml) and the mixture was stirred for 30 min at RT. Afterwards the mixture was extracted with a saturated aqueous sodium bicarbonate solution (3 x 5 ml), the phases were separated and the organic phase was dried over sodium sulphate. The organic solvent was removed under vacuo and the resulting residue was purified by flash column chromatography [amino phase silica gel, eluation gradient: Cyclohexane to EtOAc to AcOEt/MeOH, 95/5 (v/v)]. After lyophilisation from acetonitrile / water (30 ml, 4/1 (v/v)) the title compound was obtained as a solid.

HRMS [$C_{48}H_{53}N_7O_8$]: calc.: 856.40284 found: 856.4025

**117. 4-[5-(cyclopropylmethoxy)-1,3-benzodioxol-4-yl]-N-(2-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-2-oxoethyl)-5H-pyrrolo[3,2-d]pyrimidine-7-carboxamide**

To a mixture of 2-[1-(aminoacetyl)piperidin-4-yl]-6-(7-methoxy-2,2-dimethyl-2,3-dihydro-1-benzofuran-4-yl)-4,4-dimethyl-4,5-dihydropyridazin-3(2H)-one hydrochloride (180 mg; compound B149) and DIPEA (0.25 ml) in DCM (3 ml) was added 4-[5-(cyclopropylmethoxy)-1,3-benzodioxol-4-yl]-5H-pyrrolo[3,2-d]pyrimidine-7-carboxylic acid (146 mg; compound B71) and HBTU (157 mg) and the reaction mixture was stirred for 3 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified by preparative HPLC to give the title compound as a solid.

HRMS [$C_{42}H_{47}N_7O_8$]: calc.: 778.35589 found: 778.3558

**118. 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-5-(dimethylamino)-1,5-dioxopentan-2-yl]-6-methyl-5H-pyrrolo[3,2-d]pyrimidine-7-carboxamide**

4-[5-(cyclopropylmethoxy)-1,3-benzodioxol-4-yl]-6-methyl-5H-pyrrolo[3,2-d]pyrimidine-7-carboxylic acid (184 mg; compound B72), HATU (228 mg) and DIPEA (0.35 ml) were suspended in DCM (6 ml) and the mixture was stirred for 45 min at RT. A solution of (4R)-4-amino-5-{4-[3-(3,4-dimethoxyphenyl)-5,5-dimethyl-6-oxo-5,6-dihydropyridazin-1(4H)-yl]piperidin-1-yl}-N,N-dimethyl-5-oxopentanamide hydrochloride (269 mg; compound B164) in DCM (10 ml) was added to the reaction mixture and stirring was continued for 12 h. An additional batch of DIPEA (0.25 ml) and HATU (200 mg) was added and the mixture was stirred for another 3 d at RT in order to complete the reaction. Afterwards the mixture was sequentially extracted by an aqueous solution of hydrogen chloride, water and brine. The organic phase was separated using a phase separator and the organic layer was concentrated under reduced pressure. The resulting residue was purified by preparative HPLC to give the title compound as a solid.

**119. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxobutan-2-yl]-5H-pyrrolo[3,2-d]pyrimidine-7-carboxamide**

A solution of 4-[5-(cyclopropylmethoxy)-1,3-benzodioxol-4-yl]-5H-pyrrolo[3,2-d]pyrimidine-7-carboxylic acid (247 mg; compound B71), TOTU (230 mg), HOAT (95 mg) and DIPEA (0.4 ml) in DMF (3.5 ml) was stirred for 30 min at RT and afterwards (4aS,8aR)-2-{1-[(2R)-2-amino-3-methylbutanoyl]piperidin-4-yl}-4-(3,4-dimethoxyphenyl)-4a,5,6,7,8,8a-hexahydrophthalazin-1(2H)-one trifluoroacetate (330 mg; compound B168) was added. The reaction mixture was stirred for 3 h at RT. The solvent was removed under vacuo and the resulting residue was purified by flash column chromatography [amino phase silica gel, eluation gradient: EtOAc/MeOH, 100/0 to 95/5 (v/v)] and afterwards by preparative HPLC to give the title compound as a solid.

HRMS [$C_{44}H_{51}N_7O_8$]: calc.: 806.38719 found: 806.3864

**120. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxobutan-2-yl]-6-methyl-5H-pyrrolo[3,2-d]pyrimidine-7-carboxamide**

A solution of 4-[5-(cyclopropylmethoxy)-1,3-benzodioxol-4-yl]-6-methyl-5H-pyrrolo[3,2-d]pyrimidine-7-carboxylic acid (257 mg; compound B72), TOTU (230 mg), HOAT (95 mg) and DIPEA (0.4 ml) in DMF (3.5 ml) was stirred for 30 min at RT and afterwards (4aS,8aR)-2-{1-[(2R)-2-amino-3-methyl-butanoyl]piperidin-4-yl}-4-(3,4-dimethoxyphenyl)-4a,5,6,7,8,8a-hexahydrophthalazin-1(2H)-one trifluoroacetate (330 mg; compound B168) was added. The reaction mixture was stirred for 3 h at RT. The solvent was removed under vacuo and the resulting residue was purified by flash column chromatography [amino phase silica gel, eluation gradient: EtOAc / MeOH, 100/0 to 95/5 (v/v)] and afterwards by preparative HPLC to give the title compound as a solid.

HRMS [$C_{45}H_{53}N_7O_8$]: calc.: 820.4028 found: 820.4020

**121. 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3,3-dimethyl-1-oxobutan-2-yl]-5H-pyrrolo[3,2-d]pyrimidine-7-carboxamide**

A solution of 4-[5-(cyclopropylmethoxy)-1,3-benzodioxol-4-yl]-5H-pyrrolo[3,2-d]pyrimidine-7-carboxylic acid (65 mg; compound B71), TOTU (61 mg), HOAT (25 mg) and DIPEA (48 mg) in DMF (2.5 ml) was stirred for 45 min at RT and then (4aS,8aR)-2-{1-[(2R)-2-amino-3,3-dimethylbutanoyl]piperidin-4-yl}-4-(3,4-dimethoxyphenyl)-4a,5,6,7,8,8a-hexahydrophthalazin-1(2H)-one (89 mg; compound B169) was added. The reaction mixture was stirred for 2 h at RT and then for 1.5 h at 50°C. The solvent was removed under vacuo and the resulting residue was purified by flash column chromatography [silica gel, eluent: DCM/MeOH, 95/5 (v/v)] and afterwards by preparative HPLC to give the title compound as a solid.

HRMS [$C_{45}H_{53}N_7O_8$]: calc.: 820.4028 found: 820.4030

**122. 4-[5-(cyclopropylmethoxy)-1,3-benzodioxol-4-yl]-N-(2-{4-[9-(3,4-dimethoxyphenyl)-6-oxo-7,8-diazaspiro[4.5]dec-8-en-7-yl]piperidin-1-yl}-2-oxoethyl)-5H-pyrrolo[3,2-d]pyrimidine-7-carboxamide**

To a suspension of 4-[5-(cyclopropylmethoxy)-1,3-benzodioxol-4-yl]-5H-pyrrolo[3,2-d]pyrimidine-7-carboxylic acid (157 mg; compound B71) and DIPEA (0.29 ml) in DCM (3 ml) was added 7-[1-(amino-acetyl)piperidin-4-yl]-9-(3,4-dimethoxyphenyl)-7,8-diazaspiro[4.5]dec-8-en-6-one (191 mg; compound B172) and HBTU (186 mg) and the reaction mixture was stirred for 2 h at RT. The mixture was extracted with half-saturated aqueous sodium bicarbonate solution and DCM. The organic phase was dried over magnesium sulphate and concentrated under reduced pressure. The resulting residue was purified twice by flash column chromatography [first column: amino phase silica gel, eluent: EtOAc/MeOH, 99/1 (v/v); second column: silica gel, eluent: DCM/MeOH, 98/2 (v/v)] to give the title compound as a solid.

HRMS [$C_{41}H_{45}N_7O_8$]: calc.: 764.34024 found: 764.3404

**123. 4-[5-(cyclopropylmethoxy)-1,3-benzodioxol-4-yl]-N-[(2R)-1-{4-[9-(3,4-dimethoxyphenyl)-6-oxo-7,8-diazaspiro[4.5]dec-8-en-7-yl]piperidin-1-yl}-1-oxo-3-phenylpropan-2-yl]-5H-pyrrolo[3,2-d]pyrimidine-7-carboxamide**

To a suspension of 4-[5-(cyclopropylmethoxy)-1,3-benzodioxol-4-yl]-5H-pyrrolo[3,2-d]pyrimidine-7-carboxylic acid (124 mg; compound B71) and DIPEA (0.12 ml) in DCM (10 ml) was added 7-{1-[(2R)-2-amino-3-phenylpropanoyl]piperidin-4-yl}-9-(3,4-dimethoxyphenyl)-7,8-diazaspiro[4.5]dec-8-en-6-one (182 mg; compound B173) and HBTU (266 mg) and the reaction mixture was stirred for 2 h at RT. The mixture was extracted with saturated aqueous sodium bicarbonate solution (3x5 ml) and the organic phase was separated, dried over magnesium sulphate and concentrated under reduced pressure. The resulting residue was purified by flash column chromatography [amino phase silica gel, eluation gradient: Cyclohexane/EtOAc/MeOH, 100/0/0 to 0/100/0 to 0/95/5 (v/v/v)]. After lyophilisation from acetonitrile/water (20 ml, 1/1 (v/v)) the title compound was obtained as a solid.

HRMS [$C_{48}H_{51}N_7O_8$]: calc.: 854.38719 found: 854.3871

**124. 4-[5-(cyclopropylmethoxy)-1,3-benzodioxol-4-yl]-N-[(2R)-1-{4-[9-(3,4-dimethoxyphenyl)-6-oxo-7,8-diazaspiro[4.5]dec-8-en-7-yl]piperidin-1-yl}-1-oxo-3-phenylpropan-2-yl]-6-methyl-5H-pyrrolo[3,2-d]pyrimidine-7-carboxamide**

To a suspension of 4-[5-(cyclopropylmethoxy)-1,3-benzodioxol-4-yl]-6-methyl-5H-pyrrolo[3,2-d]-pyrimidine-7-carboxylic acid (129 mg; compound B72) and DIPEA (0.12 ml) in DCM (10 ml) was added 7-{1-[(2R)-2-amino-3-phenylpropanoyl]piperidin-4-yl}-9-(3,4-dimethoxyphenyl)-7,8-diazaspiro [4.5]dec-8-en-6-one (182 mg; compound B173) and HBTU (266 mg) and the reaction mixture was stirred for 2 h at RT. The mixture was extracted with saturated aqueous sodium bicarbonate solution (3x5 ml) and the organic phase was separated, dried over magnesium sulphate and concentrated under reduced pressure. The resulting residue was purified by flash column chromatography [amino phase silica gel, eluation gradient: Cyclohexane/EtOAc/MeOH, 100/0/0 to 0/100/0 to 0/95/5 (v/v/v)]. After lyophilisation from acetonitrile/water (20 ml, 1/1 (v/v)) the title compound was obtained as a solid.

HRMS [$C_{49}H_{53}N_7O_8$]: calc.: 868.40284 found: 868.4030

**125. 4-[5-(cyclopropylmethoxy)-1,3-benzodioxol-4-yl]-N-[(2S)-1-{4-[9-(3,4-dimethoxyphenyl)-6-oxo-7,8-diazaspiro[4.5]dec-8-en-7-yl]piperidin-1-yl}-1-oxo-3-(pyridin-3-yl)propan-2-yl]-5H-pyrrolo[3,2-d]pyrimidine-7-carboxamide**

To a suspension of 4-[5-(cyclopropylmethoxy)-1,3-benzodioxol-4-yl]-5H-pyrrolo[3,2-d]pyrimidine-7-carboxylic acid (106 mg; compound B71) and DIPEA (0.20 ml) in DCM (3 ml) was added HBTU (125 mg) and the reaction mixture was stirred for 5 min at RT. Then 7-{1-[(2S)-2-amino-3-(pyridin-3-yl)propanoyl]piperidin-4-yl}-9-(3,4-dimethoxyphenyl)-7,8-diazaspiro[4.5]dec-8-en-6-one (156 mg; compound B175) was added and the reaction mixture was stirred for 3 h at RT. The mixture was extracted with half-saturated aqueous sodium bicarbonate solution and DCM. The organic phase was dried over magnesium sulphate and concentrated under reduced pressure. The resulting residue was purified twice by flash column chromatography [first column: amino phase silica gel, eluent: EtOAc/MeOH, 98/2 (v/v); second column: silica gel, eluent: DCM/MeOH, 98/2 (v/v)]. After lyophilisation from acetonitrile / water the title compound was obtained as a solid.

HRMS [$C_{47}H_{50}N_8O_8$]: calc.: 855.38243 found: 855.3816

## Intermediates

**B1. (4aS,8aR)-2-(1-{(2R)-2-amino-3-[2-(trifluoromethyl)phenyl]propanoyl}piperidin-4-yl)-4-(3,4-dimethoxyphenyl)-4a,5,6,7,8,8a-hexahydrophthalazin-1(2H)-one hydrochloride**

tert-Butyl {(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[2-(trifluoromethyl)phenyl]propan-2-yl}carbamate (1.10 g; compound B61) was dissolved in a solution of hydrogen chloride in 1,4-dioxane (10 ml, 4.0 M) and the reaction mixture was stirred for 1 h at RT. Afterwards the mixture was concentrated to dryness under vacuo to yield the title compound as a solid.

MS: calc.: $C_{31}H_{37}F_3N_4O_4$ (586.64) found: [$MH^+$] = 587.3

**B2. (4aS,8aR)-2-{1-[(2S,3R)-2-amino-3-hydroxybutanoyl]piperidin-4-yl}-4-(3,4-dimethoxyphenyl)-4a,5,6,7,8,8a-hexahydrophthalazin-1(2H)-one hydrochloride**

tert-Butyl [(2R,3S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxobutan-2-yl]carbamate (4.3 g; compound B50) was dissolved in a solution of hydrogen chloride in 1,4-dioxane (50 ml, 4.0 M) at 0°C and the reaction mixture was stirred for about 45 min at 0°C and then for 1.5 h at RT. Afterwards all volatiles were removed under reduced pressure to give the title compound as a solid.

MS: calc.: $C_{25}H_{36}N_4O_5$ (472.59) found: [$MH^+$] = 473.2

**B3. 4-[(2R)-2-amino-3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl]benzonitrile**

A solution of tert-Butyl [(2R)-3-(4-cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (1.41 g; compound B47) and trifluoroacetic acid (14 ml) in DCM (14 ml) was stirred for 3 h at RT. Afterwards a saturated aqueous sodium bicarbonate solution was slowly added until the solution was alkanized. The mixture was extracted twice with, the combined organic layers were dried over magnesium sulphate and all solvents were removed under reduced pressure. The residue was dried under vacuo to give the title compound as a solid.

MS: calc.: $C_{31}H_{37}N_5O_4$ (543.66) found: [$MH^+$] = 544.2

**B4. (4aS,8aR)-2-{1-[(2R)-2-amino-3-(3-methylphenyl)propanoyl]piperidin-4-yl}-4-(3,4-dimethoxyphenyl)-4a,5,6,7,8,8a-hexahydrophthalazin-1(2H)-one** hydrochloride

To a solution of tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]carbamate (1.05 g; compound B30) in 1,4 dioxane (6 ml) was added a solution of hydrogen chloride in 1,4-dioxane (1.66 ml, 4.0 M) and the reaction mixture was stirred for 5 d at RT. Afterwards diethyl ether was added and the mixture was stirred for 0.5 h. The suspension was filtered off and washed with diethyl ether. The filter cake was dried under vacuo at 45°C to give the title compound as a solid.

MS: calc.: $C_{31}H_{40}N_4O_4$ (532.68) found: [$MH^+$] = 533.2

**B5. (4aS,8aR)-2-{1-[(2R)-2-amino-3-(4-methylphenyl)propanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]carbamate (1.47 g; compound B31) in 1,4 dioxane (10 ml) was added a solution of hydrogen chloride in 1,4-dioxane (2.32 ml, 4.0 M) and the reaction mixture was stirred for 5 d at RT. Afterwards diethyl ether was added and the mixture was stirred for 0.5 h. The suspension was filtered off and washed with diethyl ether. The filter cake was dried under vacuo at 45°C to give the title compound as a solid.
MS: calc.: $C_{31}H_{40}N_4O_4$ (532.68) found: $[MH^+]$ = 533.3

**B6. (4aS,8aR)-2-{1-[(2S)-2-amino-3-(3,4-dimethoxyphenyl)propanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2S)-3-(3,4-dimethoxyphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (1.7 g; compound B33) in 1,4 dioxane (10 ml) was added a solution of hydrogen chloride in 1,4-dioxane (2.50 ml, 4.0 M) and the reaction mixture was stirred for 3 d at RT. Afterwards diethyl ether (200 ml) was added and the mixture was stirred for 25 min. The suspension was filtered off and washed with diethyl ether. The filter cake was dried under vacuo at 55°C to give the title compound as a solid.
MS: calc.: $C_{32}H_{42}N_4O_6$ (578.70) found: $[MH^+]$ = 579.3

**B7. (4aS,8aR)-2-{1-[(2R)-2-amino-3-(3,4-difluorophenyl)propanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2R)-3-(3,4-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (1.43 g; compound B32) in 1,4 dioxane (10 ml) was added a solution of hydrogen chloride in 1,4-dioxane (3.30 ml, 4.0 M) and the reaction mixture was stirred for 2 d at RT and afterwards for 3 h at 80 °C. Afterwards diethyl ether was added and the mixture was stirred for 25 min. The suspension was filtered off and washed with diethyl ether. The filter cake was dried under vacuo at 50°C to give the title compound as a solid.
MS: calc.: $C_{30}H_{36}F_2N_4O_4$ (554.63) found: $[MH^+]$ = 555.2

**B8. (4aS,8aR)-2-{1-[(2R)-2-amino-3-(biphenyl-4-yl)propanoyl]piperidin-4-yl}-4-(3,4-dimethoxyphenyl)-4a,5,6,7,8,8a-hexahydrophthalazin-1(2H)-one**

A solution of tert-Butyl [(2R)-3-(biphenyl-4-yl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (1.44 g; compound B28) and trifluoroacetic acid (14 ml) in DCM (14 ml) was stirred for 2.5 h at RT. Afterwards a saturated aqueous sodium bicarbonate solution was slowly added until the solution was alkanized. The mixture was extracted twice with DCM, the combined organic layers were dried over magnesium sulphate and all solvents were removed under reduced pressure. The residue was dried under vacuo to give the title compound as a solid.
MS: calc.: $C_{36}H_{42}N_4O_4$ (594.75) found: $[MH^+]$ = 595.2

**B9. 4-[(2R)-2-amino-3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl]benzamide**

A solution of tert-Butyl [(2R)-3-(4-carbamoylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (1.72 g; compound B46) and trifluoroacetic acid (17 ml) in DCM (17 ml) was stirred for 3 h at RT. Afterwards a saturated aqueous sodium bicarbonate solution was slowly added until the solution was alkanized. The mixture was extracted twice with DCM, the combined organic layers were dried over magnesium sulphate and all solvents were removed under reduced pressure. The residue was dried under vacuo to give the title compound as a solid.
MS: calc.: $C_{31}H_{39}N_5O_5$ (561.67) found: $[MH^+]$ = 562.2

**B10. (4aS,8aR)-2-{1-[(2R)-2-amino-3-(4-ethoxyphenyl)propanoyl]piperidin-4-yl}-4-(3,4-dimethoxyphenyl)-4a,5,6,7,8,8a-hexahydrophthalazin-1(2H)-one**

A solution of tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]carbamate (1.68 g; compound B44) and trifluoroacetic acid (16.8 ml) in DCM (16.8 ml) was stirred for 2 h at RT. Afterwards a saturated aqueous sodium bicarbonate solution was slowly added until the solution was alkanized. The mixture was extracted twice with DCM, the combined organic layers were dried over magnesium sulphate and all solvents were removed under reduced pressure. The residue was dried under vacuo to give the title compound as a solid.

MS: calc.: $C_{32}H_{42}N_4O_5$ (562.70) found: [$MH^+$] = 563.2

**B11. (4aS,8aR)-2-{1-[(2R)-2-amino-3-(4-tert-butylphenyl)propanoyl]piperidin-4-yl}-4-(3,4-dimethoxyphenyl)-4a,5,6,7,8,8a-hexahydrophthalazin-1(2H)-one**

A solution of tert-Butyl [(2R)-3-(4-tert-butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (945 mg; compound B45) and trifluoroacetic acid (9.4 ml) in DCM (9.4 ml) was stirred for 3 h at RT. Afterwards a saturated aqueous sodium bicarbonate solution was slowly added until the solution was alkanized. The mixture was extracted twice with DCM, the combined organic layers were dried over magnesium sulphate and all solvents were removed under reduced pressure. The residue was dried under vacuo to give the title compound as a solid.

MS: calc.: $C_{34}H_{46}N_4O_4$ (574.75) found: [$MH^+$] = 575.2

**B12. 4-[2-(cyclopropylmethoxy)-5-fluoro-4-methoxyphenyl]-6-methyl-5H-pyrrolo[3,2-d]pyrimidine-7-carboxylic acid**

Compound B12 can be prepared in analogy to methods described in WO2011/023693.

MS: calc.: $C_{19}H_{18}FN_3O_4$ (371.37) found: [$MH^+$] = 372.1

**B13. (4aS,8aR)-2-{1-[(2S)-2-amino-3-(3-methylphenyl)propanoyl]piperidin-4-yl}-4-(3,4-dimethoxyphenyl)-4a,5,6,7,8,8a-hexahydrophthalazin-1(2H)-one**

A solution of tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]carbamate (1.63 g; compound B43) and trifluoroacetic acid (16.3 ml) in DCM (16.3 ml) was stirred for 2 h at RT. Afterwards a saturated aqueous sodium bicarbonate solution was slowly added until the solution was alkanized. The mixture was extracted twice with DCM, the combined organic layers were dried over magnesium sulphate and all solvents were removed under reduced pressure. The residue was dried under vacuo to give the title compound as a solid.

MS: calc.: $C_{31}H_{40}N_4O_4$ (532.67) found: [$MH^+$] = 533.2

**B14. (4aS,8aR)-2-{1-[(2R)-2-amino-3-(3,5-difluorophenyl)propanoyl]piperidin-4-yl}-4-(3,4-dimethoxyphenyl)-4a,5,6,7,8,8a-hexahydrophthalazin-1(2H)-one**

A solution of tert-Butyl [(2R)-3-(3,5-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (438 mg; compound B42) and trifluoroacetic acid (4.4 ml) in DCM (4.4 ml) was stirred for 2 h at RT. Afterwards a saturated aqueous sodium bicarbonate solution was slowly added until the solution was alkanized. The mixture was extracted twice with DCM, the combined organic layers were dried over magnesium sulphate and all solvents were removed under reduced pressure. The residue was dried under vacuo to give the title compound as a solid.

MS: calc.: $C_{30}H_{36}F_2N_4O_4$ (554.63) found: [$MH^+$] = 555.2

**B15. (4aS,8aR)-2-{1-[(2S)-2-amino-3-(4-fluorophenyl)propanoyl]piperidin-4-yl}-4-(3,4-dimethoxyphenyl)-4a,5,6,7,8,8a-hexahydrophthalazin-1(2H)-one**

A solution of tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]carbamate (643 mg; compound B49) and trifluoroacetic acid (6.4 ml) in DCM (6.4 ml) was stirred for 2 h at RT. Afterwards a saturated aqueous sodium bicarbonate solution was slowly added until the solution was alkanized. The mixturewas extracted twice with DCM, the combined organic layers were dried over magnesium sulphate and all solvents were removed under reduced pressure. The residue was dried under vacuo to give the title compound as a solid.

MS: calc.: $C_{30}H_{37}FN_4O_4$ (536.64) found: [$MH^+$] = 537.2

**B16. (4aS,8aR)-2-{1-[(2R)-2-amino-3-(2,4-dichlorophenyl)propanoyl]piperidin-4-yl}-4-(3,4-dimethoxyphenyl)-4a,5,6,7,8,8a-hexahydrophthalazin-1(2H)-one**

A solution of tert-Butyl [(2R)-3-(2,4-dichlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (1.46 g; compound B48) and trifluoroacetic acid (14 ml) in DCM (14 ml) was stirred for 2 h at RT. Afterwards a saturated aqueous sodium bicarbonate solution was slowly added until the solution was alkanized. The mixture was extracted twice with DCM, the combined organic layers were dried over magnesium sulphate and all solvents were removed under reduced pressure. The residue was dried under vacuo to give the title compound as a solid.
MS: calc.: $C_{30}H_{36}Cl_2N_4O_4$ (587.54) found: [$MH^+$] = 587.2

**B17. 4-[2-(cyclopropylmethoxy)-5-fluoro-4-methoxyphenyl]-5H-pyrrolo[3,2-d]pyrimidine-7-carboxylic acid**

Synthesis of compound B17 is described in PCT application WO2009106531.
MS: calc.: $C_{18}H_{16}FN_3O_4$ (357.34) found: [$MH^+$] = 358.0

**B18. 4-[2-(cyclopropylmethoxy)-4-fluoro-5-methoxyphenyl]-5H-pyrrolo[3,2-d]pyrimidine-7-carboxylic acid**

Synthesis of compound B18 is described in PCT application WO2009106531.
MS: calc.: $C_{18}H_{16}FN_3O_4$ (357.34) found: [$MH^+$] = 358.1

**B19. (4aS,8aR)-2-{1-[(2R)-2-amino-3-(3-chlorophenyl)propanoyl]piperidin-4-yl}-4-(3,4-dimethoxyphenyl)-4a,5,6,7,8,8a-hexahydrophthalazin-1(2H)-one**

The title compound was prepared analogously as described for example B20 using tert-Butyl [(2R)-3-(3-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (2.15 g; compound B37) and an aqueous solution of hydrogen chloride (8.31 ml, 2.0 M; 2 x1 ml, 10 M) in THF (40 ml).
MS: calc.: $C_{30}H_{37}ClN_4O_4$ (553.1) found: [$MH^+$] = 554.2

**B20. (4aS,8aR)-2-{1-[(2R)-2-amino-3-(4-chlorophenyl)propanoyl]piperidin-4-yl}-4-(3,4-dimethoxyphenyl)-4a,5,6,7,8,8a-hexahydrophthalazin-1(2H)-one**

To a solution of tert-Butyl [(2R)-3-(4-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (2.17 g; compound B36) in THF (40 ml) was added an aqueous solution of hydrogen chloride (8.31 ml, 2.0 M) and the reaction mixture was stirred for 90 min at RT and afterwards for 2 h at 65 °C and again for 12 h at RT. Since reaction control by LC-MS indicates presence of starting material tert-Butyl [(2R)-3-(4-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (ca 30 %) the reaction mixture was stirred another 9 h at 65 °C and after further addition of a concentrated solution of hydrogen chloride (2x1 ml, 10.0 M) and stirring for 2x45 min at room temperature the reaction was completed. The organic solvent was removed under reduced pressure, DCM (100 ml) and water (50 ml) were added and the mixturewas alkanized to pH 14 by addition of an aqueous solution of sodium hydroxide (5M). The mixture was extracted with DCM (5x100 ml), the organic layers were combined and dried over sodium sulphate. After evaporation of all volatiles under vacuo the title compound was obtained as a solid.
MS: calc.: $C_{30}H_{37}ClN_4O_4$ (553.1) found: [$MH^+$] = 554.3

**B21. (4aS,8aR)-2-{1-[(2S)-2-amino-3-(pyridin-2-yl)propanoyl]piperidin-4-yl}-4-(3,4-dimethoxyphenyl)-4a,5,6,7,8,8a-hexahydrophthalazin-1(2H)-one** hydrochloride

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]carbamate (1.30 g; compound B63) was dissolved in a solution of hydrogen chloride in 1,4-dioxane (10 ml, 4.0 M) and the reaction mixture was stirred for 0.5 h at RT. Afterwards the mixture was concentrated to dryness under vacuo to yield the title compound as a solid.
MS: calc.: $C_{29}H_{37}N_5O_4$ (519.64) found: [$MH^+$] = 520.2

**B22. (4aS,8aR)-2-{1-[(2S)-2-amino-3-(pyridin-3-yl)propanoyl]piperidin-4-yl}-4-(3,4-dimethoxyphenyl)-4a,5,6,7,8,8a-hexahydrophthalazin-1(2H)-one**

To a solution of tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]carbamate (1.53 g; compound B66) in THF (15 ml) was added an aqueous solution of hydrogen chloride (10 ml, 2.0 M) and the reaction mixturewas stirred for 1 h at RT, then for about 7 h at 55°C and afterwards for 2 d at RT. Afterwards an aqueous solution of sodium hydroxide (5M) was slowly added until the solution was alkanized (pH 14). The mixture was extracted with DCM (200 ml) and the organic layer was dried over sodium sulphate. All solvents were removed under reduced pressure and the residue was dried under vacuo to give the title compound as a solid.

MS: calc.: $C_{29}H_{37}N_5O_4$ (519.65) found: [$MH^+$] = 520.3

**B23. (4aS,8aR)-2-{1-[(2R)-2-amino-3-(2-chlorophenyl)propanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2R)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (1.21 g; compound B38) in THF (15 ml) was added an aqueous solution of hydrogen chloride (4.63 ml, 2.0 M) and the reaction mixture was stirred for 90 min at RT and afterwards for 3 d at 50-60 °C. Afterwards the mixturewas concentrated to dryness under vacuo and co-evaporated with DCM to yield the title compound as a solid.

MS: calc.: $C_{30}H_{37}ClN_4O_4$ (553.1) found: [$MH^+$] = 554.2

**B24. (4aS,8aR)-2-(1-{(2R)-2-amino-3-[4-(trifluoromethyl)phenyl]propanoyl}piperidin-4-yl)-4-(3,4-dimethoxyphenyl)-4a,5,6,7,8,8a-hexahydrophthalazin-1(2H)-one**

A solution of tert-Butyl {(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(trifluoromethyl)phenyl]propan-2-yl}carbamate (1.36 g; compound B41) and trifluoroacetic acid (13.6 ml) in DCM (13.6 ml) was stirred for 2 h at RT. Afterwards a saturated aqueous sodium bicarbonate solution was slowly added until the solution was alkanized. The mixture was extracted twice with DCM, the combined organic layers were dried over magnesium sulphate and all volatiles were removed under reduced pressure. The residue was dried under vacuo to give the title compound as a solid.

MS: calc.: $C_{31}H_{37}F_3N_4O_4$ (586.64) found: [$MH^+$] = 587.2

**B25. (4aS,8aR)-2-{1-[(2R)-2-amino-3-(4-methoxyphenyl)propanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxopropan-2-yl]carbamate (1.34 g; compound B35) in 1,4 dioxane (10 ml) was added a solution of hydrogen chloride in 1,4-dioxane (3.10 ml, 4.0 M) and the reaction mixture was stirred for 12 h at RT. Additional solution of hydrogen chloride in 1,4-dioxane (1.0 ml, 4.0 M) was added and the reaction mixture was stirred for 12 h at RT in order to complete the reaction. Afterwards diethyl ether (90 ml) was added and the mixture was stirred for 10 min. The suspension was filtered off and washed with diethyl ether. The filter cake was dried under vacuo at 50°C for 2 h to give the title compound as a solid.

MS: calc.: $C_{31}H_{40}N_4O_5$ (548.67) found: [$MH^+$] = 549.2

**B26. (4aS,8aR)-2-{1-[(2R)-2-amino-3-(4-fluorophenyl)propanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]carbamate (4.31 g; compound B34) in 1,4 dioxane (30 ml) was added a solution of hydrogen chloride in 1,4-dioxane (10.2 ml, 4.0 M) and the reaction mixture was stirred for 2 d at RT. Afterwards diethyl ether (250 ml) was added and the mixture was stirred for 0.5 h. The suspension was filtered off and washed with diethyl ether. The filter cake was dried under vacuo at 50°C for 4 h to give the title compound as a solid.

MS: calc.: $C_{30}H_{37}FN_4O_4$ (536.64) found: [$MH^+$] = 537.2

**B27. tert-butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxobutan-2-yl]carbamate**

To a suspension of (4aS,8aR)-4-(3,4-dimethoxyphenyl)-2-(piperidin-4-yl)-4a,5,6,7,8,8a-hexahydrophthalazin-1(2H)-one hydrochloride (653 mg; compound B76), (2S)-2-[(tert-butoxycarbonyl)amino]butanoic acid (325 mg) and HBTU (667 mg) in DCM (20 ml) was added DIPEA (1.1 ml) and the reaction mixture was stirred for 30 min at RT. Afterwards the mixture was extracted with aqueous sodium bicarbonate solution (10 ml), the organic phase was separated, dried over sodium sulphate and concentrated under reduced pressure. The resulting residue was purified by flash column chromatography [silica gel, eluation gradient: Cyclohexane/EtOAc, 30/70 to 0/100 (v/v)] to give the title compound as a solid.

MS: calc.: $C_{30}H_{44}N_4O_6$ (556.71) found: [$MH^+$] = 557.1

**B28. tert-Butyl [(2R)-3-(biphenyl-4-yl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of (2R)-3-(biphenyl-4-yl)-2-[(tert-butoxycarbonyl)amino]propanoic acid (113 mg) and DIPEA (1.92 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.2 g; compound B76)and COMU (1.38 g) and the reaction mixture was stirred for 2 h at RT. Afterwards a half-saturated aqueous sodium bicarbonate solution was added and the mixture was extracted with DCM. The combined organic phases were dried over magnesium sulphate and the organic layer was concentrated under reduced pressure. The resulting residue was purified twice by flash column chromatography [silica gel, eluent: Toluene / EtOAc, 9/1 (v/v)] to give the title compound as a solid.

MS: calc.: $C_{41}H_{50}N_4O_6$ (694.86) found: [$MH^+$] = 695.0

**B29. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a suspension of (4aS,8aR)-4-(3,4-dimethoxyphenyl)-2-(piperidin-4-yl)-4a,5,6,7,8,8a-hexahydrophthalazin-1(2H)-one hydrochloride (326 mg; compound B76), N-(tert-butoxycarbonyl)-D-alanine (151 mg) and COMU (377 mg) in DCM (10 ml) was added DIPEA (0.56 ml) and the reaction mixture was stirred for 45 min at RT. Afterwards the mixture was extracted with aqueous sodium bicarbonate solution (5 ml), the organic phase was separated, dried over sodium sulphate and concentrated under reduced pressure. The resulting residue was purified by flash column chromatography [silica gel, eluent: EtOAc] to give the title compound as a solid.

MS: calc.: $C_{29}H_{42}N_4O_6$ (542.68) found: [$MH^+$] = 543.1

**B30. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]carbamate**

To a mixture of N-(tert-butoxycarbonyl)-3-methyl-D-phenylalanine (994 mg) and DIPEA (2.30 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.45 g; compound B76) and COMU (1.68 g) and the reaction mixture was stirred for 3 h at RT. Additional COMU (360 mg) was added and the reaction mixture was stirred for 12 h in order to complete the reaction. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene / EtOAc, 9/1 (v/v)] to give the title compound as a solid.

MS: calc.: $C_{36}H_{48}N_4O_6$ (632.79) found: [$MH^+$] = 633.1; [$MH^+$ - Boc] = 533.3

**B31. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]carbamate**

To a mixture of N-(tert-butoxycarbonyl)-4-methyl-D-phenylalanine (960 mg) and DIPEA (2.25 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.40 g compound B76) and COMU (1.62 g) and the reaction mixture was stirred for 2 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue iwas purified by flash column chromatography [silica gel, eluent: Toluene / EtOAc, 9/1 (v/v)] to give the title compound as a solid.

MS: calc.: $C_{36}H_{48}N_4O_6$ (632.79) found: [$MH^+$] = 633.1

**B32. tert-Butyl [(2R)-3-(3,4-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-3,4-difluoro-D-phenylalanine (994 mg) and DIPEA (2.16 ml) in DCM (30 ml) was added (4aS,8aR)-4-(3,4-dimethoxyphenyl)-2-(piperidin-4-yl)-4a,5,6,7,8,8a-hexahydro-phthalazin-1(2H)-one hydrochloride (1.35 g; compound B76) and COMU (1.55 g) and the reaction mixture was stirred for 12 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene/EtOAc, 9/1 (v/v)] to give the title compound as a solid.

MS: calc.: $C_{35}H_{44}F_2N_4O_6$ (654.74) found: [$MH^+$] = 655.0; [$MNa^+$] = 677.1; [$MH^+$- Boc] = 555.2

**B33. tert-Butyl [(2S)-3-(3,4-dimethoxyphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-3-methoxy-O-methyl-L-tyrosine (960 mg) and DIPEA (1.93 ml) in DCM (23 ml)was added (4aS,8aR)-4-(3,4-dimethoxyphenyl)-2-(piperidin-4-yl)-4a,5,6,7,8,8a-hexa-hydrophthalazin-1(2H)-one hydrochloride (1.20 g; compound B76)and COMU (1.39 g) and the reaction mixture was stirred for 2 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene/EtOAc, 8/2 (v/v)] to give the title compound as a solid.

MS: calc.: $C_{37}H_{50}N_4O_6$ (678.81) found: [$MH^+$] = 679.0

**B34. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]carbamate**

To a mixture of N-(tert-butoxycarbonyl)-4-fluoro-D-phenylalanine (3.0 g) and DIPEA (6.93 ml) in DCM (90 ml) was added (4aS,8aR)-4-(3,4-dimethoxyphenyl)-2-(piperidin-4-yl)-4a,5,6,7,8,8a-hexahydro-phthalazin-1(2H)-one hydrochloride (4.32 g; compound B76) and COMU (4.99 g) and the reaction mixturewas stirred for 5 h at RT. Afterwards a half-saturated aqueous sodium bicarbonate solution was added and the mixture was extracted twice with DCM. The combined organic phases were dried over sodium sulphate and the organic layer was concentrated under reduced pressure. The resulting residue was purified twice by flash column chromatography [1) amino phase silica gel, eluent: Petrol ether / EtOAc / MeOH, 60/37/3 (v/v/v); 2) silica gel, eluent: Toluene/EtOAc, 8/2 (v/v)] to give the title compound as a solid.

MS: calc.: $C_{35}H_{45}FN_4O_6$ (636.75) found: [$MH^+$] = 637.0; [$MNa^+$] = 659.2; [$MH^+$ - Boc] = 537.2

**B35. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxopropan-2-yl]carbamate**

To a mixture of N-(tert-butoxycarbonyl)-O-methyl-D-tyrosine (990 mg) and DIPEA (2.20 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.37 g; compound B76) and COMU (1.58 g) and the reaction mixture was stirred for 12 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene / EtOAc, 8/2 (v/v)]. The isolated product was dissolved in acetone and treated with charcoal, filtered through a plug of Celite and washed with acetone. The filtrate was concentrated under reduced pressure to give the title compound as a solid.

MS: calc.: $C_{36}H_{48}N_4O_7$ (648.79) found: [$MH^+$] = 649.0; [$MH^+$ - Boc] = 549.3

**B36. tert-Butyl [(2R)-3-(4-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-4-chloro-D-phenylalanine (1.0 g) and DIPEA (2.27 ml) in DCM (25 ml) was added (4aS,8aR)-4-(3,4-dimethoxyphenyl)-2-(piperidin-4-yl)-4a,5,6,7,8,8a-hexahydro-phthalazin-1(2H)-one hydrochloride (1.36 g; compound B76) and COMU (1.59 g) and the reaction mixture was stirred for 1 h at RT. Afterwards a

saturated aqueous sodium bicarbonate solution (20 ml) was added and the mixture was extracted with DCM (60 ml). The organic layer was separated, dried over magnesium sulphate and the organic solvent was removed under reduced pressure. The resulting residue was purified twice by flash column chromatography [amino phase silica gel, eluent: Cyclohexane/EtOAc, 1/0 to 0/1 to EtOAc/MeOH, 9/1 (v/v) to give the title compound as a solid.

MS: calc.: $C_{35}H_{45}ClN_4O_6$ (653.21) found: [$MH^+$] = 654.0; [$MNa^+$] = 676.1; [$MH^+$ - Boc] = 553.2

**B37. tert-Butyl [(2R)-3-(3-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-3-chloro-D-phenylalanine (1.0 g) and DIPEA (2.27 ml) in DCM (25 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.36 g; compound B76) and COMU (1.59 g) and the reaction mixture was stirred for 1 h at RT. Afterwards a saturated aqueous sodium bicarbonate solution (20 ml) was added and the mixture was extracted with DCM (60 ml). The organic layer was separated, dried over magnesium sulphate and the organic solvent was removed under reduced pressure. The resulting residue was purified twice by flash column chromatography [amino phase silica gel, eluent: Cyclohexane/EtOAc, 1/0 to 0/1 to EtOAc/MeOH, 9/1 (v/v) to give the title compound as a solid.

MS: calc.: $C_{35}H_{45}ClN_4O_6$ (653.21) found: [$MH^+$] = 654.0; [$MNa^+$] = 676.2; [$MH^+$ - Boc] = 554.2

**B38. tert-Butyl [(2R)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate** -

To a mixture of N-(tert-butoxycarbonyl)-2-chloro-D-phenylalanine (567mg) and DIPEA (1.23 ml) in DCM (25 ml) was added (4aS,8aR)-4-(3,4-dimethoxyphenyl)-2-(piperidin-4-yl)-4a,5,6,7,8,8a-hexa-hydrophthalazin-1(2H)-one hydrochloride (734 mg; compound B76) and HBTU (820 mg) and the reaction mixture was stirred for 0.5 h at RT. Afterwards a saturated aqueous sodium bicarbonate solution (10 ml) was added and the mixture was extracted with DCM (30 ml + 10 ml). The combined organic layers were separated using a phase separator. The organic solvent was removed under reduced pressure and the resulting residue was purified twice by flash column chromatography [amino phase silica gel, eluent: Cyclohexane/EtOAc, 100/0 to 0/100 to EtOAc/MeOH, 93/7 (v/v) to give the title compound as a solid.

MS: calc.: $C_{35}H_{45}ClN_4O_6$ (653.21) found: [$MH^+$] = 654.1; [$MNa^+$] = 676.2; [$MH^+$ - Boc] = 554.3

**B39. tert-Butyl [(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]carbamate**

To a mixture of (betaR)-N-(tert-butoxycarbonyl)-beta-methyl-D-phenylalanine (1.0 g) and DIPEA (2.34 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.46 g; compound B76) and COMU (1.68 g) and the reaction mixturewas stirred for 2 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene/EtOAc, 9/1 (v/v)] to give the title compound as a solid.

MS: calc.: $C_{36}H_{48}N_4O_6$ (632.79) found: [$MH^+$] = 633.0; [$MNa^+$] = 655.2; [$MH^+$ - Boc] = 533.2

**B40. (4aS,8aR)-2-{1-[(2R,3R)-2-amino-3-phenylbutanoyl]piperidin-4-yl}-4-(3,4-dimethoxyphenyl)-4a,5,6,7,8,8a-hexahydrophthalazin-1(2H)-one**

A solution of tert-Butyl [(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]carbamate (1.80 g; compound B39) and trifluoroacetic acid (17 ml) in DCM (17 ml) was stirred for 1 h at RT. Afterwards a saturated aqueous sodium bicarbonate solution was slowly added until the solution was alkanized. The mixture was extracted with DCM (2 x), the combined organic layers were dried over magnesium sulphate and all solvents were removed under reduced pressure. The residue was dried under vacuo to give the title compound as a solid.

MS: calc.: $C_{31}H_{40}N_4O_4$ (532.67) found: [$MH^+$] = 533.2

**B41. tert-Butyl {(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(trifluoromethyl)phenyl]propan-2-yl}carbamate**

To a mixture of N-(tert-butoxycarbonyl)-4-(trifluoromethyl)-D-phenylalanine (1.0 g) and DIPEA (1.96 ml) in DCM

(30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.22 g; compound B76)and COMU (1.41 g) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Petrolether / EtOAc, 55/45 to 1/1 (v/v)] to give the title compound as a solid.

MS: calc.: $C_{36}H_{45}F_3N_4O_6$ (686.76) found: [$MH^+$] = 687.0; [$MNa^+$] = 709.2; [$MH^+$ - Boc] = 587.2

**B42. tert-Butyl [(2R)-3-(3,5-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-3,5-difluoro-D-phenylalanine (500 mg) and DIPEA (1.08 ml) in DCM (15 ml) was added (4aS,8aR)-4-(3,4-dimethoxyphenyl)-2-(piperidin-4-yl)-4a,5,6,7,8,8a-hexahydrophthalazin-1(2H)-one hydrochloride (677 mg; compound B76) and COMU (782 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene/EtOAc, 9/1 (v/v)] to give the title compound as a solid.

MS: calc.: $C_{35}H_{44}F_2N_4O_6$ (654.74) found: [$MH^+$] = 655.0; [$MNa^+$] = 677.1; [$MH^+$ - Boc] = 555.2

**B43. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]carbamate**

To a mixture of N-(tert-butoxycarbonyl)-3-methyl-L-phenylalanine (990 mg) and DIPEA (2.32 ml) in DCM (30 ml) was added (4aS,8aR)-4-(3,4-dimethoxyphenyl)-2-(piperidin-4-yl)-4a,5,6,7,8,8a-hexa-hydrophthalazin-1(2H)-one hydrochloride (1.45 g; compound B76) and COMU (1.67 g) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene / EtOAc, 9/1 (v/v)] to give the title compound as a solid.

MS: calc.: $C_{36}H_{48}N_4O_6$ (632.79) found: [$MH^+$] = 633.0

**B44. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]carbamate**

To a mixture of N-(tert-butoxycarbonyl)-0-ethyl-D-tyrosine (1.03 g) and DIPEA (2.18 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.36 g; compound B76) and COMU (1.57 g) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene / EtOAc, 9/1 (v/v)] to give the title compound as a solid.

MS: calc.: $C_{37}H_{50}N_4O_7$ (662.82) found: [$MH^+$] = 663.0

**B45. tert-Butyl [(2R)-3-(4-tert-butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-4-tert-butyl-D-phenylalanine dicyclohexylammonium salt (980 mg) and DIPEA (1.28 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (795 mg; compound B76)and COMU (918 mg) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene / EtOAc, 9/1 (v/v)] to give the title compound as a solid.

MS: calc.: $C_{39}H_{54}N_4O_6$ (674.87) found: [$MH^+$] = 675.1

**B46. tert-Butyl [(2R)-3-(4-carbamoylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-4-carbamoyl-D-phenylalanine (1.02 g) and DIPEA (2.17 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.35 g; compound B76) and COMU (1.55 g) and the reaction mixture was stirred for 3.5 h at RT. Afterwards a half-saturated aqueous sodium bicarbonate solution was added and the mixture was extracted twice with DCM The combined organic phases were dried over magnesium sulphate and the organic layer was concentrated under reduced pressure. The resulting residue was purified by flash column chromatography [amino phase silica gel, eluent: EtOAc / MeOH, 98/2 (v/v)] to give the title compound as a solid.

MS: calc.: $C_{36}H_{47}N_5O_7$ (661.79) found: [$MH^+$] = 662.0

**B47. tert-Butyl [(2R)-3-(4-cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-4-cyano-D-phenylalanine (1.0 g) and DIPEA (2.25 ml) in DCM (30 ml) was added (4aS,8aR)-4-(3,4-dimethoxyphenyl)-2-(piperidin-4-yl)-4a,5,6,7,8,8a-hexahydro-phthalazin-1(2H)-one hydrochloride (1.40 g; compound B76) and COMU (1.62 g) and the reaction mixture was stirred for 3 h at RT. Afterwards a half-saturated aqueous sodium bicarbonate solution was added and the mixture was extracted twice with DCM (2x). The combined organic phases were dried over magnesium sulphate and the organic layer was concentrated under reduced pressure. The resulting residue was purified by flash column chromatography [silica gel, eluent: Toluene / EtOAc, 85/15 (v/v)] to give the title compound as a solid.

MS: calc.: $C_{36}H_{45}N_5O_6$ (643.77) found: [$MH^+$] = 644.0; [$MNa^+$] = 666.1; [$MH^+$ - Boc] = 544.2

**B48. tert-Butyl [(2R)-3-(2,4-dichlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-2,4-dichloro-D-phenylalanine (1.0 g) and DIPEA (1.96 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.22 g; compound B76) and COMU (1.41 g) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Petro-lether/EtOAc, 1/1 (v/v)] to give the title compound as a solid.

MS: calc.: $C_{35}H_{44}Cl_2N_4O_6$ (687.65) found: [$MH^+$] = 688.0

**B49. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]carbamate**

To a mixture of N-(tert-butoxycarbonyl)-4-fluoro-L-phenylalanine (500 mg) and DIPEA (1.15 ml) in DCM (15 ml) was added (4aS,8aR)-4-(3,4-dimethoxyphenyl)-2-(piperidin-4-yl)-4a,5,6,7,8,8a-hexa-hydrophthalazin-1(2H)-one hydrochloride (720 mg; compound B76) and COMU (830 mg) and the reaction mixture was stirred for 4 h at RT. Afterwards a half-saturated aqueous sodium bicarbonate solution was added and the mixturewas extracted twice with DCM. The combined organic phases were dried over magnesium sulphate and the organic layer was concentrated under reduced pressure. The resulting residue was purified by flash column chromatography [silica gel, eluent: Petrolether/EtOAc, 1/1 (v/v)] to give the title compound as a solid.

MS: calc.: $C_{35}H_{45}FN_4O_6$ (636.75) found: [$MH^+$] = 637.0; [$MNa^+$] = 659.1; [$MH^+$ - Boc] = 537.2

**B50. tert-Butyl [(2R,3S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxobutan-2-yl]carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (6.12 g; compound B76), N-(tert-butoxycarbonyl)-D-threonine (3.29 g) and HBTU (8.55 g) in DCM (80 ml) was added DIPEA (10.5 ml) and the reaction mixture was stirred for 45 min at RT. Afterwards the mixture was extracted with aqueous sodium bicarbonate solution (40 ml), the organic phase was separated, dried over sodium sulphate and concentrated under reduced pressure. The resulting residue was purified by flash column chromatography [silica gel, eluation gradient: EtOAc/MeOH, 100/0 to 95/5 (v/v)] to give the title compound as a solid.

MS: calc.: $C_{30}H_{44}N_4O_7$ (572.71) found: [$MH^+$] = 573.1

**B51. (4aS,8aR)-2-{1-[(2S)-2-aminobutanoyl]piperidin-4-yl}-4-(3,4-dimethoxyphenyl)-4a,5,6,7,8,8a-hexahydrophthalazin-1(2H)-one hydrochloride**

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxobutan-2-yl]carbamate (650 mg; compound B27) was dissolved in a solution of hydrogen chloride in 1,4-dioxane (6 ml, 4.0 M) and the reaction mixture was stirred for about 45 min at RT. Afterwards all volatiles were removed under reduced pressure to give the title compound as a solid.

MS: calc.: $C_{25}H_{36}N_4O$ (456.59) found: $[MH^+]$ = 457.2

**B52. (4aS,8aR)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one**

A solution of hydrogen chloride in 1,4-dioxane (106.6 ml, 4.0 M) was added to tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate (17.59 g, compound B60) and the mixture was stirred for 90 min at RT. DCM (150 ml) was added, the suspension was filtered off and the residue was washed with DCM (100 ml). The solid was treated with water (100 ml) and DCM (250 ml) and the stirred mixture was adjusted to pH 11-13 by addition of an aqueous solution of sodium hydroxide (6M). The organic phase was separated and the aqueous phase was extracted with DCM (3x100 ml). The organic phases were combined, dried over magnesium sulfate and concentrated under reduced pressure. The resulting residue was purified by flash column chromatography [amino phase silica gel, eluent: EtOAc/Cyclohexane/MeOH, 70/30/0 to 100/0/0 to 85/0/15 (v/v/v)] to give the title compound as a solid.

MS: calc.: $C_{30}H_{38}N_4O_4$ (518.65) found: $[MH^+]$ = 519.2

**B53. {4-[5-(cyclopropylmethoxy)-1,3-benzodioxol-4-yl]-5H-pyrrolo[3,2-d]pyrimidin-7-yl}(1H-imidazol-1-yl)methanone**

A mixture of 4-[5-(cyclopropylmethoxy)-1,3-benzodioxol-4-yl]-5H-pyrrolo[3,2-d]pyrimidine-7-carboxylic acid (2.12 g; compound B71) and CDI (2.43 g) in DCM (30 ml) was stirred under reflux for 1.5 h. The suspension was filtered off and the filter cake was washed with DCM (3x5 ml). The solid was dried under vacuo at 60 °C to give the title compound.

MS: calc.: $C_{21}H_{17}N_5O_4$ (403.39) found: $[MH^+]$ = 403.9

**B54. (4aS,8aR)-2-{1-[(2R)-2-amino-3-(4-hydroxyphenyl)propanoyl]piperidin-4-yl}-4-(3,4-dimethoxyphenyl)-4a,5,6,7,8,8a-hexahydrophthalazin-1(2H)-one hydrochloride**

A solution of hydrogen chloride in 1,4-dioxane (19.65 ml, 4.0 M) was added to tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]carbamate (2.0 g; compound B56) and the mixture was stirred for 90 min at RT. All volatiles were removed under vacuo and the residue was co-evaporated with DCM (3x) to give the title compound as a solid.

MS: calc.: $C_{30}H_{38}N_4O_5$ (534.65) found: $[MH^+]$ = 535.2

**B55. (4aS,8aR)-2-{1-[(2S)-2-amino-3-(4-hydroxyphenyl)propanoyl]piperidin-4-yl}-4-(3,4-dimethoxyphenyl)-4a,5,6,7,8,8a-hexahydrophthalazin-1(2H)-one**

To a solution of tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]carbamate (2.84 g, compound B84) in THF (40 ml) was added an aqueous solution of hydrogen chloride (11.19 ml, 2M) and the mixture was stirred at 65 °C for 7.5 h. Afterwards water (50 ml) and an aqueous solution of sodium hydroxide (5M) was slowly added until the solution was alkanized (pH 14). The mixture was extracted with DCM (3 x 150ml) and the combined organic layers were dried over sodium sulphate. All solvents were removed under reduced pressure and the residue was dried under vacuo to give the title compound as a solid.

MS: calc.: $C_{30}H_{38}N_4O_5$ (534.65) found: $[MH^+]$ = 535.2

**B56. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]carbamate**

To a suspension of (4aS,8aR)-4-(3,4-dimethoxyphenyl)-2-(piperidin-4-yl)-4a,5,6,7,8,8a-hexahydrophthalazin-1(2H)-one hydrochloride (2.04 g; compound B76), N-(tert-butoxycarbonyl)-D-tyrosine (1.41 g) and COMU (2.57 g) in DCM (25 ml) was added DIPEA (3.4 ml) and the mixture was stirred for 1 h at RT. Afterwards DCM (70 ml) and saturated

aqueous sodium bicarbonate solution (25 ml) were added and the mixture was filtered using a phase separator. The organic layer was concentrated under reduced pressure and the residue was purified twice by flash column chromatography [amino phase silica gel, eluation gradient: Cyclohexane/EtOAc/MeOH, 1/0/0 to 0/1/0 to 0/9/1 (v/v/v)] to yield the title compound as a solid.

MS: calc.: $C_{35}H_{46}N_4O_7$ (634.76) found: [$MH^+$] = 635.1; [$MH^+$ - Boc] = 519.3; [$MNa^+$] = 535.2

**B57. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (612 mg; compound B76), N-(tert-butoxycarbonyl)-L-phenylalanine (398 mg) and HBTU (626mg) in DCM (15 ml) was added DIPEA (1.1 ml) and the mixture was stirred for 0.5 h at RT. Afterwards the mixture was extracted with saturated aqueous sodium bicarbonate solution (10 ml), the organic layer was separated and dried over sodium sulfate. The organic layer was concentrated under reduced pressure and the residue was purified by flash column chromatography [silica gel, eluent: EtOAc] to yield the title compound as a solid.

MS: calc.: $C_{35}H_{46}N_4O_6$ (618.78) found: [$MH^+$] = 619.1; [$MH^+$ - Boc] = 519.3; [$MNa^+$] = 641.2

**B58. (4aS,8aR)-2-{1-[(2S)-2-amino-3-phenylpropanoyl]piperidin-4-yl}-4-(3,4-dimethoxyphenyl)-4a,5,6,7,8,8a-hexahydrophthalazin-1(2H)-one hydrochloride**

To tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate (700 mg, compound B57) was added a solution of hydrogen chloride in 1,4-dioxane (5.0 ml, 4.0 M) and the reaction mixture was stirred for 1 h at RT. All volatiles were removed under vacuo to give the title compound as a solid.

MS: calc.: $C_{30}H_{38}N_4O_4$ (518.66) found: [$MH^+$] = 519.2

**B59. (4aS,8aR)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2R)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-1-oxo-3-phenylpropan-2-yl]carbamate (5.12 g, compound B60) in 1,4 dioxane (10 ml) was added a solution of hydrogen chloride in 1,4-dioxane (10.35 ml, 4.0 M) and the reaction mixture was stirred for 45 min at RT. Dichloromethane (40 ml) was added and the reaction mixture was stirred for 12 h at RT. All volatiles were evaporated and the resulting residue was co-evaporated with DCM (3x 30 ml). The residue was dried in vacuo for 90 min at RT to give the title compound as a solid.

MS: calc.: $C_{30}H_{38}N_4O_4$ (518.65) found: [$MH^+$] = 519.29

**B60. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (4.08 g; compound B76), N-(tert-butoxycarbonyl)-D-phenylalanine (2.65 g) and COMU (4.71 g) in DCM (100 ml) was added DIPEA (6.8 ml) and the mixture was stirred for 75 min at RT. Additional DCM (50 ml) and saturated aqueous sodium bicarbonate solution (20 ml) were added and the mixture was filtered using a phase separator. The organic layer was concentrated under reduced pressure and the residue was purified twice by flash column chromatography [1) amino phase silica gel, eluation gradient: Cyclohexane/EtOAc, 1/0 to 1/1 to 1/4 (v/v); 2) silica gel, eluation gradient: Cyclohexane/EtOAc, 1/0 to 3/1 to 1/1 to 1/3 (v/v/)] to yield the title compound as a solid.

MS: calc.: $C_{35}H_{46}N_4O_6$ (618.77) found: [$MH^+$] = 619.1; [$MH^+$ - Boc] = 519.3; [$MNa^+$] = 641.3

**B61. tert-Butyl {(2R)-1-{4-[(4aS,8aR)-4-(3,4-dimethoxyphenyl)-1-oxo-4a,5,6,7,8,8a-hexahydro-phthalazin-2(1H)-yl]piperidin-1-yl}-1-oxo-3-[2-(trifluoromethyl)phenyl]propan-2-yl}carbamate**

To a suspension of (4aS,8aR)-4-(3,4-dimethoxyphenyl)-2-(piperidin-4-yl)-4a,5,6,7,8,8a-hexahydrophthalazin-1(2H)-one hydrochloride (612 mg; compound B76), N-(tert-butoxycarbonyl)-2-(trifluoromethyl)-D-phenylalanine (733 mg) and COMU (1.04 g) in DCM (15 ml) was added DIPEA (1.53 ml) and the mixture was stirred for 45 min at RT. Afterwards the mixture was extracted with saturated aqueous sodium bicarbonate solution (10 ml), the organic layer was separated and dried over sodium sulfate. The organic layer was concentrated under reduced pressure and the residue was purified by flash column chromatography [amino phase silica gel, eluation gradient: EtOAc/MeOH, 100/0

to 97/3 (v/v)] to yield the title compound as a solid.

MS: calc.: $C_{36}H_{45}F_3N_4O_6$ (686.76) found: $[MH^+]$ = 687.1

### B62. (4aS,8aR)-2-{1-[(2R)-2-aminopropanoyl]piperidin-4-yl}-4-(3,4-dimethoxyphenyl)-4a,5,6,7,8,8a-hexahydrophthalazin-1(2H)-one hydrochloride

tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (350 mg; compound B29) was dissolved in a solution of hydrogen chloride in 1,4-dioxane (5 ml, 4.0 M) and the reaction mixture was stirred for about 30 min at RT. Afterwards all volatiles were removed under reduced pressure to give the title compound as a solid.

MS: calc.: $C_{24}H_{34}N_4O_4$ (442.56) found: $[MH^+]$ = 443.2

### B63. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]carbamate

To a suspension of (4aS,8aR)-4-(3,4-dimethoxyphenyl)-2-(piperidin-4-yl)-4a,5,6,7,8,8a-hexahydrophthalazin-1(2H)-one hydrochloride (1.02 g; compound B76), N-(tert-butoxycarbonyl)-3-pyridin-2-yl-L-alanine (666 mg) and HBTU (1.04 g) in DCM (15 ml) was added DIPEA (1.7 ml) and the mixture was stirred for 1.5 h at RT. Afterwards the mixture was extracted with saturated aqueous sodium bicarbonate solution (10 ml), the organic layer was separated and dried over sodium sulfate. The organic layer was concentrated under reduced pressure and the residue was purified byflash column chromatography [silica gel, eluation gradient: EtOAc/MeOH, 95/5 to 90/10 (v/v)] to yield the title compound as a solid.

MS: calc.: $C_{34}H_{45}N_5O_6$ (619.75) found: $[MH^+]$ = 620.2

### B64. (4aS,8aR)-2-{1-[(2S)-2-amino-3-(pyridin-4-yl)propanoyl]piperidin-4-yl}-4-(3,4-dimethoxyphenyl)-4a,5,6,7,8,8a-hexahydrophthalazin-1(2H)-one hydrochloride

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]carbamate (1.06 g; compound B65) was dissolved in a solution of hydrogen chloride in 1,4-dioxane (6 ml, 4.0 M) and the reaction mixture was stirred for about 2 h at RT. Afterwards all volatiles were removed under reduced pressure to give the title compound as a solid.

MS: calc.: $C_{29}H_{37}N_5O_4$ (519.65) found: $[MH^+]$ = 520.1

### B65. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]carbamate

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (612 mg; compound B76), N-(tert-butoxycarbonyl)-3-pyridin-4-yl-L-alanine (400 mg) and HBTU (626 mg) in DCM (15 ml) was added DIPEA (1.0 ml) and the mixture was stirred for 1 h at RT. Afterwards the mixture was extracted with saturated aqueous sodium bicarbonate solution (10 ml), the organic layer was separated using a phase separator. The organic layer was concentrated under reduced pressure and the residue was purified by flash column chromatography [silica gel, eluation gradient: EtOAc/MeOH, 95/5 to 90/10 (v/v)] to yield the title compound as a solid.

MS: calc.: $C_{34}H_{45}N_5O_6$ (619.77) found: $[MH^+]$ = 620.2

### B66. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]carbamate

To a suspension of (4aS,8aR)-4-(3,4-dimethoxyphenyl)-2-(piperidin-4-yl)-4a,5,6,7,8,8a-hexahydrophthalazin-1(2H)-one hydrochloride (1.53 g; compound B76), N-(tert-butoxycarbonyl)-3-pyridin-3-yl-L-alanine (1.0 g) and HBTU (1.57 g) in DCM (25 ml) was added DIPEA (1.94 ml) and the mixture was stirred for 1.5 h at RT. Afterwards the mixture was extracted wth saturated aqueous sodium bicarbonate solution (10 ml), the organic layer was separated using a phase separator. The organic layer was concentrated under reduced pressure and the residue was purified by flash column chromatography [amino phase silica gel, eluation gradient: EtOAc/Cyclohexane, 0/100 to 100/0 (v/v)] to yield the title compound as a solid.

MS: calc.: $C_{34}H_{45}N_5O_6$ (619.77) found: $[MH^+]$ = 620.2

**B67. (4aR,8aS)-2-{1-[(2S)-2-amino-3-phenylpropanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2S)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate (1.19 g, compound B69) in 1,4 dioxane (30 ml) was added a solution of hydrogen chloride in 1,4-dioxane (4.81 ml, 4.0 M) and the reaction mixture was stirred for 12 h at RT, the for 8 h at 65°C and afterwards again at RT for 12 h. Aditional solution of hydrogen chloride in 1,4-dioxane (1.6 ml, 4.0 M) was added and the reaction mixture was stirred for 4 h at 65°C in order to complete the reaction. The suspension was filtered and the filter cake was washed with dioxane. The solid was dried under vacuo to give the title compound as a solid.

MS: calc.: $C_{30}H_{38}N_4O_4$ (518.65) found: [$MH^+$] = 519.2

**B68. tert-Butyl [(2R)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (791 mg; compound B76), N-(tert-butoxycarbonyl)-D-phenylalanine (515 mg) and COMU (874 mg) in DCM (20 ml) was added DIPEA (0.83 ml) and the mixture was stirred for 0.5 h at RT. Afterwards the mixture was treated with saturated aqueous sodium bicarbonate solution (10 ml) and extracted with DCM (10 ml). The organic layer was separated using a phase separator, concentrated under reduced pressure and the residue was purified byflash column chromatography [amino phase silica gel, eluation gradient: EtOAc/Cyclohexane/MeOH, 0/100/0 to 100/0/0 to 95/0/5 (v/v/v)]. After lyophilisation from acetonitrile / water (20 ml, 3/1 (v/v)) the title compound was obtained as a solid.

MS: calc.: $C_{34}H_{45}N_5O_6$ (619.77) found: [$MH^+$] = 620.2

**B69. tert-Butyl [(2S)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (816 mg compound B76), N-(tert-butoxycarbonyl)-L-phenylalanine (531 mg) and COMU (900 mg) in DCM (20 ml) was added DIPEA (0.85 ml) and the mixture was stirred for 0.5 h at RT. Afterwards the mixture was treated with saturated aqueous sodium bicarbonate solution (10 ml) and extracted with DCM (10 ml). The organic layer was separated using a phase separator, concentrated under reduced pressure and the residue was purified byflash column chromatography [amino phase silica gel, eluation gradient: EtOAc/MeOH, 100/0 to 95/0/5 (v/v)]. After lyophilisation from acetonitrile / water (20 ml, 3/1 (v/v)) the title compound was obtained as a solid.

MS: calc.: $C_{35}H_{46}N_4O_6$ (618.78) found: [$MH^+$] = 619.0

**B70. (4aR,8aS)-2-{1-[(2R)-2-amino-3-phenylpropanoyl]piperidin-4-yl}-4-(3,4-dimethoxyphenyl)-4a,5,6,7,8,8a-hexahydrophthalazin-1(2H)-one**

To a solution of tert-Butyl [(2R)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]carbamate (745 mg, compound B68) in 1,4 dioxane (30 ml) was added a solution of hydrogen chloride in 1,4-dioxane (3.0 ml, 4.0 M) and the reaction mixture was stirred for 48 h at RT, then for 8 h at 65°C and afterwards again at RT for 12 h. Aditional solution of hydrogen chloride in 1,4-dioxane (3.0 ml, 4.0 M) was added and the reaction mixture was stirred for 4 h at 65°C. All volatiles were removed under vacuo and the residue was treated with DCM and saturated aqueous sodium bicarbonate solution (30 ml). The organic layer was separated and the solvent was removed under vacuo. The resulting residue was subjected to by flash column chromatography [amino phase silica gel, eluation gradient: EtOAc/MeOH, 100/0 to 97.5/2.5 to 95/5 (v/v)] to give the title compound together with starting material (LC-MS control indicated about 77% of title compound and about 20% starting material). The compound was used for the next reaction step without further purification.

**B71. 4-[5-(cyclopropylmethoxy)-1,3-benzodioxol-4-yl]-5H-pyrrolo[3,2-d]pyrimidine-7-carboxylic acid**

The Synthesis of Compound B71 is described in PCT application WO2009106531

MS: calc.: $C_{18}H_{15}N_3O_5$ (353.33) found: [$MH^+$] = 354.0

**B72. 4-[5-(cyclopropylmethoxy)-1,3-benzodioxol-4-yl]-6-methyl-5H-pyrrolo[3,2-d]pyrimidine-7-carboxylic acid**

Compound B72 can be prepared in analogy to methods described in WO2011/023693.

MS: calc.: $C_{19}H_{17}N_3O_5$ (367.36) found: [$MH^+$] = 368.1

**B73. (4aS,7aR)-4-(3,4-dimethoxyphenyl)-2-(piperidin-4-yl)-2,4a,5,6,7,7a-hexahydro-1H-cyclopenta[d]pyridazin-1-one**

Compound B73 can be prepared in analogy to methods described in WO2005075457.

MS: calc.: $C_{20}H_{27}N_3O_3$ (357.45) found: [$MH^+$] = 358.2

Enantiomeric excess: >97% e.e.

**B74. (4aS,8aR)-2-{1-[(2S)-2-amino-3-(2-chlorophenyl)propanoyl]piperidin-4-yl}-4-(3,4-dimethoxyphenyl)-4a,5,6,7,8,8a-hexahydrophthalazin-1(2H)-one**

tert-Butyl [(2S)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (2.3 g; compound B75) was dissolved in a solution of hydrogen chloride in 1,4-dioxane (15 ml, 4.0 M) and the reaction mixture was stirred for 30 min at RT and for 30 min at 50°C. Afterwards the mixture was extracted with DCM (50 ml) and an aqueous solution of hydrogen chloride (25 ml, 2M). The aqueous phase was separated, alkalnized and extracted with DCM. The organic phases were combined, the solvent was removed under vacuo and the residue was purified by flash column chromatography [amino phase silica gel, eluent: Cyclohexane/EtOAc/MeOH, 100/0/0 to 0/92/8 (v/v/v)] to give the title compound as a solid.

MS: calc.: $C_{30}H_{37}ClN_4O_4$ (553.09) found: [$MH^+$] = 553.2

**B75. tert-Butyl [(2S)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of N-(tert-butoxycarbonyl)-2-chloro-L-phenylalanine (1.13 g) and DIPEA (1.94 g) in DCM (35 ml) was added (4aS,8aR)-4-(3,4-dimethoxyphenyl)-2-(piperidin-4-yl)-4a,5,6,7,8,8a-hexahydro-phthalazin-1(2H)-one hydrochloride (1.53g; compound B76)) and HBTU (2.84 g) and the reaction mixture was stirred for 1 h at RT. Afterwards a saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [amino phase silica gel, eluent: Cyclohexane/EtOAc/MeOH, 1/0/0 to 0/1/0 to 0/9/1 (v/v/v)] to give the title compound as a solid.

MS: calc.: $C_{35}H_{45}ClN_4O_6$ (653.22) found: [$MH^+$] = 653.0; [$MH^+$ - Boc] = 553.2; [$MNa^+$] = 675.2

**B76. (4aS,8aR)-4-(3,4-dimethoxyphenyl)-2-(piperidin-4-yl)-4a,5,6,7,8,8a-hexahydrophthalazin-1(2H)-one hydrochloride**

The Synthesis of Compound B76 is described in PCT application WO2005075457.

MS: calc.: $C_{21}H_{29}N_3O_3$ (371.48) found: [$MH^+$] = 372.3

Enantiomeric excess: >97% e.e.

**B77. (4aR,8aS)-4-(3,4-dimethoxyphenyl)-2-(piperidin-4-yl)-4a,5,6,7,8,8a-hexahydrophthalazin-1(2H)-one hydrochloride**

Compound B77 was prepared in analogy to methods described in WO2005075457.

MS: calc.: $C_{21}H_{29}N_3O_3$ (371.48) found: [$MH^+$] = 372.3

Enantiomeric excess: >98% e.e.

**B78. (4aS,8aR)-2-{1-[(2S)-2-amino-3-hydroxypropanoyl]piperidin-4-yl}-4-(3,4-dimethoxyphenyl)-4a,5,6,7,8,8a-hexahydrophthalazin-1(2H)-one trifluoroacetate**

A solution of tert-Butyl [(2S)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-hydroxy-1-oxopropan-2-yl]carbamate (3.91 g; compound B79) in DCM (25 ml) was added trifluoroacetic acid (7.8 ml) at 0°C. The reaction mixture was stirred for 10 min at 0°C and then for 2h at RT. Afterwards all volatiles were removed to give the title compound as a solid.

MS: calc.: $C_{24}H_{34}N_4O_5$ (458.56) found: [$MH^+$] = 459.2

**B79. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxopropan-2-yl]carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (8.16 g; compound B76), N-(tert-butoxy-carbonyl)-L-serine (4.1 g) and HBTU (8.34 g) in DCM (200 ml) was added DIPEA (14 ml) and the mixture was stirred for 0.5 h at RT. Afterwards the mixture was treated with saturated aqueous sodium bicarbonate solution (100 ml), the organic layer was separated, dried over sodium sulfate, concentrated under reduced pressure and the resulting residue was purified by flash column chromatography [amino phase silica gel, eluent: EtOAc/MeOH, 95/5 (v/v)] to give the title compound as a solid.
MS: calc.: $C_{29}H_{42}N_4O_7$ (558.68) found: $[MH^+]$ = 559.1

**B80. (4aS,8aR)-2-[1-(aminoacetyl)piperidin-4-yl]-4-(3,4-dimethoxyphenyl)-4a,5,6,7,8,8a-hexahydrophthalazin-1(2H)-one hydrochloride**

tert-Butyl (2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl)carbamate (4.0 g; compound B81) was dissolved in a solution of hydrogen chloride in 1,4-dioxane (45 ml, 4.0 M) at 0°C and the reaction mixture was stirred for about 2 h at 0°C and afterwards for 1.5 h at RT. Afterwards all volatiles were removed under reduced pressure to give the title compound as a solid.
MS: calc.: $C_{23}H_{32}N_4O_4$ (428.54) found: $[MH^+]$ = 429.3

**B81. tert-Butyl (2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl)carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (6.12 g; compound B76)), N-(tert-butoxycarbonyl)glycine (2.63 g) and HBTU (8.55 g) in DCM (80 ml) was added DIPEA (10.5 ml) and the mixture was stirred for 45 min at RT. Afterwards the mixture was treated with saturated aqueous sodium bicarbonate solution (40 ml), the organic layer was separated, dried over sodium sulfate, concentrated under reduced pressure and the resulting residue was purified by flash column chromatography [silica gel, eluation gradient: EtOAc/MeOH, 100/0 to 95/5 (v/v)] to give the title compound as a solid.
MS: calc.: $C_{28}H_{40}N_4O_6$ (528.65) found: $[MH^+]$ = 529.0

**B82. (4aS,8aR)-2-{1-[(2S)-2-aminopropanoyl]piperidin-4-yl}-4-(3,4-dimethoxyphenyl)-4a,5,6,7,8,8a-hexahydrophthalazin-1(2H)-one**

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (5.82 g, compound B83) in THF (50 ml) was added an aqueous solution of hydrogen chloride (80.5 ml, 2M) and the mixture was stirred at 50 °C for 1 h and at RT for 12 h. Afterwards an aqueous solution of sodium hydroxide (10M) was slowly added until the solution was alkanized (pH 14). The mixture was extracted with DCM (3 x 300ml) and the combined organic layers were dried over sodium sulphate. All solvents were removed under reduced pressure and the residue was dried under vacuo to give the title compound as a solid.
MS: calc.: $C_{24}H_{34}N_4O_4$ (442.56) found: $[MH^+]$ = 443.2

**B83. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

A suspension of (4aS,8aR)-4-(3,4-dimethoxyphenyl)-2-(piperidin-4-yl)-4a,5,6,7,8,8a-hexahydro-phthalazin-1(2H)-one hydrochloride (5.0 g; compound B76), N-(tert-butoxycarbonyl)-L-alanine (2.44 g) HBTU (5.35 g) and DIPEA (10.5 ml) in DCM (40 ml) was stirred for 45 min at RT. Afterwards the mixturewas treated with DCM (25 ml) and saturated aqueous sodium bicarbonate solution (15 ml), the phases were separated using a phase separator and the organic layer was concentrated under reduced pressure. The resulting residue was purified twice by flash column chromatography [amino phase silica gel, eluation gradient for first run: Cyclohexane/EtOAc, 1/0 to 0/1 (v/v), eluation gradient for second run: Cyclohexane/EtOAc, 1/0 to 7/3 (v/v)] to give the title compound as a solid.
MS: calc.: $C_{29}H_{42}N_4O_6$ (542.68) found: $[MH^+]$ = 543.0

**B84. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]carbamate**

To a suspension of (4aS,8aR)-4-(3,4-dimethoxyphenyl)-2-(piperidin-4-yl)-4a,5,6,7,8,8a-hexahydro-phthalazin-1(2H)-one hydrochloride (2.04 g; compound B76), N-(tert-butoxycarbonyl)-L-tyrosine (1.41 g) and COMU (2.57 g) in DCM (25 ml) was added DIPEA (3.4 ml) and the mixture was stirred for 1 h at RT. Afterwards DCM (70 ml) and saturated aqueous sodium bicarbonate solution (25 ml) were added and the mixture was filtered using a phase separator. The organic layer was concentrated under reduced pressure and the residue was purified twice by flash column chromatography [amino phase silica gel, eluation gradient: Cyclohexane/EtOAc/MeOH, 1/0/0 to 0/1/0 to 0/9/1 (v/v/v)] to yield the title compound as a solid.

MS: calc.: $C_{35}H_{46}N_4O_7$ (634.76) found: [$MH^+$] = 635.1; [$MNa^+$] = 535.2

**B85. (4aS,8aR)-2-{1-[(2R)-2-amino-4-phenylbutanoyl]piperidin-4-yl}-4-(3,4-dimethoxyphenyl)-4a,5,6,7,8,8a-hexahydrophthalazin-1(2H)-one hydrochloride**

tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-4-phenylbutan-2-yl]carbamate (2.6 g; compound B86) was dissolved in 1,4-dioxane (15 ml) and a solution of hydrogen chloride in 1,4-dioxane (5.8 ml, 4.0 M) was added at RT and the reaction mixture was stirred for 18h. Afterwards diethyl ether (120 ml) was added, the resulting suspension was filtered off and washed with diethyl ether. The solid was dried under vacuo at 50 °C for 2 h to give the title.

MS: calc.: $C_{31}H_{40}N_4O_4$ (532.67) found: [$MH^+$] = 533.3

**B86. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-4-phenylbutan-2-yl]carbamate**

To a mixture of (2R)-2-[(tert-butoxycarbonyl)amino]-4-phenylbutanoic acid (1.1 g) and DIPEA (2.6 ml) in DCM (30 ml) was added (4aS,8aR)-4-(3,4-dimethoxyphenyl)-2-(piperidin-4-yl)-4a,5,6,7,8,8a-hexahydrophthalazin-1(2H)-one hydrochloride (1.61g; compound B76) and COMU (1.86 g) and the reaction mixture was stirred for 3 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [amino phase silica gel, eluent: Petrolether/EtOAc/MeOH, 60/37/3 (v/v/v)] to give the title compound as a solid.

MS: calc.: $C_{36}H_{48}N_4O_6$ (632.79) found: [$MH^+$] = 633.1

**B87. 2-{1-[(2R)-2-amino-3-phenylpropanoyl]piperidin-4-yl}-6-(3,4-dimethoxyphenyl)-4,4-dimethyl-4,5-dihydropyridazin-3(2H)-one**

A solution of tert-Butyl [(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]carbamate (3.6 g; compound B88) and trifluoroacetic acid (3.6 ml) in DCM (36 ml) was stirred for 48 h at RT. Afterwards a saturated aqueous sodium bicarbonate solution was slowly added until the solution was alkanized. The mixturewas extracted twice with DCM, the combined organic layers were dried over magnesium sulphate and all solvents were removed under reduced pressure. The residue was dried under vacuo to give the title compound as a solid.

MS: calc.: $C_{28}H_{36}N_4O_4$ (492.61) found: [$MH^+$] = 493.2; [$MH^+$ - Boc] = 593.0

**B88. tert-Butyl [(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]carbamate**

To a mixture of N-(tert-butoxycarbonyl)-D-phenylalanine (2.03 g) and DIPEA (5.0 ml) in DCM (75 ml) was added 6-(3,4-dimethoxyphenyl)-4,4-dimethyl-2-(piperidin-4-yl)-4,5-dihydropyridazin-3(2H)-one (2.64 g; compound B89) and COMU (3.6 g) and the reaction mixture was stirred for 2 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Toluene/EtOAc, 85/15 (v/v)] to give the title compound as a solid.

MS: calc.: $C_{33}H_{44}N_4O_6$ (592.72) found: [$MH^+$] = 593.0

**B89. 6-(3,4-dimethoxyphenyl)-4,4-dimethyl-2-(piperidin-4-yl)-4,5-dihydropyridazin-3(2H)-one**

The Synthesis of Compound B89 is described in PCT application WO2005075457.

MS: calc.: $C_{19}H_{27}N_3O_3$ (345.44) found: [$MH^+$] = 346.2

**B90. (4aS,7aR)-2-{1-[(2R)-2-amino-3-phenylpropanoyl]piperidin-4-yl}-4-(3,4-dimethoxyphenyl)-2,4a,5,6,7,7a-hexahydro-1H-cyclopenta[d]pyridazin-1-one hydrochloride**

tert-Butyl [(2R)-1-{4-[(4aS,7aR)-4-(3,4-dimethoxyphenyl)-1-oxo-1,4a,5,6,7,7a-hexahydro-2H-cyclopenta[d]pyridazin-2-yl]piperidin-1-yl}-1-oxo-3-phenylpropan-2-yl]carbamate (267 mg; compound B91) was dissolved in a solution of hydrogen chloride in 1,4-dioxane (2.5 ml, 4.0 M) and the reaction mixture was stirred for 1h at RT. All volatiles were removed under vacuo and the residue was co-evaporated with DCM to yield the title compound as a solid.

MS: calc.: $C_{29}H_{36}N_4O_4$ (504.62) found: $[MH^+]$ = 505.1

**B91. tert-Butyl [(2R)-1-{4-[(4aS,7aR)-4-(3,4-dimethoxyphenyl)-1-oxo-1,4a,5,6,7,7a-hexahydro-2H-cyclopenta[d]pyridazin-2-yl]piperidin-1-yl}-1-oxo-3-phenylpropan-2-yl]carbamate**

To a mixture of N-(tert-butoxycarbonyl)-D-phenylalanine (292 mg), (4aS,7aR)-4-(3,4-dimethoxy-phenyl)-2-(piperidin-4-yl)-2,4a,5,6,7,7a-hexahydro-1H-cyclopenta[d]pyridazin-1-one (358 mg; compound B73) and DIPEA (0.43 ml) in DCM (5 ml) was slowly added a solution of $T_3P^®$ (1.27 g, 50% solution in DCM) in DCM (1 ml). The reaction mixture was stirred for 1 h at 40 °C in a sealed tube. Afterwards a saturated aqueous sodium bicarbonate solution (5 ml) was added and the mixture was extracted with DCM (9 ml). The organic phase was separated, dried over magnesium sulphate and concentrated under reduced pressure. The resulting residue was purified by flash column chromatography [silica gel, eluent: EtOAc/MeOH, 95/5 (v/v)] to give the title compound as a solid.

MS: calc.: $C_{34}H_{44}N_4O_6$ (604.74) found: $[MH^+]$ = 605.0

**B92. tert-Butyl [(2S)-3-cyclohexyl-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a suspension of (4aS,8aR)-4-(3,4-dimethoxyphenyl)-2-(piperidin-4-yl)-4a,5,6,7,8,8a-hexahydrophthalazin-1(2H)-one hydrochloride (900 mg; compound B76), N-(tert-butoxycarbonyl)-3-cyclohexyl-L-alanine (600 mg) and COMU (1.04 g) in DCM (15 ml) was added DIPEA (1.53 ml) and the reaction mixture was stirred for 45 min at RT. Afterwards the mixture was extracted with aqueous sodium bicarbonate solution (10 ml), the organic phase was separated, dried over sodium sulphate and concentrated under reduced pressure. The resulting residue was purified by flash column chromatography [silica gel, eluation gradient: EtOAc/MeOH, 100/0 to 98/2 (v/v)] to give the title compound as a solid.

MS: calc.: $C_{35}H_{52}N_4O_6$ (624.81) found: $[MH^+]$ = 625.1

**B93. (4aS,8aR)-2-{1-[(2S)-2-amino-3-cyclohexylpropanoyl]piperidin-4-yl}-4-(3,4-dimethoxyphenyl)-4a,5,6,7,8,8a-hexahydrophthalazin-1(2H)-one hydrochloride** tert-Butyl [(2S)-3-cyclohexyl-1-{4-[(4aS,8aR)-4-(3,4-dimethoxyphenyl)-1-oxo-4a,5,6,7,8,8a-hexa-hydrophthalazin-2(1H)-yl]piperidin-1-yl}-1-oxopropan-2-yl]carbamate (1.1 g; compound B92) was dissolved in a solution of hydrogen chloride in 1,4-dioxane (10 ml, 4.0 M) and the reaction mixture was stirred for about 1 h at RT. Afterwards all volatiles were removed under reduced pressure to give the title compound as a solid.

MS: calc.: $C_{30}H_{44}N_4O_4$ (524.69) found: $[MH^+]$ = 523.3

**B94. tert-Butyl [(2S,3S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxopentan-2-yl]carbamate**

To a suspension of (4aS,8aR)-4-(3,4-dimethoxyphenyl)-2-(piperidin-4-yl)-4a,5,6,7,8,8a-hexahydrophthalazin-1(2H)-one hydrochloride (653 mg; compound B76), N-(tert-butoxycarbonyl)-L-isoleucine (370 mg) and HBTU (667 mg) in DCM (20 ml) was added DIPEA (1.1 ml) and the reaction mixture was stirred for 30 min at RT. Afterwards the mixture was extracted with aqueous sodium bicarbonate solution (10 ml), the organic phase was separated, dried over sodium sulphate and concentrated under reduced pressure. The resulting residue was purified by flash column chromatography [silica gel, eluation gradient: Cyclohexane/EtOAc, 30/70 to 0/100 (v/v)] to give the title compound as a solid.

MS: calc.: $C_{32}H_{48}N_4O_6$ (584.76) found: $[MH^+]$ = 585.1

**B95. (4aS,8aR)-2-{1-[(2S,3S)-2-amino-3-methylpentanoyl]piperidin-4-yl}-4-(3,4-dimethoxyphenyl)-4a,5,6,7,8,8a-hexahydrophthalazin-1(2H)-one** hydrochloride

tert-Butyl [(2S,3S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxopentan-2-yl]carbamate (650 mg; compound B94) was dissolved in a solution of hydrogen chloride in 1,4-dioxane (6 ml, 4.0 M) and the reaction mixture was stirred for about 45 min at RT. Afterwards

all volatiles were removed under reduced pressure to give the title compound as a solid.

MS: calc.: $C_{27}H_{40}N_4O_4$ (484.64) found: $[MH^+]$ = 485.3

**B96. tert-Butyl [(1R)-1-cyclohexyl-2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl]carbamate**

To a suspension of (4aS,8aR)-4-(3,4-dimethoxyphenyl)-2-(piperidin-4-yl)-4a,5,6,7,8,8a-hexa-hydrophthalazin-1(2H)-one hydrochloride (1.31 g; compound B76), (2R)-[(tert-butoxycarbonyl)-amino](cyclohexyl)ethanoic acid (1.03 g) and HBTU (1.82 g) in DCM (15 ml) was added DIPEA (1.63 ml) and the reaction mixture was stirred for 30 min at RT. Afterwards the mixture was extracted with aqueous sodium bicarbonate solution (3x10 ml) and DCM (50 ml), the organic phase was separated, dried over sodium sulphate and concentrated under reduced pressure. The resulting residue was purified twice by flash column chromatography [first column: silica gel, eluation gradient: Cyclohexane/EtOAc, 80/20 to 0/100 (v/v); second column: amino phase silica gel, eluent: EtOAc] to give the title compound as a solid.

MS: calc.: $C_{34}H_{50}N_4O_6$ (610.78) found: $[MH^+]$ = 611.1

**B97. (4aS,8aR)-2-{1-[(2R)-2-amino-2-cyclohexylacetyl]piperidin-4-yl}-4-(3,4-dimethoxyphenyl)-4a,5,6,7,8,8a-hexahydrophthalazin-1(2H)-one hydrochloride**

tert-Butyl [(1R)-1-cyclohexyl-2-{4-[(4aS,8aR)-4-(3,4-dimethoxyphenyl)-1-oxo-4a,5,6,7,8,8a-hexa-hydrophthalazin-2(1H)-yl]piperidin-1-yl}-2-oxoethyl]carbamate (1.93 g; compound B96)was dissolved in a solution of hydrogen chloride in 1,4-dioxane (19.8 ml, 4.0 M) and the reaction mixture was stirred for 90 min at RT. Afterwards all volatiles were removed under reduced pressure and the residue was treated with DCM and co-evaporated (3x) to give the title compound as a solid.

MS: calc.: $C_{29}H_{42}N_4O_4$ (510.67) found: $[MH^+]$ = 511.2

**B98. tert-Butyl [(1S)-1-cyclohexyl-2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl]carbamate**

To a suspension of (4aS,8aR)-4-(3,4-dimethoxyphenyl)-2-(piperidin-4-yl)-4a,5,6,7,8,8a-hexa-hydrophthalazin-1(2H)-one hydrochloride (2.04 g; compound B76), (2S)-[(tert-butoxycarbonyl)-amino](cyclohexyl)ethanoic acid (1.61 g) and HBTU (2.37 g) in DCM (25 ml) was added DIPEA (2.55 ml) and the reaction mixture was stirred for 2 h at RT. Afterwards the mixture was extracted with aqueous sodium bicarbonate solution (3x10 ml), the organic phase was separated and concentrated under reduced pressure. The resulting residue was purified twice by flash column chromatography [first column: silica gel, eluation gradient: EtOAc/n-hexane, 30/70 to 100/100 to 70/30 to100/0 (v/v); second column: amino phase silica gel, eluent: EtOAc] to give the title compound as a solid.

MS: calc.: $C_{34}H_{50}N_4O_6$ (610.78) found: $[MH^+]$ = 611.0

**B99. (4aS,8aR)-2-{1-[(2S)-2-amino-2-cyclohexylacetyl]piperidin-4-yl}-4-(3,4-dimethoxyphenyl)-4a,5,6,7,8,8a-hexahydrophthalazin-1(2H)-one hydrochloride**

tert-Butyl [(1S)-1-cyclohexyl-2-{4-[(4aS,8aR)-4-(3,4-dimethoxyphenyl)-1-oxo-4a,5,6,7,8,8a-hexa-hydrophthalazin-2(1H)-yl]piperidin-1-yl}-2-oxoethyl]carbamate (3.0 g; compound B98) was dissolved in a solution of hydrogen chloride in 1,4-dioxane (18.4 ml, 4.0 M) and the reaction mixture was stirred for about 20 min at RT. Afterwards all volatiles were removed under reduced pressure and the residue was treated with DCM and co-evaporated (3x) to give the title compound as a solid.

MS: calc.: $C_{29}H_{42}N_4O_4$ (510.67) found: $[MH^+]$ = 511.2

**B100. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(thiophen-2-yl)propan-2-yl]carbamate**

To a suspension of (4aS,8aR)-4-(3,4-dimethoxyphenyl)-2-(piperidin-4-yl)-4a,5,6,7,8,8a-hexa-hydrophthalazin-1(2H)-one hydrochloride (734 mg; compound B76), N-(tert-butoxycarbonyl)-3-thiophen-2-yl-L-alanine (513 mg) and HBTU (820 mg) in DCM (25 ml) was added DIPEA (1.23 ml) and the reaction mixture was stirred for 30 min at RT. Afterwards the mixture was extracted with aqueous sodium bicarbonate solution (10 ml) and DCM (2x20 ml), the organic phase was separated and concentrated under reduced pressure. The resulting residue was purified by flash column chromatography [amino phase silica gel, eluation gradient: Cyclohexane/EtOAc/MeOH, 100/0/0 to 0/100/0 to 0/90/10 (v/v/v)] to give the title compound as a solid.

MS: calc.: $C_{33}H_{44}N_4O_6S$ (624.81) found: $[MH^+]$ = 625.0; $[MNa^+]$ = 647.2; $[MH^+ - Boc]$ = 525.2

**B101. (4aS,8aR)-2-{1-[(2S)-2-amino-3-(thiophen-2-yl)propanoyl]piperidin-4-yl}-4-(3,4-dimethoxyphenyl)-4a,5,6,7,8,8a-hexahydrophthalazin-1(2H)-one hydrochloride**

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(thiophen-2-yl)propan-2-yl]carbamate (1.16 g; compound B100) was dissolved in THF (15 ml) and an aqueous solution of hydrogen chloride (4.68 ml, 2.0 M) was added. The reaction mixture was stirred for 2 d at 55 °C. Afterwards all volatiles were removed under reduced pressure and the residue was treated with DCM and co-evaporated to give the title compound as a solid.

MS: calc.: $C_{28}H_{36}N_4O_4S$ (524.69) found: [$MH^+$] = 525.2

**B102. (4aS,8aR)-2-[1-(3-aminopropanoyl)piperidin-4-yl]-4-(3,4-dimethoxyphenyl)-4a,5,6,7,8,8a-hexahydrophthalazin-1(2H)-one hydrochloride**

tert-Butyl (3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl)carbamate (1.38 g; compound B103) was dissolved in THF (20 ml) and an aqueous solution of hydrogen chloride (6.34 ml, 2.0 M) was added. The reaction mixture was stirred for about 1 d at 55 °C and afterwards for 2 d at RT. Afterwards all volatiles were removed under reduced pressure and the residue was treated with DCM and co-evaporated to give the title compound as a solid.

MS: calc.: $C_{24}H_{34}N_4O_4$ (442.56) found: [$MH^+$] = 443.3

**B103. tert-Butyl (3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl)carbamate**

To a suspension of (4aS,8aR)-4-(3,4-dimethoxyphenyl)-2-(piperidin-4-yl)-4a,5,6,7,8,8a-hexa-hydrophthalazin-1(2H)-one hydrochloride (979 mg; compound B76), N-(tert-butoxycarbonyl)-beta-alanine (454 mg) and HBTU (1.0 g) in DCM (25 ml) was added DIPEA (1.63 ml) and the reaction mixture was stirred for 2 h at RT. Afterwards the mixture was extracted with aqueous sodium bicarbonate solution (10 ml) and DCM (50 ml), the organic phase was separated and concentrated under reduced pressure. The resulting residue was purified by flash column chromatography [amino phase silica gel, eluation gradient: Cyclohexane/EtOAc/MeOH, 100/0/0 to 0/100/0 to 0/93/7 (v/v/v)] to give the title compound as a solid.

MS: calc.: $C_{29}H_{42}N_4O_6$ (542.68) found: [$MH^+$] = 543.1; [$MNa^+$] = 565.2; [$MH^+$ - Boc] = 443.3

**B104. tert-Butyl [(2R)-4-{4-[(4aS,8aR)-4-(3,4-dimethoxyphenyl)-1-oxo-4a,5,6,7,8,8a-hexahydrophthalazin-2(1H)-yl]piperidin-1-yl}-4-oxo-1-phenylbutan-2-yl]carbamate**

To a mixture of (3R)-3-[(tert-butoxycarbonyl)amino]-4-phenylbutanoic acid (0.5 g) and DIPEA (1.17 ml) in DCM (30 ml) was added COMU (0.84 g) and (4aS,8aR)-4-(3,4-dimethoxyphenyl)-2-(piperidin-4-yl)-4a,5,6,7,8,8a-hexahydrophthalazin-1(2H)-one hydrochloride (730 mg; compound B76) and the mixture was stirred for 3 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified by flash column chromatography [amino phase silica gel, eluent: petrolether/ EtOAc, 1/1 (v/v) to give the title compound as a solid.

MS: calc.: $C_{36}H_{48}N_4O_6$ (632.79) found: [$MH^+$] = 633.1

**B105. (4aS,8aR)-2-{1-[(3R)-3-amino-4-phenylbutanoyl]piperidin-4-yl}-4-(3,4-dimethoxyphenyl)-4a,5,6,7,8,8a-hexahydrophthalazin-1(2H)-one**

A solution of tert-Butyl [(2R)-4-{4-[(4aS,8aR)-4-(3,4-dimethoxyphenyl)-1-oxo-4a,5,6,7,8,8a-hexa-hydrophthalazin-2(1H)-yl]piperidin-1-yl}-4-oxo-1-phenylbutan-2-yl]carbamate (0.75 g; compound B104) and trifluoroacetic acid (7.5 ml) in DCM (7.5 ml) was stirred for 1.5 h at RT. Afterwards a saturated aqueous sodium bicarbonate solution was slowly added until the solution was alkanized. The mixture was extracted with DCM, the combined organic layers were dried over magnesium sulphate and all solvents were removed under reduced pressure. The residue was dried under vacuo to give the title compound as a solid.

MS: calc.: $C_{31}H_{40}N_4O_4$ (532.67) found: [$MH^+$] = 532.2

**B106. tert-Butyl [(2R)-4-{4-[3-(3,4-dimethoxyphenyl)-5,5-dimethyl-6-oxo-5,6-dihydropyridazin-1(4H)-yl]piperidin-1-yl}-4-oxo-1-phenylbutan-2-yl]carbamate**

To a mixture of (3R)-3-[(tert-butoxycarbonyl)amino]-4-phenylbutanoic acid (0.5 g) and DIPEA (1.17 ml) in DCM (30 ml) was added COMU (0.84 g) and 6-(3,4-dimethoxyphenyl)-4,4-dimethyl-2-(piperidin-4-yl)-4,5-dihydropyridazin-3(2H)-one (0.62 g; compound B89) and the mixture was stirred for 2 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified by flash column chromatography [amino phase silica gel, eluent: Petrolether/EtOAc, 1/1 (v/v) to give the title compound as a solid.
MS: calc.: $C_{34}H_{46}N_4O_6$ (606.75) found: $[MH^+]$ = 607.1

**B107. 2-{1-[(3R)-3-amino-4-phenylbutanoyl]piperidin-4-yl}-6-(3,4-dimethoxyphenyl)-4,4-dimethyl-4,5-dihydropyridazin-3(2H)-one**

A solution of tert-Butyl [(2R)-4-{4-[3-(3,4-dimethoxyphenyl)-5,5-dimethyl-6-oxo-5,6-dihydropyridazin-1(4H)-yl]piperidin-1-yl}-4-oxo-1-phenylbutan-2-yl]carbamate (0.74 g; compound B106) and trifluoroacetic acid (7.5 ml) in DCM (7.5 ml) was stirred for 2 h at RT. Afterwards saturated aqueous sodium bicarbonate solution was slowly added until the solution was alkanized. The mixture was extracted with DCM, the combined organic layers were dried over magnesium sulphate and all solvents were removed under reduced pressure. The residue was dried under vacuo to give the title compound as a solid.
MS: calc.: $C_{29}H_{38}N_4O_4$ (506.64) found: $[MH^+]$ = 507.2

**B108. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]carbamate**

To a mixture of N-(tert-butoxycarbonyl)-3-(1,3-thiazol-4-yl)-D-alanine (1 g), COMU (1.73 g) and (4aS,8aR)-4-(3,4-dimethoxyphenyl)-2-(piperidin-4-yl)-4a,5,6,7,8,8a-hexahydrophthalazin-1(2H)-one hydrochloride (1.5 g; compound B76) in DCM (20 ml) was added DIPEA (2.56 ml) and the mixture was stirred for 0.5 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified by flash column chromatography [amino phase silica gel, eluent: EtOAc/MeOH, 100/0 to 95/5 (v/v) to give the title compound as a solid.
MS: calc.: $C_{32}H_{43}N_5O_6S$ (625.79) found: $[MH^+]$ = 626.1

**B109. (4aS,8aR)-2-{1-[(2R)-2-amino-3-(1,3-thiazol-4-yl)propanoyl]piperidin-4-yl}-4-(3,4-dimethoxyphenyl)-4a,5,6,7,8,8a-hexahydrophthalazin-1(2H)-one hydrochloride**

tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]carbamate (313 mg; compound B108) was dissolved in a solution of hydrogen chloride in 1,4-dioxane (3 ml, 4.0 M) and the reaction mixture was stirred for about 20 min at RT. Afterwards all volatiles were removed under reduced pressure to give the title compound as a solid.
MS: calc.: $C_{27}H_{35}N_5O_4S$ (525.68) found: $[MH^+]$ = 526.2

**B110. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]carbamate**

To a mixture of N-(tert-butoxycarbonyl)-3-(1,3-thiazol-4-yl)-L-alanine (545 mg), COMU (942 mg) and (4aS,8aR)-4-(3,4-dimethoxyphenyl)-2-(piperidin-4-yl)-4a,5,6,7,8,8a-hexahydrophthalazin-1(2H)-one hydrochloride (816 mg; compound B76) in DCM (15 ml) was added DIPEA (1.4 ml) and the mixture was stirred for 45 min at RT. Afterwards a half-saturated aqueous sodium bicarbonate solution (10 ml) was added and the mixture was extracted several times with DCM. The combined organic phases were dried over magnesium sulphate and the organic layer was concentrated under reduced pressure. The resulting residue was purified by flash column chromatography [amino phase silica gel, eluation gradient: EtOAc/MeOH, 97/3 to 90/10 (v/v) to give the title compound as a solid.
MS: calc.: $C_{32}H_{43}N_5O_6S$ (625.79) found: $[MH^+]$ = 626.1

**B111. (4aS,8aR)-2-{1-[(2S)-2-amino-3-(1,3-thiazol-4-yl)propanoyl]piperidin-4-yl}-4-(3,4-dimethoxyphenyl)-4a,5,6,7,8,8a-hexahydrophthalazin-1(2H)-one hydrochloride**

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]carbamate (1.0 g; compound B110) was dissolved in a solution of hydrogen chloride in 1,4-dioxane (10 ml, 4.0 M) and the reaction mixture was stirred for about 30 min at RT. Afterwards all volatiles were removed under reduced pressure to give the title compound as a solid which was directly used for the next reaction step without further purification.

**B112. (4aS,8aR)-2-{1-[(2S)-2-amino-3-(1H-pyrazol-1-yl)propanoyl]piperidin-4-yl}-4-(3,4-dimethoxyphenyl)-4a,5,6,7,8,8a-hexahydrophthalazin-1(2H)-one hydrochloride**

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1H-pyrazol-1-yl)propan-2-yl]carbamate (4.88 g; compound B113) was dissolved in a solution of hydrogen chloride in 1,4-dioxane (45 ml, 4.0 M) and the reaction mixture was stirred for about 30 min at 0°C. The ice-bath was removed and the mixture was stirred for 3 h at RT. All volatiles were removed under reduced pressure to give the title compound as a solid MS: calc.: $C_{27}H_{36}N_6O_4$ (508.63) found: [$MH^+$] = 509.3

**B113. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1H-pyrazol-1-yl)propan-2-yl]carbamate**

To a mixture of N-(tert-butoxycarbonyl)-3-(1H-pyrazol-1-yl)-L-alanine (3.0 g), HBTU (4.9 g) and (4aS,8aR)-4-(3,4-dimethoxyphenyl)-2-(piperidin-4-yl)-4a,5,6,7,8,8a-hexahydrophthalazin-1(2H)-one hydrochloride (4.8 g; compound B76) in DCM (100 ml) was added DIPEA (8.2 ml) and the mixture was stirred for 45 min at RT. Afterwards a half-saturated aqueous sodium bicarbonate solution (30 ml) was added and the mixture was extracted several times with DCM. The combined organic phases were dried over sodium sulphate and the organic layer was concentrated under reduced pressure. The resulting residue was purified by flash column chromatography [silica gel, eluent: EtOAc/MeOH, 100/0 to 95/5 (v/v) to give the title compound as a solid.
MS: calc.: $C_{32}H_{44}N_6O_6$ (608.74) found: [$MH^+$] = 609.2

**B114. (4aS,8aR)-2-{1-[(2R)-2-amino-3-(1H-imidazol-4-yl)propanoyl]piperidin-4-yl}-4-(3,4-dimethoxyphenyl)-4a,5,6,7,8,8a-hexahydrophthalazin-1(2H)-one trifluoroacetate**

Step1:

To a solution of N-(tert-butoxycarbonyl)-D-histidine (962 mg) in DMF (35 ml) was added TBTU (4.0 g), HOBt x $H_2O$ (1.68 g) and 4-methylmorpholine (1.37 ml) and the mixture was stirred for 5 min at RT. Afterwards (4aS,8aR)-4-(3,4-dimethoxyphenyl)-2-(piperidin-4-yl)-4a,5,6,7,8,8a-hexahydrophthalazin-1(2H)-one hydrochloride (1.54 g; compound B76) was added and the reaction mixture was stirred for 12 h at RT. All volatiles were removed under reduced pressure and the resulting residue was purified by flash column chromatography [silica gel, eluent: DCM/MeOH, 10/1 (v/v) + 2% aqueous solution of ammonium hydroxid) to give tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]carbamate as a solid.

Step2:

A solution of tert-Butyl [(2R)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-(1H-imidazol-4-yl)-1-oxopropan-2-yl]carbamate (1.08 g; step 1) and trifluoroacetic acid (20 ml) in DCM (20 ml) was stirred for 3 h at RT. Afterwards all volatiles were removed under vacuo, the resulting residue was treated with diethyl ether, filtered off and dried under vacuo to give the title compound as a solid.
MS: calc.: $C_{27}H_{36}N_6O_4$ (508.62) found: [$MH^+$] = 509.3

**B115. 9-(3,4-dimethoxyphenyl)-7-(piperidin-4-yl)-7,8-diazaspiro[4.5]dec-8-en-6-one hydrochloride**

Step 1:

Aluminium trichloride (7.8 g) was suspended in DCM (60 ml) under nitrogen atmosphere. A solution of 1,2-dimethoxybenzene (5 ml) in DCM (10 ml) was slowly added at 0°C and subsequently a solution of 2-oxaspiro[4.4]nonane-1,3-dione (2.0 g) in DCM (20 ml) was slowly added to the reaction mixture at 0°C. The reaction mixture was stirred for 12 h at RT. The reaction mixture was poured into water and was extracted with DCM (3x200 ml). The combined organic phase was washed with brine, dried over sodium sulphate and was evaporated under vacuo. The resulting residue was treated with diethyl ether, filtered and dried under vacuo to give 1-[2-(3,4-dimethoxyphenyl)-2 oxoethyl]cyclopentanecarboxylic acid as a solid.

Step 2:

A mixture of 1-[2-(3,4-dimethoxyphenyl)-2-oxoethyl]cyclopentanecarboxylic acid (1 g; step 1), 4-hydrazinylpiperidine dihydrochloride (0.77 g) and triethylamine (3 ml) in ethanol (15 ml) wassubjected to microwave irradiation at 160 °C for 5 h. Afterwards water was added to the reaction mixture at RT and the mixture was extracted with DCM (3x100 ml). The combined organic phase was dried over sodium sulphate and was evaporated under vacuo to give the title compound as a solid.

MS: calc.: $C_{21}H_{29}N_3O_3$ (371.48) found: $[MH^+]$ = 372.4

**B116. (4aS,8aR)-2-{1-[(2S)-2-amino-3-(1H-imidazol-4-yl)propanoyl]piperidin-4-yl}-4-(3,4-dimethoxyphenyl)-4a,5,6,7,8,8a-hexahydrophthalazin-1(2H)-one trifluoroacetate**

Step1:

To a solution of N-(tert-butoxycarbonyl)-L-histidine (962 mg) in DMF (35 ml) was added TBTU (4.0 g), HOBt x $H_2O$ (1.68 g) and 4-methylmorpholine (1.37 ml) and the mixture was stirred for 5 min at RT. Afterwards (4aS,8aR)-4-(3,4-dimethoxyphenyl)-2-(piperidin-4-yl)-4a,5,6,7,8,8a-hexahydrophthalazin-1(2H)-one hydrochloride (1.54 g; compound B76) was added and the reaction mixture was stirred for 12 h at RT. All volatiles were removed under reduced pressure and the resulting residue was purified by flash column chromatography [silica gel, eluent: DCM/MeOH, 10/1 (v/v) + 2% aqueous solution of ammonium hydroxid) to give tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]carbamate as a solid.

Step 2:

A solution of tert-Butyl [(2S)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-(1H-imidazol-4-yl)-1-oxopropan-2-yl]carbamate (1.05 g; step 1) and trifluoroacetic acid (5 ml) in DCM (5 ml) was stirred for 3 h at RT. All volatiles were removed under vacuo, the resulting residue was treated with diethyl ether, filtered off and dried under vacuo to give the title compound as a solid.

MS: calc.: $C_{27}H_{36}N_6O_4$ (508.62) found: $[MH^+]$ = 509.3

**B117. tert-Butyl [(2S)-1-{4-[9-(3,4-dimethoxyphenyl)-6-oxo-7,8-diazaspiro[4.5]dec-8-en-7-yl]piperidin-1-yl}-1-oxo-3-(pyridin-3-yl)propan-2-yl]carbamate**

To a mixture of N-(tert-butoxycarbonyl)-3-pyridin-3-yl-L-alanine (652 mg) and DIPEA (1.6 ml) in DCM (20 ml) was added HBTU (1.03 g) and 9-(3,4-dimethoxyphenyl)-7-(piperidin-4-yl)-7,8-diazaspiro[4.5]-dec-8-en-6-one hydrochloride (1.0 g; compound B115) and the mixture was stirred for 2 h at RT. Afterwards half-saturated aqueous sodium bicarbonate solution was added and the mixture was extracted with DCM. The organic phase was dried over magnesium sulphate and concentrated under reduced pressure. The resulting residue was purified twice by flash column chromatography [first column: amino phase silica gel, eluent: EtOAc/MeOH, 99/1 (v/v); second column: silica gel, eluent: toluene/EtOAc, 70/30 (v/v)] to give the title compound as a solid.

MS: calc.: $C_{34}H_{45}N_5O_6$ (619.75) found: $[MH^+]$ = 620.1

**B118. (4aS,8aR)-2-{1-[(2S)-2-amino-3-(1H-indol-3-yl)propanoyl]piperidin-4-yl}-4-(3,4-dimethoxyphenyl)-4a,5,6,7,8,8a-hexahydrophthalazin-1(2H)-one**

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]carbamate (1.46 g; compound B120)was dissolved in a solution of hydrogen chloride in 1,4-dioxane (20 ml, 4.0 M) and the reaction mixture was stirred for 24 h at RT. Afterwards a saturated aqueous sodium bicarbonate solution was slowly added until the solution was neutralized and the solution was extracted with DCM (300 ml). The organic phase was separated, dried over sodium sulphate and all solvents were removed under reduced pressure. The resulting residue was purified by flash column chromatography [amino phase silica gel, eluent: DCM/EtOH, 100/0 to 90/10 (v/v)] to give the title compound as a solid.

**B119. (4aR,8aS)-2-{1-[(2R)-2-amino-3-(1H-indol-3-yl)propanoyl]piperidin-4-yl}-4-(3,4-dimethoxyphenyl)-4a,5,6,7,8,8a-hexahydrophthalazin-1(2H)-one**

tert-Butyl [(2R)-1-{4-[(4aR,8aS)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]carbamate (1.2 g; compound B121) was dissolved in a solution of hydrogen chloride in 1,4-dioxane (20 ml, 4.0 M) and the reaction mixture was stirred for 12 h at RT. Afterwards a saturated aqueous sodium bicarbonate solution (80 ml) was slowly added until the solution was neutralized and the solution was extracted with DCM (100 ml). The organic phase was separated, dried over sodium sulphate and all solvents were removed under reduced pressure. The resulting residue was purified by flash column chromatography [amino phase silica gel, eluent: DCM/EtOH, 95/5 (v/v)] to give the title compound as a solid.

**B120. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]carbamate**

To a mixture of (4aS,8aR)-4-(3,4-dimethoxyphenyl)-2-(piperidin-4-yl)-4a,5,6,7,8,8a-hexahydro-phthalazin-1(2H)-one hydrochloride (1.0 g; compound B76) and triethylamine (0.34 ml) in DMF (20 ml) was added N-(tert-butoxycarbonyl)-L-tryptophan (746 mg) and HOBt (375 mg). After stirring for 10 min EDC (470 mg) was added and the reaction mixture was stirred for 12 h at RT. Afterwards all volatiles were removed under vacuo and the resulting residue was extracted with DCM (500 ml) and water (200 ml). The organic phase was separated, dried over sodium sulphate and concentrated to dryness. The residue was purified by flash column chromatography [silica gel, eluation gradient: DCM/EtOH, 100/0 to 90/10 (v/v)] to give the title compound as a solid.

**B121. tert-Butyl [(2R)-1-{4-[(4aR,8aS)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]carbamate**

A mixture of N-(tert-butoxycarbonyl)-D-tryptophan (746 mg), (4aS,8aR)-4-(3,4-dimethoxyphenyl)-2-(piperidin-4-yl)-4a,5,6,7,8,8a-hexahydrophthalazin-1(2H)-one hydrochloride (1.0 g; compound B76), EDC (470 mg) and HOBt (375 mg) in DMF (20 ml)was stirred for 12 h at RT under nitrogen atmosphere. Afterwards water was added (40 ml) and the mixture was extracted with DCM (3 x 30 ml). The combined organic layers were dried with sodium sulfate, the organic solvent was evaporated under vacuo and the resulting residue was purified by flash column chromatography [silica gel, eluent: DCM/EtOH, 95/5 (v/v)] to give the title compound as a solid.

**B122. (4aS,8aR)-2-{1-[(2R)-2-amino-3-(1-methyl-1H-indol-3-yl)propanoyl]piperidin-4-yl}-4-(3,4-dimethoxyphenyl)-4a,5,6,7,8,8a-hexahydrophthalazin-1(2H)-one**

To a solution of tert-Butyl [(2R)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-(1-methyl-1H-indol-3-yl)-1-oxopropan-2-yl]carbamate (1.53 g; compound B123) in DCM (15 ml) was added trifluoroacetic acid (15 ml) and the reaction mixture was stirred for 2 h at RT. Afterwards all volatiles were removed under vacuo; the resulting residue was treated with diethyl ether and filtered off. The obtained solid was purified by flash column chromatography [silica gel, eluent: DCM/MeOH, 95/5 (v/v) + 1% aqueous solution of ammonium hydroxid) to give the title compound as a solid.
MS: calc.: $C_{33}H_{41}N_5O_4$ (571.72) found: $[MH^+]$ = 572.3

**B123. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1-methyl-1H-indol-3-yl)-1-oxopropan-2-yl]carbamate**

Step 1:

To a solution of 1-methyl-D-tryptophan (2.0 g) in ethanol (29 ml) was added triethylamine (1.53 ml) and di-tert-butyl dicarbonate (2.4 g) and the reaction mixture was stirred for 3 d at RT. The solvent was removed under vacuo and the resulting residue was treated with DCM and extracted with 10 % aqueous solution of citric acid. The aqueous phase was washed with DCM and the combined organic phase was dried over sodium sulphate, filtered and concentrated under vacuo to give N-(tert-butoxycarbonyl)-1-methyl-D-tryptophan as a solid.

Step 2:

N-(tert-butoxycarbonyl)-1-methyl-D-tryptophan (1.5 g; step 1) was dissolved in DCM (37 ml) at 0°C and then DIPCDI (683 mg), HOBt x H2O (837 mg) and 4-methylmorpholine (837 mg) were added. After stirring for 5 min (4aS,8aR)-4-(3,4-dimethoxyphenyl)-2-(piperidin-4-yl)-4a,5,6,7,8,8a-hexahydro-phthalazin-1(2H)-one hydrochloride (2.02 g; compound B76) was added and the reaction mixture was stirred for 3 d at RT. All volatiles were removed under vacuo and the resulting residue was purified by flash column chromatography [silica gel, eluent: DCM + 1% aqueous solution of ammonium hydroxid] to give the title compound as a solid.

**B124. tert-Butyl [(2R)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-4-methyl-1-oxopentan-2-yl]carbamate**

To a mixture of N-(tert-butoxycarbonyl)-D-leucine (580 mg), HBTU (1.04 g) and (4aS,8aR)-4-(3,4-dimethoxyphenyl)-2-(piperidin-4-yl)-4a,5,6,7,8,8a-hexahydrophthalazin-1(2H)-one hydrochloride (1.02 g; compound B76) in DCM (25 ml) was added DIPEA (1.75 ml) and the mixture was stirred for 30 min at RT. Afterwards a half-saturated aqueous sodium bicarbonate solution (10 ml) was added and the mixture was extracted several times with DCM. The combined organic phases were dried oversodium sulphate and the organic layer was concentrated under reduced pressure. The resulting residue was purified by flash column chromatography [silica gel, eluent: EtOAc] to give the title compound as a solid.

MS: calc.: $C_{32}H_{48}N_4O_6$ (584.76) found: [$MH^+$] = 585.1

**B125. (4aS,8aR)-2-{1-[(2R)-2-amino-4-methylpentanoyl]piperidin-4-yl}-4-(3,4-dimethoxyphenyl)-4a,5,6,7,8,8a-hexahydrophthalazin-1(2H)-one**

tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-methyl-1-oxopentan-2-yl]carbamate (760 mg; compound B124) was dissolved in a solution of hydrogen chloride in 1,4-dioxane (6 ml, 4.0 M) and the reaction mixture was stirred for 30 min at RT. All volatiles were removed under vacuo to give the title compound as a solid.

MS: calc.: $C_{27}H_{40}N_4O_4$ (484.64) found: [$MH^+$] = 485.2

**B126. (4aS,8aR)-2-{1-[(2R)-2-amino-3-(4-bromophenyl)propanoyl]piperidin-4-yl}-4-(3,4-dimethoxyphenyl)-4a,5,6,7,8,8a-hexahydrophthalazin-1(2H)-one**

To a solution of tert-Butyl [(2R)-3-(4-bromophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate (2.26 g; compound B127) in DCM (15 ml) was added trifluoroacetic acid (15 ml) and the reaction mixture was stirred for 3 h at RT. The mixture was slowly poured in a saturated aqueous sodium bicarbonate solution. After extraction with DCM, the combined organic layers were dried over magnesium sulphate and all solvents were removed under reduced pressure. The residue was dried under vacuo to give the title compound as a solid.

MS: calc.: $C_{30}H_{37}BrN_4O_4$ (597.54) found: [$MH^+$] = 599.1

**B127. tert-Butyl [(2R)-3-(4-bromophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]carbamate**

To a mixture of 4-bromo-N-(tert-butoxycarbonyl)-D-phenylalanine (1.5 g) and DIPEA (2.85 ml) in DCM (30 ml) was added COMU (2.1 g) and (4aS,8aR)-4-(3,4-dimethoxyphenyl)-2-(piperidin-4-yl)-4a,5,6,7,8,8a-hexahydrophthalazin-1(2H)-one hydrochloride (1.78 g; compound B76) and the mixture was stirred for 3 h at RT. Afterwards half-saturated aqueous sodium bicarbonate solution was added and the mixture was extracted with DCM. The combined organic phases were dried over magnesium sulphate and the organic layer was concentrated under reduced pressure. The resulting residue was purified by flash column chromatography [silica gel, eluent: Petrolether/EtOAc, 6/4 (v/v)] to give the title compound as a solid.

MS: calc.: $C_{35}H_{45}BrN_4O_6$ (697.66) found: [$MH^+$] = 696.9

**B128. (2S)-2-amino-1-{4-[(4aS,8aR)-4-(3,4-dimethoxyphenyl)-1-oxo-4a,5,6,7,8,8a-hexahydrophthalazin-2(1H)-yl]piperidin-1-yl}-5-(piperidin-1-yl)pentane-1,5-dione hydrochloride**

tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]carbamate (839 mg; compound B129) was dissolved in THF (13 ml) and an aqueous solution of hydrogen chloride (9.6 ml, 2.0 M) was added. The reaction mixture was stirred for 1.5 h at 60 °C. The volatiles were removed under vacuo to give the title compound as a solid.

MS: calc.: $C_{31}H_{45}N_5O_5$ (567.72) found: [$MH^+$] = 568.3

**B129. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]carbamate**

To a stirred mixture of (2S)-2-[(tert-butoxycarbonyl)amino]-5-oxo-5-(piperidin-1-yl)pentanoic acid (0.45 g) and DIPEA (0.93 ml) in DCM (30 ml) was added HATU (0.65 g). After 20 min (4aS,8aR)-4-(3,4-dimethoxyphenyl)-2-(piperidin-4-yl)-4a,5,6,7,8,8a-hexahydrophthalazin-1(2H)-one hydrochloride (0.53 g; compound B76) was added and the reaction mixture was stirred for 12 h at RT. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography to give the title compound as a solid.

MS: calc.: $C_{36}H_{53}N_5O_7$ (667.84) found: [$MH^+$] = 668.2

**B130. (2S)-2-[(tert-butoxycarbonyl)amino]-5-oxo-5-(piperidin-1-yl)pentanoic acid**

To a solution of benzyl (2S)-2-[(tert-butoxycarbonyl)amino]-5-oxo-5-(piperidin-1-yl)pentanoate in MeOH (40 ml) was added Pd/C (10%) (80 mg) and the reaction mixture was stirred for 2 h under a hydrogen atmosphere. The suspension was filtered off and the filtrate was evaporated to dryness under vacuo to give the title compound as a solid.

MS: calc.: $C_{15}H_{26}N_2O_5$ (314.38) found: [$MH^+$] = 315.0

**B131. benzyl (2S)-2-[(tert-butoxycarbonyl)amino]-5-oxo-5-(piperidin-1-yl)pentanoate**

To a stirred solution of DIPEA (1.94 ml) in DCM (35 ml) was added N-tert-Butoxycarbonyl-L-glutamic acid 2-benzyl ester (1 g) and HATU (1.35 g). After 30 min piperidine (0.59 ml) was added the reaction mixture was stirred at RT for 12 h. Afterwards a half-saturated aqueous sodium bicarbonate solution was added and the mixture was extracted twice with DCM. The combined organic phases were dried over magnesium sulphate and the organic layer was concentrated under reduced pressure. The resulting residue was purified by flash column chromatography [silica gel, eluent: Petrolether/EtOAc, 6/4 (v/v)] to give the title compound as a solid.

MS: calc.: $C_{22}H_{32}N_2O_5$ (404.50) found: [$MH^+$] = 405.0; [$MH^+$ - Boc] = 305.1

**B132. Benzyl (4S)-4-[(tert-butoxycarbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate**

To a mixture of N-tert-Butoxycarbonyl-L-glutamic acid 5-benzyl ester (3.37 g), COMU (4.28 g) and (4aS,8aR)-4-(3,4-dimethoxyphenyl)-2-(piperidin-4-yl)-4a,5,6,7,8,8a-hexahydrophthalazin-1(2H)-one hydrochloride (4.08 g; compound B76) in DCM (50 ml) was added DIPEA (6.8 ml) and the mixture was stirred for 1 h at RT. Afterwards a saturated aqueous sodium bicarbonate solution (50 ml) was added and the mixture was extracted with DCM (150 ml). The combined organic phases were dried over sodium sulphate and the organic layer was concentrated under reduced pressure. The resulting residue was purified twice by flash column chromatography [first column: silica gel, eluation gradient: Cyclo-hexane/EtOAc, 100/0 to 60/40 (v/v); second column: amino phase silica gel, eluation gradient Cyclo-hexane/EtOAc, 100/0 to 60/40 (v/v)] to give the title compound as a solid.

MS: calc.: $C_{38}H_{50}N_4O_8$ (690.82) found: [$MH^+$] = 691.1

**B133. Benzyl (4S)-4-amino-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate**

Benzyl (4S)-4-[(tert-butoxycarbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate (2.69 g; compound B132) was dissolved in a solution of hydrogen chloride in 1,4-dioxane (19.5 ml, 4.0 M) and the reaction mixture was stirred for 2.5 h at RT. The mixture was partitioned between an aqueous solution of sodium hydroxide (2M), a saturated aqueous sodium bicarbonate solution (pH level of 8) and DCM (70 ml). The organic phase was separated, concentrated to dryness under vacuo and co-evaporated with DCM to give the title compound as a solid.

MS: calc.: $C_{33}H_{42}N_4O_6$ (590.71) found: [$MH^+$] = 591.2

**B134. 2-{1-[(2R)-2-amino-3-(4-fluorophenyl)propanoyl]piperidin-4-yl}-6-(3,4-dimethoxyphenyl)-4,4-dimethyl-4,5-dihydropyridazin-3(2H)-one**

tert-Butyl [(2R)-1-{4-[3-(3,4-dimethoxyphenyl)-5,5-dimethyl-6-oxo-5,6-dihydropyridazin-1(4H)-yl]-piperidin-1-yl}-3-(4-fluorophenyl)-1-oxopropan-2-yl]carbamate (1.05 g; compound B135) was dissolved in a solution of hydrogen

chloride in 1,4-dioxane (6.4 ml, 4.0 M) and the reaction mixture was stirred for 1.5 h at RT. The mixture was partitioned between an aqueous solution of sodium hydroxide (6M), a saturated aqueous sodium bicarbonate solution and DCM. The organic phase was separated, concentrated to dryness and the resulting residue was purified by flash column chromatography [amino phase silica gel, eluation gradient: Cyclohexane to EtOAc/MeOH/DCM, 65/20/15 (v/v/v)] to give the title compound as a solid.

MS: calc.: $C_{28}H_{35}FN_4O_4$ (510.60) found: [$MH^+$] = 511.4

**B135. tert-Butyl [(2R)-1-{4-[3-(3,4-dimethoxyphenyl)-5,5-dimethyl-6-oxo-5,6-dihydropyridazin-1(4H)-yl]piperidin-1-yl}-3-(4-fluorophenyl)-1-oxopropan-2-yl]carbamate**

A mixture of N-(tert-butoxycarbonyl)-4-fluoro-D-phenylalanine (666 mg), DIPEA (1.60 ml), COMU (2.01 g) and 6-(3,4-dimethoxyphenyl)-4,4-dimethyl-2-(piperidin-4-yl)-4,5-dihydropyridazin-3(2H)-one (0.81 g; compound B89) in DCM (12 ml) was stirred for 45 min at RT. Afterwards the mixture was partitioned between a saturated aqueous sodium bicarbonate solution (20 ml) and DCM (25 ml), the phases were separated and the organic phase was dried over sodium sulphate. The organic solvent was removed under vacuo and the resulting residue was purified twice by flash column chromatography [first column: amino phase silica gel, eluation gradient: Cyclohexane/EtOAc, 100/0 to 80/20 to 70/30 to 60/40 to 50/50 (v/v) ; second column: silica gel, eluation gradient: Cyclohexane/EtOAc/DCM, 100/0/0 to 90/0/10 to 60/30/10 to 40/50/10 (v/v/v)] to give the title compound as a solid.

MS: calc.: $C_{33}H_{43}FN_4O_6$ (610.72) found: [$MH^+$] = 611.1; [$MH^+$ - Boc] = 511.3

**B136. (4aS,8aR)-2-{1-[(2R)-2-amino-3-(pyridin-4-yl)propanoyl]piperidin-4-yl}-4-(3,4-dimethoxyphenyl)-4a,5,6,7,8,8a-hexahydrophthalazin-1(2H)-one**

tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]carbamate (980 mg; compound B137) was dissolved in a solution of hydrogen chloride in 1,4-dioxane (5.9 ml, 4.0 M) and the reaction mixture was stirred for 1.5 h at RT. The mixture was partitioned between an aqueous solution of sodium hydroxide (6M), a saturated aqueous sodium bicarbonate solution and DCM. The organic phase was separated, concentrated to dryness and the resulting residue was purified by flash column chromatography [amino phase silica gel, eluation gradient: Cyclohexane to EtOAc/MeOH/DCM, 65/20/15 (v/v/v)] to give the title compound as a solid.

MS: calc.: $C_{29}H_{37}N_5O_4$ (519.64) found: [$MH^+$] = 520.4

**B137. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]carbamate**

To a mixture of N-(tert-butoxycarbonyl)-3-pyridin-4-yl-D-alanine (666 mg), COMU (2.14 g) and (4aS,8aR)-4-(3,4-dimethoxyphenyl)-2-(piperidin-4-yl)-4a,5,6,7,8,8a-hexahydrophthalazin-1(2H)-one hydrochloride (1.02 g; compound B76) in DCM (12 ml) was added DIPEA (1.70 ml) and the mixture was stirred for 45 min at RT. Afterwards the mixture was partitioned between a saturated aqueous sodium bicarbonate solution (20 ml) and DCM (25 ml), the phases were separated and the organic phase was dried over sodium sulphate. The organic solvent was removed under vacuo and the resulting residue was purified twice by flash column chromatography [first column: amino phase silica gel, eluation gradient: Cyclohexane/EtOAc, 100/0 to 85/15 to 70/30 to 50/50 (v/v) ; second column: silica gel, eluation gradient: Cyclohexane/EtOAc, 100/0 to 70/30 to 100/100 to 10/90 to 0/100 (v/v)] to give the title compound as a solid.

MS: calc.: $C_{34}H_{45}N_5O_6$ (619.75) found: [$MH^+$] = 620.2

**B138. tert-Butyl [(2S)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-(pyridin-3-yl)propan-2-yl]carbamate**

To a stirred mixture of N-(tert-butoxycarbonyl)-3-pyridin-3-yl-L-alanine (294 mg) and DIPEA (0.72 ml) in DCM (10 ml) was added COMU (520 mg) and 6-(7-methoxy-2,2-dimethyl-2,3-dihydro-1-benzofuran-4-yl)-4,4-dimethyl-2-(piperidin-4-yl)-4,5-dihydropyridazin-3(2H)-one hydrochloride (465 mg). The mixture was stirred for 3 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified by flash column chromatography [amino phase silica gel, eluent: EtOAc/MeOH, 99/1 (v/v)] to give the title compound as a solid.

MS: calc.: $C_{35}H_{47}N_5O_6$ (633.78) found: [$MH^+$] = 634.2

**B139. 2-{1-[(2S)-2-amino-3-(pyridin-3-yl)propanoyl]piperidin-4-yl}-6-(7-methoxy-2,2-dimethyl-2,3-dihydro-1-benzofuran-4-yl)-4,4-dimethyl-4,5-dihydropyridazin-3(2H)-one**

To a solution of tert-Butyl [(2S)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-(pyridin-3-yl)propan-2-yl]carbamate (0.64 g; compound B138) in DCM (6 ml) was added trifluoroacetic acid (6 ml) and the reaction mixture was stirred for 1 h at RT. The mixture was slowly poured in a saturated aqueous sodium bicarbonate solution. After extraction with DCM, the combined organic layers were dried over magnesium sulphate and all solvents were removed under reduced pressure. After lyophilisation from acetonitrile / water the title compound was obtained as a solid.

MS: calc.: $C_{30}H_{39}N_5O_4$ (533.66) found: $[MH^+]$ = 534.3

**B140. tert-Butyl [(2R)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-phenylpropan-2-yl]carbamate**

To a stirred mixture of N-(tert-butoxycarbonyl)-D-phenylalanine (876 mg) and DIPEA (1.53 ml) in DCM (15 ml) was added HBTU (1.42 g) and 6-(7-methoxy-2,2-dimethyl-2,3-dihydro-1-benzofuran-4-yl)-4,4-dimethyl-2-(piperidin-4-yl)-4,5-dihydropyridazin-3(2H)-one hydrochloride (1.27 g). The mixture was stirred for 1 h at RT. Afterwards the mixture was extracted with a saturated aqueous sodium bicarbonate solution (3 x 5 ml), the phases were separated and the organic phase was dried over sodium sulphate. The organic solvent was removed under vacuo and the resulting residue was purified twice by flash column chromatography [amino phase silica gel, eluent: EtOAc] to give the title compound as a solid.

MS: calc.: $C_{36}H_{48}N_4O_6$ (632.79) found: $[MH^+]$ = 633.0; $[MH^+$ - Boc] = 533.2

**B141. 2-{1-[(2R)-2-amino-3-phenylpropanoyl]piperidin-4-yl}-6-(7-methoxy-2,2-dimethyl-2,3-dihydro-1-benzofuran-4-yl)-4,4-dimethyl-4,5-dihydropyridazin-3(2H)-one**

tert-Butyl [(2R)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-phenylpropan-2-yl]carbamate (2.14 g; compound B140) was dissolved in a solution of hydrogen chloride in 1,4-dioxane (12.7 ml, 4.0 M) and the reaction mixture was stirred for about 1 h at RT. DCM (20 ml) was added and the mixture was sequentially extracted with an aqueous hydrochloride acid solution (2M) and with sodium hydroxide (15 ml, 1 M). The organic phase was evaporated under vacuo and the resulting residue was purified by flash column chromatography [amino phase silica gel, eluation gradient: Cyclohexane/EtOAc/MeOH, 100/0/0 to 0/100/0 to 0/90/10 (v/v/v)] to give the title compound as a solid.

MS: calc.: $C_{31}H_{40}N_4O_4$ (532.67) found: $[MH^+]$ = 533.2

**B142. 2-{1-[(2R)-2-amino-3-phenylpropanoyl]piperidin-4-yl}-6-(3,4-dimethoxyphenyl)-4,4-diethyl-4,5-dihydropyridazin-3(2H)-one**

tert-Butyl [(2R)-1-{4-[3-(3,4-dimethoxyphenyl)-5,5-diethyl-6-oxo-5,6-dihydropyridazin-1(4H)-yl]piperidin-1-yl}-1-oxo-3-phenylpropan-2-yl]carbamate (352 mg; compound B143) was dissolved in a solution of hydrogen chloride in 1,4-dioxane (2.1 ml, 4.0 M) and the reaction mixture was stirred for about 1 h at RT. DCM (20 ml) was added and the mixture was sequentially extracted with an aqueous hydrochloric acid solution (2M) and with sodium hydroxide (10 ml, 1M). The organic phase was separated,evaporated under vacuo and the resulting residue was purified by flash column chromatography [amino phase silica gel, eluation gradient: Cyclohexane/EtOAc/MeOH, 100/0/0 to 0/100/0 to 0/90/10 (v/v/v)] to give the title compound as a solid.

MS: calc.: $C_{30}H_{40}N_4O_4$ (520.66) found: $[MH^+]$ = 521.2

**B143. tert-Butyl [(2R)-1-{4-[3-(3,4-dimethoxyphenyl)-5,5-diethyl-6-oxo-5,6-dihydropyridazin-1(4H)-yl]piperidin-1-yl}-1-oxo-3-phenylpropan-2-yl]carbamate**

To a stirred mixture of N-(tert-butoxycarbonyl)-D-phenylalanine (566 mg) and DIPEA (0.70 ml) in DCM (10 ml) was added HBTU (656 mg) and 6-(3,4-dimethoxyphenyl)-4,4-diethyl-2-(piperidin-4-yl)-4,5-dihydropyridazin-3(2H)-one hydrochloride. The mixture was stirred for 1 h at RT. Afterwards the mixture was extracted with a saturated aqueous sodium bicarbonate solution (3 x 5 ml), the phases were separated and the organic phase was dried over sodium sulphate. The organic solvent was removed under vacuo and the resulting residue was purified twice by flash column chromatography [amino phase silica gel, eluent: EtOAc] to give the title compound as a solid.

MS: calc.: $C_{35}H_{48}N_4O_6$ (620.78) found: $[MH^+]$ = 621.0; $[MH^+$ - Boc] = 521.2

**B144. (4aS,8aR)-2-{1-[(2R)-2-amino-3-(dimethylamino)propanoyl]piperidin-4-yl}-4-(3,4-dimethoxyphenyl)-4a,5,6,7,8,8a-hexahydrophthalazin-1(2H)-one trifluoroacetate**

A solution of tert-Butyl [(2R)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-(dimethylamino)-1-oxopropan-2-yl]carbamate (1.25 g; compound B145) and trifluoroacetic acid (10 ml) in DCM (10 ml) was stirred for 0.5 h at RT. Afterwards all volatiles were removed under vacuo, the resulting residue was treated with diethyl ether, filtered off and dried under vacuo to give the title compound as a solid.
MS: calc.: $C_{26}H_{39}N_5O_4$ (485.63) found: $[MH^+]$ = 486.4

**B145. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]carbamate**

To a suspension of (4aS,8aR)-4-(3,4-dimethoxyphenyl)-2-(piperidin-4-yl)-4a,5,6,7,8,8a-hexa-hydrophthalazin-1(2H)-one hydrochloride (3.97 g; compound B76), N-(tert-butoxycarbonyl)-3-(dimethylamino)-D-alanine (2.26 g) and HATU (4.07 g) in DCM (45 ml) was added DIPEA (4.96 ml) and the reaction mixture was stirred for 45 min at RT. Afterwards DCM (150 ml) was added and the mixture was sequentially extracted with a saturated aqueous sodium bicarbonate solution (50 ml) and water (50 ml), the phases were separated and the organic phase was dried over sodium sulphate. The organic solvent was removed under vacuo and the resulting residue was purified by flash column chromatography [amino phase silica gel, eluation gradient: Cyclohexane to EtOAc] to give the title compound as a solid.
MS: calc.: $C_{31}H_{47}N_5O_6$ (585.73) found: $[MH^+]$ = 586.2

**B146. N-(tert-butoxycarbonyl)-3-(1H-pyrazol-1-yl)-L-alanine**

To a solution of 3-(1H-pyrazo-1-yl)-L-alanine (1.77 g) in MeOH (40 ml) was added di-tert-butyl dicarbonate (2.98 g) and TEA (1.38 g) and the reaction mixture was stirred for 3 d at RT. All solvents were removed under vacuo and the resulting residue was treated with DCM and washed with citric acid solution. The aqueous layer was extracted with DCM (2x) and the combined organic phases were dried over sodium sulphate. After filtration and evaporation of the solvent under vacuo the title compound was obtained as a solid.
MS: calc.: $C_{11}H_{17}N_3O_4$ (255.27) found: $[MH^+]$ = 255.9

**B147. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1H-pyrazol-1-yl)propan-2-yl]carbamate**

To a suspension of (4aS,8aR)-4-(3,4-dimethoxyphenyl)-2-(piperidin-4-yl)-4a,5,6,7,8,8a-hexa-hydrophthalazin-1(2H)-one hydrochloride (4.80 g; compound B76), N-(tert-butoxycarbonyl)-3-(1H-pyrazol-1-yl)-L-alanine (3.0 g, compound B146) and HBTU (4.9 g) in DCM (100 ml) was added DIPEA (8.2 ml) and the reaction mixture was stirred for 45 min at RT. The mixture was extracted with a saturated aqueous sodium bicarbonate solution (30 ml), the phases were separated and the organic phase was dried over sodium sulphate. The organic solvent was removed under vacuo and the resulting residue was purified by flash column chromatography [silica gel, eluation gradient: EtOAc/MeOH 100/0 to 95/5 (v/v)] to give the title compound as a solid.
MS: calc.: $C_{32}H_{44}N_6O_6$ (608.74) found: $[MH^+]$ = 609.2

**B148. (4aS,8aR)-2-{1-[(2S)-2-amino-3-(1H-pyrazol-1-yl)propanoyl]piperidin-4-yl}-4-(3,4-dimethoxyphenyl)-4a,5,6,7,8,8a-hexahydrophthalazin-1(2H)-one**

A solution of tert-Butyl [(2S)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-1-oxo-3-(1H-pyrazol-1-yl)propan-2-yl]carbamate (2.36 g; compound B147) and trifluoroacetic acid (15 ml) in DCM (20 ml) was stirred for 3 h at RT. Afterwards all volatiles were removed under vacuo, the resulting residue was treated with diethyl ether, filtered off and dried under vacuo to give the title compound as a solid.
MS: calc.: $C_{27}H_{36}N_6O_4$ (508.62) found: $[MH^+]$ = 509.3

**B149. 2-[1-(aminoacetyl)piperidin-4-yl]-6-(7-methoxy-2,2-dimethyl-2,3-dihydro-1-benzofuran-4-yl)-4,4-dimethyl-4,5-dihydropyridazin-3(2H)-one hydrochloride**

To a solution of tert-Butyl (2-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-2-oxoethyl)carbamate (434 mg; compound B150) in THF (3.9 ml) was added an aqueous solution of hydrogen chloride (2M, 6.25 ml) and the reaction mixture was stirred for 1 h at 50 °C and subsequently for 12 h at RT. All volatiles were removed under reduced pressure to give the title compound as a solid.

MS: calc.: $C_{24}H_{34}N_4O_4$ (442.55) found: [$MH^+$] = 443.3; [$MNa^+$] = 465.3

**B150. tert-Butyl (2-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-2-oxoethyl)carbamate**

To a solution of 6-(7-methoxy-2,2-dimethyl-2,3-dihydro-1-benzofuran-4-yl)-4,4-dimethyl-2-(piperidin-4-yl)-4,5-dihydropyridazin-3(2H)-one hydrochloride (391 mg) in DCM (10 ml) was added N-(tert-butoxycarbonyl)glycine (178 mg), HBTU (423 mg) and DIPEA (0.69 ml) and the reaction mixture was stirred for 2 h at RT. Afterwards a half-saturated aqueous sodium bicarbonate solution was added and the mixture was extracted several times with DCM. The combined organic phases were dried over magnesium sulphate and the organic layer was concentrated under reduced pressure. The resulting residue was purified by flash column chromatography [silica gel, eluent: Toluene/EtOAc, 9/1 (v/v)] to give the title compound as a solid.

MS: calc.: $C_{29}H_{42}N_4O_6$ (542.68) found: [$MH^+$] = 543.0; [$MNa^+$] = 565.2; [$MH^+$- Boc] = 443.3

**B151. 2-{1-[(2R)-2-aminopropanoyl]piperidin-4-yl}-6-(3,4-dimethoxyphenyl)-4,4-dimethyl-4,5-dihydropyridazin-3(2H)-one**

tert-Butyl [(2R)-1-{4-[3-(3,4-dimethoxyphenyl)-5,5-dimethyl-6-oxo-5,6-dihydropyridazin-1(4H)-yl]-piperidin-1-yl}-1-oxopropan-2-yl]carbamate(1.24 g; compound B152) was dissolved in a solution of hydrogen chloride in 1,4-dioxane (9 ml, 4.0 M) and the reaction mixture was stirred for about 1.5 h at RT. The mixture was partitioned between an aqueous solution of sodium hydroxide (6M), a saturated aqueous sodium bicarbonate solution and DCM. The organic phase was separated, concentrated to dryness under vacuo. The resulting residue was purified by flash column chromatography [amino phase silica gel, eluation gradient: Cyclohexane to EtOAc/MeOH/DCM, 65/20/15 (v/v/v)] to give the title compound as a solid.

MS: calc.: $C_{22}H_{32}N_4O_4$ (416.51) found: [$MH^+$] = 417.2

**B152. tert-Butyl[(2R)-1-{4-[3-(3,4-dimethoxyphenyl)-5,5-dimethyl-6-oxo-5,6-dihydropyridazin-1(4H)-yl]piperidin-1-yl}-1-oxopropan-2-yl]carbamate**

To a mixture of N-(tert-butoxycarbonyl)-D-alanine (445 mg) and DIPEA (1.60 ml) in DCM (12 ml) was added COMU (2.01 g) and 6-(3,4-dimethoxyphenyl)-4,4-dimethyl-2-(piperidin-4-yl)-4,5-dihydro-pyridazin-3(2H)-one (812 mg; compound B89) and the mixture was stirred for 45 min at RT. Afterwards saturated aqueous sodium bicarbonate solution (20 ml) was added and the mixture was extracted with DCM (25 ml). The combined organic phases were dried over magnesium sulphate and the organic layer was concentrated under reduced pressure. The resulting residue was purified twice by flash column chromatography [first column: amino phase silica gel, eluation gradient: Cyclohexane/EtOAc, 100/0 to 85/15 to 60/40 to 50/50 (v/v); second column: Cyclohexane/EtOAc/DCM/MeOH, 100/0/0/0 to 90/0/10/0 to 60/30/10/0 to 40/50/10/0 (v/v/v/v)] to give the title compound as a solid.

MS: calc.: $C_{27}H_{40}N_4O_6$ (516.63) found: [$MH^+$] = 517.1

**B153. 2-{1-[(2S)-2-amino-3-phenylpropanoyl]piperidin-4-yl}-6-(3,4-dimethoxyphenyl)-4,4-dimethyl-4,5-dihydropyridazin-3(2H)-one**

tert-Butyl[(2S)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]carbamate(940 mg; compound B154) was dissolved in a solution of hydrogen chloride in 1,4-dioxane (5.95 ml, 4.0 M) and the reaction mixture was stirred for about 1.5 h at RT. The mixture was partitioned between an aqueous solution of sodium hydroxide (6M), a saturated aqueous sodium bicarbonate solution and DCM. The organic phase was separated, concentrated to dryness under vacuo. The resulting residue was purified by flash column chromatography [amino phase silica gel, eluation gradient: Cyclohexane to EtOAc/MeOH/DCM, 65/20/15 (v/v/v)] to give the title compound as a solid.

MS: calc.: $C_{28}H_{36}N_4O_4$ (492.61) found: [$MH^+$] = 493.5

**B154. tert-Butyl[(2S)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]carbamate**

To a mixture of N-(tert-butoxycarbonyl)-L-phenylalanine (624 mg) and DIPEA (1.60 ml) in DCM (12 ml) was added COMU (2.01 g) and 6-(3,4-dimethoxyphenyl)-4,4-dimethyl-2-(piperidin-4-yl)-4,5-dihydro-pyridazin-3(2H)-one (812 mg; compound B89) and the mixture was stirred for 45 min at RT. Afterwards saturated aqueous sodium bicarbonate solution (20 ml) was added and the mixture was extracted with DCM (25 ml). The combined organic phases were dried

over magnesium sulphate and the organic layer was concentrated under reduced pressure. The resulting residue was purified twice by flash column chromatography [first column: amino phase silica gel, eluation gradient: Cyclohexane/EtOAc, 100/0 to 85/15 to 60/40 to 50/50 (v/v); second column: Cyclohexane/EtOAc/DCM, 100/0/0 to 90/0/10 to 60/30/10 to 40/50/10 (v/v/v)] to give the title compound as a solid.

MS: calc.: $C_{33}H_{44}N_4O_6$ (592.72) found: [$MH^+$] = 593.1; [$MNa^+$] = 616.3; [$MH^+$- Boc] = 493.3

**B155. Benzyl (4R)-4-amino-5-{4-[(4aS,8aR)-4-(3,4-dimethoxyphenyl)-1-oxo-4a,5,6, 7,8,8a-hexahydrophthalazin-2(1H)-yl]piperidin-1-yl}-5-oxopentanoate**

A solution of Benzyl (4R)-4-[(tert-butoxycarbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate (1.9 g; compound B156) and trifluoroacetic acid (19 ml) in DCM (19 ml) was stirred for 2.5 h at RT. Afterwards a saturated aqueous sodium bicarbonate solution was slowly added until the solution was alkanized. The mixture was extracted with DCM, the combined organic layers were dried over magnesium sulphate and all solvents were removed under reduced pressure. The residue was dried under vacuo to give the title compound as a solid.

MS: calc.: $C_{33}H_{42}N_4O_6$ (590.71) found: [$MH^+$] = 591.2

**B156. Benzyl (4R)-4-[(tert-butoxycarbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate**

To a mixture of N-tert-Butoxycarbonyl-D-glutamic acid 5-benzyl ester (1.5 g) and DIPEA (2.91 ml) in DCM (30 ml) was added COMU (2.1 g) and (4aS,8aR)-4-(3,4-dimethoxyphenyl)-2-(piperidin-4-yl)-4a,5,6,7,8,8a-hexahydrophthalazin-1(2H)-one hydrochloride (1.81 g; compound B76) and the mixture was stirred for 1.5 h at RT. Afterwards half-saturated aqueous sodium bicarbonate solution was added and the mixture was extracted with DCM. The combined organic phases were dried over magnesium sulphate and the organic layer was concentrated under reduced pressure. The resulting residue was purified by flash column chromatography [silica gel, eluent: Toluene/EtOAc, 85/15 (v/v)] to give the title compound as a solid.

MS: calc.: $C_{38}H_{50}N_4O_8$ (690.82) found: [$MH^+$] = 691.1

**B157. 2-{1-[(2S)-2-amino-3-cyclohexylpropanoyl]piperidin-4-yl}-6-(3,4-dimethoxyphenyl)-4,4-dimethyl-4,5-dihydropyridazin-3(2H)-one**

A solution of tert-Butyl [(2S)-3-cyclohexyl-1-{4-[3-(3,4-dimethoxyphenyl)-5,5-dimethyl-6-oxo-5,6-di-hydropyridazin-1(4H)-yl]piperidin-1-yl}-1-oxopropan-2-yl]carbamate (651 mg; compound B158) and trifluoroacetic acid (6.5 ml) in DCM (6.5 ml) was stirred for 2 h at RT. Afterwards a saturated aqueous sodium bicarbonate solution was slowly added until the solution was alkanized. The mixture was extracted with DCM, the combined organic layers were dried over magnesium sulphate and all solvents were removed under reduced pressure. The residue was dried under vacuo to give the title compound as a solid.

MS: calc.: $C_{28}H_{42}N_4O_4$ (498.66) found: [$MH^+$] = 499.2

**B158. tert-Butyl [(2S)-3-cyclohexyl-1-(4-[3-(3,4-dimethoxyphenyl)-5,5-dimethyl-6-oxo-5,6-dihydropyridazin-1 (4H)-yl]piperidin-1-yl}-1-oxopropan-2-yl]carbamate**

To a mixture of N-(tert-butoxycarbonyl)-3-cyclohexyl-L-alanine x $H_2O$ (450 mg) and DIPEA (1.02 ml) in DCM (15 ml) was added COMU (733 mg) and 6-(3,4-dimethoxyphenyl)-4,4-dimethyl-2-(piperidin-4-yl)-4,5-dihydropyridazin-3(2H)-one (537 mg; compound B89) and the mixture was stirred for 3 h at RT. Afterwards half-saturated aqueous sodium bicarbonate solution was added and the mixture was extracted with DCM. The combined organic phases were dried over magnesium sulphate and the organic layer was concentrated under reduced pressure. The resulting residue was purified by flash column chromatography [silica gel, eluent: petrolether/EtOAc, 6/4 (v/v)] to give the title compound as a solid.

MS: calc.: $C_{34}H_{51}N_3O_6$ (597.78) found: [$MH^+$] = 599.1; [$MNa^+$] = 621.3; [$MH^+$- Boc] = 499.2

**B159. (4aS,8aR)-2-{1-[(2S)-2-amino-3-(dimethylamino)propanoyl]piperidin-4-yl}-4-(3,4-dimethoxyphenyl)-4a,5,6,7,8,8a-hexahydrophthalazin-1(2H)-one trifluoroacetate**

A solution of tert-Butyl [(2S)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-(dimethylamino)-1-oxopropan-2-yl]carbamate (546 mg; compound B162) and trifluoroacetic acid (10 ml) in DCM (10 ml) was stirred for 0.5 h at RT. Afterwards all volatiles were removed under vacuo, the

resulting residue was treated with diethyl ether, filtered off and dried under vacuo to give the title compound as a solid.

MS: calc.: $C_{26}H_{39}N_5O_4$ (485.63) found: [$MH^+$] = 486. 3

**B160. (4aS,8aR)-2-{1-[(2R)-2-amino-4-(methylsulfanyl)butanoyl]piperidin-4-yl}-4-(3,4-dimethoxyphenyl)-4a,5,6,7,8,8a-hexahydrophthalazin-1(2H)-one hydrochloride**

To a solution of tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-(methylsulfanyl)-1-oxobutan-2-yl]carbamate (205 mg; compound B161) in THF (10 ml) was added an aqueous solution of hydrogen chloride (2M, 0.85 ml) and the reaction mixture was stirred for 30 h at 55-60 °C. Afterwards all volatiles were removed under reduced pressure and the residue was treated with DCM and co-evaporated to give the title compound as a solid.

MS: calc.: $C_{26}H_{38}N_4O_4S$ (502.68) found: [$MH^+$] = 503.2

**B161. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-(methylsulfanyl)-1-oxobutan-2-yl]carbamate**

To a mixture of N-2-(tert-butoxycarbonyl)-D-methioninamide (598 mg) and DIPEA (1.63 ml) in DCM (25 ml) was added HBTU (1.0 g) and (4aS,8aR)-4-(3,4-dimethoxyphenyl)-2-(piperidin-4-yl)-4a,5,6,7,8,8a-hexahydrophthalazin-1(2H)-one hydrochloride (979 mg; compound B76) and the mixture was stirred for 2 h at RT. Afterwards saturated aqueous sodium bicarbonate solution (10 ml) was added and the mixture was extracted with DCM (50 ml). The phases were separated using a phase separator and the organic layer was concentrated under reduced pressure. The resulting residue was purified by flash column chromatography [amino phase silica gel, eluation gradient: Cyclohexane to EtOAc to EtOAc/ MeOH, 93:7 (v/v)] to give the title compound as a solid.

MS: calc.: $C_{31}H_{46}N_4O_6$ (602.80) found: [$MH^+$] = 603.0; [$MNa^+$] = 625.2; [$MH^+$ - Boc] = 503.2

**B162. tert-Butyl [(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]carbamate**

To a stirred solution of N-(tert-butoxycarbonyl)-3-(dimethylamino)-L-alanine (0.4 g) in DCM (10 ml) was added DIPCDI (0.43 ml), HOBt x $H_2O$ (426 mg) and N-methylmorpholine (348 mg) at 0°C. After stirring for 5 min at 0°C (4aS,8aR)-4-(3,4-dimethoxyphenyl)-2-(piperidin-4-yl)-4a,5,6,7,8,8a-hexahydro-phthalazin-1(2H)-one hydrochloride (718 mg; compound B76) was added and the reaction mixture was stirred for 1 d at RT. The solvent was removed under vacuo and the resulting residue was purified by flash column chromatography [silica gel, eluent: DCM/dioxane, 5/1+1% $NH_4OH$ solution (v/v)] to give the title compound as a solid.

**B163. tert-Butyl[(2R)-1-{4-[3-(3,4-dimethoxyphenyl)-5,5-dimethyl-6-oxo-5,6-dihydropyridazin-1(4H)-yl]piperidin-1-yl}-5-(dimethylamino)-1,5-dioxopentan-2-yl]carbamate**

To a stirred suspension of N-2-(tert-butoxycarbonyl)-N,N-dimethyl-D-glutamine (150 mg), DIPEA (0.38 ml) and HATU (249 mg) in DCM (10 ml) was added 6-(3,4-dimethoxyphenyl)-4,4-dimethyl-2-(piperidin-4-yl)-4,5-dihydropyridazin-3(2H)-one hydrochloride (209 mg; hydrochloride salt of compound B89) and the mixture was stirred for 4 d at RT. Afterwards the mixture was sequentially extracted by an aqueous solution of hydrogen chloride, water and brine. The organic phase was separated using a phase separator and the organic layer was concentrated under reduced pressure to give the title compound as a solid.

MS: calc.: $C_{31}H_{47}N_5O_7$ (601.75) found: [$MH^+$] = 602.1

**B164. (4R)-4-amino-5-{4-[3-(3,4-dimethoxyphenyl)-5,5-dimethyl-6-oxo-5,6-dihydropyridazin-1(4H)-yl]piperidin-1-yl}-N,N-dimethyl-5-oxopentanamide hydrochloride**

To a solution of tert-Butyl [(2R)-1-{4-[3-(3,4-dimethoxyphenyl)-5,5-dimethyl-6-oxo-5,6-dihydropyridazin-1(4H)-yl]piperidin-1-yl}-5-(dimethylamino)-1,5-dioxopentan-2-yl]carbamate (285 mg; compound B163) in DCM (15 ml) was added a solution of hydrogen chloride in 1,4-dioxane (3.0 ml, 4.0 M) and the reaction mixture was stirred 4 d at RT. The solvent was removed under vacuo and the resulting residue was used for the next reaction step without further purification.

**B165. Benzyl N~2~-(tert-butoxycarbonyl)-N,N-dimethyl-D-glutaminate**

To a stirred solution of DIPEA (1.03 ml) in DCM (16 ml) was added (4R)-5-(benzyloxy)-4-[(tert-butoxycarbonyl)amino]-5-oxopentanoic acid (0.5 g) and HATU (674 mg). After 1 h dimethylamine hydrochloride (242 mg) was added the reaction mixture was stirred at RT for 12 h. Afterwards the mixture was sequentially extracted by an aqueous solution of hydrogen chloride, brine and water. The organic phase was separated using a phase separator and the organic layer was concentrated under reduced pressure to give the title compound as a solid.

MS: calc.: $C_{19}H_{28}N_2O_5$ (364.45) found: [$MH^+$] = 364.9

**B166. N~2~-(tert-butoxycarbonyl)-N,N-dimethyl-D-glutamine**

To a solution of Benzyl N-2-(tert-butoxycarbonyl)-N,N-dimethyl-D-glutaminate in MeOH (30 ml) was added Pd/C (10%) (75 mg) and the reaction mixture was stirred for 12 h under a hydrogen atmosphere. The suspension was filtered off and the filtrate was evaporated to dryness under vacuo to give the title compound as a solid.

MS: calc.: $C_{12}H_{22}N_2O_5$ (274.32) found: [$MH^+$] = 274.9

**B167. tert-Butyl[(2R)-1-{4-[(4aS,8aR)-4-(3,4-dimethoxyphenyl)-1-oxo-4a,5,6,7 ,8,8a-hexahydrophthalazin-2(1H)-yl]piperidin-1-yl}-3-methyl-1-oxobutan-2-yl]carbamate**

To a stirred mixture of N-(tert-butoxycarbonyl)-D-valine (543 mg), HBTU (1.04 g) and (4aS,8aR)-4-(3,4-dimethoxyphenyl)-2-(piperidin-4-yl)-4a,5,6,7,8,8a-hexahydrophthalazin-1(2H)-one hydrochloride (1.02 g; compound B76) in DCM (20 ml) was added DIPEA (1.29 g) and the mixture was stirred for 20 min at RT. Afterwards saturated aqueous sodium bicarbonate solution (20 ml) was added and the mixture was extracted with DCM (15 ml). The combined organic phases were dried over sodium sulphate and the organic layer was concentrated under reduced pressure. The resulting residue was purified by flash column chromatography [silica gel, eluent: EtOAc] to give the title compound as a solid.

MS: calc.: $C_{31}H_{46}N_4O_6$ (570.73) found: [$MH^+$] = 571.1; [$MNa^+$] = 593.2

**B168. (4aS,8aR)-2-{1-[(2R)-2-amino-3-methylbutanoyl]piperidin-4-yl}-4-(3,4-dimethoxyphenyl)-4a,5,6,7,8,8a-hexahydrophthalazin-1(2H)-one trifluoroacetate**

To a solution of tert-Butyl [(2R)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-methyl-1-oxobutan-2-yl]carbamate (1.2 g; compound B167) in DCM (7 ml) was added trifluoroacetic acid (2.3 ml) at 0°C. The reaction mixture was stirred for 30 min at 0°C and then for 1.5 h at RT. All volatiles were removed under reduced pressure to give the title compound as a solid which was used for the next step without further purification.

MS: calc.: $C_{26}H_{38}N_4O_4$ (470.62) found: [$MH^+$] = 471.2

**B169. (4aS,8aR)-2-{1-[(2R)-2-amino-3,3-dimethylbutanoyl]piperidin-4-yl}-4-(3,4-dimethoxyphenyl)-4a,5,6,7,8,8a-hexahydrophthalazin-1(2H)-one**

tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3,3-dimethyl-1-oxobutan-2-yl]carbamate (330m g; compound B170) was dissolved in a solution of hydrogen chloride in 1,4-dioxane (5 ml, 4.0 M) and the reaction mixture was stirred for 16 h at RT. Afterwards all volatiles were removed under reduced pressure and the resulting residue was purified by flash column chromatography [silica gel, eluation gradient: Cyclohexane to EtOAc to DCM/MeOH, 9/1 (v/v)] to give the title compound as a solid.

MS: calc.: $C_{27}H_{40}N_4O_4$ (484.64) found: [$MH^+$] = 485.2

**B170. tert-Butyl [(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3,3-dimethyl-1-oxobutan-2-yl]carbamate**

A solution of N-(tert-butoxycarbonyl)-3-methyl-D-valine (301 mg), TOTU (427 mg) and HOAT (177 mg) in DMF (9 ml) was stirred for 30 min at RT and then (4aS,8aR)-4-(3,4-dimethoxyphenyl)-2-(piperidin-4-yl)-4a,5,6,7,8,8a-hexahydrophthalazin-1(2H)-one hydrochloride (530 mg; compound B76) and DIPEA (0.45 ml) were added. The reaction mixture was stirred for 16 h at RT and then for about 2 h at 70°C. The mixture was concentrated under vacuo and the resulting residue was purified twice by flash column chromatography [amino phase silica gel, eluation gradient: Cyclohexane/EtOAc, 100/0 to 70/30 (v/v)] to give the title compound as a waxy solid.

MS: calc.: $C_{32}H_{48}N_4O_6$ (584.76) found: [$MH^+$] = 585.9; [$MNa^+$] = 607.2; [$MH^+$ - Boc] = 485.2

**B171. tert-Butyl (2-{4-[9-(3,4-dimethoxyphenyl)-6-oxo-7,8-diazaspiro[4.5]dec-8-en-7-yl]piperidin-1-yl}-2-oxoethyl)carbamate**

To a mixture of N-(tert-butoxycarbonyl)glycine (386 mg) and DIPEA (1.44 g) in DCM (20 ml) was added HBTU (0.92 g) and 9-(3,4-dimethoxyphenyl)-7-(piperidin-4-yl)-7,8-diazaspiro[4.5]dec-8-en-6-one hydrochloride (0.9 g; compound 177) and the mixture was stirred for 2 h at RT. Afterwards half-saturated aqueous sodium bicarbonate solution was added and the mixture was extracted with DCM. The organic phase was dried over magnesium sulphate and concentrated under reduced pressure. The resulting residue was purified by flash column chromatography [silica gel, eluent: petrolether/EtOAc, 1/1 (v/v)] to give the title compound as a solid.
MS: calc.: $C_{28}H_{40}N_4O_6$ (528.64) found: [$MH^+$] = 529.1; [$MNa^+$] = 551.2

**B172. 7-[1-(aminoacetyl)piperidin-4-yl]-9-(3,4-dimethoxyphenyl)-7,8-diazaspiro[4.5]dec-8-en-6-one**

To a solution of tert-Butyl (2-{4-[9-(3,4-dimethoxyphenyl)-6-oxo-7,8-diazaspiro[4.5]dec-8-en-7-yl]piperidin-1-yl}-2-oxoethyl)carbamate (1.01 g; compound B171) in DCM (10 ml) was added trifluoroacetic acid (10 ml) and the reaction mixture was stirred for 1 h at RT. The mixture was slowly poured in a saturated aqueous sodium bicarbonate solution. After extraction with DCM, the combined organic layers were dried over magnesium sulphate and all solvents were removed under reduced pressure. After lyophilisation from acetonitrile / water the title compound was obtained as a solid.
MS: calc.: $C_{23}H_{32}N_4O_4$ (428.52) found: [$MH^+$] = 429.2

**B173. 7-{1-[(2R)-2-amino-3-phenylpropanoyl]piperidin-4-yl}-9-(3,4-dimethoxyphenyl)-7,8-diazaspiro[4.5]dec-8-en-6-one**

tert-Butyl [(2R)-1-{4-[9-(3,4-dimethoxyphenyl)-6-oxo-7,8-diazaspiro[4.5]dec-8-en-7-yl]piperidin-1-yl}-1-oxo-3-phenylpropan-2-yl]carbamate (1.72 g; compound B174) was dissolved in a solution of hydrogen chloride in 1,4-dioxane (10.4 ml, 4.0 M) and the reaction mixture was stirred for 45 min at RT. DCM (20 ml) was added and the mixture was sequentially extracted with an aqueous hydrochloric acid solution (2M) and with sodium hydroxide (15 ml, 1M). The organic phase was separated, evaporated under vacuo and the resulting residue was purified by flash column chromatography [amino phase silica gel, eluation gradient: Cyclohexane to EtOAc to EtOAc/MeOH 9/1 (v/v)] to give the title compound as an oil.
MS: calc.: $C_{30}H_{38}N_4O_4$ (518.65) found: [$MH^+$] = 519.2

**B174. tert-Butyl [(2R)-1-{4-[9-(3,4-dimethoxyphenyl)-6-oxo-7,8-diazaspiro[4.5]dec-8-en-7-yl]piperidin-1-yl}-1-oxo-3-phenylpropan-2-yl]carbamate**

To a mixture of N-(tert-butoxycarbonyl)-D-phenylalanine (1.02 g) and DIPEA (1.36 g) in DCM (15 ml) was added HBTU (1.66 g) and 9-(3,4-dimethoxyphenyl)-7-(piperidin-4-yl)-7,8-diazaspiro[4.5]dec-8-en-6-one hydrochloride (1.43 g; compound B115) and the mixture was stirred for 1 h at RT. The mixture was extracted with saturated aqueous sodium bicarbonate solution (3x5 ml) and the organic phase was separated, dried over magnesium sulphate and concentrated under reduced pressure. The resulting residue was purified by flash column chromatography [amino phase silica gel, eluation gradient: EtOAc/n-hexane, 70/30 to 100/0 (v/v)] to give the title compound as a solid.
MS: calc.: $C_{35}H_{46}N_4O_6$ (618.76) found: [$MH^+$] = 619.0; [$MNa^+$] = 641.2

**B175. 7-{1-[(2S)-2-amino-3-(pyridin-3-yl)propanoyl]piperidin-4-yl}-9-(3,4-dimethoxyphenyl)-7,8-diazaspiro[4.5]dec-8-en-6-one**

To a solution of tert-Butyl [(2S)-1-{4-[9-(3,4-dimethoxyphenyl)-6-oxo-7,8-diazaspiro[4.5]dec-8-en-7-yl]piperidin-1-yl}-1-oxo-3-(pyridin-3-yl)propan-2-yl]carbamate (1.13 g; compound B117) in DCM (11 ml) was added trifluoroacetic acid (11 ml) and the reaction mixture was stirred for 1 h at RT. The mixture was slowly poured in a saturated aqueous sodium bicarbonate solution. After extraction with DCM, the combined organic layers were dried over magnesium sulphate and all solvents were removed under reduced pressure. After lyophilisation from acetonitrile / water the title compound was obtained as a solid.
MS: calc.: $C_{29}H_{37}N_5O_4$ (519.64) found: [$MH^+$] = 520.3

**Commercial Utility**

**Medical Uses**

The compounds of formula (1) and the stereoisomers of the compounds of formula (1) according to the invention are hereinafter referred to as the compounds of the invention. In particular, the compounds of the invention are pharmaceutically acceptable.

The compounds of the invention have-as dual-selective type 4/type 5 phosphodiesterase (PDE4/5) inhibitors - valuable pharmaceutical properties, which make them commercially utilizable.

PDE4 inhibitors are thought to be useful in the treatment or prophylaxis of a variety of diseases and disorders. They are thought to be suitable on the one hand as bronchial therapeutics (for the treatment of airway obstructions on account of their effects to curb pulmonary inflammation, lung fibrotic remodling, lung parenchymal destruction, mucociliary malfunction, oxidative stress, pulmonary vascular remodelling), but on the other hand especially for the treatment of disorders, in particular of an inflammatory nature, e.g. of the airways, of the skin, of the intestine, of the eyes, of the CNS and of the joints, which are mediated by mediators such as histamine, PAF (platelet-activating factor), arachidonic acid derivatives such as leukotrienes and prostaglandins, cytokines, interleukins, chemokines, alpha-, beta- and gamma-interferon, tumor necrosis factor $\alpha$ (TNF$\alpha$) or oxygen free radicals and proteases.

In particular, PDE4 inhibitors are thought to be useful in the treatment or prophylaxis of a variety of diseases and disorders, such as for example:

acute and chronic airway diseases, such as, but not limited to, chronic bronchitis, allergic bronchitis, bronchial asthma, emphysema, COPD (chronic obstructive pulmonary disease), bronchiolitis obliterans (BOS) and interstitial lung disease such as pulmonary fibrosis;

pulmonary hypertension;

diseases which are based on allergic and/or chronic, immunological false reactions in the region of the upper airways (pharynx, nose) and the adjacent regions (paranasal sinuses, eyes), such as, but not limited to, allergic rhinitis/sinusitis, chronic rhinitis/sinusitis, allergic conjunctivitis and also nasal polyps; ocular inflammatory diseases such as, but not limited to, uveitis, scleritis, keratitis, retinal vasculitis, age-related macula degeneration, diabetic nephropathy, and chronic and allergic conjunctivitis; dermatological diseases especially of proliferative, inflammatory and allergic type, such as, but not limited to psoriasis (vulgaris), toxic and allergic contact eczema, atopic dermatitis (eczema), seborrhoeic eczema, Lichen simplex, sunburn, pruritus in the anogenital area, alopecia areata, hypertrophic scars, discoid lupus erythematosus, follicular and widespread pyodermias, endogenous and exogenous acne, acne rosacea and other proliferative, inflammatory and allergic skin disorders;

diseases to which excessive release of TNF$\alpha$ and leukotrienes may contribute, such as, for example, diseases of the arthritis type like rheumatoid arthritis, rheumatoid spondylitis, osteoarthritis and other arthritic conditions;

fibrotic diseases, such as, but not limited to, cystic fibrosis, pulmonary fibrosis, hepatic fibrosis renal fibrosis, myelofibrosis, retroperitoneal fibrosis, endomyocardial fibrosis, mediastinal fibrosis, nephro-genic systemic fibrosis, hypertrophic scars or toxic liver damage;

viral, alcoholic or drug-induced acute and fulminant hepatitis, hepatic steatosis (alcoholic and non-alcoholic steatiohepatitis);

diseases of the immune system, such as, but not limited to, AIDS, multiple sclerosis, graft versus host reaction, acute allograft rejections, but also chronic graft versus host disease (CGVHD) after allogeneic hematopoietic stem-cells transplantation (HSCT);

cachexia, cancer cachexia, AIDS cachexia;

types of shock, such as, but not limited to, septic shock, endotoxin shock, gram-negative sepsis, toxic shock syndrome and ARDS (adult respiratory distress syndrome);

diseases in the gastrointestinal region, such as Crohn's disease and ulcerative colitis;

diseases of the heart which can be treated by PDE inhibitors, such as cardiac insufficiency;

diseases which can be treated on account of the tissue-relaxant action of the PDE4 inhibitors, such as, for example, oncolytic action (to treat preterm delivery);

renal diseases such as nephritis such as glomerulonephritis, diabetic nephropathy and urinary tract infections;

diabetes insipidus, diabetes mellitus (type I and in particular type II); cancer (in particular lymphoid and myeloid leukaemia); osteoporosis;

conditions associated with cerebral metabolic inhibition, such as, but not limited to, cerebral senility, senile dementia (Alzheimer's disease), memory impairment associated with Parkinson's disease or multiinfarct dementia;

and also diseases of the central nervous system, such as, but not limited to, depressions, anxiety states, spinal cord injury, schizophrenia or arteriosclerotic dementia.

PDE5 inhibitors are thought to be able to influence the physiological and pathophysiological function of various cells, e.g., but not limited to, smooth muscle cells, fibroblasts, myofibroblasts and platelets, which are involved in a great variety of physiological and pathophysiological mechanisms. In particular, PDE5 inhibitors are thought to be able to effect relaxation of the vasculature, thus increasing blood flow, induce neurogenesis, inhibit platelet function, such as aggregation, adhesion and mediator release and, thus, have an anti-inflammatory effect.

In particular, PDE5 inhibitors are thought to be useful in the treatment or prophylaxis of a variety of diseases and disorders, such as for example:

male and female sexual dysfunction, such as, but not limited to, male erectile dysfunction, premature ejaculation, Peyronie's disease;

acute and chronic airway diseases, such as, but not limited to, COPD (chronic obstructive pulmonary disease), bronchitis, emphysema, pulmonary vascular remodeling, interstitial lung disease such as idiopathic pulmonary lung fibrosis (IPF), asthma, cystic fibrosis, bronchiectasis, bronchiolitis obliterans,

connective tissue diseases, sarcoidosis, kyphoscoliosis, pneumoconiosis, amyotrophic lateral sclerosis, thoracoplasty, extrinsic allergic alveolitis;

pulmonary hypertension;

inflammatory diseases, such as, but not limited to, vasculature inflammation, acute respiratory distress syndrome, nephritis, mesangial glomerulonephritis, chronic inflammatory bowel disease, disseminated intravascular inflammation, allergic vasculitis, dermatoses (e.g., but not limited to, psoriasis, toxic and allergic contact eczema, atopic eczema, seborrhoeic eczema, Lichen simplex, sunburn, pruritus in the anogenital area, alopecia areata, hypertrophic scars, discoid lupus erythematosus, follicular and widespread pyodermias, endogenous and exogenous acne, acne rosacea), disorders of the arthritis type (e.g., but not limited to, rheumatoid arthritis, rheumatoid spondylitis, osteoarthritis), disorders of the immune system [e.g., but not limited to, AIDS (acquired immunodeficiency syndrome), multiple sclerosis], graft versus host reaction, allograft rejections, shock [e.g., but not limited to, septic shock, endotoxin shock, gram-negative sepsis shock, toxic shock syndrome and ARDS (adult respiratory distress syndrome)], gastrointestinal inflammations (e.g., but not limited to, Crohn's disease and ulcerative colitis); disorders which are based on allergic and/or chronic, immunological false reactions (e.g., but not limited to, allergic rhinitis, allergic sinusitis, chronic rhinitis, chronic sinusitis, allergic conjunctivitis, nasal polyps);

pain, such as, but not limited to, inflammatory pain;

right-heart failure, right heart hypertrophy (cor pulmonale), hypertension, hypercholesterolemia, hypertriglyceridemia; diabetes mellitus (type I and type II);

ischaemic diseases, such as, but not limited to, stroke, coronary artery disease, angina (including, but not limited to, vasospastic angina), myocardial infarction, peripheral artery disease, cerebrovascular obstruction, sleep apnea, macular ischaemia, arterial and venous occlusion, congestive heart failure; ocular inflammatory diseases such as, but not limited to, uveitis, scleritis, keratitis, retinal vasculitis, age-related macula degeneration, diabetic nephropathy, and chronic and allergic conjunctivitis; diabetic gastroparesis and diseases with symptoms of gastroparesis;

diseases or conditions in which it is desirable to suppress platelet function, for example, but not limited to, after stent implantations (e.g., but not limited to, coronary stenting), after bypass operations, in pulmonary hypertension, thrombotic diseases, post-angioplasty stenosis, coronary artery disease, infarction (e.g., but not limited to, myocardial infarction), instable angina pectoris, stroke, and arterial and venous occlusion diseases (e.g., but not limited to, claudicatio intermittens);

diseases or conditions with an impairment or dysfunction of cerebral vascular reactivity and/or neurovascular coupling, such as, but not limited to, arteriosclerotic dementia, multi-infarct dementia, cerebral senility;

diseases which are based on neuronal damage or degradation, such as but not limited to, stroke, spinal cord injury, brain injury, morbus parkinson, amyotrophic lateral sclerosis, morbus alzheimer, amyloidosis, prion diseases and neuropathy;

peripheral arterial diseases, chronic renal failure, chronic heart failure, sepsis, senile dementia (Alzheimer's disease), Creutzfeld-Jacob disease, septic encephalopathy, arteriosclerotic encephalopathy, diabetes associated encephalopathy, toxic encephalopathy, vascular and neuronal dementia, Huntington's disease, Parkinson's disease, multiple sclerosis and preeclampsia;

portal hypertension, liver cirrhosis, toxic liver damage (e.g., but not limited to, alcohol-induced liver damage), hepatitis, thrombosis of the portal vein, Budd-Chiari syndrome, malformation of liver veins, compression of liver veins (e.g., but without limitation, due to tumors), arteriovenous fistula, diseases associated with an enlarged spleen, schistosomiasis (bilharziosis), sarcoidosis and other granulomatous diseases, primary biliary cirrhosis, myeloproliferative disorders (e.g., but not limited to, chronic myeloid leukemia, osteomyelofibrosis), lymphatic systemic diseases, collagenosis (e.g., but not limited to, systemic lupus erythematodes, sclerodermia), morbus Osler (congenital arteriovenous malformations, inter alia in the liver), nodular regenerative hyperplasia, tricuspid insufficiency, pericarditis constrictiva, veno-occlusive disease (VOD), non-alcoholic steatohepatitis (NASH);

fibrotic diseases, such as, but not limited to, cystic fibrosis, pulmonary fibrosis, hepatic fibrosis renal fibrosis, myelofibrosis, retroperitoneal fibrosis, endomyocardial fibrosis, mediastinal fibrosis, nephrogenic systemic fibrosis, hypertrophic scars or toxic liver damage;
benign prostatic hyperplasia;
insufficient uteroplacental blood flow in pregnancies with fetal growth restriction;
insufficient brain skills, such as but not limited to, verbal attainment, attention, concentration, deductive thinking, central auditory processing, cognition, learning, vigilance, apprehension and reagibility; Overactive Bladder; LUTS = lower urinary tract symptoms; Raynauds syndrome/phenomenon

In this respect, the term "pulmonary hypertension" in particular embraces

- pulmonary arterial hypertension including primary pulmonary hypertension (e.g. sporadic or familial) and pulmonary arterial hypertension related, for example, but without limitation, to collagen vascular disease, congenital systemic-to-pulmonary shunts, portal hypertension, human immunodeficiency virus infection, drugs or toxins (e.g., but not limited to, anorexigens), persistent pulmonary hypertension of the newborn;
- pulmonary venous hypertension due to, for example, but without limitation, left-sided atrial or ventricular heart disease, left-sided valvular heart disease, extrinsic compression of central pulmonary veins (e.g. fibrosing mediastinitis, adenopathy in relation to tumors), pulmonary veno-occlusive disease;
- pulmonary hypertension associated with disorders of the respiratory system or hypoxemia including, for example, but without limitation, chronic obstructive pulmonary disease (COPD), interstitial lung disease, sleep-disordered breathing, alveolar hypoventilation disorders, chronic exposure to high altitude, neonatal lung disease, alveolar-capillary dysplasia;
- pulmonary hypertension caused by chronic thrombotic or embolic diseases including thromboembolic obstruction of proximal pulmonary arteries and obstruction of distal pulmonary arteries, such as pulmonary embolism (due to thrombus, tumor, ova, parasites, or foreign material), in situ thrombosis and sickle-cell disease, in particular chronic thromboembolic pulmonary hypertension (CTEPH);
- pulmonary hypertension caused by disorders directly affecting the pulmonary vasculature including inflammatory disorders (e.g., but not limited to, schistosomiasis, sarcoidosis) and pulmonary capillary hemangiomatosis.

It is noteworthy that compounds of the invention, which are inhibitors of type 4 phosphodiesterase (PDE4) and of type 5 phosphodiesterase (PDE5), have the potential to be more effective in treatment of distinct disease identities than compounds inhibiting only one of those two enzymes, since inhibition of PDE4 and PDE5 might address diverse and different pathophysiologies occuring within one disease state as e.g. lung fibrosis.

In respect to lung fibrosis it has been described that inhibitors of type 4 phosphodiesterase inhibit THF-β induced transition of lung fibroblasts to myofibroblasts (Dunkern et al., Eur. J. Pharmacol., 572(1): 12-22, 2007; Sabatini et al., Pulm Pharmacol Ther 23: 283-91, 2010), which is a hallmark of fibrosis progression. They have further been described to inhibit matrix metalloproteinase production from lung fibroblasts (Martin-Chouly CA et al., Life Sci. 75(7): 823-40, 2004) and to prevent chemotaxis as well as collagen gel contraction by these cells (Kohyama T et al., Am. J. Respir. Cell Mol. Biol., 26(6): 694-701, 2002; Togo et al., Am J Physiol Lung Cell Mol Physiol 296: L959-69, 2009), which are important pathophysiological aspects of lung fibrosis. In addition the selective type 4 phosphodiesterase inhibitor roflumilast was shown to alleviate bleomycin-induced lung fibrotic remodeling in mice and rat in preventive and therapeutic protocols outperforming glucocorticoids in the latter to inhibit fibrosis development (Cortijo J et al., Br. J. Pharmacol., 156(3): 534-44, 2009).

On the other hand it has been shown in respect to lung fibrosis that PDE5 inhibition by means of the selective PDE5 inhibitor sildenafil attenuates bleomycin-induced pulmonary fibrosis and pulmonary hypertension through inhibition of ROS generation and RhoA/Rho kinase activation (Hemnes AR, Zaiman A, Champion HC, Am. J. Physiol. Lung Cell. Mol. Physiol. 2008 Jan;294(1):L24-33. Epub 2007 Oct 26) and it has been shown in clinical human open-label trials that sildenafil improves lung hemodynamic parameters (vascular resistance and ventilation/perfusion matching) and increases exercise tolerance in patients with pulmonary fibrosis (Ghofrani et al., Lancet 360, 895-900, 2002; Collard et al., Chest 131, 897-899, 2007).

Accordingly, the invention further relates to the compounds of the invention for use in the treatment or prophylaxis of diseases, especially diseases alleviated by inhibition of type 4 and type 5 phosphodiesterase, in particular the diseases exemplified above.

Preferably, the invention relates to the compounds of the invention for use in the treatment or prophylaxis of the following diseases:

acute and chronic airway diseases, such as interstitial lung disease such as pulmonary fibrosis, cystic fibrosis, bronchial asthma, chronic bronchitis, allergic bronchitis, allergic rhinitis, emphysema, chronic obstructive pulmonary

disease (COPD) and COPD associated with pulmonary hypertension;
pulmonary hypertension, in particular thromboembolic pulmonary hypertension;
dermatological diseases, such as psoriasis and atopic dermatitis (eczema);
ocular diseases, such as uveitis, scleritis, keratitis, retinal vasculitis, age-related macula degeneration, diabetic nephropathy, and chronic and allergic conjunctivitis;
rheumatoid arthritis; and
inflammations in the gastrointestinal region, such as Crohn's disease and ulcerative colitis.

The invention also relates to the use of a compound of the invention in the manufacture of a pharmaceutical composition inhibiting the type 4 and type 5 phosphodiesterase, in particular a pharmaceutical composition for the treatment or prophylaxis of diseases alleviated by inhibition of type 4 and type 5 phosphodiesterase, preferably, a pharmaceutical composition for the treatment or prophylaxis of the diseases exemplified above.
In particular, the invention relates to the use of a compound of the invention in the manufacture of a pharmaceutical composition for the treatment or prophylaxis of an acute or chronic airway disease, such as, but not limited to, interstitial lung disease, pulmonary fibrosis, cystic fibrosis, bronchial asthma, chronic bronchitis, emphysema, chronic obstructive pulmonary disease (COPD) or COPD associated with pulmonary hypertension.
The invention also relates to the use of a compound of the invention in the manufacture of a pharmaceutical composition for the treatment or prophylaxis of pulmonary hypertension or thromboembolic pulmonary hypertension.
The invention relates also to the use of a compound of the invention in the manufacture of a pharmaceutical composition for the treatment or prophylaxis of allergic rhinitis or allergic asthma.
Furthermore, the invention relates to the use of a compound of the invention in the manufacture of a pharmaceutical composition for the treatment or prophylaxis of dermatological diseases, such as, but not limited to, psoriasis or atopic dermatitis (eczema).
In addition, the invention relates to the use of a compound of the invention in the manufacture of a pharmaceutical composition for the treatment or prophylaxis of ocular diseases, such as, but not limited to uveitis, scleritis, keratitis, retinal vasculitis, age-related macula degeneration, diabetic nephropathy, or chronic or allergic conjunctivitis.
The invention relates as well to the use of a compound of the invention in the manufacture of a phar-maceutical composition for the treatment or prophylaxis of rheumatoid arthritis.
Additionally, the invention relates to the use of a compound of the invention in the manufacture of a pharmaceutical composition for the treatment or prophylaxis of inflammations in the gastrointestinal region, such as, but not limited to, Crohn's disease or ulcerative colitis.
In a particularly preferred embodiment of the invention, in the above-mentioned uses the compound of the invention is a compound of the examples according to the invention.

Pharmaceutical compositions

The invention furthermore relates to a pharmaceutical composition, which comprises at least one of the compounds of the invention together with at least one pharmaceutically acceptable auxiliary.
Preferably, the pharmaceutical composition comprises one or two of the compounds of the invention. More preferably, the pharmaceutical composition comprises one of the compounds of the invention.
In a particularly preferred embodiment of the invention, the pharmaceutical composition comprises a compound of the examples according to the present invention together with at least one pharmaceutically acceptable auxiliary.
The invention furthermore relates to a pharmaceutical composition according to the invention inhibiting the type 4 and type 5 phosphodiesterase, especially for (use in) the treatment or prophylaxis of diseases alleviated by inhibition of type 4 and type 5 phosphodiesterase, in particular for the treatment or prophylaxis of the diseases exemplified above.
The invention encompasses pharmaceutical compositions according to the invention, as defined above, in particular for (use in) the treatment or prophylaxis of one or more of the following diseases:

interstitial lung disease such as pulmonary fibrosis, cystic fibrosis, bronchial asthma, chronic bronchitis, allergic bronchitis, allergic rhinitis, emphysema, chronic obstructive pulmonary disease (COPD) and COPD associated with pulmonary hypertension;
pulmonary hypertension, in particular thromboembolic pulmonary hypertension;
dermatological diseases, such as psoriasis and atopic dermatitis (eczema);
ocular diseases, such as uveitis, scleritis, keratitis, retinal vasculitis, age-related macula degeneration, diabetic nephropathy, and chronic and allergic conjunctivitis
rheumatoid arthritis; and
inflammations in the gastrointestinal region, such as Crohn's disease and ulcerative colitis.

Although the compounds of the invention may be administered orally, oral administration is not presently thought to be a preferred route of administration. This is because, without intending to be bound by this data, preliminary tests appear to indicate low systemic exposure after oral administration of the compounds of the invention in rats at a dose level of about 10 μmol/kg of the compound of the invention per kg bodyweight when formulated in aqueous suspension with polyethylenglycol 400 (1.3%) and hypromellose (4%).

The compounds of the invention respectively the pharmaceutical compositions comprising the compounds of the invention therefore preferably may be administered, for example, by external topical (i.e. through the skin/ transdermal or via the eye), parenteral (e.g. intravenous, subcutaneous, intraarterial, intraperitoneal, intraarticular, or intramuscular), inhaled or nasal administration. The compounds may also be administered via the rectal route, for example in form of a suppository or a foam.

Accordingly, the pharmaceutical composition can be suitable for (e.g. adapted for) external topical (i.e. through the skin / transdermal or via the eye), parenteral (e.g. intravenous, subcutaneous, intraarterial, intraperitoneal, intraarticular, or intramuscular), inhaled or nasal administration. The pharmaceutical composition is preferably suitable for inhaled administration. Inhaled administration involves topical administration to the lung e.g. by aerosol or dry powder composition.

Inhalable and intranasal pharmaceutical compositions

Formulations for inhalation include powder compositions, which will preferably contain lactose, and spray compositions which may be formulated, for example, as aqueous solutions or suspensions or as aerosols delivered from pressurised packs, with the use of a suitable propellant, e. g. 1, 1, 1, 2-tetrafluorethane, 1, 1, 1, 2, 3, 3, 3-heptafluoropropane, carbon dioxide or other suitable gas.

A class of propellants, which is believed to have minimal ozone-depleting effects in comparison to conventional chlorofluorocarbons comprise hydrofluorocarbons and a number of medicinal aerosol formulations using such propellant systems are disclosed in, for example, EP 0372777, WO91/04011, WO91/11173, WO91/11495, WO91/14422, WO93/11743, and EP-0553298. These applications are all concerned with the preparation of pressurised aerosols for the administration of medicaments and seek to overcome problems associated with the use of this new class of propellants, in particular the problems of stability associated with the pharmaceutical formulations prepared. The applications propose, for example, the addition of one or more of excipients such as polar cosolvents or wetting agents (e.g. alcohols such as ethanol), alkanes, dimethyl ether, surfactants (including fluorinated and non-fluorinated surfactants, carboxylic acids such as oleic acid, polyethoxylates etc.) or bulking agents such as a sugar (see for example WO02/30394) and vehicles such as cromoglicic acid and/or nedocromil which are contained at concentrations, which are not therapeutically and prophylactically active (see WO00/07567). The aerosol dosage form can also take the form of a pump-atomizer.

For suspension aerosols, the compound of the invention should be micronised so as to permit inhalation of substantially all of the compound of the invention into the lungs upon administration of the aerosol formulation, thus the compound of the invention will have a mean particle size of less than 100 μm, desirably less than 20 μm, and preferably in the range of 1 to 10 μm (D50 value, e.g. as measured using laser diffraction).

Dry powder inhalable compositions: For pharmaceutical compositions suitable (e.g. adapted for) inhaled administration, the pharmaceutical composition may for example be a dry powder inhalable composition. The dry powder comprises finely divided compound of the invention optionally together with a finely divided pharmaceutically acceptable carrier, which is preferably present and may be one or more materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran or mannitol. An especially preferred carrier is lactose, particularly in the form of the monohydrate.

The dry powder may be in capsules of gelatine or plastic, or in blisters, for use in a dry powder inhalation device, preferably in dosage units of the compound of the invention together with the carrierin amounts to bring the total weight of powder in each capsule to from 5mg to 50mg. Alternatively the dry powder may be contained in a reservoir of a multi-dose dry powder inhalation device. Capsules and cartridges of for example gelatin, or blisters of for example laminated aluminium foil, for use in an inhaler or insulator may be formulated containing a powder mix of the compounds of the invention and a suitable powder base such as lactose or starch, preferably lactose. In this aspect, the compound of the invention is suitably micronised so as to permit inhalation of substantially all of the compound of the invention into the lungs upon administration of the dry powder formulation, thus the compound of the invention will have a particle size of less than 100μm, desirably less than 20μm, and preferably in the range 1 to 10μm (D50 value, e.g. as measured using laser diffraction). The solid carrier, where present, generally has a maximum particle diameter of 300μm, preferably 200μm, and conveniently has a mean particle diameter of 40 to 100μm, preferably 50 to 75μm. The particle size of the ccmpound of the invention and that of a solid carrier where present in dry powder compositions, can be reduced to the desired level by conventional methods, for example by grinding in an air-jet mill, ball mill or vibrator mill, microprecipitation, spray drying, lyophilisation or recrystallisation from supercritical media.

Where the inhalable form of the composition of the invention is the finely divided particulate form, the inhalation device may be, for example a dry powder inhalation device adapted to deliver dry powder from a capsule or blister containing a dosage unit of the dry powder or a multi-dose dry powder inhalation device. Such dry powder inhalation devices are known in the art. Examples which may be mentioned are Cyclohaler®, Diskhaler®, Rotadisk®, Turbohaler®, Novolizer®, Easyhaler®, Jethaler®, Clickhaler® or the dry powder inhalation devices disclosed in EP 0 505 321, EP 407028, EP 650410, EP 691865 or EP 725725 (Ultrahaler®).

Formulations for inhalation by nebulization may be formulated with an aqueous vehicle with the addition of agents such as acid or alkali, buffer salts, isotonicity adjusting agents or antimicrobials. They may be sterilised by filtration or heating in an autoclave. Suitable technologies for this type of administration are known in the art. As an example the Mystic® technology is to be mentioned (see for example US6397838, US6454193 and US6302331).

Preferred unit dosage formulations are those containing a pharmaceutical effective dose, as hereinbelow recited, or an appropriate fraction thereof, of the active ingredient. Thus, in the case of formulations designed for delivery by metered dose pressurised aerosols, one actuation of the aerosol may deliver half of the therapeutical effective amount such that two actuations are necessary to deliver the therapeutically effective dose.

In the dry powder inhalable composition, the compound of the invention can for example be present in about 0.1% to about 70% (e.g. about 1% to about 50%, e.g. about 5% to about 40%, e.g. about 20 to about 30%) by weight of the composition.

In case of intranasal administration, for example, sprays and solutions to be applied in drop form are preferred formulations. Intranasal sprays or nasal drops may be formulated with aqueous or non-aqueous vehicles with or without the addition of agents such as thickening agents, buffer salts or acid or alkali to adjust the pH, isotonicity adjusting agents, preservatives or anti-oxidants. Pharmaceutical compositions suitable for external topical administration

"External topical" administration means topical administration to an external body part (i.e. excluding, for example, the lung or mouth, but including the lips or the eye). External topical adminstration (e.g. through the skin / transdermal) can for example be to those parts of the skin affected by or susceptible to a dermatological disease, such as for example, atopic dermatitis or psoriasis.

In case of external topical administration (i.e. through the skin / transdermal), suitable pharmaceutical formulations are, for example, ointments, creams (usually an oil-in-water or water-in-oil pharmaceutical composition, usually an emulsion), lotions, pastes, gels, powders, solutions, emulsions, suspensions, oils, sprays and patches (e.g., but not limited to, transdermal therapeutic systems).

In an external-topical pharmaceutical composition, e.g. an ointment or an oil-in-water or water-in-oil composition, the compound of the invention is suitably present in 0.05 to 10%, preferably 0.1 to 5%, more preferably 0.1 to 3%, still more preferably 0.5 to about 2.5 %, by weight of the composition (w/w).

External topical adminstration (e.g. via the eye) can for example be to the eye affected by or susceptible to an ocular disease, such as for example, uveitis, scleritis, keratitis, retinal vasculitis, age-related macula degeneration, diabetic nephropathy, and chronic and allergic conjunctivitis.

Examples, which may be mentioned in connection with pharmaceutical formulations for the eye are eyebaths or eye lotions, eye inserts, eye ointments, eye sprays, eye drops, preparations for intraocular application [e.g. intravitreale application, intraocular injection] and eyelid ointments.

Pharmaceutical compositions for oral or parenteral administration

For parenteral modes of administration such as, for example, intravenous, subcutaneous or intramuscular administration, preferably solutions (e.g., but not limited to, sterile solutions, isotonic solutions) are used. They are preferably administered by injection or infusion techniques.

A pharmaceutical composition suitable for parenteral (e.g. intravenous, subcutaneous or intramuscular) administration can comprise a solution or suspension of the compound of the invention in a sterile parenterally acceptable carrier (e.g. sterile water) or parenterally acceptable oil. Alternatively, the solution can be lyophilised. A lyophilised pharmaceutical composition suitable for parenteral administration may, in use, optionally be reconstituted with a suitable solvent, e.g. sterile water or a sterile parenterally acceptable aqueous solution, just prior to administration.

Oral administration is not preferred, as described above. However, a pharmaceutical composition for oral administration may be liquid or solid; for example, it may be a syrup, suspension or emulsion; as well it may be, for example, a tablet, coated tablet (dragee), pill, cachet, capsule (caplet), or in form of granules.

A liquid formulation may optionally consist of a suspension or solution of the compound of the invention in a pharmaceutically acceptable liquid carrier, for example an aqueous solvent such as water, ethanol or glycerine, or a non-aqueous solvent, such as polyethylene glycol or an oil. The formulation may contain in addition, a suspending agent, a preservative, a flavouring and/or a colouring agent.

A pharmaceutical composition for oral administration being a tablet, through not preferred, may comprise one or more pharmaceutically acceptable auxiliaries (for example, carriers and/or excipients) suitable for preparing tablet formulations. The carrier may, for example, be or include lactose, cellulose or mannitol. The tablet may also or instead contain one or more pharmaceutically acceptable excipients, for example, a binding agent, a lubricant and/or a tablet

disintegrant.

The pharmaceutical compositions according to the invention for oral or parenteral administration preferably contain the compound or compounds of the invention in a total amount of from 0.1 to 99.9%, more preferably 5 to 95%, in particular 20 to 80% by weight of the composition (w/w).

In general, as pharmaceutically acceptable auxiliaries, any auxiliaries known to be suitable for preparing a particular pharmaceutical composition can be used. Examples thereof include, but are not limited to, solvents, excipients, dispersants, emulsifiers, solubilizers, gel formers, ointment bases, antioxidants, preservatives, stabilizers, carriers, fillers, binders, thickeners, complexing agents, disintegrating agents, buffers, permeation promoters, polymers, lubricants, coating agents, propellants, tonicity adjusting agents, surfactants, colorants, flavorings, sweeteners and dyes. In particular, auxiliaries of a type appropriate to the desired formulation and the desired mode of administration are used.

The pharmaceutical compositions/formulations can be manufactured in a manner known to a person skilled in the art, e.g. by dissolving, mixing, granulating, dragee-making, levigating, emulsifying, encapsulating, entrapping or lyophilizing processes.

Dosages

Generally, the pharmaceutical compositions according to the invention can be administered such that the dose of the compound of the invention is in the range customary for type 4 phosphodiesterase inhibitors.

The pharmaceutically acceptable compounds of the invention are preferably administered in a daily dose (for an adult patient) of, for example an oral or parenteral dose of 0.01 mg to 250 mg per day, preferably 0.05 mg to 100 mg per day, more preferably 0.05 mg to 10 mg per day, or a nasal or inhaled dose of 0.001 mg to 30 mg per day, preferably 0.01 mg to 10 mg per day, more preferably 0.1 mg to 4 mg per day, of the compound of the invention, calculated as the free compound (= the unsolvated, unhydrated, non-salt form of the compound).

In this respect, it is to be noted that the dose is dependent, for example, on the specific compound used, the species treated, age, body weight, general health, sex and diet of the subject treated, mode and time of administration, rate of excretion, severity of the disease to be treated and drug combination.

The pharmaceutical compositions of the invention can be administered in a single dose per day or in multiple subdoses, for example, 2 to 4 doses per day. A single dose unit of the pharmaceutical composition can contain, in case of inhalative administration e.g. from 0.001 mg to 10 mg, preferably 0.01 mg to 7.5 mg, more preferably 0.1 mg to 4 mg of the compound of the invention. Administration of the pharmaceutical composition in a single dose per day is preferred.

Combinations

Depending on the particular disease to be treated or prevented, additionally therapeutic agents, which are normally administered to treat or prevent that disease, may optionally be co-administered with the compounds of the invention.

In a preferred embodiment, at least one of the compounds of the invention is co-administered with at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides.

In this respect, the "therapeutic agent" includes the corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides in form of the free compounds, the pharmaceutically acceptable salts thereof, the pharmaceutically acceptable derivatives thereof (e.g., but not limited to, ester derivatives, N-oxides etc.), the solvates (hydrates) thereof and the stereoisomers of the compounds, salts, derivatives and solvates.

Co-administration of at least one of the compounds of the invention with at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides can take place in form of a fixed combination, a non-fixed combination or a kit of parts.

A "fixed combination" is defined as a combination wherein the compound of the invention and the therapeutic agent intended for co-administration are present in one dosing unit or in a single entity. One example of a fixed combination is a pharmaceutical composition wherein the compound of the invention and the therapeutic agent are present in admixture for simultaneous administration. Another example of a fixed combination is a pharmaceutical composition wherein the compound of the invention and the therapeutic compound are present in one dosing unit without being in admixture.

A "non-fixed combination" or "kit of parts" is defined as a combination wherein the compound of the invention and the therapeutic agent are present in more than one dosing unit. In a non-fixed combination or a kit of parts the compound of the invention and the therapeutic agent are provided as separate formulations. They might be packaged and presented together as separate components of a combination pack for simultaneous, sequential or separate use in combination therapy. Simultaneous or sequential administration of the compound of the invention and the therapeutic agent are preferred. In case of sequential or separate administration of the compound of the invention and the therapeutic agent, the compound of the invention can be administered before or after administration of the therapeutic agent.

Sequential administration encompasses a short time period between the administration of the compound of the invention and the therapeutic agent or vice versa (for example, the time that is needed to swallow one tablet after the other).

Separate administration encompasses longer time periods between the administration of the compound of the invention and the therapeutic agent. In a preferred embodiment of the invention, the compound of the invention is administered while the therapeutic agent (or vice versa) still has an therapeutic effect on the patient being treated.

In a particularly preferred embodiment of the invention the co-administration of at least one of the compounds of the invention with at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides leads to a therapeutic effect that is greater than the sum of the therapeutic effects that will be achieved in case the compound of the invention respectively the additional therapeutic agent are given alone.

The type of formulation of the compound of the invention and the therapeutic agent of a non-fixed combination or a kit of parts can be identical, i.e. both, the compound of the invention and the therapeutic agent are formulated, for example, as powder, solution or suspension suitable for inhalative administration, or can be different, i.e. suited for different administration forms, such as e.g. the compound of the invention is formulated as powder, solution or suspension suitable for inhalative administration and the therapeutic agent is formulated as tablet or capsule for oral administration.

Accordingly, the invention additionally relates to a pharmaceutical composition presented either as a fixed combination, a non-fixed combination or kit of parts comprising at least one of the compounds of the invention, at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylylcyclase activators/stimulators, tetrahydrobiopterin and tetrahydrobiopterin derivatives, anticoagulants, diuretics, pirfenidone and digitalis glycosides, and at least one pharmaceutically acceptable auxiliary.

In a preferred embodiment, the above-mentioned fixed combination, non-fixed combination or kit of parts comprise a compound of the invention (in particular the compound of the invention is one of the examples of the invention), a $\beta_2$-adrenoreceptor agonist and at least one pharmaceutically acceptable auxiliary. In a particularly preferred embodiment, the above-mentioned fixed combination, non-fixed combination or kit of parts comprise:

a compound of the invention and salbutamol,
a compound of the invention and milveterol,
a compound of the invention and indacaterol,
a compound of the invention and carmoterol,
a compound of the invention and salmeterol,
a compound of the invention and formoterol,
a compound of the invention and vilanterol, or
a compound of the invention and olodaterol,
and at least one pharmaceutically acceptable auxiliary.

In a preferred embodiment, the pharmaceutically acceptable salt of salbutamol is salbutamol sulfate. In a preferred embodiment, the pharmaceutically acceptable salt of milveterol is milveterol hydrochloride. In a preferred embodiment, the pharmaceutically acceptable salt of carmoterol is carmoterol hydrochloride. In a preferred embodiment, the pharmaceutically acceptable salt of salmeterol is salmeterol xinafoate. In another preferred embodiment, the pharmaceutically acceptable salt of formoterol is formoterol hemifumarate monohydrate. In another preferred embodiment, the stereoisomer of formoterol is R,R-formoterol. In another preferred embodiment, the pharmaceutically acceptable salt of R,R-formoterol is R,R-formoterol L-tartrate. In a preferred embodiment, the pharmaceutically acceptable salt of vilanterol is vilanterol trifenatate. In another preferred embodiment, the pharmaceutically acceptable salt of vilanterol is vilanterol α-phenyl cinnamate. In a preferred embodiment, the pharmaceutically acceptable salt of olodaterol is olodaterol hydrochloride.

Preferably the β2-adrenoreceptor agonist is a long-acting β2-adrenoreceptor agonist; particularly preferred in this respect are those β2-adrenoreceptor agonists having a therapeutic effect over a 12-24 hours period. Furthermore, the β2-adrenoreceptor agonist is preferably for inhaled administration, for once daily administration and for simultaneous inhaled administration.

Preferably, the combination comprising a compound of the invention and a β2-adrenoreceptor agonist is for the treatment or prophylaxis of bronchial asthma and COPD.

In a preferred embodiment, the above-mentioned fixed combination, non-fixed combination or kit of parts comprise a compound of the invention (in particular the compound of the invention is one of the examples of the invention), a corticosteroid and at least one pharmaceutically acceptable auxiliary. In a particularly preferred embodiment, the above-mentioned fixed combination, non-fixed combination or kit of parts comprise:

a compound of the invention and budesonide,
a compound of the invention and fluticasone,
a compound of the invention and beclometasone,
a compound of the invention and mometasone,
a compound of the invention and triamcinolone acetonide, or
a compound of the invention and ciclesonide,
and at least one pharmaceutically acceptable auxiliary.

In a preferred embodiment, the pharmaceutically acceptable derivative of fluticasone is fluticasone-17-propionate. In another preferred embodiment, the pharmaceutically acceptable derivative of fluticasone is fluticasone-17-furoate. In another preferred embodiment, the pharmaceutically acceptable derivative of beclometasone is beclometasone 17, 21-dipropionate ester. In a preferred embodiment, the pharmaceutically acceptable derivative of mometasone is mometasone furoate.

The combination comprising a compound of the invention and a corticosteroid preferably is for the treatment and prophylaxis of bronchial asthma, COPD, allergic rhinitis or a dermatological disease, such as for example atopic dermatitis. Preferably the corticosteroid is used for external topical, intranasal or inhaled administration; in severe cases, the corticosteroid may also be used orally.
In a preferred embodiment, the above-mentioned fixed combination, non-fixed combination or kit of parts comprise a compound of the invention (in particular the compound of the invention is one of the examples of the invention), an anticholinergic and at least one pharmaceutically acceptable auxiliary. In a particularly preferred embodiment, the above-mentioned fixed combination, non-fixed combination or kit of parts comprise:

a compound of the invention and glycopyrronium bromide,
a compound of the invention and aclidinium bromide,
a compound of the invention and tiotropium bromide,
a compound of the invention and ipratropium bromide, or
a compound of the invention and darotropium bromide,
and at least one pharmaceutically acceptable auxiliary.

In a preferred embodiment, the stereoisomer of glycopyrronium bromide is (R,R)-glycopyrronium bromide. In a preferred embodiment, tiotropium bromide is used in form of its monohydrate.

Preferably, the anticholinergic is for inhaled administration. The combination comprising a compound of the invention and an anticholinergic is preferably for the treatment or prophylaxis of COPD.

In a preferred embodiment, the above-mentioned fixed combination, non-fixed combination or kit of parts comprise a compound of the invention (in particular the compound of the invention is one of the examples of the invention), a H1 receptor antagonist and at least one pharmaceutically acceptable auxiliary. In a particularly preferred embodiment, the above-mentioned fixed combination, non-fixed combination or kit of parts comprise:

a compound of the invention and azelastine,
a compound of the invention and olopatadine,
a compound of the invention and loratadine,
a compound of the invention and desloratadine, or
a compound of the invention and cetirizine,
and at least one pharmaceutically acceptable auxiliary.

In a preferred embodiment, the pharmaceutically acceptable salt of azelastine is is azelastine hydrochloride. In a preferred embodiment, the pharmaceutically acceptable salt of olapatadine is olapatadine hydrochloride. In a preferred embodiment, the pharmaceutically acceptable salt of cetirizine is cetirizine dihydrochloride. In a preferred embodiment, the stereoisomer of cetirizine is levocetirizine. In another preferred embodiment, the pharmaceutically acceptable salt of levocetirizine is levocetirizine dihydrochloride.

The combination comprising a compound of the invention and a H1 receptor agonist is preferably for the treatment or prophylaxis of allergic rhinitis.

In a preferred embodiment, the above-mentioned fixed combination, non-fixed combination or kit of parts comprise a compound of the invention (in particular the compound of the invention is one of the examples of the invention), a leukotriene receptor antagonist and at least one pharmaceutically acceptable auxiliary. In a particularly preferred embodiment, the above-mentioned fixed combination, non-fixed combination or kit of parts comprise:

a compound of the invention and montelukast,
a compound of the invention and pranlukast, or
a compound of the invention and zafirlukast,
and at least one pharmaceutically acceptable auxiliary.

In a preferred embodiment, the pharmaceutically acceptable salt of montelukast is montelukast sodium. In another preferred embodiment, pranlukast is used in form of its monohydrate.

The combination comprising a compound of the invention and a leukotriene receptor antagonist is preferably for the treatment or prophylaxis of bronchial asthma.

In a preferred embodiment, the above-mentioned fixed combination, non-fixed combination or kit of parts comprise a compound of the invention (in particular the compound of the invention is one of the examples of the invention), a 5-lipoxygenase inhibitor and at least one pharmaceutically acceptable auxiliary. In a particularly preferred embodiment, the above-mentioned fixed combination, non-fixed combination or kit of parts comprise:

a compound of the invention and zileuton,
and at least one pharmaceutically acceptable auxiliary.

The combination comprising a compound of the invention and a 5-lipoxygenase inhibitor is preferably for the treatment or prophylaxis of bronchial asthma.

In a preferred embodiment, the above-mentioned fixed combination, non-fixed combination or kit of parts comprise a compound of the invention (in particular the compound of the invention is one of the examples of the invention), an endothelin antagonist and at least one pharmaceutically acceptable auxiliary. In a particularly preferred embodiment, the above-mentioned fixed combination, non-fixed combination or kit of parts comprise:

a compound of the invention and bosentan,
a compound of the invention and ambrisentan,
a compound of the invention and atrasentan,
a compound of the invention and darusentan,
a compound of the invention and clazosentan, or
a compound of the invention and avosentan,
and at least one pharmaceutically acceptable auxiliary.

In another preferred embodiment, bosentan is used in form of its monohydrate. In another preferred embodiment the pharmaceutically acceptable salt of clazosentan is the disodium salt of clazosentan. In another preferred embodiment the pharmaceutically acceptable salts of atrasentan are atrasentan hydrochloride or the sodium salt of atrasentan. In another preferred embodiment the R-enantiomer of atrasentan is used. In another preferred embodiment the S-enantiomer of darusentan is used.

The combination comprising a compound of the invention and an endothelin antagonist is preferably for the treatment or prophylaxis of pulmonary hypertension and COPD.

In a preferred embodiment, the above-mentioned fixed combination, non-fixed combination or kit of parts comprises a compound of the invention invention (in particular the compound of the invention is one of the examples of the invention), a prostacyclin and at least one pharmaceutically acceptable auxiliary. In a particularly alternative embodiment, the above-mentioned fixed combination, non-fixed combination or kit of parts comprise:

a compound of the invention and iloprost,
a compound of the invention and epoprostenol, or
a compound of the invention and triprostinil,
and at least one pharmaceutically acceptable auxiliary.

The combination comprising a compound of the invention and a prostacyclin is preferably for the treatment or prophylaxis of pulmonary hypertension.

In a preferred embodiment, the above-mentioned fixed combination, non-fixed combination or kit of parts comprises a compound of the invention invention (in particular the compound of the invention is one of the examples of the invention), a calcium channel blocker and at least one pharmaceutically acceptable auxiliary. In a particularly alternative embodiment, the above-mentioned fixed combination, non-fixed combination or kit of parts comprise:

a compound of the invention and amlodipine,
a compound of the invention and nifedipine,
a compound of the invention and diltiazem,
a compound of the invention and verapamil, or
a compound of the invention and felodipine,
and at least one pharmaceutically acceptable auxiliary.

In a preferred embodiment, the above-mentioned fixed combination, non-fixed combination or kit of parts comprise a compound of the invention invention (in particular the compound of the invention is one of the examples of the invention), a beta-blocker and at least one pharmaceutically acceptable auxiliary. In a particularly preferred embodiment, the above-mentioned fixed combination, non-fixed combination or kit of parts comprise:

a compound of the invention and bisoprolol,
a compound of the invention and nebivolol,
a compound of the invention and metoprolol,
a compound of the invention and carvedilol,
a compound of the invention and atenolol, or
a compound of the invention and nadolol,
and at least one pharmaceutically acceptable auxiliary.

In a preferred embodiment, the above-mentioned fixed combination, non-fixed combination or kit of parts comprises a compound of the invention invention (in particular the compound of the invention is one of the examples of the invention), a type 4 phosphodiesterase inhibitor and at least one pharmaceutically acceptable auxiliary. In a particularly preferred embodiment, the above-mentioned fixed combination, non-fixed combination or kit of parts comprise:

a compound of the invention and roflumilast,
a compound of the invention and roflumilast N-oxide,
a compound of the invention and apremilast,
a compound of the invention and oglemilast,
a compound of the invention and revamilast,or
a compound of the invention and 6-({3- [(dimethylamino) carbonyl]phenyl}sulfonyl)-8-methyl-4-{[3-methyloxy)phenyl]amino}-3-quinolinecarboxamide (GSK256066)
and at least one pharmaceutically acceptable auxiliary.

The combination comprising a compound of the invention and an additional PDE4 inhibitor is preferably for the treatment or prophylaxis of pulmonary hypertension and COPD.

In a preferred embodiment, the above-mentioned fixed combination, non-fixed combination or kit of parts comprise a compound of the invention (in particular the compound of the invention is one of the examples of the invention), a type 5 phosphodiesterase inhibitor and at least one pharmaceutically acceptable auxiliary. In a particularly preferred embodiment, the above-mentioned fixed combination, non-fixed combination or kit of parts comprise:

a compound of the invention and sildenafil,
a compound of the invention and vardenafil,
a compound of the invention and tadalafil,
a compound of the invention and udenafil, or
a compound of the invention and avanafil,
and at least one pharmaceutically acceptable auxiliary.

In another preferred embodiment, the pharmaceutically acceptable salts of sildenafil are sildenafil hemi-citrate, sildenafil citrate and sildenafil mesilate; particularly preferred is the citrate salt of sildenafil. In another preferred embodiment, the pharmaceutically acceptable salts of vardenafil are vardenafil hydrochloride or vardenafil dihyrochloride. In another preferred embodiment, the pharmaceutically acceptable salt of avanafil is avanafil besilate.

The combination comprising a compound of the invention and an additional PDE5 inhibitor is preferably for the treatment or prophylaxis of pulmonary hypertension and COPD.

In a preferred embodiment, the above-mentioned fixed combination, non-fixed combination or kit of parts comprise a compound of the invention (in particular the compound of the invention is one of the examples of the invention), a guanyl-cyclase activator/stimulator and at least one pharmaceutically acceptable auxiliary. In a particularly preferred embodiment, the above-mentioned fixed combination, non-fixed combination or kit of parts comprise:

a compound of the present subject matter and BAY63-2521 (Riociguat), or
a compound of the present subject matter and Ataciguat,
and at least one pharmaceutically acceptable auxiliary.

In a preferred embodiment, the above-mentioned fixed combination, non-fixed combination or kit of parts comprise a compound of the invention (in particular the compound of the invention is one of the examples of the invention), tetrahydrobiopterin or a tetrahydrobiopterin derivative and at least one pharmaceutically acceptable auxiliary. In a particularly preferred embodiment, the above-mentioned fixed combination, non-fixed combination or kit of parts comprise:

a compound of the invention and (6R)-L-erythro-5,6,7,8-tetrahydrobiopterin,
a compound of the invention and (6R,S)-5,6,7,8-tetrahydrobiopterin,
a compound of the invention and 1',2'-diacetyl-5,6,7,8-tetrahydrobiopterin,
a compound of the invention and sepiapterin,
a compound of the invention and 6-methyl-5,6,7,8-tetrahydropterin,
a compound of the invention and 6-hydroxymethyl-5,6,7,8-tetrahydropterin, or
a compound of the invention and 6-phenyl-5,6,7,8-tetrahydropterin,
and at least one pharmaceutically acceptable auxiliary.

In a preferred embodiment, the pharmaceutically acceptable derivative of (6R)-L-erythro-5,6,7,8-tetrahydrobiopterin is (6R)-L-erythro-5,6,7,8-tetrahydrobiopterin dihydrochloride.

In a preferred embodiment, the above-mentioned fixed combination, non-fixed combination or kit of parts comprise a compound of the invention (in particular the compound of the invention is one of the examples of the invention), a HMG-CoA reductase inhibitor and at least one pharmaceutically acceptable auxiliary. In a particularly preferred embodiment, the above-mentioned fixed combination, non-fixed combination or kit of parts comprise:

a compound of the invention and lovastatin,
a compound of the invention and pravastatin,
a compound of the invention and simvastatin,
a compound of the invention and atorvastatin,
a compound of the invention and fluvastatin,
a compound of the invention and rosuvastatin,
a compound of the invention and pitavastatin,
a compound of the invention and bervastatin,
a compound of the invention and dalvastatin, or
a compound of the invention and glenvastatin,
and at least one pharmaceutically acceptable auxiliary.

In a preferred embodiment the pharmaceutically acceptable salts of pravastatin are the potassium, lithium, sodium and hemi-calcium salt of pravastatin. A particularly preferred pharmaceutically acceptable salt of pravastatin is the sodium salt of pravastatin. In a preferred embodiment the pharmaceutically acceptable salt of simvastatin is the

sodium salt of simvastatin. In a preferred embodiment the pharmaceutically acceptable salts of atorvastatin are the potassium, sodium and the hemi-calcium salt of atorvastatin. A particularly preferred pharmaceutically acceptable salt of atorvastatin is the hemi-calcium salt of atorvastatin. As an example for a hydrate of atorvastatin may be mentioned the trihydrate and the sesqui-hydrate of the hemi-calcium salt of atorvastatin. In a preferred embodiment of the pharmaceutically acceptable salt of fluvastatin is the sodium salt of fluvastatin. In a preferred embodiment the pharmaceutically acceptable salts of rosuvastatin are the potassium, lithium, sodium, hemi-magnesium and the hemi-calcium salt of rosuvastatin. A particularly preferred pharmaceutically acceptable salt of rosuvastatin is the hemi-calcium salt of rosuvastatin. Another particularly preferred pharmaceutically acceptable salt of rosuvastatin is the sodium salt of rosuvastatin. In a preferred embodiment the pharmaceutically acceptable salts of pitavastatin are the potassium, sodium and the hemi-calcium salt of pitavastatin. A particularly preferred pharmaceutically acceptable salt of pitavastatin is the hemi-calcium salt of pitavastatin.

The combination comprising a compound of the invention and a HMG-CoA reductase inhibitor is preferably for the treatment or prophylaxis of COPD.

In a preferred embodiment, the above-mentioned fixed combination, non-fixed combination or kit of parts comprise a compound of the invention (in particular the compound of the invention is one of the examples of the invention), a PPARγ agonist and at least one pharmaceutically acceptable auxiliary. In a particularly preferred embodiment, the above-mentioned fixed combination, non-fixed combination or kit of parts comprise:

a compound of the invention and pioglitazone,
a compound of the invention and rosiglitazone,
a compound of the invention and troglitazone,
a compound of the invention and rivoglitazone, or
a compound of the invention and ciglitazone,
and at least one pharmaceutically acceptable auxiliary.

The combination comprising a compound of the invention and an additional PPARγ agonist is preferably for the treatment or prophylaxis of COPD and comorbidities.

In a preferred embodiment, the above-mentioned fixed combination, non-fixed combination or kit of parts comprise a compound of the invention (in particular the compound of the invention is one of the examples of the invention), a ACE inhibitor and at least one pharmaceutically acceptable auxiliary. In a particularly preferred embodiment, the above-mentioned fixed combination, non-fixed combination or kit of parts comprise:

a compound of the invention and captopril,
a compound of the invention and enalapril,
a compound of the invention and fosinopril,
a compound of the invention and lisinopril,
a compound of the invention and moexipril,
a compound of the invention and benazepril,
a compound of the invention and perindopril
a compound of the invention and ramipril
a compound of the invention and trandolapril, or
a compound of the invention and quinapril,
and at least one pharmaceutically acceptable auxiliary.

The combination comprising a compound of the invention and an additional ACE inhibitor is preferably for the treatment or prophylaxis of COPD and comorbidities.

In a preferred embodiment, the above-mentioned fixed combination, non-fixed combination or kit of parts comprise a compound of the invention (in particular the compound of the invention is one of the examples of the invention), a angiotensin II receptor antagonist and at least one pharmaceutically acceptable auxiliary. In a particularly preferred embodiment, the above-mentioned fixed combination, non-fixed combination or kit of parts comprise:

a compound of the invention and losartan,
a compound of the invention and azilsartan,
a compound of the invention and valsartan,
a compound of the invention and olemsartan,
a compound of the invention and telmisartan, or
a compound of the invention and irbesartan,
and at least one pharmaceutically acceptable auxiliary.

The combination comprising a compound of the invention and an additional angiotensin II receptor antagonist is preferably for the treatment or prophylaxis of COPD and comorbidities.

In a preferred embodiment, the above-mentioned fixed combination, non-fixed combination or kit of parts comprises a compound of the invention (in particular the compound of the invention is one of the examples of the invention), a lung surfactant and at least one pharmaceutically acceptable auxiliary. In a particularly preferred embodiment, the above-mentioned fixed combination, non-fixed combination or kit of parts comprise:

a compound of the invention and lusupultide,
a compound of the invention and poracant alfa,
a compound of the invention and sinapultide,
a compound of the invention and beracant,
a compound of the invention and bovacant,
a compound of the invention and colfosceril palmitate,
a compound of the invention and surfactant-TA, or
a compound of the invention and calfacant,
and at least one pharmaceutically acceptable auxiliary.

The combination comprising a compound of the invention and a lung surfactant is preferably for the treatment or prophylaxis of bronchial asthma or COPD.

In a preferred embodiment, the above-mentioned fixed combination, non-fixed combination or kit of parts comprise a compound of the invention (in particular the compound of the invention is one of the examples of the invention), an antibiotic and at least one pharmaceutically acceptable auxiliary. In a particularly preferred embodiment, the above-mentioned fixed combination, non-fixed combination or kit of parts comprise:

a compound of the invention and amoxicillin,
a compound of the invention and ampicillin,
a compound of the invention and levofloxacin,
a compound of the invention and clarithromycin,
a compound of the invention and ciprofloxacin,
a compound of the invention and telithromycin, or
a compound of the invention and azithromycin,
and at least one pharmaceutically acceptable auxiliary.

In a preferred embodiment, amoxicillin is used in form of its trihydrate. In another preferred embodiment, ampicillin is used in form of its trihydrate. In another preferred embodiment, the pharmaceutically acceptable salt of ampicillin is ampicillin natrium. In another preferred embodiment levofloxacin is used in form of its hemi hydrate. In another preferred embodiment, the pharmaceutically acceptable salt of ciprofloxacin is ciprofloxacin hydrochloride monohydrate. In another preferred embodiment, azithromycin is used in form of its monohydrate.

The combination comprising a compound of the invention and an antibiotic is preferably for the treatment or prophylaxis of exacerbations associated with bronchial asthma and COPD.

In a preferred embodiment, the above-mentioned fixed combination, non-fixed combination or kit of parts comprises a compound of the invention (in particular the compound of the invention is one of the examples of the invention), an anticoagulant and at least one pharmaceutically acceptable auxiliary. In a particularly preferred embodiment, the above-mentioned fixed combination, non-fixed combination or kit of parts comprise:

a compound of the invention and clopidogrel,
a compound of the invention and enoxaparin,
a compound of the invention and cilostazol,
a compound of the invention and nadroparin,
a compound of the invention and warfarin, or
a compound of the invention and abciximab,
and at least one pharmaceutically acceptable auxiliary.

In a preferred embodiment, the above-mentioned fixed combination, non-fixed combination or kit of parts comprise a compound of the invention (in particular the compound of the invention is one of the examples of the invention), a diuretic and at least one pharmaceutically acceptable auxiliary. In a particularly preferred embodiment, the above-mentioned fixed combination, non-fixed combination or kit of parts comprise:

a compound of the invention and furosemide,
a compound of the invention and bumetanide, or
a compound of the invention and torsemide,
and at least one pharmaceutically acceptable auxiliary.

The combination comprising a compound of the invention and a diuretic preferably is for the treatment and prophylaxis of cystic fibrosis.

In a preferred embodiment, the above-mentioned fixed combination, non-fixed combination or kit of parts comprises a compound of the invention (in particular the compound of the invention is one of the examples of the invention), pirfenidone and at least one pharmaceutically acceptable auxiliary. In a particularly preferred embodiment, the above-mentioned fixed combination, non-fixed combination or kit of parts comprise:

a compound of the invention and pirfenidone,
and at least one pharmaceutically acceptable auxiliary.

The combination comprising a compound of the invention and pirfenidone preferably is for the treatment and prophylaxis of lung fibrosis.

In a preferred embodiment, the above-mentioned fixed combination, non-fixed combination or kit of parts comprise a compound of the invention (in particular the compound of the invention is one of the examples of the invention), a digitalis glycoside and at least one pharmaceutically acceptable auxiliary. In a particularly preferred embodiment, the above-mentioned fixed combination, non-fixed combination or kit of parts comprise:

a compound of the invention and digoxin, or
a compound of the invention and digitoxin,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a corticosteroid, a $\beta_2$-adrenoreceptor agonist and at least one pharmaceutically acceptable auxiliary. In a particularly preferred embodiment, the above-mentioned fixed combination, non-fixed combination or kit of parts comprise:

a compound of the invention, budesonide and salbutamol,
a compound of the invention, budesonide and milveterol,
a compound of the invention, budesonide and indacaterol,
a compound of the invention, budesonide and carmoterol,
a compound of the invention, budesonide and salmeterol,
a compound of the invention, budesonide and formoterol,
a compound of the invention, budesonide and vilanterol,
a compound of the invention, budesonide and olodaterol,
a compound of the invention, fluticasone and salbutamol,
a compound of the invention, fluticasone and milveterol,
a compound of the invention, fluticasone and indacaterol,
a compound of the invention, fluticasone and carmoterol,
a compound of the invention, fluticasone and salmeterol,
a compound of the invention, fluticasone and formoterol,
a compound of the invention, fluticasone and vilanterol,
a compound of the invention, fluticasone and olodaterol,
a compound of the invention, beclometasone and salbutamol,
a compound of the invention, beclometasone and milveterol,
a compound of the invention, beclometasone and indacaterol,
a compound of the invention, beclometasone and carmoterol,
a compound of the invention, beclometasone and salmeterol,
a compound of the invention, beclometasone and formoterol,
a compound of the invention, beclometasone and vilanterol,
a compound of the invention, beclometasone and olodaterol,
a compound of the invention, mometasone and salbutamol,
a compound of the invention, mometasone and milveterol,
a compound of the invention, mometasone and indacaterol,

a compound of the invention, mometasone and carmoterol,
a compound of the invention, mometasone and salmeterol,
a compound of the invention, mometasone and formoterol,
a compound of the invention, mometasone and vilanterol,
a compound of the invention, mometasone and olodaterol,
a compound of the invention, triamcinolone acetonide and salbutamol,
a compound of the invention, triamcinolone acetonide and milveterol,
a compound of the invention, triamcinolone acetonide and indacaterol,
a compound of the invention, triamcinolone acetonide and carmoterol,
a compound of the invention, triamcinolone acetonide and salmeterol,
a compound of the invention, triamcinolone acetonide and formoterol,
a compound of the invention, triamcinolone and vilanterol,
a compound of the invention, triamcinolone and olodaterol,
a compound of the invention, ciclesonide and salbutamol,
a compound of the invention, ciclesonide and milveterol,
a compound of the invention, ciclesonide and indacaterol,
a compound of the invention, ciclesonide and carmoterol,
a compound of the invention, ciclesonide and salmeterol,
a compound of the invention, ciclesonide and formoterol,
a compound of the invention, ciclesonide and vilanterol, or
a compound of the invention, ciclesonide and olodaterol,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a $\beta_2$-adrenoreceptor agonist, an anticholinergic and at least one pharmaceutically acceptable auxiliary. In a particularly preferred embodiment, the above-mentioned fixed combination, non-fixed combination or kit of parts comprise:

a compound of the invention, salbutamol and glycopyrronium bromide,
a compound of the invention, salbutamol and aclidinium bromide,
a compound of the invention, salbutamol and tiotropium bromide,
a compound of the invention, salbutamol and ipratropium bromide,
a compound of the invention, salbutamol and darotropium bromide,
a compound of the invention, milveterol and glycopyrronium bromide,
a compound of the invention, milveterol and aclidinium bromide,
a compound of the invention, milveterol and tiotropium bromide,
a compound of the invention, milveterol and ipratropium bromide,
a compound of the invention, milveterol and darotropium bromide,
a compound of the invention, salmeterol and glycopyrronium bromide,
a compound of the invention, salmeterol and aclidinium bromide,
a compound of the invention, salmeterol and tiotropium bromide,
a compound of the invention, salmeterol and ipratropium bromide,
a compound of the invention, salmeterol and darotropium bromide,
a compound of the invention, formoterol and glycopyrronium bromide,
a compound of the invention, formoterol and aclidinium bromide,
a compound of the invention, formoterol and tiotropium bromide,
a compound of the invention, formoterol and ipratropium bromide,
a compound of the invention, formoterol and darotropium bromide,
a compound of the invention, indacaterol and glycopyrronium bromide,
a compound of the invention, indacaterol and aclidinium bromide,
a compound of the invention, indacaterol and tiotropium bromide,
a compound of the invention, indacaterol and ipratropium bromide,
a compound of the invention, indacaterol and darotropium bromide,
a compound of the invention, carmoterol and glycopyrronium bromide,
a compound of the invention, carmoterol and aclidinium bromide,
a compound of the invention, carmoterol and tiotropium bromide,
a compound of the invention, carmoterol and ipratropium bromide,
a compound of the invention, carmoterol and darotropium bromide,

a compound of the invention, vilanterol and glycopyrronium bromide,
a compound of the invention, vilanterol and aclidinium bromide,
a compound of the invention, vilanterol and tiotropium bromide,
a compound of the invention, vilanterol and ipratropium bromide,
a compound of the invention, vilanterol and darotropium bromide,
a compound of the invention, olodaterol and glycopyrronium bromide,
a compound of the invention, olodaterol and aclidinium bromide,
a compound of the invention, olodaterol and tiotropium bromide,
a compound of the invention, olodaterol and ipratropium bromide, or
a compound of the invention, olodaterol and darotropium bromide,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a corticosteroid, an anticholinergic and at least one pharmaceutically acceptable auxiliary. In a particularly preferred embodiment, the above-mentioned fixed combination, non-fixed combination or kit of parts comprise:

a compound of the invention, budesonide and glycopyrronium bromide,
a compound of the invention, budesonide and aclidinium bromide,
a compound of the invention, budesonide and tiotropium bromide,
a compound of the invention, budesonide and ipratropium bromide,
a compound of the invention, budesonide and darotropium bromide,
a compound of the invention, fluticasone and glycopyrronium bromide,
a compound of the invention, fluticasone and aclidinium bromide,
a compound of the invention, fluticasone and tiotropium bromide,
a compound of the invention, fluticasone and ipratropium bromide,
a compound of the invention, fluticasone and darotropium bromide,
a compound of the invention, beclometasone and glycopyrronium bromide,
a compound of the invention, beclometasone and aclidinium bromide,
a compound of the invention, beclometasone and tiotropium bromide,
a compound of the invention, beclometasone and ipratropium bromide,
a compound of the invention, beclometasone and darotropium bromide,
a compound of the invention, mometasone and glycopyrronium bromide,
a compound of the invention, mometasone and aclidinium bromide,
a compound of the invention, mometasone and tiotropium bromide,
a compound of the invention, mometasone and ipratropium bromide,
a compound of the invention, mometasone and darotropium bromide,
a compound of the invention, triamcinolone acetonide and glycopyrronium bromide,
a compound of the invention, triamcinolone acetonide and aclidinium bromide,
a compound of the invention, triamcinolone acetonide and tiotropium bromide,
a compound of the invention, triamcinolone acetonide and ipratropium bromide,
a compound of the invention, triamcinolone acetonide and darotropium bromide,
a compound of the invention, ciclesonide and glycopyrronium bromide,
a compound of the invention, ciclesonide and aclidinium bromide,
a compound of the invention, ciclesonide and tiotropium bromide,
a compound of the invention, ciclesonide and ipratropium bromide, or
a compound of the invention, ciclesonide and darotropium bromide,
and at least one pharmaceutically acceptable auxiliary.

The above-mentioned triple combinations may preferably be used in the treatment or prophylaxis of bronchial asthma or COPD.

Exemplary combinations, in particular for external topical administration (for example versus atopic dermatitis or psoriasis), may include a compound of the invention and an immunosuppressant, for example a calcineurin inhibitor, such as pimecrolimus or tacrolimus.

Therefore, in another preferred embodiment, the above-mentioned fixed combination, non-fixed combination or kit of parts comprise a compound of the invention (in particular the compound of the invention is one of the examples of the invention or a pharmaceutically acceptable salt thereof), an immunosuppressant and at least one pharmaceutically acceptable auxiliary. In a particularly preferred embodiment, the above mentioned fixed combination, non-fixed combination or kit of parts comprise:

a compound of the invention and pimecrolimus,
a compound of the invention and tacrolimus,
a compound of the invention and methotrexate,
a compound of the invention and ascomycin, or
a compound of the invention and cyclosporin A,
and at least one pharmaceutically acceptable auxiliary.

The externally topically administrable immunosuppressant can be administered or administrable in a external-topical composition separately from the compound of the invention (non-fixed combination or kit of parts) or it can be contained with the compound of the invention in a combined externally-topically administrable composition (fixed combination). In a preferred embodiment the externally topically administrable composition is a cream containing pimecrolimus at ca. 1% w/w concentration. In another preferred embodiment the externally topically administrable composition is an ointment containing tacrolimus at from about 0.03% to about 0.1% w/w concentration).

Other combinations for external topical adminstration, in particular for the treatment or prophylaxis of atopic dermatitis and psoriasis, may include a compound of the invention and a corticosteroid. Beside the corticosteroid combinations mentioned above also the following corticosteroid combinations may be useful.

In another preferred embodiment, the above-mentioned fixed combination, non-fixed combination or kit of parts comprise a compound of the invention (in particular the compound of the invention is one of the examples of the invention or a pharmaceutically acceptable salt thereof), a corticosteroid and at least one pharmaceutically acceptable auxiliary. In a particularly preferred embodiment, the above mentioned fixed combination, non-fixed combination or kit of parts comprise:

a compound of the invention and prednisolone,
a compound of the invention and dexamethasone,
a compound of the invention and betamethasone, or
a compound of the invention and hydrocortisone,
and at least one pharmaceutically acceptable auxiliary.

In another preferred embodiment, the above-mentioned corticosteroids are used in form of an ester, such as, for example, prednisolone valerate acetate, hydrocortisone butyrate, hydrocortisone acetate, dexamethasone valerate, dexamethasone propionate, dexamethasone dipropionate, betamethasone butyrate propionate or prednisolone valerate acetate.

Further combinations for external topical combination, in particular for the treatment of psoriasis, may include a compound of the invention and a vitamin D analogue.
Therefore, in another preferred embodiment the above-mentioned fixed combination, non-fixed combination or kit of parts comprise a compound of the invention (in particular the compound of the invention is one of the examples of the invention or a pharmaceutically acceptable salt thereof), a vitamin D analogue and at least one pharmaceutically acceptable auxiliary. In a particularly preferred embodiment, the above mentioned fixed combination, non-fixed combination or kit of parts comprise:

a compound of the invention and calcitriol,
a compound of the invention and calcipotriol, or
a compound of the invention and tacalcitol,
and at least one pharmaceutically acceptable auxiliary.

In case, both (or all) combination partners - the compound of the invention as well as the therapeutic agent(s) -of the above-defined combinations are both (or all) suitable for inhalative administration, a preferred embodiment of the invention is the simultaneous inhaled administration of both (or all) combination partners by use of a combination inhalation device. Such a combination inhalation device can comprise a combined pharmaceutical composition for simultaneous inhaled administration, the composition comprising both (or all) individual compounds of the particular combination.

In an alternative, the combination inhalation device can be such that the individual compounds of the particular combination are administrable simultaneously but are stored separately (or wholly or partly separated for triple combinations), for example in separate pharmaceutical compositions.

In case of non-fixed combinations or kit of parts comprising at least one of the compounds of the invention and at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, lung surfactants, antibiotics, guanylyl-cyclase activators/stimulators, tetrahydrobiopterin and tetrahydrobiopterin derivatives, anticoagulants, diuretics, pirfenidone and digitalis glycosides, the compound of the invention and the therapeutic agent may be administered by the same route, e.g., without limitation, by inhalation (or external topical), or by different routes, e.g., without limitation, the compound of the invention may be, for example, administered by inhalation and the therapeutic agent may be administered orally.

In case of co-administration of at least one compound of the invention with at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, lung surfactants, antibiotics, guanylyl-cyclase activators/stimulators, tetrahydrobiopterin and tetrahydrobiopterin derivatives, anticoagulants, diuretics, pirfenidone and digitalis glycosides, in form of a fixed combination, non-fixed combination or kit of parts the dose of the compound of the invention as well as the dose of the therapeutic agent will be in a range customary for the mono-therapy, it more likely being possible, on account of the individual action, which are mutually positively influencing and reinforcing, to reduce the respective doses in case of co-administration of the compound(s) of the invention and the therapeutic agent.

In case of co-administration of at least one compound of the invention and at least one therapeutic compound selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, lung surfactants, antibiotics, guanylyl-cyclase activators/stimulators, tetrahydrobiopterin and tetrahydrobiopterin derivatives, anticoagulants, diuretics, pirfenidone and digitalis glycosides, in form of a fixed combination, a non-fixed combination or a kit of parts a single dose unit of the respective pharmaceutical composition/formulation can contain, in case of oral or parenteral administration 0.01 mg to 250 mg, preferably 0.05 mg to 100 mg, more preferably 0.05 mg to 10 mg, or in case of nasal or inhalative administration 0.001 mg to 10 mg, preferably 0.01 mg to 7.5 mg, more preferably 0.1 mg to 4 mg of the compound of the invention and from 0.01 mg to 4000 mg, preferably 0.1 mg to 2000 mg, more preferably 0.5 mg to 1000 mg, most preferably 1 mg to 500 mg, of the therapeutic agent, depending on the therapeutic agent being used the disease to be treated and the administration route selected. Preferably, the at least one compound of the invention and the at least one therapeutic agent are present in the pharmaceutical compositions/formulations in a weight ratio of from 1000:1 to 1:1000, more preferably in a weight ratio of from 100:1 to 1:100, even more preferably in a weight ratio of from 25:1 to 1:25.

## Biological Investigations

### Method for measuring inhibition of PDE4 activity

The PDE4B1 (GB no. L20966) was a gift of Prof. M. Conti (Stanford University, USA). It was amplified from the original plasmid (pCMV5) via PCR with primers Rb18 (5'- CAGACATCCTAAGAGGGGAT -3') and Rb10 (5'- AGAGGGGGATTATGTATCCAC -3') and cloned into the pCR-Bac vector (Invitrogen, Groningen, NL).

The recombinant baculovirus was prepared by means of homologous recombination in SF9 insect cells. The expression plasmids were cotransfected with Baculo-Gold DNA (Pharmingen, Hamburg) using a standard protocol (Pharmingen, Hamburg). Wt virus-free recombinant virus supernatants were selected using plaque assay methods. After that, high-titre virus supernatants were prepared by amplifying 3 times. PDE4B1 was expressed in SF21 cells by infecting 2 x $10^6$ cells/ml with an MOI (multiplicity of infection) between 1 and 10 in the serum-free SF900 medium (GIBCO Life Technologies, Karlsruhe, Germany) The cells were cultured at 28°C for 48 - 72 hours, after which they were pelleted for 5-10 min at 1000xg and 4°C.

The SF21 insect cells were resuspended, at a concentration of approx. $10^7$ cells/ml, in ice-cold (4°C) homogenization buffer (20 mM Tris, pH 8.2, containing the following additions: 140 mM NaCl, 3.8 mM KCl, 1 mM EGTA, 1 mM $MgCl_2$, 10 mM $\beta$-mercaptoethanol, 2 mM benzamidine, 0.4 mM Pefablock, 10 $\mu$M leupeptin, 10 $\mu$M pepstatin A, 5 $\mu$M trypsin inhibitor) and disrupted by ultrasonication. The homogenate was then centrifuged for 10 min at 1000xg and the supernatant was stored at-80°C until subsequent use (see below). The protein content was determined by the Bradford method (BioRad, Munich) using BSA (Bovine serum albumin) as the standard.

PDE4B1 activities were measured in a 96-well platform using SPA (scintillation proximity assay) yttrium silicate beads (RPNQ1050 from GE Healthcare). In a first step the PDE activity operated hydrolysis of either [$^3$H] cAMP (substrate) into [$^3$H] 5'AMP. In a second step substrate and product were distinguished following addition of SPA yttrium silicate beads. Indeed, in the presence of zinc sulphate the linear [$^3$H] 5'AMP bound to the beads while the cyclic [$^3$H] cAMP did not. Close proximity of bound [$^3$H] 5'AMP then allowed radiation from the tritium to the scintillant within the beads

resulting in a measureable signal while the unbound, hence distant [$^3$H] cAMP did not generate this signal. The test volume was 100 μl and finally contained 20 mM Tris buffer (pH 7.4), 0.1 mg /ml of BSA, 5 mM $Mg^{2+}$, 0.5 μM cAMP (including about 50,000 cpm of [3H]cAMP) as substrate, 1 μl of the respective substance dilution in DMSO and sufficient recombinant PDE (1000xg supernatant, see above) to ensure that 10-20% of the cAMP was converted under the said experimental conditions. The final concentration of DMSO in the assays (1 % v/v) did not substantially affect the activity of the PDE investigated. After a preincubation of 5 min at 37°C, the reaction was commenced by adding the substrate (cAMP) and the assays were incubated for a further 15 min. Reactions were terminated by adding SPA beads (50 μl). In agreement with the manufacturer's instructions, the SPA beads had previously been resuspended in water, but were then diluted 1:3 (v/v) in water. This diluted solution further contained 3 mM IBMX (isobutylmethylxanthine) to ensure a complete block of PDE activity. Once the beads were sedimented (> 30 min), the MTP's (micro titerplate) were analyzed in commercially available luminescence detection devices. The corresponding $IC_{50}$ values of the compounds for inhibition of PDE4B1 activity were determined from the concentration-effect curves by means of non-linear regression.

For the following compounds PDE4B1 inhibitory values [measured as -log$IC_{50}$ (mol/l)] below 8, between 8 and 9 and above 9 have been determined. The numbers of the compounds correspond to the numbers of the examples.

| **PDE4B1 inhibitory values measured as -logIC50 (mol/l)** | | |
|---|---|---|
| Below 8 | Between 8 and 9 | Above 9 |
| Example 44, 45, | Example 2, 4, 5, 6, 7, 8, 12, 14, 16, 19, 20, 21, 22, 23, 24, 25, 26, 27, 29, 30, 31, 32, 33, 36, 37, 38, 40, 46, 47, 49, 55, 68, 69, 73, 76, 77, 78, 79, 83, 84, 88, 94, 96, 97, 99, 100, 101, 102, 104, 105, 106, 107, 110, 111, 114, 116, 117, 118, 120, 121, 122, 123, 124, 125 | 1, 3, 9, 10, 11, 13, 15, 17, 18, 28, 34, 35, 39, 41, 42, 43, 48, 50, 51, 52, 53, 59, 60, 61, 62, 63, 64, 65, 66, 67, 71, 72, 74, 75, 85, 89, 90, 91, 92, 93, 95, 98, 108, 109, 112, 113, 115, 119 |

**Method for measuring inhibition of PDE5 activity**

As a source for human PDE5, platelets were used. For that purpose, 150 ml fresh blood from human donors anticoagulated with citrate [final concentration 0.3% (w/v)] was centrifuged at 200 g for 10 min to obtain the so-called platelet-rich-plasma (PRP) as a supernatant. 1/10 volume of ACD solution (85 mM $Na_3$-citrate, 111 mM D-glucose, 71 mM citric acid, pH 4.4) was added to 9/10 volume of PRP. After centrifugation (1,400 g, 10 min) the cell pellet was resuspended in 3 ml homogenization buffer (NaCl 140 mM, KCl 3.8 mM, EGTA (ethylene glycol tetraacetic acid) 1 mM, $MgCl_2$ 1 mM, Tris-HCl 20 mM, beta-mercaptoethanol 1 mM, pH 8.2) plus protease-inhibitor mix resulting in final concentrations of 0.5 mM Pefablock (Roche), 10 μM Leupeptin, 5 μM Trypsine inhibitor, 2 mM Benzamidin and 10 μM Pepstatin A. The suspension was sonicated and thereafter centrifuged for 15 min at 10,000 g. The resulting supernatant (platelet lysate) was used for enzymatic assays.

PDE5 activities were measured in a 96-well platform using SPA (scintillation proximity assay) yttrium silicate beads (RPNQ1050 from GE Healthcare). In a first step the PDE activity operated hydrolysis of either [$^3$H] cGMP (substrate) into [$^3$H] 5'GMP. In a second step substrate and product were distinguished following addition of SPA yttrium silicate beads. Indeed, in the presence of zinc sulphate the linear [$^3$H] 5'GMP bound to the beads while the cyclic [$^3$H] cGMP did not. Close proximity of bound [$^3$H] 5'GMP then allowed radiation from the tritium to the scintillant within the beads resulting in a measureable signal while the unbound, hence distant [$^3$H] cGMP did not generate this signal. The test volume was 100 μl and contained 20 mM Tris buffer (pH 7.4), 0.1 mg of BSA (bovine serum albumin)/ml, 5 mM $Mg^{2+}$, 1 uM motapizone (PDE3 Inhibitor), 10 nM PDE2 inhibitor 2-(3,4-dimethoxybenzyl)-7-[(1*R*,2*R*)-2-hydroxy-1-(2-phenylethyl)propyl]-5-methylimidazo[5,1-*f*][1,2,4]triazin-4(3*H*)-one, 0.5 μM cGMP (cyclic guanosine monophosphate) (including about 50,000 cpm of [3H]cGMP as a tracer) (substrate), 1 μl of the respective compound dilution in dimethylsulfoxide (DMSO) and sufficient PDE5-containing platelet lysat (10,000×g supernatant, see above) to ensure that 10-20% of the cGMP was converted under the said experimental conditions. The final concentration of DMSO in the assay (1% v/v) did not substantially affect the activity of the PDE investigated. After a preincubation of 5 min at 37°C, the reaction was commenced by adding the substrate (cGMP) and the assay was incubated for a further 15 min. The reaction was terminated by adding SPA beads (50 μl). In agreement with the manufacturer's instructions, the SPA beads had previously been resuspended in water, but were then diluted 1:3 (v/v) in water. This diluted solution also contained 3 mM 8-methoxymethyl-3-isobutyl-1-methylxanthine (IBMX) to ensure a complete block of PDE activity. Once the beads were sedimented (> 30 min), the MTP's were analyzed in commercially available luminescence detection devices. The corresponding $IC_{50}$ values of the compounds for inhibition of PDE activity were determined from the concentration-effect curves by means of non-linear regression.

For the following compounds PDE5 inhibitory values [measured as -log$IC_{50}$ (mol/l)] below 8, between 8 and 9

and above 9 have been determined. The numbers of the compounds correspond to the numbers of the examples.

| PDE5 inhibitory values measured as -logIC50 (mol/l) | | |
|---|---|---|
| Below 8 | Between 8 and 9 | Above 9 |
| Example 14 | Example 1, 2, 3, 4, 5, 6, 11, 12, 13, 15, 16, 18, 19, 22, 23, 24, 25, 30, 32, 36, 37, 44, 45, 46, 47, 49, 50, 51, 53, 55, 59, 60, 61, 62, 67, 68, 69, 71, 72, 73, 75, 76, 77, 78, 79, 88, 97, 93, 96, 99, 100, 101, 102, 104, 105, 106, 107, 110, 111, 115, 116, 117, 120, 121, 122, 123, 124, 125 | 7, 8, 9, 10, 17, 20, 21, 26, 27, 28, 29, 31, 33, 34, 35, 38, 39, 40, 41, 42, 43, 48, 52, 63, 64, 65, 66, 74, 83, 84, 85, 87, 89, 90, 91, 92, 94, 95, 98, 108, 109, 112, 113, 114, 118, 119 |

**In Vivo Assay: LPS-induced pulmonary inflammation model in rats (Method A+B)**

**Introduction**

Exposure of rats to aerosolized lipopolysaccharide (LPS) causes a pulmonary mainly neutrophilic inflammation, which can be assessed by bronchoalveolar lavage (BAL). LPS-induced pulmonary inflammation models are robust and are commonly used for the evaluation of test compounds modulating the immediate immune response. Selective phosphodiesterase-4 inhibitors are administered by intratracheal instillation 1 h prior nose-only LPS challenge in rats. The anti-inflammatory activity of the selective phosphodiesterase inhibitors is assessed based on pulmonary total leukocyte and neutrophil counts in the bronchoalveolar lavage fluid 4 h after LPS exposure

**Materials and Methods**

**Animals**

Male Sprague Dawley rats weighing 200 - 300 g were used. Rats were delivered 1 weak prior to the experiments and had free access to water and food.

**Intratracheal compound instillation**

**Compound preparation**

The test compound was suspended in Aqua ad injectabilia (Braun, Melsungen, Germany) or 0.9 % NaCl (Saline) (Braun, Melsungen, Germany) supplemented with 0.02% Tween20 (Sigma-Aldrich, Schnelldorf, Germany) for intratracheal administration. Suspensions of test compound were treated in an ultrasonic bath or a Covaris S2x high-energetic ultrasonic bath (KBiosciences, Hoddesdon Herts, UK) to obtain a homogenous suspension. The aimed doses were prepared by dilution series from the stock suspension, which was prepared for the administration of the highest dose in each experiment.

**Compound instillation technique (Method A)**

The compound suspension was administered intratracheally via a microsprayer device (Penn Century, distributed by EMMS, Bordon Hants, UK). Therefore, the rats were intubated by inserting the microsprayer into the trachea. The length of the microsprayer device was adjusted to avoid disruption of the tracheal bifurcation. The intubation was guided by sight and was done under a short time isoflurane anesthesia. A syringe, filled with the compound suspension, was connected to the microsprayer device via the Luer Lock adapter and the compound suspension was directly administered to the lungs.

**Compound instillation technique (Methode B)**

The compound suspension was administered intratracheally. The intubation was guided by sight and was done under a short time isoflurane anesthesia. The compound suspension was administered to the lungs by liquid instillation. Therefore, the trachea was intubated with a device consisting of a catheter which contained a blunted cannula (size 14G, Dispomed, Gelnhausen, Germany). The length of the catheter was adjusted to avoid disruption of the tracheal bifurcation. A 1 ml syringe, filled with the compound suspension and air, was connected to the intubation device via the

Luer Lock adapter and the whole content of the syringe was directly administered to the lungs.

**Compound dosing**

The administered volume of the compound suspension was 0.5 - 1 ml/kg. Control animals received drug-free Aqua/Tween20 or NaCl/Tween20 solution as placebo. Test compounds and placebo were administered 1 h prior to LPS challenge.

**LPS challenge**

Conscious and restrained animals were connected to a nose-only exposure system (CR equipment SA, Tannay, Switzerland) and were exposed to the LPS aerosol for 30 min. The LPS-containing aerosol was generated using a compressed air driven medication nebulizer device (Pari master in combination with Pari LC Sprint Star, Pari GmbH, Starnberg, Germany). The LPS solution (E. coli, Serotype 055B5, Art.# L2880, Lot# L048K4126 or 109K4075, Sigma-Aldrich, Germany, 1 mg/ml - 3 mg/ml diluted in Phosphate-Buffered Saline (PBS)) was prepared 30 minutes in advance. The aerosol was dispersed and transported to the exposure tower by a sheath air flow of 600l/h. All rats except negative controls were exposed to LPS.

**Bronchoalveolar lavage**

4 hours after LPS challenge, animals were anesthetized by isoflurane and sacrificed by cervical dislocation. BALs were performed. For the BAL, the trachea was exposed and cannulated, followed by gently lavage of the lungs two times in situ with 5 ml PBS buffer supplemented with 0.5% Bovine Serum Albumin (Serva, Darmstadt, Germany).

**Total and differential cell counts**

Determination of total leukocyte and neutrophil counts in BAL fluid was performed with an automated haemocytometer (XT-2000iV, Sysmex, Norderstedt, Germany).

**Data Analysis**

The baseline correction was done for each sample according to the formula:

*Baseline-corrected cell count value = cell count - Median (negative control group)*

All further calculations were performed with the baseline-corrected values.

The effect of a compound on LPS-induced total cell and neutrophil influx into the lungs was calculated in % using the median of the cell count of each treatment group in relation to the median of the control group according to the formula:

$$\% \text{ effect} = (Y-K)/K*100$$

With defining:

Y= Median of the baseline-corrected cell count value of compound-treated group
K= Median of the baseline-corrected cell count value of placebo-treated group

Statistical analysis was performed on the primary cell count data using one-way ANOVA and Dunnett's multiple comparison post test vs. positive control. The Grubbs test was used to detect statistical out-liers.

**Exemplary Results for compounds tested using Method A** (the numbers of the compounds correspond to the numbers of the examples):

**The compounds 60 and 61** showed at a dosage of 1 mg/kg a reduction in the range of 28 to 55 % of the total cell count, respectively a reduction in the range of 27 to 49 % of neutrophils in comparison to the placebo group.

**Exemplary Results for compounds tested using Method B** (the numbers of the compounds correspond to the numbers of the examples):

The compounds **17, 31 and 59** showed at a dosage of 1 mg/kg a reduction in the range of 36 to 75 % of the total cell count, respectively a reduction in the range of 30 to 67 % of neutrophils in comparison to the placebo group.

## Claims

**1.** A compound of formula (1)

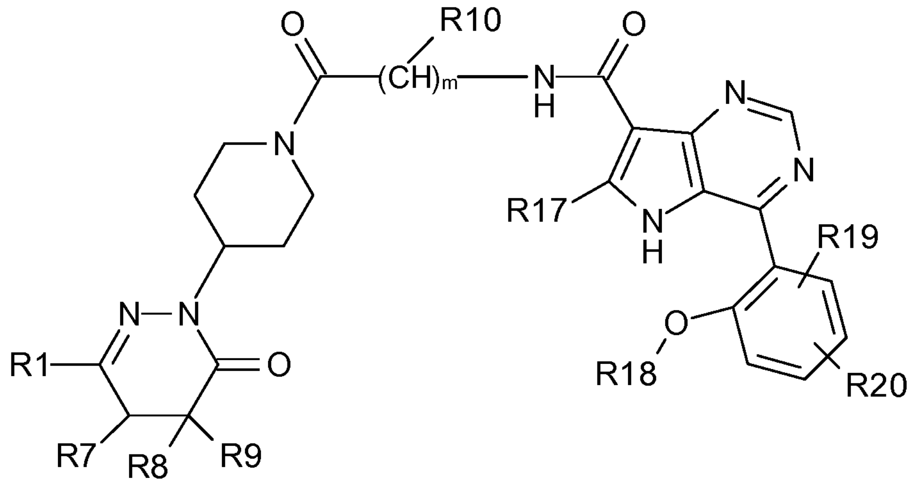

(1),

wherein

R1 represents a phenyl derivative of formulae (a) or (b)

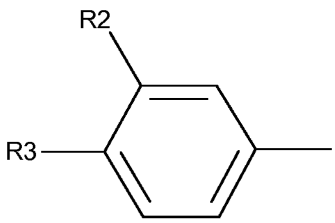

(a)

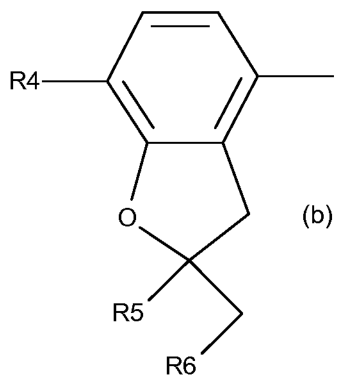

(b)

wherein

R2 is 1-4C-alkoxy or 1-4C-alkoxy predominantly or completely substituted by fluorine,
R3 is 1-4C-alkoxy, 3-5C-cycloalkoxy, 3-5C-cycloalkoxymethoxy or 1-4C-alkoxy predominantly or completely substituted by fluorine,
R4 is 1-4C-alkoxy or 1-4C-alkoxy predominantly or completely substituted by fluorine,
R5 is 1-4C-alkyl,
R6 is hydrogen or 1-4C-alkyl or wherein R5 and R6 together and with inclusion of the two carbon atoms, to which they are bonded, form a spiro-linked 5-, 6- or 7-membered hydrocarbon ring, optionally interrupted by an oxygen or sulphur atom,
R7 is hydrogen,

R8 is 1-4C-alkyl,
or R7 and R8 together form a 3C- to 5C-alkylene group,
R9 is hydrogen or 1-4C-alkyl,
or wherein R8 and R9 together and with inclusion of the carbon atom, to which they are bonded, form a spiro-linked 5-, 6- or 7-membered hydrocarbon ring,

m is 1 or 2,

R10 is independently from each other hydrogen, 5-7C-cycloalkyl, 1-6C-alkyl, -CH($CH_3$)-R11 or -$CH_2$-R12, wherein
R11 is unsubstituted phenyl or hydroxyl,
R12 is hydroxyl, 5-7C-cycloalkyl, -N-(1-2C-alkyl)$_2$, -$CH_2$-S-(1-2C-alkyl), benzyl, unsubstituted phenyl, phenyl substituted by R13, phenyl substituted by R13 and R14,
wherein

R13 is halogen, 1-4C-alkoxy, 1-6C-alkyl, 1-4C-fluoroalkyl, hydroxyl, phenyl, -C(O)$NH_2$, -CN, 2-oxoazetidin-1-yl or 2-oxopyrrolidin-1-yl,
R14 is halogen, 1-4C-alkoxy, 1-6C-alkyl, 1-4C-fluoroalkyl, hydroxyl, phenyl, -C(O)$NH_2$ or -CN,

or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is -$CH_2$-C(O)-R15,
wherein

R15 is hydroxyl, -N(R16)$_2$, piperidin-1-yl, pyrrolidin-1-yl or benzyloxy, wherein
R16 is independently from each other hydrogen or 1-4C-alkyl,

R17 is hydrogen or methyl,
R18 is -$CH_2$-3-6C-cycloalkyl,
R19 is halogen, hydroxyl, 1-4C-alkoxy, 1-4C-alkoxy predominantly or completely substituted by fluorine or 1-4C-fluoroalkyl,
R20 is halogen, hydroxyl, 1-4C-alkoxy or 1-4C-alkoxy predominantly or completely substituted by fluorine,
or R19 and R20 together form a 1-2C-alkylenedioxy group

or a stereoisomer of the compound or a salt of the compound or a salt of the stereoisomer of the compound.

**2.** A compound of formula (1) according to claim 1, wherein

R1 represents a phenyl derivative of formulae (a) or (b),
wherein

R2 is 1-4C-alkoxy or 1-4C-alkoxy predominantly or completely substituted by fluorine,
R3 is 1-4C-alkoxy or 1-4C-alkoxy predominantly or completely substituted by fluorine,
R4 is 1-2C-alkoxy or 1-2C-alkoxy predominantly or completely substituted by fluorine,
R5 is 1-2C-alkyl,
R6 is hydrogen or 1-2C-alkyl,
or wherein R5 and R6 together and with inclusion of the two carbon atoms, to which they are bonded, form a spiro-linked 5-, 6- or 7-membered hydrocarbon ring,

R7 is hydrogen,
R8 is 1-4C-alkyl,
or R7 and R8 together form a 3C- to 5C-alkylene group,
R9 is hydrogen or 1-4C-alkyl,
or wherein R8 and R9 together and with inclusion of the carbon atom, to which they are bonded, form a spiro-linked 5-, 6- or 7-membered hydrocarbon ring,
R10 is independently from each other hydrogen, 5-7C-cycloalkyl, 1-4C-alkyl, -CH($CH_3$)-R11 or -$CH_2$-R12, wherein

R11 is unsubstituted phenyl or hydroxyl,
R12 is hydroxyl, 5-7C-cycloalkyl, -N-(1-2C-alkyl)$_2$, -$CH_2$-S-(1-2C-alkyl), benzyl, unsubstituted phenyl, phenyl substituted by R13, phenyl substituted by R13 and R14,
wherein

R13 is halogen, 1-4C-alkoxy, 1-4C-alkyl, 1-4C-fluoroalkyl, hydroxyl, phenyl, -C(O)$NH_2$, -CN, 2-oxoazetidin-1-yl or 2-oxopyrrolidin-1-yl,
R14 is halogen, 1-4C-alkoxy, 1-4C-alkyl, 1-4C-fluoroalkyl, hydroxyl, phenyl, -C(O)$NH_2$ or -CN,

or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is -$CH_2$-C(O)-R15,
wherein

R15 is hydroxyl, -N(R16)$_2$, piperidin-1-yl, pyrrolidin-1-yl or benzyloxy, wherein
R16 is independently from each other hydrogen or 1-4C-alkyl,

R17 is hydrogen or methyl,
R18 is -$CH_2$-3-6C-cycloalkyl,
R19 is halogen, hydroxyl, 1-4C-alkoxy, 1-4C-alkoxy predominantly or completely substituted by fluorine or 1-4C-fluoroalkyl,
R20 is halogen, hydroxyl, 1-4C-alkoxy or 1-4C-alkoxy predominantly or completely substituted by fluorine,
or R19 and R20 together form a 1-2C-alkylenedioxy group,

or a stereoisomer of the compound or a salt of the compound or a salt of the stereoisomer of the compound.

**3.** A compound of formula (1) according to claim 1, wherein

R1 represents a phenyl derivative of formulae (a) or (b),
wherein

R2 is 1-2C-alkoxy or 1-2C-alkoxy predominantly or completely substituted by fluorine,
R3 is 1-2C-alkoxy or 1-2C-alkoxy predominantly or completely substituted by fluorine,
R4 is 1-2C-alkoxy or 1-2C-alkoxy predominantly or completely substituted by fluorine,
R5 is 1-2C-alkyl,
R6 is hydrogen or 1-2C-alkyl,
or wherein R5 and R6 together and with inclusion of the two carbon atoms, to which they form 5- 6- membered

R7 are bonded, a spiro-linked or hydrocarbon ring, is hydrogen,
R8 is 1-2C-alkyl,
or R7 and R8 together form a 3C- to 5C-alkylene group,
R9 is hydrogen or 1-2C-alkyl,
or wherein R8 and R9 together and with inclusion of the carbon atom, to which they are bonded, form a spiro-linked 5- or 6- membered hydrocarbon ring,
m is 1 or 2,
R10 is independently from each other hydrogen, 5-7C-cycloalkyl, 1-4C-alkyl, -CH($CH_3$)-R11 or -$CH_2$-R12,
wherein

R11 is unsubstituted phenyl or hydroxyl,
R12 is hydroxyl, 5-7C-cycloalkyl, -N-(1-2C-alkyl)$_2$, -$CH_2$-S-(1-2C-alkyl), benzyl, unsubstituted phenyl, phenyl substituted by R13, phenyl substituted by R13 and R14,
wherein

R13 is halogen, 1-4C-alkoxy, 1-4C-alkyl, 1-4C-fluoroalkyl, hydroxyl, phenyl, -C(O)$NH_2$, -CN, 2-oxoazetidin-1-yl or 2-oxopyrrolidin-1-yl,
R14 is halogen or 1-4C-alkoxy, 1-4C-alkyl,

or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is $-CH_2-C(O)-R15$,
wherein

R15 is hydroxyl, $-N(R16)_2$, piperidin-1-yl, pyrrolidin-1-yl or benzyloxy, wherein
R16 is independently from each other hydrogen or 1-3C-alkyl,

R17 is hydrogen or methyl,
R18 is $-CH_2$-3-5C-cycloalkyl,
R19 is halogen or 1-4C-alkoxy,
R20 is halogen or 1-4C-alkoxy,
or R19 and R20 together form a 1-2C-alkylenedioxy group,

or a stereoisomer of the compound or a salt of the compound or a salt of the stereoisomer of the compound.

**4.** A compound of formula (1) according to claim 1, wherein

R1 represents a phenyl derivative of formulae (a) or (b),
wherein

R2 is methoxy,
R3 is methoxy,
R4 is methoxy,
R5 is methyl,
R6 is hydrogen,

R7 is hydrogen,
R8 is 1-2C-alkyl,
or R7 and R8 together form a 3C- to 5C-alkylene group,
R9 is hydrogen or 1-2C-alkyl,
or wherein R8 and R9 together and with inclusion of the carbon atom, to which they are bonded, form a spiro-linked 5- membered hydrocarbon ring,
m is 1 or 2,
R10 is independently from each other hydrogen, 5-7C-cycloalkyl, 1-4C-alkyl, $-CH(CH_3)-R11$ or $-CH_2-R12$,
wherein

R11 is unsubstituted phenyl or hydroxyl,
R12 is hydroxyl, 5-7C-cycloalkyl, $-N-(1\text{-}2C\text{-alkyl})_2$, $-CH_2$-S-(1-2C-alkyl), benzyl, unsubstituted phenyl, phenyl substituted by R13, phenyl substituted by R13 and R14,
wherein

R13 is fluorine, chlorine, bromine, 1-2C-alkoxy, 1-4C-alkyl, 1-2C-fluoroalkyl, hydroxyl, phenyl, $-C(O)NH_2$, -CN, 2-oxoazetidin-1-yl or 2-oxopyrrolidin-1-yl,
R14 is fluorine, chlorine, bromine or 1-2C-alkoxy,

or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is $-CH_2-C(O)-R15$,
wherein

R15 is hydroxyl, $-N(R16)_2$, piperidin-1-yl, pyrrolidin-1-yl or benzyloxy, wherein
R16 is independently from each other hydrogen or 1-3C-alkyl,

R17 is hydrogen or methyl,
R18 is $-CH_2$-3-4C-cycloalkyl,

R19 is 1-2C-alkoxy,
R20 is fluorine, chlorine or bromine,
or R19 and R20 together form a methylenedioxy group,

or a stereoisomer of the compound or a salt of the compound or a salt of the stereoisomer of the compound.

**5.** A compound of formula (1) according to claim 1, wherein

R1 represents a phenyl derivative of formulae (a) or (b),
wherein

R2 is methoxy,
R3 is methoxy,
R4 is methoxy,
R5 is methyl,
R6 is hydrogen,

R7 is hydrogen,
R8 is 1-2C-alkyl,
or R7 and R8 together form a 3C- or 4C-alkylene group,
R9 is hydrogen or 1-2C-alkyl,
or wherein R8 and R9 together and with inclusion of the carbon atom, to which they are bonded, form a spiro-linked 5- membered hydrocarbon ring,
m is 1 or 2,
R10 is independently from each other hydrogen, cyclohexyl, 1-4C-alkyl, $-CH(CH_3)$-R11 or $-CH_2$-R12,
wherein

R11 is unsubstituted phenyl or hydroxyl,
R12 is hydroxyl, cyclohexyl, $-N-(CH_3)_2$, $-CH_2-S-CH_3$, benzyl, unsubstituted phenyl, phenyl substituted by R13, phenyl substituted by R13 and R14,
wherein

R13 is fluorine, chlorine, 1-2C-alkoxy, methyl, tert-butyl, trifluoromethyl, hydroxyl, phenyl, $-C(O)NH_2$, -CN, 2-oxoazetidin-1-yl or 2-oxopyrrolidin-1-yl,
R14 is fluorine, chlorine or methoxy,

or R12 is selected from imidazol-2-yl, imidazol-4-yl, pyrazol-1-yl, thiophen-2-yl, thiophen-3-yl, thiazol-2-yl, thiazol-4-yl, thiazol-5-yl, pyridin-2-yl, pyridin-3-yl, pyridin-4-yl, indol-2-yl, indol-3-yl, 1-methyl-indol-2-yl or 1-methyl-indol-3-yl, or
R12 is $-CH_2-C(O)$-R15,
wherein

R15 is hydroxyl, $-N(R16)_2$, piperidin-1-yl, pyrrolidin-1-yl or benzyloxy, wherein
R16 is independently from each other hydrogen or isopropyl,

R17 is hydrogen or methyl,
R18 is $-CH_2$-3C-cycloalkyl,
R19 is methoxy,
R20 is fluorine,
or R19 and R20 together form a methylenedioxy group,

or a stereoisomer of the compound or a salt of the compound or a salt of the stereoisomer of the compound.

**6.** A compound of formula (1) according to claim 5, which is selected from the group consisting of 4-[5-(cyclopropylmethoxy)-1,3-benzodioxol-4-yl]-N-[(2R)-3-(3,5-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide; 4-[2-(cyclopropylmethoxy)-5-fluoro-4-methoxyphenyl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-5H-pyrrolo[3,2-d]pyrimidine-7-carbox-amide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide; N-[(2R)-3-(4-tert-butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[2-(cyclopropylmethoxy)-5-fluoro-4-methoxyphenyl]-6-methyl-5H-pyrrolo[3,2-d]pyrimidine-7-carboxamide; N-[(2R)-3-(4-tert-butyl-phenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; N-[(2R)-3-(4-carbamoylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-6-methyl-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-6-methyl-5H-pyrrolo[3,2-d]pyrimidine-7-carboxamide; N-[(2R)-3-(4-carbamoylphenyl)-1-{4-[(4aS,8aR)-4-(3,4-dimethoxy-phenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide; N-[(2R)-3-(biphenyl-4-yl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropyl-methoxy)-1,3-benzodioxol-4-yl]-6-methyl-5H-pyrrolo[3,2-d]pyrimidine-7-carboxamide; N-[(2R)-3-(biphenyl-4-yl)-1-{4-[(4aS,8aR)-4-(3,4-dimethoxyphenyl)-1-oxo-4a,5,6,7,8,8a-hexahydro-phthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; N-[(2R)-3-(4-cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]-6-methyl-5H-pyrrolo[3,2-d]pyrimidine-7-carboxamide; N-[(2R)-3-(4-cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide; 4-[5-(cyclopropylmethoxy)-1,3-benzodioxol-4-yl]-N-[(2R)-3-(3,4-difluorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-6-methyl-5H-pyrrolo[3,2-d]pyrimidine-7-carboxamide; 4-[5-(cyclopropylmethoxy)-1,3-benzodioxol-4-yl]-N-[(2R)-3-(3,4-difluorophenyl)-1-{4-[(4aS,8aR)-4-(3,4-dimethoxyphenyl)-1-oxo-4a,5,6,7,8,8a-hexahydro-phthalazin-2(1H)-yl]piperidin-1-yl}-1-oxopropan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxy-phenyl)-1-oxo-4a,5,6,7,8,8a-hexahydrophthalazin-2(1H)-yl]piperidin-1-yl}-3-(3-methylphenyl)-1-oxopropan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]-5H-pyrrolo[3,2-d]pyrimidine-7-carboxamide; 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-6-methyl-5H-pyrrolo[3,2-d]pyrimidine-7-carboxamide; 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide; 4-[2-(cyclopropylmethoxy)-4-fluoro-5-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide; 4-[5-(cyclopropylmethoxy)-1,3-benzodioxol-4-yl]-N-[(2S)-3-(3,4-dimethoxyphenyl)-1-{4-[(4aS,8aR)-4-(3,4-dimethoxyphenyl)-1-oxo-4a,5,6,7,8,8a-hexahydro-phthalazin-2(1H)-yl]piperidin-1-yl}-1-oxopropan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxy-phenyl)-1-oxo-4a,5,6,7,8,8a-hexahydrophthalazin-2(1H)-yl]piperidin-1-yl}-1-oxo-3-phenylpropan-2-yl]-6-methyl-5H-pyrrolo[3,2-d]pyrimidine-7-carboxamide; N-[(2R)-3-(4-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxo-propan-2-yl]-5H-pyrrolo[3,2-d]pyrimidine-7-carboxamide; N-[(2R)-3-(3-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide; 4-[5-(cyclopropyl-methoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[2-(trifluoromethyl)phenyl]-propan-2-yl}-6-methyl-5H-pyrrolo[3,2-d]pyrimidine-7-carboxamide; 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[2-(trifluoromethyl)phenyl]propan-2-yl}-5H-pyrrolo[3,2-d]pyrimidine-7-carboxamide; N-[(2R)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-propan-2-yl]-4-[5-(cyclopropylmethoxy)-1,3-benzodioxol-4-yl]-6-methyl-5H-pyrrolo[3,2-d]pyrimidine-7-carboxamide; - N-[(2R)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide; 4-[2-(cyclopropylmethoxy)-5-fluoro-4-methoxyphenyl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-6-methyl-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]-6-methyl-5H-pyrrolo[3,2-d]pyrimidine-7-carboxamide; 4-[5-(cyclopropyl-methoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-6-methyl-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]-6-methyl-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]-5H-pyrrolo[3,2-d]pyrimidine-7-carboxamide; 4-[5-(cyclopropylmethoxy)-1,3-benzodioxol-4-yl]-N-[(2S)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide; N-[(2R)-3-(4-tert-butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide; 4-[5-(cyclopropyl-methoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorophenyl)-1-oxopropan-2-yl]-5H-pyrrolo[3,2-d]pyrimidine-7-carboxamide; 4-[5-(cyclopropylmethoxy)-1,3-benzodioxol-4-yl]-N-[(2R)-3-(2,4-dichlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide; 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(trifluoromethyl)phenyl]propan-2-yl}-5H-pyrrolo[3,2-d]pyrimidine-7-carboxamide; 4-[5-(cyclo-propylmethoxy)-1,3-benzodioxol-4-yl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-5H-pyrrolo[3,2-d]pyrimidine-7-carboxamide; 4-[5-(cyclopropylmethoxy)-1,3-benzodioxol-4-yl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-6-methyl-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxo-propan-2-yl]-5H-pyrrolo[3,2-d]pyrimidine-7-carboxamide; 4-[5-(cyclopropyl-methoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]-5H-pyrrolo[3,2-d]pyrimidine-7-carboxamide; N-[(2S)-3-(2-chlorophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,7aR)-4-(3,4-dimethoxyphenyl)-1-oxo-1,4a,5,6,7,7a-hexahydro-2H-cyclopenta[d]pyridazin-2-yl]piperidin-1-yl}-1-oxo-3-phenylpropan-2-yl]-5H-pyrrolo[3,2-d]pyrimidine-7-carboxamide; N-[(2S)-3-(2-chloro-phenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide; 4-[2-(cyclopropylmethoxy)-5-fluoro-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxopropan-2-yl]-6-methyl-5H-pyrrolo[3,2-d]pyrimidine-7-carboxamide; 4-[5-(cyclopropylmethoxy)-1,3-benzodioxol-4-yl]-N-(2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl)-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxopropan-2-yl]-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidine-7-carboxamide; 4-[5-(cyclopropylmethoxy)-1,3-benzodioxol-4-yl]-N-[(2S,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxobutan-2-yl]-5H-pyrrolo[3,2-d]pyrimidine-7-carboxamide; 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxobutan-2-yl]-6-methyl-5H-pyrrolo[3,2-d]pyrimidine-7-carboxamide; 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-4-phenylbutan-2-yl]-5H-pyrrolo[3,2-d]pyrimidine-7-carboxamide; 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidine-7-carboxamide; 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide; 4-[2-(cyclopropylmethoxy)-5-fluoro-4-methoxyphenyl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide or 4-[2-(cyclopropylmethoxy)-5-fluoro-4-methoxyphenyl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidine-7-carboxamide,4-[5-(cyclopropylmethoxy)-1,3-benzodioxol-4-yl]-N-[(2S,3S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxopentan-2-yl]-5H-pyrrolo[3,2-d]pyrimidine-7-carboxamide, N-[(2S)-3-cyclo-hexyl-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidine-7-carboxamide, N-[(1R)-1-cyclohexyl-2-{4-[(4aS,8aR)-4-(3,4-dimethoxy-phenyl)-1-oxo-4a,5,6,7,8,8a-hexahydrophthalazin-2(1H)-yl]piperidin-1-yl}-2-oxoethyl]-4-[5-(cyclo-propylmethoxy)-1,3-benzodioxol-4-yl]-5H-pyrrolo[3,2-d]pyrimidine-7-carboxamide, N-[(1S)-1-cyclohexyl-2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl]-4-[5-(cyclopropylmethoxy)-1,3-benzodioxol-4-yl]-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxy-phenyl)-1-oxo-4a,5,6,7,8,8a-hexahydrophthalazin-2(1H)-yl]piperidin-1-yl}-1-oxo-3-(thiophen-2-yl)propan-2-yl]-6-methyl-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(thiophen-2-yl)propan-2-yl]-5H-pyrrolo[3,2-d]pyrimidine-7-carboxamide, 4-[5-(cyclopropyl-methoxy)-1,3-benzodioxol-4-yl]-N-(3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl)-5H-pyrrolo[3,2-d]pyrimidine-7-carboxamide, 4-[5-(cyclopropylmethoxy)-1,3-benzodioxol-4-yl]-N-[(2R)-4-{4-[(4aS,8aR)-4-(3,4-dimethoxyphenyl)-1-oxo-4a,5,6,7,8,8a-hexahydrophthalazin-2(1H)-yl]piperidin-1-yl}-4-oxo-1-phenylbutan-2-yl]-5H-pyrrolo[3,2-d]pyrimidine-7-carboxamide,4-[5-(cyclopropyl-methoxy)-1,3-benzodioxol-4-yl]-N-[(2R)-4-{4-[3-(3,4-dimethoxyphenyl)-5,5-dimethyl-6-oxo-5,6-dihydropyridazin-1(4H)-yl]piperidin-1-yl}-4-oxo-1-phenylbutan-2-yl]-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-5H-pyrrolo[3,2-d]pyrimidine-7-carboxamide, 4-[5-(cyclopropyl-methoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-6-methyl-5H-pyrrolo[3,2-d]pyrimidine-7-carboxamide, 4-[2-(cyclopropylmethoxy)-5-fluoro-4-methoxyphenyl]-N-[(2S)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-1,5-dioxo-5-(piperidin-1-yl)pentan-2-yl]-6-methyl-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-6-methyl-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1H-pyrazol-1-yl)propan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]-5H-pyrrolo[3,2-d]pyrimidine-7-carboxamide, 4-[5-(cyclopropyl-methoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydro-phthalazin-2(1H)-yl]piperidin-1-yl}-3-(1H-indol-3-yl)-1-oxopropan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexa-hydrophthalazin-2(1H)-yl]piperidin-1-yl}-3-(1-methyl-1 H-indol-3-yl)-1-oxopropan-2-yl]-5H-pyrrolo[3,2-d]pyrimidine-7-carboxamide, N-[(2S)-3-cyclohexyl-1-{4-[3-(3,4-dimethoxyphenyl)-5,5-dimethyl-6-oxo-5,6-dihydropyridazin-1(4H)-yl]piperidin-1-yl}-1-oxopropan-2-yl]-4-[5-(cyclopropyl-methoxy)-1,3-benzodioxol-4-yl]-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-methyl-1-oxopentan-2-yl]-5H-pyrrolo[3,2-d]pyrimidine-7-carboxamide,4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]-5H-pyrrolo[3,2-d]pyrimidine-7-carboxamide, 4-[5-(cyclo-propylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-(methylsulfanyl)-1-oxobutan-2-yl]-5H-pyrrolo[3,2-d]pyrimidine-7-carboxamide, N-[(2R)-3-(4-bromophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidine-7-carboxamide, Benzyl (4R)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate, (4R)-4-[({4-[5-(cyclopropyl-methoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoic acid, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(pyrrolidin-1-yl)pentan-2-yl]-5H-pyrrolo[3,2-d]pyrimidine-7-carboxamide, (4S)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoic acid, Benzyl (4S)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentanoate, 4-[5-(cyclopropyl-methoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(pyrrolidin-1-yl)pentan-2-yl]-5H-pyrrolo[3,2-d]pyrimidine-7-carboxamide, 4-[5-(cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(propan-2-ylamino)pentan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(propan-2-ylamino)pentan-2-yl]-5H-pyrrolo[3,2-d]pyrimidine-7-carboxamide, 4-[5-(cyclopropyl-methoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-3-(4-fluorophenyl)-1-oxopropan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-5H-pyrrolo[3,2-d]pyrimidine-7-carboxamide, 4-[5-(cyclo-propylmethoxy)-1,3-benzodioxol-4-yl]-N-[(2S)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxopropan-2-yl]-5H-pyrrolo[3,2-d]pyrimidine-7-carboxamide, 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(2-oxoazetidin-1-yl)phenyl]propan-2-yl}-5H-pyrrolo[3,2-d]pyrimidine-7-carboxamide, 4-[5-(cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1 H)-yl]piperidin-1-yl}-1-oxo-3-[4-(2-oxopyrrolidin-1-yl)phenyl]propan-2-yl}-5H-pyrrolo[3,2-d]pyrimidine-7-carboxamide, 4-[5-(cyclopropylmethoxy)-1,3-benzodioxol-4-yl]-N-[(2S)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-(pyridin-3-yl)propan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidine-7-carboxamide, 4-[5-(cyclo-propylmethoxy)-1,3-benzodioxol-4-yl]-N-[(2R)-1-{4-[3-(3,4-dimethoxyphenyl)-5,5-diethyl-6-oxo-5,6-dihydropyridazin-1(4H)-yl]piperidin-1-yl}-1-oxo-3-phenylpropan-2-yl]-5H-pyrrolo[3,2-d]pyrimidine-7-carboxamide, 4-[5-(cyclopropylmethoxy)-1,3-benzodioxol-4-yl]-N-(2-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-2-oxoethyl)-5H-pyrrolo[3,2-d]pyrimidine-7-carboxamide, 4-[5-(cyclopropyl-methoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-5-(dimethylamino)-1,5-dioxopentan-2-yl]-6-methyl-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxobutan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxobutan-2-yl]-6-methyl-5H-pyrrolo[3,2-d]pyrimidine-7-carboxamide, 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3,3-dimethyl-1-oxobutan-2-yl]-5H-pyrrolo[3,2-d]pyrimidine-7-carboxamide, 4-[5-(cyclopropylmethoxy)-1,3-benzodioxol-4-yl]-N-(2-{4-[9-(3,4-dimethoxyphenyl)-6-oxo-7,8-diazaspiro[4.5]dec-8-en-7-yl]piperidin-1-yl}-2-oxoethyl)-5H-pyrrolo[3,2-d]pyrimidine-7-carboxamide, 4-[5-(cyclopropylmethoxy)-1,3-benzodioxol-4-yl]-N-[(2R)-1-{4-[9-(3,4-dimethoxy-phenyl)-6-oxo-7,8-diazaspiro[4.5]dec-8-en-7-yl]piperidin-1-yl}-1-oxo-3-phenylpropan-2-yl]-5H-pyrrolo[3,2-d]pyrimidine-7-carboxamide, 4-[5-(cyclopropylmethoxy)-1,3-benzodioxol-4-yl]-N-[(2R)-1-{4-[9-(3,4-dimethoxyphenyl)-6-oxo-7,8-diazaspiro[4.5]dec-8-en-7-yl]piperidin-1-yl}-1-oxo-3-phenylpropan-2-yl]-6-methyl-5H-pyrrolo[3,2-d]pyrimidine-7-carboxamide and 4-[5-(cyclopropyl-methoxy)-1,3-benzodioxol-4-yl]-N-[(2S)-1-{4-[9-(3,4-dimethoxyphenyl)-6-oxo-7,8-diazaspiro[4.5]dec-8-en-7-yl]piperidin-1-yl}-1-oxo-3-(pyridin-3-yl)propan-2-yl]-5H-pyrrolo[3,2-d]pyrimidine-7-carboxamide.

7. Compound of formula (1) according to claim 1 which is4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidine-7-carboxamide or a salt thereof.

8. Compound of formula (1) according to claim 1 which is 4-[5-(cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidine-7-carboxamide or a salt thereof.

9. Compound of formula (1) according to claim 1 which is 4-[5-(cyclopropylmethoxy)-1,3-benzodioxol-4-yl]-N-(2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl)-5H-pyrrolo[3,2-d]pyrimidine-7-carboxamide or a salt thereof.

10. A compound of formula (1) or a stereoisomer of the compound or a salt of the compound or a salt of the stereoisomer of the compound according to any one of claims 1 to 6 for use in the treatment or prophylaxis of diseases.

11. The compound of formula (1) or a salt thereof according to any one of claims 7 to 9 for use in the treatment or prophylaxis of diseases.

12. Pharmaceutical composition comprising at least one of the compounds of formula (1) or a stereoisomer of the compound or a salt of the compound or a salt of the stereoisomer of the compound according to any one of claims 1 to 6 together with at least one pharmaceutically acceptable auxiliary.

13. Pharmaceutical composition the compound of formula (1) or a salt thereof according to any one of claims 7 to 9

together with at least one pharmaceutically acceptable auxiliary.

**14.** Fixed combination, non-fixed combination or kit of parts comprising at least one compound of formula (1) or a stereoisomer of the compound or a salt of the compound or a salt of a stereoisomer of the compound according to anyone of claims 1 to 6, at least one therapeutic agent selected from the group consisting of corticosteroids, anti-cholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, lung surfactants, antibiotics, guanylyl-cyclase activators/stimulators, tetrahydrobiopterin and tetrahydrobiopterin derivatives, anticoagulants, diuretics, pirfenidone and digitalis glycosides, and at least one pharmaceutically acceptable auxiliary.

**15.** Fixed combination, non-fixed combination or kit of parts comprising the compound of formula (1) or a salt thereof according to any one of claims 7 to 9, at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, lung surfactants, antibiotics, guanylyl-cyclase activators/stimulators, tetrahydrobiopterin and tetrahydrobiopterin derivatives, anticoagulants, diuretics, pirfenidone and digitalis glycosides, and at least one pharmaceutically acceptable auxiliary.

**16.** Use of a compound of formula (1) or a stereoisomer of the compound or a salt of the compound or a salt of the stereoisomer of the compound according to any one of claims 1 to 6 in the manufacture of a pharmaceutical composition for the treatment or prophylaxis of an acute or chronic airway disease.

**17.** Use of the compound of formula (1) or a salt thereof according to any one of claims 7 to 9 in the manufacture of a pharmaceutical composition for the treatment or prophylaxis of an acute or chronic airway disease.

**18.** Use according to any one of claims 16 to 17, wherein the acute or chronic airway disease is selected from the group consisting of interstitial lung disease, pulmonary fibrosis, cystic fibrosis, bronchial asthma, chronic bronchitis, emphysema, chronic obstructive pulmonary disease (COPD) and COPD associated with pulmonary hypertension.

**19.** A compound of formula (1) or a stereoisomer of the compound or a salt of the compound or salt of the stereoisomer of the compound according to any one of claims 1 to 6 for use in the treatment or prophylaxis of an acute or chronic airway disease.

**20.** The compound of formula (1) or a salt thereof according to any one of claims 7 to 9 for use in the treatment or prophylaxis of an acute or chronic airway disease.

**21.** A compound of formula (1) or a stereoisomer of the compound or a saltof the compound or a salt of the stereoisomer of the compound according to any one of claims 1 to 6 for the treatment or prophylaxis of an acute or chronic airway disease selected from the group consisting of interstitial lung disease, pulmonary fibrosis, cystic fibrosis, bronchial asthma, chronic bronchitis, emphysema, chronic obstructive pulmonary disease (COPD) and COPD associated with pulmonary hypertension.

**22.** The compound of formula (1) or a salt thereof according to any one of claims 7 to 9 for the treatment or prophylaxis of an acute or chronic airway disease selected from the group consisting of interstitial lung disease, pulmonary fibrosis, cystic fibrosis, bronchial asthma, chronic bronchitis, emphysema, chronic obstructive pulmonary disease (COPD) and COPD associated with pulmonary hypertension.

## Patentansprüche

**1.** Verbindung der Formel (1)

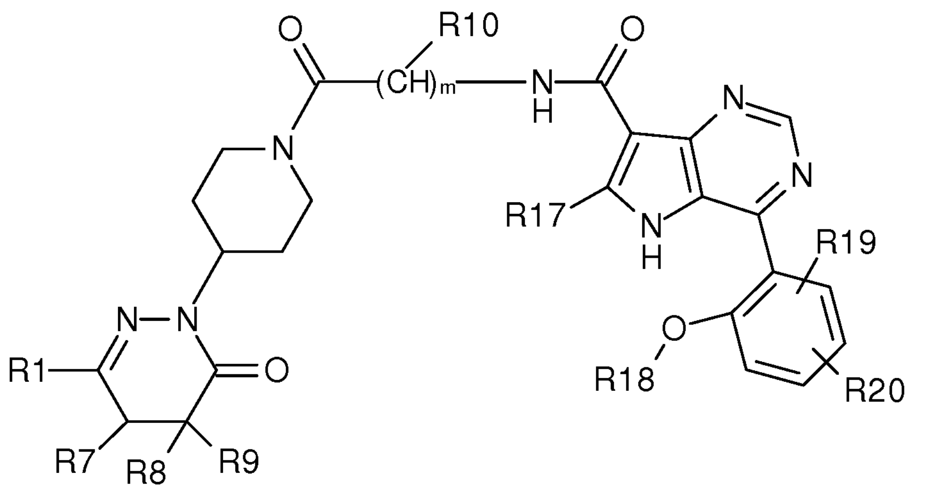

(1),

wobei

R1 für ein Phenylderivat der Formel (a) oder (b) steht

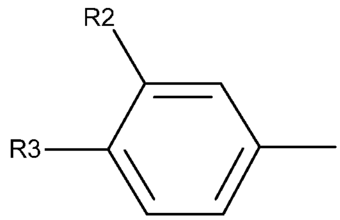

(a)

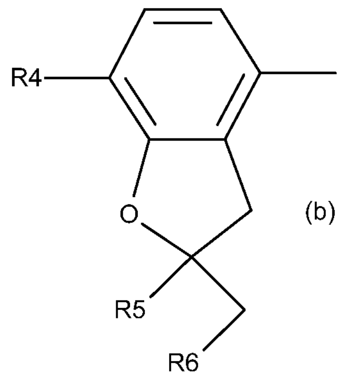

(b)

,

wobei

R2 für 1-4C-Alkoxy oder überwiegend oder vollständig durch Fluor substituiertes 1-4C-Alkoxy steht,

R3 für 1-4C-Alkoxy, 3-5C-Cycloalkoxy, 3-5C-Cycloalkoxymethoxy oder überwiegend oder vollständig durch Fluor substituiertes 1-4C-Alkoxy steht,
R4 für 1-4C-Alkoxy oder überwiegend oder vollständig durch Fluor substituiertes 1-4C-Alkoxy steht,
R5 für 1-4C-Alkyl steht,
R6 für Wasserstoff oder 1-4C-Alkyl steht,
oder wobei R5 und R6 zusammen und einschließlich der beiden Kohlenstoffatome, an die sie gebunden sind, einen spiro-verknüpften 5-, 6- oder 7-gliedrigen Kohlenwasserstoffring bilden, der gegebenenfalls durch ein Sauerstoff- oder Schwefelatom unterbrochen ist,

R7 für Wasserstoff steht,
R8 für 1-4C-Alkyl steht,
oder R7 und R8 zusammen eine 3C- bis 5C-Alkylengruppe bilden,
R9 für Wasserstoff oder 1-4C-Alkyl steht,
oder wobei R8 und R9 zusammen und einschließlich des Kohlenstoffatoms, an das sie gebunden sind, einen spiro-verknüpften 5-, 6- oder 7-gliedrigen Kohlenwasserstoffring bilden,
m für 1 oder 2 steht,
R10 unabhängig voneinander für Wasserstoff, 5-7C-Cycloalkyl, 1-6C-Alkyl, $-CH(CH_3)$-R11 oder $-CH_2$-R12 steht,

wobei

R11 für unsubstituiertes Phenyl oder Hydroxy steht,
R12 für Hydroxy, 5-7C-Cycloalkyl, -N-(1-2C-Alkyl)$_2$,-$CH_2$-S-(1-2C-Alkyl),Benzyl, unsubstituiertes Phenyl, durch R13 substituiertes Phenyl oder durch R13 und R14 substituiertes Phenyl steht,

wobei

R13 für Halogen, 1-4C-Alkoxy, 1-6C-Alkyl, 1-4C- Fluoralkyl, Hydroxy, Phenyl, -C(O)$NH_2$, -CN, 2-Oxoazetidin-1-yl oder 2-Oxopyrrolidin-1-yl steht,
R14 für Halogen, 1-4C-Alkoxy, 1-6C-Alkyl, 1-4C-Fluoralkyl, Hydroxy, Phenyl, -C(O)$NH_2$ oder -CN steht,
oder R12 ausgewählt ist aus Imidazol-2-yl, Imidazol-4-yl, Pyrazol-1-yl, Thiophen-2-yl, Thiophen-3-yl, Thiazol-2-yl, Thiazol-4-yl, Thiazol-5-yl, Pyridin-2-yl, Pyridin-3-yl, Pyridin-4-yl, Indol-2-yl, Indol-3-yl, 1-Methylindol-2-yl oder 1-Methylindol-3-yl, oder
R12 für -$CH_2$-C(O)-R15 steht,

wobei

R15 für Hydroxy, -N(R16)$_2$, Piperidin-1-yl, Pyrrolidin-1-yl oder Benzyloxy steht, wobei R16 unabhängig voneinander für Wasserstoff oder 1-4C-Alkyl steht,

R17 für Wasserstoff oder Methyl steht,
R18 für -$CH_2$-3-6C-Cycloalkyl steht,
R19 für Halogen, Hydroxy, 1-4C-Alkoxy, überwiegend oder vollständig durch Fluor substituiertes 1-4C-Alkoxy oder 1-4C-Fluoralkyl steht,
R20 für Halogen, Hydroxy, 1-4C-Alkoxy oder überwiegend oder vollständig durch Fluor substituiertes 1-4C-Alkoxy steht,
oder R19 und R20 zusammen eine 1-2C-Alkylendioxygruppe bilden,
oder ein Stereoisomer der Verbindung oder ein Salz der Verbindung oder ein Salz des Stereoisomers der Verbindung.

2. Verbindung der Formel (1) nach Anspruch 1, wobei R1 für ein Phenylderivat der Formel (a) oder (b) steht, wobei

R2 für 1-4C-Alkoxy oder überwiegend oder vollständig durch Fluor substituiertes 1-4C-Alkoxy steht,

R3 für 1-4C-Alkoxy oder überwiegend oder vollständig durch Fluor substituiertes 1-4C-Alkoxy steht,
R4 für 1-2C-Alkoxy oder überwiegend oder vollständig durch Fluor substituiertes 1-2C-Alkoxy steht,
R5 für 1-2C-Alkyl steht,
R6 für Wasserstoff oder 1-2C-Alkyl steht,
oder wobei R5 und R6 zusammen und einschließlich der beiden Kohlenstoffatome, an die sie gebunden sind, einen spiro-verknüpften 5-, 6- oder 7-gliedrigen Kohlenwasserstoffring bilden,

R7 für Wasserstoff steht,
R8 für 1-4C-Alkyl steht,
oder R7 und R8 zusammen eine 3C- bis 5C-Alkylengruppe bilden,
R9 für Wasserstoff oder 1-4C-Alkyl steht,
oder wobei R8 und R9 zusammen und einschließlich des Kohlenstoffatoms, an das sie gebunden sind, einen spiro-verknüpften 5-, 6- oder 7-gliedrigen Kohlenwasserstoffring bilden,
R10 unabhängig voneinander für Wasserstoff, 5-7C-Cycloalkyl, 1-4C-Alkyl, -CH($CH_3$)-R11 oder -$CH_2$-R12 steht, wobei

R11 für unsubstituiertes Phenyl oder Hydroxy steht,
R12 für Hydroxy, 5-7C-Cycloalkyl, -N-(1-2C-Alkyl)$_2$,-$CH_2$-S-(1-2C-Alkyl),Benzyl, unsubstituiertes Phenyl, durch R13 substituiertes Phenyl oder durch R13 und R14 substituiertes Phenyl steht,
wobei

R13 für Halogen, 1-4C-Alkoxy, 1-4C-Alkyl, 1-4C Fluoralkyl, Hydroxy, Phenyl, -C(O)$NH_2$, -CN, 2-Oxoa-

zetidin-1-yl oder 2-Oxopyrrolidin-1-yl steht,
R14 für Halogen, 1-4C-Alkoxy, 1-4C-Alkyl, 1-4C-Fluoralkyl, Hydroxy, Phenyl, -C(O)$NH_2$ oder -CN steht,

oder R12 ausgewählt ist aus Imidazol-2-yl, Imidazol-4-yl, Pyrazol-1-yl, Thiophen-2-yl, Thiophen-3-yl, Thiazol-2-yl, Thiazol-4-yl, Thiazol-5-yl, Pyridin-2-yl, Pyridin-3-yl, Pyridin-4-yl, Indol-2-yl, Indol-3-yl, 1-Methylindol-2-yl oder 1-Methylindol-3-yl, oder R12 für -$CH_2$-C(O)-R15 steht,
wobei

R15 für Hydroxy, -N$(R16)_2$, Piperidin-1-yl, Pyrrolidin-1-yl oder Benzyloxy steht, wobei R16 unabhängig voneinander für Wasserstoff oder 1-4C-Alkyl steht,

R17 für Wasserstoff oder Methyl steht,
R18 für -$CH_2$-3-6C-Cycloalkyl steht,
R19 für Halogen, Hydroxy, 1-4C-Alkoxy, überwiegend oder vollständig durch Fluor substituiertes 1-4C-Alkoxy oder 1-4C-Fluoralkyl steht,
R20 für Halogen, Hydroxy, 1-4C-Alkoxy oder überwiegend oder vollständig durch Fluor substituiertes 1-4C-Alkoxy steht,
oder R19 und R20 zusammen eine 1-2C-Alkylendioxygruppe bilden,
oder ein Stereoisomer der Verbindung oder ein Salz der Verbindung oder ein Salz des Stereoisomers der Verbindung.

**3.** Verbindung der Formel (1) nach Anspruch 1, wobei R1 für ein Phenylderivat der Formel (a) oder (b) steht, wobei

R2 für 1-2C-Alkoxy oder überwiegend oder vollständig durch Fluor substituiertes 1-2C-Alkoxy steht,
R3 für 1-2C-Alkoxy oder überwiegend oder vollständig durch Fluor substituiertes 1-2C-Alkoxy steht,

R4 für 1-2C-Alkoxy oder überwiegend oder vollständig durch Fluor substituiertes 1-2C-Alkoxy steht,
R5 für 1-2C-Alkyl steht,
R6 für Wasserstoff oder 1-2C-Alkyl steht,
oder wobei R5 und R6 zusammen und einschließlich der beiden Kohlenstoffatome, an die sie gebunden sind, einen spiro-verknüpften 5- oder 6-gliedrigen Kohlenwasserstoffring bilden,

R7 für Wasserstoff steht,
R8 für 1-2C-Alkyl steht,
oder R7 und R8 zusammen eine 3C- bis 5C-Alkylengruppe bilden,
R9 für Wasserstoff oder 1-2C-Alkyl steht,
oder wobei R8 und R9 zusammen und einschließlich des Kohlenstoffatoms, an das sie gebunden sind, einen spiro-verknüpften 5- oder 6-gliedrigen Kohlenwasserstoffring bilden,
m für 1 oder 2 steht,
R10 unabhängig voneinander für Wasserstoff, 5-7C-Cycloalkyl, 1-4C-Alkyl, -CH$(CH_3)$-R11 oder -$CH_2$-R12 steht, wobei

R11 für unsubstituiertes Phenyl oder Hydroxy steht,
R12 für Hydroxy, 5-7C-Cycloalkyl, -N-$(1\text{-}2C\text{-}Alkyl)_2$,-$CH_2$-S-(1-2C-Alkyl),Benzyl, unsubstituiertes Phenyl, durch R13 substituiertes Phenyl oder durch R13 und R14 substituiertes Phenyl steht,

wobei

R13 für Halogen, 1-4C-Alkoxy, 1-4C-Alkyl, 1-4C-Fluoralkyl, Hydroxy, Phenyl, -C(O)$NH_2$, -CN, 2-Oxoazetidin-1-yl oder 2-Oxopyrrolidin-1-yl steht,
R14 für Halogen oder 1-4C-Alkoxy, 1-4C-Alkyl steht,
oder R12 ausgewählt ist aus Imidazol-2-yl, Imidazol-4-yl, Pyrazol-1-yl, Thiophen-2-yl, Thiophen-3-yl, Thiazol-2-yl, Thiazol-4-yl, Thiazol-5-yl, Pyridin-2-yl, Pyridin-3-yl, Pyridin-4-yl, Indol-2-yl, Indol-3-yl, 1-Methylindol-2-yl oder 1-Methylindol-3-yl, oder
R12 für -$CH_2$-C(O)-R15 steht,

wobei

R15 für Hydroxy, $-N(R16)_2$, Piperidin-1-yl, Pyrrolidin-1-yl oder Benzyloxy steht, wobei R16 unabhängig voneinander für Wasserstoff oder 1-3C-Alkyl steht,

R17 für Wasserstoff oder Methyl steht,
R18 für $-CH_2$-3-5C-Cycloalkyl steht,
R19 für Halogen oder 1-4C-Alkoxy steht,
R20 für Halogen oder 1-4C-Alkoxy steht,
oder R19 und R20 zusammen eine 1-2C-Alkylendioxygruppe bilden,
oder ein Stereoisomer der Verbindung oder ein Salz der Verbindung oder ein Salz des Stereoisomers der Verbindung.

**4.** Verbindung der Formel (1) nach Anspruch 1, wobei

R1 für ein Phenylderivat der Formel (a) oder (b) steht,
wobei

R2 für Methoxy steht,
R3 für Methoxy steht,
R4 für Methoxy steht,
R5 für Methyl steht,
R6 für Wasserstoff steht,

R7 für Wasserstoff steht,
R8 für 1-2C-Alkyl steht,
oder R7 und R8 zusammen eine 3C- bis 5C-Alkylengruppe bilden,
R9 für Wasserstoff oder 1-2C-Alkyl steht,
oder wobei R8 und R9 zusammen und einschließlich des Kohlenstoffatoms, an das sie gebunden sind, einen spiro-verknüpften 5-gliedrigen Kohlenwasserstoffring bilden,
m für 1 oder 2 steht,
R10 unabhängig voneinander für Wasserstoff, 5-7C-Cycloalkyl, 1-4C-Alkyl, $-CH(CH_3)$-R11 oder $-CH_2$-R12 steht, wobei

R11 für unsubstituiertes Phenyl oder Hydroxy steht,
R12 für Hydroxy, 5-7C-Cycloalkyl, -N-(1-2C-Alkyl)$_2$,$-CH_2$-S-(1-2C-Alkyl), Benzyl, unsubstituiertes Phenyl, durch R13 substituiertes Phenyl, durch R13 und R14 substituiertes Phenyl steht,

wobei

R13 für Fluor, Chlor, Brom, 1-2C-Alkoxy, 1-4C-Alkyl, 1-2C-Fluoralkyl, Hydroxy, Phenyl, $-C(O)NH_2$, -CN, 2-Oxoazetidin-1-yl oder 2-Oxopyrrolidin-1-yl steht,
R14 für Fluor, Chlor, Brom oder 1-2C-Alkoxy steht,
oder R12 ausgewählt ist aus Imidazol-2-yl, Imidazol-4-yl, Pyrazol-1-yl, Thiophen-2-yl, Thiophen-3-yl, Thiazol-2-yl, Thiazol-4-yl, Thiazol-5-yl, Pyridin-2-yl, Pyridin-3-yl, Pyridin-4-yl, Indol-2-yl, Indol-3-yl, 1-Methylindol-2-yl oder 1-Methylindol-3-yl, oder
R12 für $-CH_2$-C(O)-R15 steht,

wobei

R15 für Hydroxy, $-N(R16)_2$, Piperidin-1-yl, Pyrrolidin-1-yl oder Benzyloxy steht,
wobei
R16 unabhängig voneinander für Wasserstoff oder 1-3C-Alkyl steht,

R17 für Wasserstoff oder Methyl steht,
R18 für $-CH_2$-3-4C-Cycloalkyl steht,
R19 für 1-2C-Alkoxy steht,
R20 für Fluor, Chlor oder Brom steht,
oder R19 und R20 zusammen eine Methylenedioxygruppe bilden,

oder ein Stereoisomer der Verbindung oder ein Salz der Verbindung oder ein Salz des Stereoisomers der Verbindung.

**5.** Verbindung der Formel (1) nach Anspruch 1, wobei,

R1 für ein Phenylderivat der Formel (a) oder (b) steht, wobei

R2 für Methoxy steht,
R3 für Methoxy steht,
R4 für Methoxy steht,
R5 für Methyl steht,
R6 für Wasserstoff steht,

R7 für Wasserstoff steht,
R8 für 1-2C-Alkyl steht,
oder R7 und R8 zusammen eine 3C- oder 4C-Alkylengruppe bilden,
R9 für Wasserstoff oder 1-2C-Alkyl steht,
oder wobei R8 und R9 zusammen und einschließlich des Kohlenstoffatoms, an das sie gebunden sind, einen spiro-verknüpften 5-gliedrigen Kohlenwasserstoffring bilden,
m für 1 oder 2 steht,
R10 unabhängig voneinander für Wasserstoff, Cyclohexyl, 1-4C-Alkyl, -$CH(CH_3)$-R11 oder -$CH_2$-R12 steht, wobei

R11 für unsubstituiertes Phenyl oder Hydroxy steht,
R12 für Hydroxy, Cyclohexyl, -N-$(CH_3)_2$, -$CH_2$-S-$CH_3$, Benzyl, unsubstituiertes Phenyl, durch R13 substituiertes Phenyl oder durch R13 und R14 substituiertes Phenyl steht,

wobei

R13 für Fluor, Chlor, 1-2C-Alkoxy, Methyl, tert.-Butyl, Trifluormethyl, Hydroxy, Phenyl, -C(O)$NH_2$, -CN, 2-Oxoazetidin-1-yl oder 2-Oxopyrrolidin-1-yl steht,
R14 für Fluor, Chlor oder Methoxy steht,
oder R12 ausgewählt ist aus Imidazol-2-yl, Imidazol-4-yl, Pyrazol-1-yl, Thiophen-2-yl, Thiophen-3-yl, Thiazol-2-yl, Thiazol-4-yl, Thiazol-5-yl, Pyridin-2-yl, Pyridin-3-yl, Pyridin-4-yl, Indol-2-yl, Indol-3-yl, 1-Methylindol-2-yl oder 1-Methylindol-3-yl, oder
R12 für -$CH_2$-C(O)-R15 steht,

wobei

R15 für Hydroxy, -$N(R16)_2$, Piperidin-1-yl, Pyrrolidin-1-yl oder Benzyloxy steht, wobei
R16 unabhängig voneinander für Wasserstoff oder Isopropyl steht,

R17 für Wasserstoff oder Methyl steht,
R18 für -$CH_2$-3C-Cycloalkyl steht,
R19 für Methoxy steht,
R20 für Fluor steht,
oder R19 und R20 zusammen eine Methylendioxygruppe bilden,
oder ein Stereoisomer der Verbindung oder ein Salz der Verbindung oder ein Salz des Stereoisomers der Verbindung.

**6.** Verbindung der Formel (1) nach Anspruch 5, ausgewählt aus der Gruppe bestehend aus 4-[5-(Cyclopropylmethoxy)-1,3-benzodioxol-4-yl]-N-[(2R)-3-(3,5-difluorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidin-7-carbonsäureamid; 4-[2-(Cyclopropylmethoxy)-5-fluor-4-methoxyphenyl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-5H-pyrrolo[3,2-d]pyrimidin-7-carboxamid; 4-[5-(Cyclopropylmethoxy)-1,3-benzo-

dioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidin-7-carbonsäureamid; N-[(2R)-3-(4-tert.-Butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[2-(cyclopropylmethoxy)-5-fluor-4-methoxyphenyl]-6-methyl-5H-pyrrolo[3,2-d]pyrimidin-7-carbonsäureamid; N-[(2R)-3-(4-tert.-Butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidin-7-carbonsäureamid; N-[(2R)-3-(4-Carbamoylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(CyClopropylmethoxy)-1,3-benzodioxol-4-yl]-5H-pyrrolo[3,2-d]pyrimidin-7-carbonsäureamid; 4-[2-(Cyclopropylmethoxy)-5-fluor-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-ethoxyphenyl)-1-oxopropan-2-yl]-6-methyl-5H-pyrrolo[3,2-d]pyrimidin-7-carbonsäureamid; N-[(2R)-3-(4-Carbamoylphenyl)-1-{4-[(4aS,8aR)-4-(3,4-dimethoxy-phenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidin-7-carbonsäureamid; N-[(2R)-3-(Biphenyl-4-yl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidin-7-carbonsäureamid; N-[(2R)-3-(Biphenyl-4-yl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidin-7-carbonsäureamid; N-[(2R)-3-(4-Cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]-6-methyl-5H-pyrrolo[3,2-d]pyrimidin-7-carbonsäureamid; N-[(2R)-3-(4-Cyanophenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2R)-3-(3,4-difluorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2R)-3-(3,4-difluorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methylphenyl)-1-oxopropan-2-yl]-5H-pyrrolo[3,2-d]pyrimidin-7-carbonsäureamid; 4-[2-(Cyclopropylmethoxy)-5-fluor-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-6-methyl-5H-pyrrolo[3,2-d]pyrimidin-7-carbonsäureamid; 4-[2-(Cyclopropylmethoxy)-5-fluor-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidin-7-carbonsäureamid; 4-[2-(Cyclopropylmethoxy)-4-fluor-5-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidin-7-carbonsäureamid; 4-[5-(Cyclopropylmethoxy)-1,3-benzodioxol-4-yl]-N-[(2S)-3-(3,4-dimethoxyphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxy-phenyl)-1-oxo-4a,5,6,7,8,8a-hexahydrophthalazin-2(1H)-yl]piperidin-1-yl}-1-oxo-3-phenylpropan-2-yl]-6-methyl-5H-pyrrolo[3,2-d]pyrimidin-7-carbonsäureamid; N-[(2R)-3-(4-Chlorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorphenyl)-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxopropan-2-yl]-5H-pyrrolo[3,2-d]pyrimidin-7-carbonsäureamid; N-[(2R)-3-(3-Chlorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yll-4-[5-(cyclopropylmethoxy)-1,3-benzodioxol-4-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidin-7-carbonsäureamid; 4-[5-(Cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[2-(trifluormethyl)phenyl]-propan-2-yl}-6-methyl-5H-pyrrolo[3,2-d]pyrimidin-7-carbonsäureamid; 4-[5-(Cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[2-(trifluormethyl)phenyl]propan-2-yl}-5H-pyrrolo[3,2-d]pyrimidin-7-carbonsäureamid; N-[(2R)-3-(2-Chlorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidin-7-carbonsäureamid; N-[(2R)-3-(2-Chlorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidin-7-carbonsäureamid; 4-[2-(Cyclopropylmethoxy)-5-fluor-4-methoxyphenyl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-2-yl)propan-2-yl]-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-3-yl)propan-2-yl]-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aR,8aS)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidin-7-carbonsäureamid; N-[(2R)-3-(4-tert.-Butylphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-fluorphenyl)-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2R)-3-(2,4-dichlorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidin-7-carbonsäureamid; 4-[5-(Cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(trifluormethyl)phenyl]propan-2-yl}-5H-pyrrolo[3,2-d]pyrimidin-7-carbonsäureamid; 4-[5-(Cyclopropylmethoxy)-1,3-benzodioxol-4-yl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-5H-pyrrolo[3,2-d]pyrimidin-7-carbonsäureamid; 4-[5-(Cyclopropylmethoxy)-1,3-benzodioxol-4-yl]-N-[(2R,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylbutan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-hydroxyphenyl)-1-oxopropan-2-yl]-5H-pyrrolo[3,2-d]pyrimidin-7-carbonsäureamid; N-[(2S)-3-(2-Chlorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidin-7-carbonsäure-

amid; 4-[5-(Cyclopropylmethoxy)-1,3-benzodioxol-4-yl]-N-[(2R)-1-{4-[(4aS,7aR)-4-(3,4-dimethoxyphenyl)-1-oxo-1,4a,5,6,7,7a-hexahydro-2H-cyclopenta[d]pyridazin-2-yl]piperidin-1-yl}-1-oxo-3-phenylpropan-2-yl]-5H-pyrrolo[3,2-d]pyrimidin-7-carbonsäureamid; N-[(2S)-3-(2-Chlorphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidin-7-carbonsäureamid; 4-[2-(Cyclopropylmethoxy)-5-fluor-4-methoxyphenyl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(4-methoxyphenyl)-1-oxopropan-2-yl]-6-methyl-5H-pyrrolo[3,2-d]pyrimidin-7-carbonsäureamid; 4-[5-(Cyclopropylmethoxy)-1,3-benzodioxol-4-yl]-N-(2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl)-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-5H-pyrrolo[3,2-d]pyrimidin-7-carbonsäureamid; 4-[5-(Cyclopropylmethoxy)-1,3-benzodioxol-4-yl]-N-[(2S,3R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-hydroxy-1-oxobutan-2-yl]-5H-pyrrolo[3,2-d]pyrimidin-7-carbonsäureamid; 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxobutan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-4-phenylbutan-2-yl]-5H-pyrrolo[3,2-d]pyrimidin-7-carbonsäureamid; 4-[5-(Cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidin-7-carbonsäureamid; 4-[5-(Cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidin-7-carbonsäureamid; 4-[2-(Cyclopropylmethoxy)-5-fluor-4-methoxyphenyl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidin-7-carbonsäureamid oder 4-[2-(Cyclopropylmethoxy)-5-fluor-4-methoxyphenyl]-N-[(2R)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidin-7-carbonsäureamid, 4-[5-(Cyclopropylmethoxy)-1,3-benzodioxol-4-yl]-N-[(2S,3S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxopentan-2-yl]-5H-pyrrolo[3,2-d]pyrimidin-7-carbonsäureamid, N-[(2S)-3-Cyclohexyl-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-6-methyl-5H-pyrrolo[3,2-d]pyrimidin-7-carbonsäureamid, N-[(1R)-1-Cyclohexyl-2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl]-4-[5-(cyclopropylmethoxy)-1,3-benzodioxol-4-yl]-5H-pyrrolo[3,2-d]pyrimidin-7-carbonsäureamid, N-[(1S)-1-Cyclohexyl-2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl]-4-[5-(cyclopropylmethoxy)-1,3-benzodioxol-4-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(thiophen-2-yl)propan-2-yl]-6-methyl-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(thiophen-2-yl)propan-2-yl]-5H-pyrrolo[3,2-d]pyrimidin-7-carbonsäureamid, 4-[5-(Cyclopropylmethoxy)-1,3-benzodioxol-4-yl]-N-(3-{4-[(4aS,8aR)-4-(3,4-dimethoxyphenyl)-1-oxo-4a,5,6,7,8,8a-hexahydrophthalazin-2(1H)-yl]piperidin-1-yl}-3-oxopropyl)-5H-pyrrolo[3,2-d]pyrimidin-7-carbonsäureamid, 4-[5-(Cyclopropylmethoxy)-1,3-benzodioxol-4-yl]-N-[(2R)-4-{4-[(4aS,8aR)-4-(3,4-dimethoxyphenyl)-1-oxo-4a,5,6,7,8,8a-hexahydrophthalazin-2(1H)-yl]piperidin-1-yl}-4-oxo-1-phenylbutan-2-yl]-5H-pyrrolo[3,2-d]pyrimidin-7-carbonsäureamid,4-[5-(Cyclopropylmethoxy)-1,3-benzodioxol-4-yl]-N-[(2R)-4-{4-[3-(3,4-dimethoxyphenyl)-5,5-dimethyl-6-oxo-5,6-dihydropyridazin-1(4H)-yl]piperidin-1-yl}-4-oxo-1-phenylbutan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-6-methyl-5H-pyrrolo[3,2-d]pyrimidin-7-carbonsäureamid, 4-[2-(Cyclopropylmethoxy)-5-fluor-4-methoxyphenyl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(piperidin-1-yl)pentan-2-yl]-6-methyl-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1,3-thiazol-4-yl)propan-2-yl]-6-methyl-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(1H-pyrazol-1-yl)propan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-imidazol-4-yl)-1-oxopropan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1H-indol-3-yl)-1-oxopropan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(1-methyl-1H-indol-3-yl)-1-oxopropan-2-yl]-5H-pyrrolo[3,2-d]pyrimidin-7-carbonsäureamid, N-[(2S)-3-Cyclohexyl-1-{4-[3-(3,4-dimethoxyphenyl)-5,5-dimethyl-6-oxo-5,6-dihydropyridazin-1(4H)-yl]piperidin-1-yl}-1-oxopropan-2-yl]-4-[5-(CyClopropylmethoxy)-1,3-benzodioxol-4-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-methyl-1-oxopentan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(dimethylamino)-1-oxopropan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-4-(methylsulfanyl)-1-oxobutan-2-yl]-5H-pyrrolo[3,2-d]pyrimidin-7-carbonsäureamid, N-[(2R)-3-(4-Bromphenyl)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxopropan-2-yl]-4-[5-(cyclopropylmethoxy)-1,3-benzodioxol-4-yl]-5H-pyrrolo[3,2-d]pyrimidin-7-carbonsäureamid, (4R)-4-[({4-[5-(Cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentansäurebenzylester, (4R)-4-[({4-[5-(cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentansäure, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(pyrrolidin-1-yl)pentan-2-yl]-5H-pyrrolo[3,2-d]pyrimidin-7-carbonsäureamid, (4S)-4-[({4-[5-(Cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentansäure, (4S)-4-[({4-[5-(Cyclopropylmethoxy)-1,3-benzodioxol-4-yl]-5H-pyrrolo[3,2-d]pyrimidin-7-yl}carbonyl)amino]-5-{4-[(4aS,8aR)-4-(3,4-dimethoxyphenyl)-1-oxo-4a,5,6,7,8,8a-hexahydrophthalazin-2(1H)-yl]piperidin-1-yl}-5-oxopentansäurebenzylester, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(pyrrolidin-1-yl)pentan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(propan-2-ylamino)pentan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1,5-dioxo-5-(propan-2-ylamino)pentan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-3-(4-fluorphenyl)-1-oxopropan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-(pyridin-4-yl)propan-2-yl]-5H-pyrrolo[3,2-d]pyrimidin-7-carbonsäureamid, 4-[5-(Cyclopropylmethoxy)-1,3-benzodioxol-4-yl]-N-[(2S)-1-{4-[3-(3,4-dimethoxyphenyl)-5,5-dimethyl-6-oxo-5,6-dihydropyridazin-1(4H)-yl]piperidin-1-yl}-1-oxo-3-phenylpropan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-1-oxopropan-2-yl]-5H-pyrrolo[3,2-d]pyrimidin-7-carbonsäureamid, 4-[5-(Cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-[4-(2-oxoazetidin-1-yl)phenyl]propan-2-yl}-5H-pyrrolo[3,2-d]pyrimidin-7-carbonsäureamid, 4-[5-(Cyclopropylmethoxy)-1,3-benzodioxol-4-yl]-N-{(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-l-oxo-3-[4-(2-oxopyrrolidin-l-yl)phenyl]propan-2-yl}-5H-pyrrolo[3,2-d]pyrimidin-7-carbonsäureamid, 4-[5-(Cyclopropylmethoxy)-1,3-benzodioxol-4-yl]-N-[(2S)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-(pyridin-3-yl)propan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-1-oxo-3-phenylpropan-2-yl]-6-methyl-5H-pyrrolo[3,2-d]pyrimidin-7-carbonsäureamid, 4-[5-(Cyclopropylmethoxy)-1,3-benzodioxol-4-yl]-N-[(2R)-1-{4-[3-(3,4-dimethoxyphenyl)-5,5-diethyl-6-oxo-5,6-dihydropyridazin-1(4H)-yl]piperidin-1-yl}-1-oxo-3-phenylpropan-2-yl]-5H-pyrrolo[3,2-d]pyrimidin-7-carbonsäureamid, 4-[5-(Cyclopropylmethoxy)-1,3-benzodioxol-4-yl]-N-(2-{4-[3-(7-methoxy-2,2-dimethyl-2,3-dihydro-1-benzofuran-4-yl)-5,5-dimethyl-6-oxo-5,6-dihydropyridazin-1(4H)-yl]piperidin-1-yl}-2-oxoethyl)-5H-pyrrolo[3,2-d]pyrimidin-7-carbonsäureamid, 4-[5-(Cyclopropylmethoxy)-1,3-benzodioxol-4-yl]-N-[(2R)-1-{4-[3-(3,4-dimethoxyphenyl)-5,5-dimethyl-6-oxo-5,6-dihydropyridazin-1(4H)-yl]piperidin-1-yl}-5-(dimethylamino)-1,5-dioxopentan-2-yl]-6-methyl-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxobutan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-methyl-1-oxobutan-2-yl]-6-methyl-5H-pyrrolo[3,2-d]pyrimidin-7-carbonsäureamid, 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3,3-dimethyl-1-oxobutan-2-yl]-5H-pyrrolo[3,2-d]pyrimidin-7-carbonsäureamid, 4-[5-(Cyclopropylmethoxy)-1,3-benzodioxol-4-yl]-N-(2-{4-[9-(3,4-dimethoxyphenyl)-6-oxo-7,8-diazaspiro[4.5]dec-8-en-7-yl]piperidin-1-yl}-2-oxoethyl)-5H-pyrrolo[3,2-d]pyrimidin-7-carbonsäureamid, 4-[5-(Cyclopropylmethoxy)-1,3-benzodioxol-4-yl]-N-[(2R)-1-{4-[9-(3,4-dimethoxy-phenyl)-6-oxo-7,8-diazaspiro[4.5]dec-8-en-7-yl]piperidin-1-yl}-1-oxo-3-phenylpropan-2-yl]-5H-pyrrolo[3,2-d]pyrimidin-7-carbonsäureamid, 4-[5-(Cyclopropylmethoxy)-1,3-benzodioxol-4-yl]-N-[(2R)-1-{4-[9-(3,4-dimethoxyphenyl)-6-oxo-7,8-diazaspiro[4.5]dec-8-en-7-yl]piperidin-1-yl}-1-oxo-3-phenylpropan-2-yl]-6-methyl-5H-pyrrolo[3,2-d]pyrimidin-7-carbonsäureamid und 4-[5-(Cyclopropylmethoxy)-1,3-benzodioxol-4-yl]-N-[(2S)-1-{4-[9-(3,4-dimethoxyphenyl)-6-oxo-7,8-diazaspiro[4.5]dec-8-en-7-yl]piperidin-1-yl}-1-oxo-3-(pyridin-3-yl)propan-2-yl]-5H-pyrrolo[3,2-d]pyrimidin-7-carbonsäureamid.

**7.** Verbindung der Formel (1) nach Anspruch 1, bei der es sich um 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-3-(3-methylphenyl)-1-oxopropan-2-yl]-6-methyl-5H-pyrrolo[3,2-d]pyrimidin-7-carbonsäure-amid handelt, oder ein Salz davon.

**8.** Verbindung der Formel (1) nach Anspruch 1, bei der es sich um 4-[5-(Cyclopropylmethoxy)-1,3-benzodioxol-4-yl]-N-[(2R)-1-{4-[(4aS,8aR)-4-(3,4-dimethoxyphenyl)-1-oxo-4a,5,6,7,8,8a-hexahydrophthalazin-2(1H)-yl]piperidin-1-yl}-1-oxo-3-phenylpropan-2-yl]-5H-pyrrolo[3,2-d]pyrimidin-7-carbonsäureamid handelt, oder ein Salz davon.

**9.** Verbindung der Formel (1) nach Anspruch 1, bei der es sich um 4-[5-(Cyclopropylmethoxy)-1,3-benzodioxol-4-yl]-N-(2-{4-[(4aS,8aR)-4-(3,4-dimethoxyphenyl)-1-oxo-4a,5,6,7,8,8a-hexahydrophthalazin-2(1H)-yl]piperidin-1-yl}-2-oxoethyl)-5H-pyrrolo[3,2-d]pyrimidin-7-carbonsäureamid handelt, oder ein Salz davon.

**10.** Verbindung der Formel (1) oder ein Steroeisomer der Verbindung oder ein Salz der Verbindung oder ein Salz des Stereoisomers der Verbindung nach einem der Ansprüche 1 bis 6 zur Verwendung bei der Behandlung oder Prophylaxe von Krankheiten.

**11.** Verbindung der Formel (1) oder ein Salz davon nach einem der Ansprüche 7 bis 9 zur Verwendung bei der Behandlung oder Prophylaxe von Krankheiten.

**12.** Pharmazeutische Zusammensetzung, umfassend mindestens eine der Verbindungen der Formel (1) oder ein Stereoisomer der Verbindung oder ein Salz der Verbindung oder ein Salz des Stereoisomers der Verbindung nach einem der Ansprüche 1 bis 6 zusammen mit mindestens einem pharmazeutisch unbedenklichen Hilfsstoff.

**13.** Pharmazeutische Zusammensetzung der Verbindung der Formel (1) oder eines Salzes davon nach einem der Ansprüche 7 bis 9 zusammen mit mindestens einem pharmazeutisch unbedenklichen Hilfsstoff.

**14.** Fixe Kombination, nichtfixe Kombination oder Kit von Teilen, umfassend mindestens eine Verbindung der Formel (1) oder ein Stereoisomer der Verbindung oder ein Salz der Verbindung oder ein Salz eines Stereoisomers der Verbindung nach einem der Ansprüche 1 bis 6, mindestens ein therapeutisches Mittel ausgewählt aus der Gruppe bestehend aus Kortikosteroiden, Anticholinergika, $\beta_2$-Adrenorezeptor-Agonisten, H1-Rezeptor-Antagonisten, Leukotrienrezeptor-Antagonisten, 5-Lipoxygenase-Inhibitoren, Endothelinrezeptor-Antagonisten, Prostacyclinen, Calciumkanalblockern, beta-Blockern, Typ-4-Phosphodiesterase-Inhibitoren, Typ-5-Phosphodiesterase-Inhibitoren, Immunsuppresiva, Vitamin-D-Analoga, HMG-CoA-Reduktase-Inhibitoren, Lungen-Surfactants, Antibiotika, Aktivatoren/Stimulatoren der Guanylylcyclase, Tetrahydrobiopterin und Tetrahydrobiopterinderivaten, Antikoagulantien, Diuretika, Pirfenidon und digitalis-Glykosiden, und mindestens einen pharmazeutisch unbedenklichen Hilfsstoff.

**15.** Fixe Kombination, nichtfixe Kombination oder Kit von Teilen, umfassend die Verbindung der Formel (1) oder ein Salz davon nach einem der Ansprüche 7 bis 9, mindestens ein therapeutisches Mittel ausgewählt aus der Gruppe bestehend aus Kortikosteroiden, Anticholinergika, $\beta_2$-Adrenorezeptor-Agonisten, H1-Rezeptor-Antagonisten, Leukotrienrezeptor-Antagonisten, 5-Lipoxygenase-Inhibitoren, Endothelinrezeptor-Antagonisten, Prostacyclinen, Calciumkanalblockern, beta-Blockern, Typ-4-Phosphodiesterase-Inhibitoren, Typ-5-Phosphodiesterase-Inhibitoren, Immunsuppresiva, Vitamin-D-Analoga, HMG-CoA-Reduktase-Inhibitoren, Lungen-Surfactants, Antibiotika, Aktivatoren/Stimulatoren der Guanylylcyclase, Tetrahydrobiopterin und Tetrahydrobiopterinderivaten, Antikoagulantien, Diuretika, Pirfenidon und digitalis-Glykosiden, und mindestens einen pharmazeutisch unbedenklichen Hilfsstoff.

**16.** Verwendung einer Verbindung der Formel (1) oder eines Stereoisomers der Verbindung oder eines Salzes der Verbindung oder eines Salzes des Stereoisomers der Verbindung nach einem der Ansprüche 1 bis 6 zur Herstellung einer pharmazeutischen Zusammensetzung zur Behandlung oder Prophylaxe einer akuten oder chronischen Atemwegserkrankung.

**17.** Verwendung der Verbindung der Formel (1) oder eines Salzes davon nach einem der Ansprüche 7 bis 9 zur Herstellung einer pharmazeutischen Zusammensetzung zur Behandlung oder Prophylaxe einer akuten oder chronischen Atemwegserkrankung.

**18.** Verwendung nach einem der Ansprüche 16 bis 17, wobei die akute oder chronische Atemwegserkrankung ausgewählt ist aus der Gruppe bestehend aus interstitieller Lungenkrankheit, Lungenfibrose, zystischer Fibrose, Bronchialasthma, chronischer Bronchitis, Emphysem, chronischer obstruktiver Lungenerkrankung (Chronic Obstructive Pulmonary Disease, COPD) und mit pulmonaler Hypertonie assoziierter COPD.

**19.** Verbindung der Formel (1) oder ein Stereoisomer der Verbindung oder ein Salz der Verbindung oder ein Salz des Stereoisomers der Verbindung nach einem der Ansprüche 1 bis 6 zur Verwendung bei der Behandlung oder Prophylaxe einer akuten oder chronischen Atemwegserkrankung.

**20.** Verbindung der Formel (1) oder ein Salz davon nach einem der Ansprüche 7 bis 9 zur Verwendung bei der Behandlung oder Prophylaxe einer akuten oder chronischen Atemwegserkrankung.

**21.** Verbindung der Formel (1) oder ein Stereoisomer der Verbindung oder ein Salz der Verbindung oder ein Salz des Stereoisomers der Verbindung nach einem der Ansprüche 1 bis 6 zur Behandlung oder Prophylaxe einer akuten oder chronischen Atemwegserkrankung ausgewählt aus der Gruppe bestehend aus interstitieller Lungenkrankheit, Lungenfibrose, zystischer Fibrose, Bronchialasthma, chronischer Bronchitis, Emphysem, chronischer obstruktiver Lungenerkrankung (Chronic Obstructive Pulmonary Disease, COPD) und mit pulmonaler Hypertonie assoziierter COPD.

**22.** Verbindung der Formel (1) oder ein Salz davon nach einem der Ansprüche 7 bis 9 zur Behandlung oder Prophylaxe einer akuten oder chronischen Atemwegserkankung ausgewählt aus der Gruppe bestehend aus interstitieller Lungenkrankheit, Lungenfibrose, zystischer Fibrose, Bronchialasthma, chronischer Bronchitis, Emphysem, chronischer obstruktiver Lungenerkrankung (Chronic Obstructive Pulmonary Disease, COPD) und mit pulmonaler Hypertonie assoziierter COPD.

## Revendications

1. Composé de formule (1)

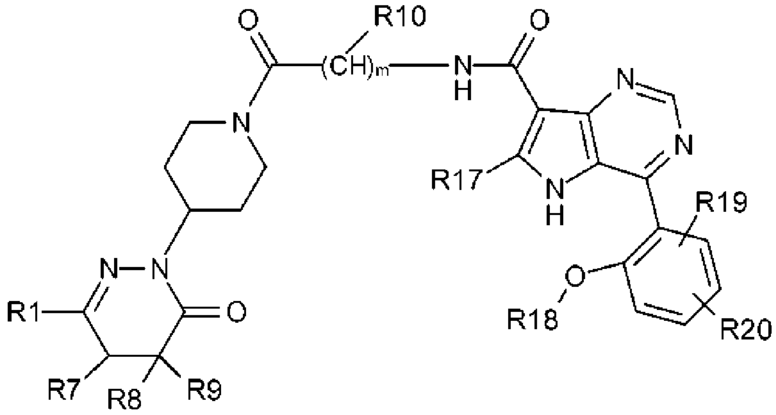

(1),

dans lequel

R1 représente un dérivé de phényle de formule (a) ou (b)

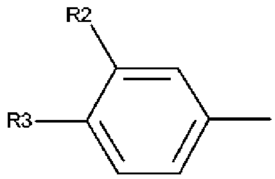

(a)

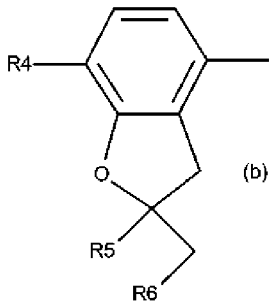

(b)

où

R2 est alcoxy en C1-4 ou alcoxy en C1-4 principalement ou totalement substitué par fluor,

R3 est alcoxy en C1-4, 3-5C-cycloalcoxy, 3-5C-cycloalcoxyméthoxy ou alcoxy en C1-4 principalement ou totalement substitué par fluor, R4 est alcoxy en C1-4 ou alcoxy en C1-4 principalement ou totalement substitué par fluor, R5 est alkyle en C1-4,
R6 est hydrogène ou alkyle en C1-4

ou dans lequel R5 et R6 conjointement et avec inclusion des deux atomes de carbone, auxquels ils sont liés, forment un cycle hydrocarboné de 5, 6 ou 7 chaînons à liaison spiro, facultativement interrompu par un atome d'oxygène de soufre,
R7 est hydrogène,
R8 est alkyle en C1-4,
ou R7 et R8 forment conjointement un groupe alkylène en C3 à C5,
R9 est hydrogène ou alkyle en C1-4,
ou dans lequel R8 et R9 conjointement et avec inclusion de l'atome de carbone, auquel ils sont liés, forment un cycle hydrocarboné de 5, 6 ou 7 chaînons à liaison spiro,
m est 1 ou 2,
R10 est indépendamment l'un de l'autre hydrogène, cycloalkyle en C5-7, alkyle en C1-6, $-CH(CH_3)$-R11 ou $-CH_2$-R12,
où

R11 est phényle non substitué ou hydroxyle,
R12 est hydroxyle, cycloalkyle en C5-7, -N-(alkyle en C1-2)$_2$, -$CH_2$-S-(alkyle en C1-2), benzyle, phényle non substitué, phényle substitué par R13, phényle substitué par R13 et R14, où

R13 est halogène, alcoxy en C1-4, alkyle en C1-6, fluoroalkyle en C1-4, hydroxyle, phényle, -C(O)$NH_2$, -CN, 2-oxoazétidin-1-yle ou 2-oxopyrrolidin-1-yle,
R14 est halogène, alcoxy en C1-4, alkyle en C1-6, fluoroalkyle en C1-4, hydroxyle, phényle, -C(O)$NH_2$ ou -CN,

ou R12 est choisi parmi imidazol-2-yle, imidazol-4-yle, pyrazol-1-yle, thiophén-2-yle, thiophén-3-yle, thiazol-2-yle, thiazol-4-yle, thiazol-5-yle, pyridin-2-yle, pyridin-3-yle, pyridin-4-yle, indol-2-yle, indol-3-yle, 1-méthyl-indol-2-yle ou 1-méthyl-indol-3-yle, ou
R12 est -$CH_2$-C(O)-R15, où

R15 est hydroxyle, -N(R16)$_2$, pipéridin-1-yle, pyrrolidin-1-yle ou benzyloxy, où
R16 est indépendamment l'un de l'autre hydrogène ou alkyle en C1-4,

R17 est hydrogène ou méthyle,
R18 est -$CH_2$-(cycloalkyle en C3-6),
R19 est halogène, hydroxyle, alcoxy en C1-4, alcoxy en C1-4 principalement ou totalement substitué par fluor ou fluoroalkyle en C1-4,
R20 est halogène, hydroxyle, alcoxy en C1-4 ou alcoxy en C1-4 principalement ou totalement substitué par fluor,
ou R19 et R20 forment conjointement un groupe alkylènedioxy en C1-2
ou un stéréoisomère du composé ou un sel du composé ou un sel du stéréoisomère du composé.

**2.** Composé de formule (1) selon la revendication 1, dans lequel

R1 représente un dérivé de phényle de formule (a) ou (b),
où

R2 est alcoxy en C1-4 ou alcoxy en C1-4 principalement ou totalement substitué par fluor,
R3 est alcoxy en C1-4 ou alcoxy en C1-4 principalement ou totalement substitué par fluor,
R4 est alcoxy en C1-2 ou alcoxy en C1-2 principalement ou totalement substitué par fluor,
R5 est alkyle en C1-2,
R6 est hydrogène ou alkyle en C1-2,
ou dans lequel R5 et R6 conjointement et avec inclusion des deux atomes de carbone, auxquels ils sont liés, forment un cycle hydrocarboné de 5, 6 ou 7 chaînons à liaison spiro,

R7 est hydrogène,
R8 est alkyle en C1-4,
ou R7 et R8 forment conjointement un groupe alkylène en C3 à C5,
R9 est hydrogène ou alkyle en C1-4,
ou dans lequel R8 et R9 conjointement et avec inclusion de l'atome de carbone, auquel ils sont liés, forment un cycle hydrocarboné de 5, 6 ou 7 chaînons à liaison spiro,
R10 est indépendamment l'un de l'autre hydrogène, cycloalkyle en C5-7, alkyle en C1-4, -CH($CH_3$)-R11 ou -$CH_2$-R12, où

R11 est phényle non substitué ou hydroxyle,
R12 est hydroxyle, cycloalkyle en C5-7, -N-(alkyle en C1-2)$_2$, -$CH_2$-S-(alkyle en C1-2), benzyle, phényle non substitué, phényle substitué par R13, phényle substitué par R13 et R14, où

R13 est halogène, alcoxy en C1-4, alkyle en C1-4, fluoroalkyle en C1-4, hydroxyle, phényle, -C(O)$NH_2$, -CN, 2-oxoazétidin-1-yle ou 2-oxopyrrolidin-1-yle,
R14 est halogène, alcoxy en C1-4, alkyle en C1-4, fluoroalkyle en C1-4, hydroxyle, phényle, -C(O)$NH_2$ ou -CN,

ou R12 est choisi parmi imidazol-2-yle, imidazol-4-yle, pyrazol-1-yle, thiophén-2-yle, thiophén-3-yle, thiazol-2-yle, thiazol-4-yle, thiazol-5-yle, pyridin-2-yle, pyridin-3-yle, pyridin-4-yle, indol-2-yle, indol-3-yle, 1-méthyl-indol-2-yle ou 1-méthyl-indol-3-yle, ou

R12 est $-CH_2-C(O)-R15$, où
R15 est hydroxyle, $-N(R16)_2$, pipéridin-1-yle, pyrrolidin-1-yle ou benzyloxy, où

R16 est indépendamment l'un de l'autre hydrogène ou alkyle en C1-4,

R17 est hydrogène ou méthyle,
R18 est $-CH_2$-(cycloalkyle en 3-6),
R19 est halogène, hydroxyle, alcoxy en C1-4, alcoxy en C1-4 principalement ou totalement substitué par fluor ou fluoroalkyle en C1-4,
R20 est halogène, hydroxyle, alcoxy en C1-4 ou alcoxy en C1-4 principalement ou totalement substitué par fluor,
ou R19 et R20 forment conjointement un groupe alkylènedioxy en C1-2,
ou un stéréoisomère du composé ou un sel du composé ou un sel du stéréoisomère du composé.

**3.** Composé de formule (1) selon la revendication 1, dans lequel

R1 représente un dérivé de phényle de formule (a) ou (b), où

R2 est alcoxy en C1-2 ou alcoxy en C1-2 principalement ou totalement substitué par fluor,
R3 est alcoxy en C1-2 ou alcoxy en C1-2 principalement ou totalement substitué par fluor,
R4 est alcoxy en C1-2 ou alcoxy en C1-2 principalement ou totalement substitué par fluor,
R5 est alkyle en C1-2,
R6 est hydrogène ou alkyle en C1-2,

ou dans lequel R5 et R6 conjointement et avec inclusion des deux atomes de carbone, auxquels ils sont liés, forment un cycle hydrocarboné de 5 ou 6 chaînons à liaison spiro,
R7 est hydrogène,
R8 est alkyle en C1-2,
ou R7 et R8 forment conjointement un groupe alkylène en C3 à C5,
R9 est hydrogène ou alkyle en C1-2,
ou dans lequel R8 et R9 conjointement et avec inclusion de l'atome de carbone, auquel ils sont liés, forment un cycle hydrocarboné de 5 ou 6 chaînons à liaison spiro,
m est 1 ou 2,
R10 est indépendamment l'un de l'autre hydrogène, cycloalkyle en C5-7, alkyle en C1-4, $-CH(CH_3)-R11$ ou $-CH_2-R12$, où

R11 est phényle non substitué ou hydroxyle,
R12 est hydroxyle, cycloalkyle en C5-7, -N-(alkyle en C1-2)$_2$, $-CH_2$-S-(alkyle en C1-2), benzyle, phényle non substitué, phényle substitué par R13, phényle substitué par R13 et R14, où

R13 est halogène, alcoxy en C1-4, alkyle en C1-4, fluoroalkyle en C1-4, hydroxyle, phényle, $-C(O)NH_2$, -CN, 2-oxoazétidin-1-yle ou 2-oxopyrrolidin-1-yle,
R14 est halogène ou alcoxy en C1-4, alkyle en C1-4,

ou R12 est choisi parmi imidazol-2-yle, imidazol-4-yle, pyrazol-1-yle, thiophén-2-yle, thiophén-3-yle, thiazol-2-yle, thiazol-4-yle, thiazol-5-yle, pyridin-2-yle, pyridin-3-yle, pyridin-4-yle, indol-2-yle, indol-3-yle, 1-méthyl-indol-2-yle ou 1-méthyl-indol-3-yle, ou R12 est $-CH_2-C(O)-R15$, où

R15 est hydroxyle, $-N(R16)_2$, pipéridin-1-yle, pyrrolidin-1-yle ou benzyloxy, où
R16 est indépendamment l'un de l'autre hydrogène ou alkyle en C1-3,

R17 est hydrogène ou méthyle,
R18 est $-CH_2$-(cycloalkyle en C3-5),
R19 est halogène ou alcoxy en C1-4,
R20 est halogène ou alcoxy en C1-4,
ou R19 et R20 forment conjointement un groupe alkylènedioxy en C1-2,
ou un stéréoisomère du composé ou un sel du composé ou un sel du stéréoisomère du composé.

**4.** Composé de formule (1) selon la revendication 1, dans lequel

R1 représente un dérivé de phényle de formule (a) ou (b), où

R2 est méthoxy,
R3 est méthoxy,
R4 est méthoxy,
R5 est méthyle,
R6 est hydrogène,
R7 est hydrogène,
R8 est alkyle en C1-2,
ou R7 et R8 forment conjointement un groupe alkylène en C3 à C5,
R9 est hydrogène ou alkyle en C1-2,
ou dans lequel R8 et R9 conjointement et avec inclusion de l'atome de carbone, auquel ils sont liés, forment un cycle hydrocarboné de 5 chaînons à liaison spiro,
m est 1 ou 2,
R10 est indépendamment l'un de l'autre hydrogène, cycloalkyle en C5-7, alkyle en C1-4, $-CH(CH_3)$-R11 ou $-CH_2$-R12, où

R11 est phényle non substitué ou hydroxyle,
R12 est hydroxyle, cycloalkyle en C5-7, -N-(alkyle en C1-2)$_2$, $-CH_2$-S-(alkyle en C1-2), benzyle, phényle non substitué, phényle substitué par R13, phényle substitué par R13 et R14, où

R13 est fluor, chlore, brome, alcoxy en C1-2, alkyle en C1-4, fluoroalkyle en C1-2, hydroxyle, phényle, $-C(O)NH_2$, -CN, 2-oxoazétidin-1-yle ou 2-oxopyrrolidin-1-yle, R14 est fluor, chlore, brome ou alcoxy en C1-2,

ou R12 est choisi parmi imidazol-2-yle, imidazol-4-yle, pyrazol-1-yle, thiophén-2-yle, thiophén-3-yle, thiazol-2-yle, thiazol-4-yle, thiazol-5-yle, pyridin-2-yle, pyridin-3-yle, pyridin-4-yle, indol-2-yle, indol-3-yle, 1-méthyl-indol-2-yle ou 1-méthyl-indol-3-yle, ou
R12 est $-CH_2-C(O)$-R15, où

R15 est hydroxyle, -N(R16)$_2$, pipéridin-1-yle, pyrrolidin-1-yle ou benzyloxy, où
R16 est indépendamment l'un de l'autre hydrogène ou alkyle en C1-3,

R17 est hydrogène ou méthyle,
R18 est $-CH_2$-(cycloalkyle en C3-4),
R19 est alcoxy en C1-2,
R20 est fluor, chlore ou brome,
ou R19 et R20 forment conjointement un groupe méthylènedioxy,
ou un stéréoisomère du composé ou un sel du composé ou un sel du stéréoisomère du composé.

**5.** Composé de formule (1) selon la revendication 1, dans lequel

R1 représente un dérivé de phényle de formule (a) ou (b), où

R2 est méthoxy,
R3 est méthoxy,
R4 est méthoxy,
R5 est méthyle,
R6 est hydrogène,

R7 est hydrogène,
R8 est alkyle en C1-2,
ou R7 et R8 forment conjointement un groupe alkylène en C3 ou C4,
R9 est hydrogène ou alkyle en C1-2,
ou dans lequel R8 et R9 conjointement et avec inclusion de l'atome de carbone, auquel ils sont liés, forment un cycle hydrocarboné de 5 chaînons à liaison spiro,

m est 1 ou 2,
R10 est indépendamment l'un de l'autre hydrogène, cyclohexyle, alkyle en C1-4, $-CH(CH_3)$-R11 ou $-CH_2$-R12, où

R11 est phényle non substitué ou hydroxyle,
R12 est hydroxyle, cyclohexyle, $-N-(CH_3)_2$, $-CH_2-S-CH_3$, phényle non substitué, benzyle, phényle substitué par R13, phényle substitué par R13 et R14, où

R13 est fluor, chlore, alcoxy en C1-2, méthyle, tert-butyle, trifluorométhyle, hydroxyle, phényle, $-C(O)NH_2$, -CN, 2-oxoazétidin-1-yle ou 2-oxopyrrolidin-1-yle, R14 est fluor, chlore ou méthoxy,

ou R12 est choisi parmi imidazol-2-yle, imidazol-4-yle, pyrazol-1-yle, thiophén-2-yle, thiophén-3-yle, thiazol-2-yle, thiazol-4-yle, thiazol-5-yle, pyridin-2-yle, pyridin-3-yle, pyridin-4-yle, indol-2-yle, indol-3-yle, 1-méthyl-indol-2-yle ou 1-méthyl-indol-3-yle, ou

R12 est $-CH_2-C(O)$-R15, où
R15 est hydroxyle, $-N(R16)_2$, pipéridin-1-yle, pyrrolidin-1-yle ou benzyloxy, où
R16 est indépendamment l'un de l'autre hydrogène ou isopropyle,

R17 est hydrogène ou méthyle,
R18 est $-CH_2$-(cycloalkyle en C3),
R19 est méthoxy,
R20 est fluor,
ou R19 et R20 forment conjointement un groupe méthylènedioxy,
ou un stéréoisomère du composé ou un sel du composé ou un sel du stéréoisomère du composé.

**6.** Composé de formule (1) selon la revendication 5, qui est choisi dans le groupe constitué de 4-[5-(cyclopropylméthoxy)-1,3-benzodioxol-4-yl]-N-[(2R)-3-(3,5-difluorophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-5H-pyrrolo[3,2-d]pyrimidine-7-carboxamide ; 4-[2-(cyclopropylméthoxy)-5-fluoro-4-méthoxyphényl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(3-méthylphényl)-1-oxopropan-2-yl]-6-méthyl-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(3-méthylphényl)-1-oxopropan-2-yl]-5H-pyrrolo[3,2-d]pyrimidine-7-carbox-amide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(3-méthylphényl)-1-oxopropan-2-yl]-6-méthyl-5H-pyrrolo[3,2-d]pyrimidine-7-carboxamide ; N-[(2R)-3-(4-tert-butylphényl)-1-{4-[(4aS,8aR)-4-(3,4-diméthoxyphényl)-1-oxo-4a,5,6,7,8,8a-hexahydro-phtalazin-2(1H)-yl]pipéridin-1-yl}-1-oxopropan-2-yl]-4-[2-(cyclopropylméthoxy)-5-fluoro-4-méthoxyphényl]-6-méthyl-5H-pyrrolo[3,2-d]pyrimidine-7-carboxamide ; N-[(2R)-3-(4-tert-butyl-phényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; N-[(2R)-3-(4-carbamoylphényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; 4-[2-(cyclopropylméthoxy)-5-fluoro-4-méthoxyphényl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-éthoxyphényl)-1-oxopropan-2-yl]-6-méthyl-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-éthoxyphényl)-1-oxopropan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-éthoxyphényl)-1-oxopropan-2-yl]-6-méthyl-5H-pyrrolo[3,2-d]pyrimidine-7-carboxamide ; N-[(2R)-3-(4-carbamoylphényl)-1-{4-[(4aS,8aR)-4-(3,4-diméthoxy-phényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-6-méthyl-5H-pyrrolo[3,2-d]pyrimidine-7-carboxamide ; N-[(2R)-3-(biphényl-4-yl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropyl-méthoxy)-1,3-benzodioxol-4-yl]-6-méthyl-5H-pyrrolo[3,2-d]pyrimidine-7-carboxamide ; N-[(2R)-3-(biphényl-4-yl)-1-{4-[(4aS,8aR)-4-(3,4-diméthoxyphényl)-1-oxo-4a,5,6,7,8,8a-hexahydro-phtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; N-[(2R)-3-(4-cyanophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-propan-2-yl]-4-[5-(cyclopropylméthoxy)-1,3-benzodioxol-4-yl]-6-méthyl-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-méthylphényl)-1-oxopropan-2-yl]-6-méthyl-5H-pyrrolo[3,2-d]pyrimidine-7-carboxamide ; N-[(2R)-3-(4-cyanophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(3-méthylphényl)-1-oxopropan-2-yl]-6-méthyl-5H-pyrrolo[3,2-d]pyrimidine-7-carboxamide ; 4-[5-(cyclopropylméthoxy)-1,3-benzodioxol-4-yl]-N-[(2R)-3-(3,4-difluorophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-6-méthyl-5H-pyrrolo[3,2-d]pyrimidine-7-carboxamide ; 4-[5-(cyclopropylméthoxy)-1,3-benzodioxol-4-yl]-N-[(2R)-3-(3,4-difluorophényl)-1-{4-[(4aS,8aR)-4-(3,4-diméthoxyphényl)-1-oxo-4a,5,6,7,8,8a-hexahydro-phtalazin-2(1H)-yl]pipéridin-1-yl}-1-oxopropan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxy-phényl)-1-oxo-4a,5,6,7,8,8a-hexahydrophtalazin-2(1H)-yl]pipéridin-1-yl}-3-(3-méthylphényl)-1-oxopropan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-méthylphényl)-1-oxopropan-2-yl]-5H-pyrrolo[3,2-d]pyrimidine-7-carboxamide ; 4-[2-(cyclopropylméthoxy)-5-fluoro-4-méthoxyphényl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-6-méthyl-5H-pyrrolo[3,2-d]pyrimidine-7-carboxamide ; 4-[2-(cyclopropylméthoxy)-5-fluoro-4-méthoxyphényl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-5H-pyrrolo[3,2-d]pyrimidine-7-carboxamide ; 4-[2-(cyclopropylméthoxy)-4-fluoro-5-méthoxyphényl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-5H-pyrrolo[3,2-d]pyrimidine-7-carboxamide ; 4-[5-(cyclopropylméthoxy)-1,3-benzodioxol-4-yl]-N-[(2S)-3-(3,4-diméthoxyphényl)-1-{4-[(4aS,8aR)-4-(3,4-diméthoxyphényl)-1-oxo-4a,5,6,7,8,8a-hexahydro-phtalazin-2(1H)-yl]pipéridin-1-yl}-1-oxopropan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxy-phényl)-1-oxo-4a,5,6,7,8,8a-hexahydrophtalazin-2(1H)-yl]pipéridin-1-yl}-1-oxo-3-phénylpropan-2-yl]-6-méthyl-5H-pyrrolo[3,2-d]pyrimidine-7-carboxamide ; N-[(2R)-3-(4-chlorophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-fluorophényl)-1-oxopropan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-méthoxyphényl)-1-oxo-propan-2-yl]-5H-pyrrolo[3,2-d]pyrimidine-7-carboxamide ; N-[(2R)-3-(3-chlorophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-5H-pyrrolo[3,2-d]pyrimidine-7-carboxamide ; 4-[5-(cyclopropyl-méthoxy)-1,3-benzodioxol-4-yl]-N-{(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-[2-(trifluorométhyl)phényl]-propan-2-yl}-6-méthyl-5H-pyrrolo[3,2-d]pyrimidine-7-carboxamide ; 4-[5-(cyclopropylméthoxy)-1,3-benzodioxol-4-yl]-N-{(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-[2-(trifluorométhyl)phényl]propan-2-yl}-5H-pyrrolo[3,2-d]pyrimidine-7-carboxamide ; N-[(2R)-3-(2-chlorophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-propan-2-yl]-4-[5-(cyclopropylméthoxy)-1,3-benzodioxol-4-yl]-6-méthyl-5H-pyrrolo[3,2-d]pyrimidine-7-carboxamide ; -N-[(2R)-3-(2-chlorophényl)-1-{4-[(4aS,8aR)-4-(3,4-diméthoxy-phényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-5H-pyrrolo[3,2-d]pyrimidine-7-carboxamide ; 4-[2-(cyclopropylméthoxy)-5-fluoro-4-méthoxyphényl]-N-[(2R,3R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylbutan-2-yl]-6-méthyl-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(pyridin-2-yl)propan-2-yl]-6-méthyl-5H-pyrrolo[3,2-d]pyrimidine-7-carboxamide ; 4-[5-(cyclopropyl-méthoxy)-1,3-benzodioxol-4-yl]-N-[(2S)-1-{4-[(4aS,8aR)-4-(3,4-diméthoxyphényl)-l-oxo-4a,5,6,7,8,8a-hexahydrophtalazin-2(1H)-yl]pipéridin-1-yl}-1-oxo-3-(pyridin-2-yl)propan-2-yl]-5H-pyrrolo[3,2-d]pyrimidine-7-carboxamide ; 4-[5-(cyclopropylméthoxy)-1,3-benzo-

dioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(pyridin-4-yl)propan-2-yl]-6-méthyl-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(pyridin-4-yl)propan-2-yl]-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(pyridin-3-yl)propan-2-yl]-6-méthyl-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(pyridin-3-yl)propan-2-yl]-5H-pyrrolo[3,2-d]pyrimidine-7-carboxamide ; 4-[5-(cyclopropylméthoxy)-1,3-benzodioxol-4-yl]-N-[(2S)-1-{4-[(4aR,8aS)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aR,8aS)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-5H-pyrrolo[3,2-d]pyrimidine-7-carboxamide ; N-[(2R)-3-(4-tert-butylphényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-6-méthyl-5H-pyrrolo[3,2-d]pyrimidine-7-carboxamide ; 4-[5-(cyclopropyl-méthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-fluorophényl)-1-oxopropan-2-yl]-5H-pyrrolo[3,2-d]pyrimidine-7-carboxamide ; 4-[5-(cyclopropylméthoxy)-1,3-benzodioxol-4-yl]-N-[(2R)-3-(2,4-dichlorophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-5H-pyrrolo[3,2-d]pyrimidine-7-carboxamide ; 4-[5-(cyclopropylméthoxy)-1,3-benzodioxol-4-yl]-N-{(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-[4-(trifluorométhyl)phényl]propan-2-yl}-5H-pyrrolo[3,2-d]pyrimidine-7-carboxamide ; 4-[5-(cyclo-propylméthoxy)-1,3-benzodioxol-4-yl]-N-[(2R,3R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylbutan-2-yl]-5H-pyrrolo[3,2-d]pyrimidine-7-carboxamide ; 4-[5-(cyclopropylméthoxy)-1,3-benzodioxol-4-yl]-N-[(2R,3R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylbutan-2-yl]-6-méthyl-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-hydroxyphényl)-1-oxopropan-2-yl]-5H-pyrrolo[3,2-d]pyrimidine-7-carboxamide ; 4-[5-(cyclopropyl-méthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-hydroxyphényl)-1-oxopropan-2-yl]-5H-pyrrolo[3,2-d]pyrimidine-7-carboxamide ; N-[(2S)-3-(2-chlorophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,7aR)-4-(3,4-diméthoxyphényl)-1-oxo-1,4a,5,6,7,7a-hexahydro-2H-cyclopenta[d]pyridazin-2-yl]pipéridin-1-yl}-1-oxo-3-phénylpropan-2-yl]-5H-pyrrolo[3,2-d]pyrimidine-7-carboxamide ; N-[(2S)-3-(2-chloro-phényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-6-méthyl-5H-pyrrolo[3,2-d]pyrimidine-7-carboxamide ; 4-[2-(cyclopropylméthoxy)-5-fluoro-4-méthoxyphényl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(3-méthylphényl)-1-oxopropan-2-yl]-6-méthyl-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(4-méthoxyphényl)-1-oxopropan-2-yl]-6-méthyl-5H-pyrrolo[3,2-d]pyrimidine-7-carboxamide ; 4-[5-(cyclopropylméthoxy)-1,3-benzodioxol-4-yl]-N-(2-{4-[(4aS,8aR)-4-(3,4-diméthoxyphényl)-1-oxo-4a,5,6,7,8,8a-hexahydrophtalazin-2(1H)-yl]pipéridin-1-yl}-2-oxoéthyl)-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-hydroxy-1-oxopropan-2-yl]-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-5H-pyrrolo[3,2-d]pyrimidine-7-carboxamide ; 4-[5-(cyclopropylméthoxy)-1,3-benzodioxol-4-yl]-N-[(2S,3R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-hydroxy-1-oxobutan-2-yl]-5H-pyrrolo[3,2-d]pyrimidine-7-carboxamide ; 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxobutan-2-yl]-6-méthyl-5H-pyrrolo[3,2-d]pyrimidine-7-carboxamide ; 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-4-phénylbutan-2-yl]-5H-pyrrolo[3,2-d]pyrimidine-7-carboxamide ; 4-[5-(cyclopropylméthoxy)-1,3-benzodioxol-4-yl]-N-[(2R)-1-{4-[3-(3,4-diméthoxyphényl)-5,5-diméthyl-6-oxo-5,6-di-hydropyridazin-1(4H)-yl]pipéridin-1-yl}-1-oxo-3-phénylpropan-2-yl]-6-méthyl-5H-pyrrolo[3,2-d]pyrimidine-7-carboxamide ; 4-[5-(cyclopropylméthoxy)-1,3-benzodioxol-4-yl]-N-[(2R)-1-{4-[3-(3,4-diméthoxyphényl)-5,5-diméthyl-6-oxo-5,6-dihydropyridazin-1(4H)-yl]pipéridin-1-yl}-1-oxo-3-phénylpropan-2-yl]-5H-pyrrolo[3,2-d]pyrimidine-7-carboxamide ; 4-[2-(cyclopropylméthoxy)-5-fluoro-4-méthoxyphényl]-N-[(2R)-1-{4-[3-(3,4-diméthoxyphényl)-5,5-diméthyl-6-oxo-5,6-dihydropyridazin-1(4H)-yl]pipéridin-1-yl}-1-oxo-3-phénylpropan-2-yl]-5H-pyrrolo[3,2-d]pyrimidine-7-carboxamide ou 4-[2-(cyclopropylméthoxy)-5-fluoro-4-méthoxyphényl]-N-[(2R)-1-{4-[3-(3,4-diméthoxyphényl)-5,5-diméthyl-6-oxo-5,6-dihydropyridazin-1(4H)-yl]pipéridin-1-yl}-1-oxo-3-phénylpropan-2-yl]-6-méthyl-5H-pyrrolo[3,2-d]pyrimidine-7-carboxamide,4-[5-(cyclopropylméthoxy)-1,3-benzodioxol-4-yl]-N-[(2S,3S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-méthyl-1-oxopentan-2-yl]-5H-pyrrolo[3,2-d]pyrimidine-7-carboxamide, N-[(2S)-3-cyclo-hexyl-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy)-1,3-benzodioxol-4-yl]-6-méthyl-5H-pyrrolo[3,2-d]pyrimidine-7-carboxamide, N-[(1R)-1-cyclohexyl-2-{4-[(4aS,8aR)-4-(3,4-diméthoxy-phényl)-1-oxo-4a,5,6,7,8,8a-hexahydrophtalazin-2(1H)-yl]pipéridin-1-yl}-2-oxoéthyl]-4-[5-(cyclopropylméthoxy)-1,3-benzodioxol-4-yl]-5H-pyrrolo[3,2-d]pyrimidine-7-carboxamide, N-[(1S)-1-cyclohexyl-2-{4-[(4aS,8aR)-4-(3,4-diméthoxyphényl)-1-oxo-4a,5,6,7,8,8a-hexahydrophtalazin-2(1H)-yl]pipéridin-1-yl}-2-oxoéthyl]-4-[5-(cyclopropylméthoxy)-1,3-benzodioxol-4-yl]-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxy-phényl)-1-oxo-4a,5,6,7,8,8a-hexahydrophtalazin-2(1H)-yl]pipéridin-1-yl}-1-oxo-3-(thiophén-2-yl)propan-2-yl]-6-méthyl-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-l-oxo-3-(thiophén-2-yl)propan-2-yl]-5H-pyrrolo[3,2-d]pyrimidine-7-carboxamide, 4-[5-(cyclopropyl-méthoxy)-1,3-benzodioxol-4-yl]-N-(3-{4-[(4aS,8aR)-4-(3,4-diméthoxyphényl)-1-oxo-4a,5,6,7,8,8a-hexahydrophtalazin-2(1H)-yl]pipéridin-1-yl}-3-oxopropyl)-5H-pyrrolo[3,2-d]pyrimidine-7-carboxamide, 4-[5-(cyclopropylméthoxy)-1,3-benzodioxol-4-yl]-N-[(2R)-4-{4-[(4aS,8aR)-4-(3,4-diméthoxyphényl)-1-oxo-4a,5,6,7,8,8a-hexahydrophtalazin-2(1H)-yl]pipéridin-1-yl}-4-oxo-1-phénylbutan-2-yl]-5H-pyrrolo[3,2-d]pyrimidine-7-carboxamide,4-[5-(cyclopropyl-méthoxy)-1,3-benzodioxol-4-yl]-N-[(2R)-4-{4-[3-(3,4-diméthoxyphényl)-5,5-diméthyl-6-oxo-5,6-dihydropyridazin-1(4H)-yl]pipéridin-1-yl}-4-oxo-1-phénylbutan-2-yl]-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1,5-dioxo-5-(pipéridin-1-yl)pentan-2-yl]-5H-pyrrolo[3,2-d]pyrimidine-7-carboxamide, 4-[5-(cyclopropyl-méthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1,5-dioxo-5-(pipéridin-1-yl)pentan-2-yl]-6-méthyl-5H-pyrrolo[3,2-d]pyrimidine-7-carboxamide, 4-[2-(cyclopropylméthoxy)-5-fluoro-4-méthoxyphényl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1,5-dioxo-5-(pipéridin-1-yl)pentan-2-yl]-6-méthyl-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(1,3-thiazol-4-yl)propan-2-yl]-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(1,3-thiazol-4-yl)propan-2-yl]-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(1,3-thiazol-4-yl)propan-2-yl]-6-méthyl-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(1H-pyrazol-1-yl)propan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(1H-imidazol-4-yl)-1-oxopropan-2-yl]-5H-pyrrolo[3,2-d]pyrimidine-7-carboxamide, 4-[5-(cyclopropyl-méthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(1H-imidazol-4-yl)-1-oxopropan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(1H-indol-3-yl)-1-oxopropan-2-yl]-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(1H-indol-3-yl)-1-oxopropan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexa-hydrophtalazin-2(1H)-yl]pipéridin-1-yl}-3-(1-méthyl-1H-indol-3-yl)-1-oxopropan-2-yl]-5H-pyrrolo[3,2-d]pyrimidine-7-carboxamide, N-[(2S)-3-cyclohexyl-1-{4-[3-(3,4-diméthoxyphényl)-5,5-diméthyl-6-oxo-5,6-dihydropyridazin-1(4H)-yl]pipéridin-1-yl}-1-oxopropan-2-yl]-4-[5-(cyclopropyl-méthoxy)-1,3-benzodioxol-4-yl]-5H-pyrrolo[3,2-d]pyrimidine-7-carboxamide, 4-[5-(cyclopropyl-méthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-4-méthyl-1-oxopentan-2-yl]-5H-pyrrolo[3,2-d]pyrimidine-7-carboxamide,4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(diméthylamino)-1-oxopropan-2-yl]-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(diméthylamino)-1-oxopropan-2-yl]-5H-pyrrolo[3,2-d]pyrimidine-7-carboxamide, 4-[5-(cyclo-propylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-4-(méthylsulfanyl)-1-oxobutan-2-yl]-5H-pyrrolo[3,2-d]pyrimidine-7-carboxamide, N-[(2R)-3-(4-bromophényl)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxopropan-2-yl]-4-[5-(cyclopropylméthoxy) -1,3-benzodioxol-4-yl]-5H-pyrrolo[3,2-d]pyrimidine-7-carboxamide, (4R)-4-[({4-[5-(cyclopropylméthoxy)-1,3-benzodioxol-4-yl]-5H-pyrrolo[3,2-d]pyrimidin-7-yl}carbonyl)amino]-5-{4-[(4aS,8aR)-4-(3,4-diméthoxyphényl)-1-oxo-4a,5,6,7,8,8a-hexahydrophtalazin-2(1H)-yl]pipéridin-1-yl}-5-oxopentanoate de benzyle, acide (4R)-4-[({4-[5-(cyclopropyl-méthoxy)-1,3-benzodioxol-4-yl]-5H-pyrrolo[3,2-d]pyrimidin-7-yl}carbonyl)amino]-5-{4-[(4aS,8aR)-4-(3,4-diméthoxyphényl)-1-oxo-4a,5,6,7,8,8a-hexahydrophtalazin-2(1H)-yl]pipéridin-1-yl}-5-oxopentanoïque, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1,5-dioxo-5-(pyrrolidin-1-yl)pentan-2-yl]-5H-pyrrolo[3,2-d]pyrimidine-7-carboxamide, acide (4S)-4-[({4-[5-(cyclopropylméthoxy)-1,3-benzodioxol-4-yl]-5H-pyrrolo[3,2-d]pyrimidin-7-yl}carbonyl)amino]-5-{4-[(4aS,8aR)-4-(3,4-diméthoxyphényl)-1-oxo-4a,5,6,7,8,8a-hexahydrophtalazin-2(1H)-yl]pipéridin-1-yl}-5-oxopentanoïque, (4S)-4-[({4-[5-(cyclopropylméthoxy)-1,3-benzodioxol-4-yl]-5H-pyrrolo[3,2-d]pyrimidin-7-yl}carbonyl)amino]-5-{4-[(4aS,8aR)-4-(3,4-diméthoxyphényl)-1-oxo-4a,5,6,7,8,8a-hexahydrophtalazin-2(1H)-yl]pipéridin-1-yl}-5-oxopentanoate de benzyle, 4-[5-(cyclopropyl-méthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1,5-dioxo-5-(pyrrolidin-1-yl)pentan-2-yl]-5H-pyrrolo[3,2-d]pyrimidine-7-carboxamide, 4-[5-(cyclopropylméthoxy)-1,3-benzodioxol-4-yl]-N-[(2S)-1-{4-[(4aS,8aR)-4-(3,4-diméthoxyphényl)-1-oxo-4a,5,6,7,8,8a-hexahydrophtalazin-2(1H)-yl]pipéridin-1-yl}-1,5-dioxo-5-(propan-2-ylamino)pentan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1,5-dioxo-5-(propan-2-ylamino)pentan-2-yl]-5H-pyrrolo[3,2-d]pyrimidine-7-carboxamide, 4-[5-(cyclopropyl-méthoxy)-1,3-benzodioxol-4-yl]-N-[(2R)-1-{4-[3-(3,4-diméthoxyphényl)-5,5-diméthyl-6-oxo-5,6-dihydropyridazin-1(4H)-yl]pipéridin-1-yl}-3-(4-fluorophényl)-1-oxopropan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-(pyridin-4-yl)propan-2-yl]-5H-pyrrolo[3,2-d]pyrimidine-7-carboxamide, 4-[5-(cyclo-propylméthoxy)-1,3-benzodioxol-4-yl]-N-[(2S)-1-{4-[3-(3,4-diméthoxyphényl)-5,5-diméthyl-6-oxo-5,6-dihydropyridazin-1(4H)-yl]pipéridin-1-yl}-1-oxo-3-phénylpropan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[3-(3,4-diméthoxyphényl)-5,5-diméthyl-6-oxo-5,6-dihydropyridazin-1(4H)-yl]pipéridin-1-yl}-1-oxopropan-2-yl]-5H-pyrrolo[3,2-d]pyrimidine-7-carboxamide, 4-[5-(cyclopropylméthoxy)-1,3-benzodioxol-4-yl]-N-{(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-[4-(2-oxoazétidin-1-yl)phényl]propan-2-yl}-5H-pyrrolo[3,2-d]pyrimidine-7-carboxamide, 4-[5-(cyclopropylméthoxy)-1,3-benzodioxol-4-yl]-N-{(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-[4-(2-oxopyrrolidin-1-yl)phényl]propan-2-yl}-5H-pyrrolo[3,2-d]pyrimidine-7-carboxamide, 4-[5-(cyclopropylméthoxy)-1,3-benzodioxol-4-yl]-N-[(2S)-1-{4-[3-(7-méthoxy-2,2-diméthyl-2,3-dihydro-1-benzofuran-4-yl)-5,5-diméthyl-6-oxo-5,6-dihydropyridazin-1(4H)-yl]pipéridin-1-yl}-1-oxo-3-(pyridin-3-yl)propan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[3-(7-méthoxy-2,2-diméthyl-2,3-dihydro-1-benzofuran-4-yl)-5,5-diméthyl-6-oxo-5,6-dihydropyridazin-1(4H)-yl]pipéridin-1-yl}-1-oxo-3-phénylpropan-2-yl]-6-méthyl-5H-pyrrolo[3,2-d]pyrimidine-7-carboxamide, 4-[5-(cyclo-propylméthoxy)-1,3-benzodioxol-4-yl]-N-[(2R)-1-{4-[3-(3,4-diméthoxyphényl)-5,5-diéthyl-6-oxo-5,6-dihydropyridazin-1(4H)-yl]pipéridin-1-yl}-1-oxo-3-phénylpropan-2-yl]-5H-pyrrolo[3,2-d]pyrimidine-7-carboxamide, 4-[5-(cyclopropylméthoxy)-1,3-benzodioxol-4-yl]-N-(2-{4-[3-(7-méthoxy-2,2-diméthyl-2,3-dihydro-1-benzofuran-4-yl)-5,5-diméthyl-6-oxo-5,6-dihydropyridazin-1(4H)-yl]pipéridin-1-yl}-2-oxoéthyl)-5H-pyrrolo[3,2-d]pyrimidine-7-carboxamide, 4-[5-(cyclopropyl-méthoxy)-1,3-benzodioxol-4-yl]-N-[(2R)-1-{4-[3-(3,4-diméthoxyphényl)-5,5-diméthyl-6-oxo-5,6-dihydropyridazin-1(4H)-yl]pipéridin-1-yl}-5-(diméthylamino)-1,5-dioxopentan-2-yl]-6-méthyl-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-méthyl-1-oxobutan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-méthyl-1-oxobutan-2-yl]-6-méthyl-5H-pyrrolo[3,2-d]pyrimidine-7-carboxamide, 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3,3-diméthyl-1-oxobutan-2-yl]-5H-pyrrolo[3,2-d]pyrimidine-7-carboxamide, 4-[5-(cyclopropylméthoxy)-1,3-benzodioxol-4-yl]-N-(2-{4-[9-(3,4-diméthoxyphényl)-6-oxo-7,8-diazaspiro[4.5]déc-8-én-7-yl]pipéridin-1-yl}-2-oxoéthyl)-5H-pyrrolo[3,2-d]pyrimidine-7-carboxamide, 4-[5-(cyclopropylméthoxy)-1,3-benzodioxol-4-yl]-N-[(2R)-1-{4-[9-(3,4-diméthoxy-phényl)-6-oxo-7,8-diazaspiro[4.5]déc-8-én-7-yl]pipéridin-1-yl}-1-oxo-3-phénylpropan-2-yl]-5H-pyrrolo[3,2-d]pyrimidine-7-carboxamide, 4-[5-(cyclopropylméthoxy)-1,3-benzodioxol-4-yl]-N-[(2R)-1-{4-[9-(3,4-diméthoxyphényl)-6-oxo-7,8-diazaspiro[4.5]déc-8-én-7-yl]pipéridin-1-yl}-1-oxo-3-phénylpropan-2-yl]-6-méthyl-5H-pyrrolo[3,2-d]pyrimidine-7-carboxamide et 4-[5-(cyclopropyl-méthoxy)-1,3-benzodioxol-4-yl]-N-[(2S)-1-{4-[9-(3,4-diméthoxy-phényl)-6-oxo-7,8-diazaspiro[4.5]déc-8-én-7-yl]pipéridin-1-yl}-1-oxo-3-(pyridin-3-yl)propan-2-yl]-5H-pyrrolo[3,2-d]pyrimidine-7-carboxamide.

**7.** Composé de formule (1) selon la revendication 1 qui est 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-3-(3-méthylphényl)-1-oxopropan-2-yl]-6-méthyl-5H-pyrrolo[3,2-d]pyrimidine-7-carboxamide ou un sel de celui-ci.

**8.** Composé de formule (1) selon la revendication 1 qui est 4-[5-(cyclopropylméthoxy)-1,3-benzodioxol-4-yl]-N-[(2R)-1-{4-[(4aS,8aR)-4-(3,4-diméthoxyphényl)-1-oxo-4a,5,6,7,8,8a-hexahydrophtalazin-2(1H)-yl]pipéridin-1-yl}-1-oxo-3-phénylpropan-2-yl]-5H-pyrrolo[3,2-d]pyrimidine-7-carboxamide ou un sel de celui-ci.

**9.** Composé de formule (1) selon la revendication 1 qui est 4-[5-(cyclopropylméthoxy)-1,3-benzodioxol-4-yl]-N-(2-{4-[(4aS,8aR)-4-(3,4-diméthoxyphényl)-1-oxo-4a,5,6,7,8,8a-hexahydrophtalazin-2(1H)-yl]pipéridin-1-yl}-2-oxoéthyl)-5H-pyrrolo[3,2-d]pyrimidine-7-carboxamide ou un sel de celui-ci.

**10.** Composé de formule (1) ou un stéréoisomère du composé ou un sel du composé ou un sel du stéréoisomère du composé selon l'une quelconque des revendications 1 à 6 pour utilisation dans le traitement ou la prophylaxie de maladies.

**11.** Composé de formule (1) ou un sel de celui-ci selon l'une quelconque des revendications 7 à 9 pour utilisation dans le traitement ou la prophylaxie de maladies.

**12.** Composition pharmaceutique comprenant au moins un des composés de formule (1) ou un stéréoisomère du composé ou un sel du composé ou un sel du stéréoisomère du composé selon l'une quelconque des revendications 1 à 6 conjointement avec au moins un auxiliaire pharmaceutiquement acceptable.

**13.** Composition pharmaceutique le composé de formule (1) ou un sel de celui-ci selon l'une quelconque des revendications 7 à 9 conjointement avec au moins un auxiliaire pharmaceutiquement acceptable.

**14.** Combinaison fixe, combinaison non fixe ou kit de composants comprenant au moins un composé de formule (1) ou un stéréoisomère du composé ou un sel du composé ou un sel d'un stéréoisomère du composé selon l'une quelconque des revendications 1 à 6, au moins un agent thérapeutique choisi dans le groupe constitué de corticostéroïdes, anticholinergiques, agonistes β2-adrénergiques, antagonistes de récepteur H1, antagonistes de récepteur des leucotriènes, inhibiteurs de 5-lipoxygénase, antagonistes de récepteur d'endothéline, prostacyclines, inhibiteurs calciques, bêta-bloquants, inhibiteurs de phosphodiestérase de type 4, inhibiteurs de phosphodiestérase de type 5, immunosuppresseurs, analogues de vitamine D, inhibiteurs de HMG-CoA réductase, tensioactifs pulmonaires, antibiotiques, activateurs/stimulateurs de guanylyle cyclase, tétrahydrobioptérine et dérivés de tétrahydrobioptérine, anticoagulants, diurétiques, pirfénidone et glycosides de digitale, et au moins un auxiliaire pharmaceutiquement acceptable.

**15.** Combinaison fixe, combinaison non fixe ou kit de composants comprenant le composé de formule (1) ou un sel de celui-ci selon l'une quelconque des revendications 7 à 9, au moins un agent thérapeutique choisi dans le groupe constitué de corticostéroïdes, anticholinergiques, agonistes $\beta_2$-adrénergiques, antagonistes de récepteur H1, antagonistes de récepteur des leucotriènes, inhibiteurs de 5-lipoxygénase, antagonistes de récepteur d'endothéline, prostacyclines, inhibiteurs calciques, bêta-bloquants, inhibiteurs de phosphodiestérase de type 4, inhibiteurs de phosphodiestérase de type 5, immunosuppresseurs, analogues de vitamine D, inhibiteurs de HMG-CoA réductase, tensioactifs pulmonaires, antibiotiques, activateurs/stimulateurs de guanylyle cyclase, tétrahydrobioptérine et dérivés de tétrahydrobioptérine, anticoagulants, diurétiques, pirfénidone et glycosides de digitale, et au moins un auxiliaire pharmaceutiquement acceptable.

**16.** Utilisation d'un composé de formule (1) ou un stéréoisomère du composé ou un sel du composé ou un sel du stéréoisomère du composé selon l'une quelconque des revendications 1 à 6 dans la fabrication d'une composition pharmaceutique pour le traitement ou la prophylaxie d'une maladie aiguë ou chronique des voies respiratoires.

**17.** Utilisation du composé de formule (1) ou un sel de celui-ci selon l'une quelconque des revendications 7 à 9 dans la fabrication d'une composition pharmaceutique pour le traitement ou la prophylaxie d'une maladie aiguë ou chronique des voies respiratoires.

**18.** Utilisation selon l'une quelconque des revendications 16 à 17, dans laquelle la maladie aiguë ou chronique des voies respiratoires est choisie dans le groupe constitué d'une maladie pulmonaire interstitielle, la fibrose pulmonaire, la mucoviscidose, l'asthme bronchique, la bronchite chronique, l'emphysème, la bronchopneumopathie chronique obstructive (BPCO) et BPCO associée à l'hypertension pulmonaire.

**19.** Composé de formule (1) ou un stéréoisomère du composé ou un sel du composé ou sel du stéréoisomère du composé selon l'une quelconque des revendications 1 à 6 pour utilisation dans le traitement ou la prophylaxie d'une maladie aiguë ou chronique des voies respiratoires.

**20.** Composé de formule (1) ou un sel de celui-ci selon l'une quelconque des revendications 7 à 9 pour utilisation dans le traitement ou la prophylaxie d'une maladie aiguë ou chronique des voies respiratoires.

**21.** Composé de formule (1) ou un stéréoisomère du composé ou un sel du composé ou un sel du stéréoisomère du composé selon l'une quelconque des revendications 1 à 6 pour le traitement ou la prophylaxie d'une maladie aiguë ou chronique des voies respiratoires choisie dans le groupe constitué d'une maladie pulmonaire interstitielle, la fibrose pulmonaire, la mucoviscidose, l'asthme bronchique, la bronchite chronique, l'emphysème, la bronchopneumopathie chronique obstructive (BPCO) et BPCO associée à l'hypertension pulmonaire.

**22.** Composé de formule (1) ou un sel de celui-ci selon l'une quelconque des revendications 7 à 9 pour le traitement ou la prophylaxie d'une maladie aiguë ou chronique des voies respiratoires choisi dans le groupe constitué d'une maladie pulmonaire interstitielle, la fibrose pulmonaire, la mucoviscidose, l'asthme bronchique, la bronchite chronique, l'emphysème, la bronchopneumopathie chronique obstructive (BPCO) et BPCO associée à l'hypertension pulmonaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02064584 A
- WO 02085906 A
- WO 04017974 A
- WO 04018449 A
- WO 04018451 A
- WO 04018457 A
- WO 05075456 A
- WO 05075457 A
- WO 2009106531 A
- WO 2011023693 A
- WO 2005075457 A
- WO 2005075456 A
- EP 0372777 A
- WO 9104011 A
- WO 9111173 A
- WO 9111495 A
- WO 9114422 A
- WO 9311743 A
- EP 0553298 A
- WO 0230394 A
- WO 0007567 A
- EP 0505321 A
- EP 407028 A
- EP 650410 A
- EP 691865 A
- EP 725725 A
- US 6397838 B
- US 6454193 B
- US 6302331 B
- GB L20966 A


### Non-patent literature cited in the description


- **C. A. G. N. MONTALBETTI ; V. FALQUE.** *Tetrahedron,* 2005, vol. 61, 10827-10852
- **A. EL-FAHAM ; R. S. FUNOSAS ; R. PROHENS ; F. ALBERICIO.** *Chemistry- A European Journal,* 2009, vol. 15, 9404-9416
- **J. GLAUDER.** *Speciality Chemicals Magazine,* 2004, vol. 24, 30-31
- **A. EL-FAHAM ; R. S. FUNOSAS ; R. PROHENS ; F. ALBERICIO.** *Chemistry - A European Journal,* 2009, vol. 15, 9404-9416
- *J. Glauder, Speciality Chemicals Magazine,* 2004, vol. 24, 30-31
- **T. W. GREENE.** Protective Groups in Organic Synthesis. John Wiley & Sons, 1999
- **P. KOCIENSKI.** Protecting Groups. Thieme Medical Publishers, 2000
- **DUNKERN et al.** *Eur. J. Pharmacol.,* 2007, vol. 572 (1), 12-22
- **SABATINI et al.** *Pulm Pharmacol Ther,* 2010, vol. 23, 283-91
- **MARTIN-CHOULY CA et al.** *Life Sci.,* 2004, vol. 75 (7), 823-40
- **KOHYAMA T et al.** *Am. J. Respir. Cell Mol. Biol.,* 2002, vol. 26 (6), 694-701
- **TOGO et al.** *Am J Physiol Lung Cell Mol Physiol,* 2009, vol. 296, L959-69
- **CORTIJO J et al.** *Br. J. Pharmacol.,* 2009, vol. 156 (3), 534-44
- **HEMNES AR ; ZAIMAN A ; CHAMPION HC.** *Am. J. Physiol. Lung Cell. Mol. Physiol.,* 26 October 2007, vol. 294 (1), L24-33
- **GHOFRANI et al.** *Lancet,* 2002, vol. 360, 895-900
- **COLLARD et al.** *Chest,* 2007, vol. 131, 897-899